(19) Europäisches Patentamt
European Patent Office
Office européen des brevets

(11) **EP 3 782 599 B1**

(12) **EUROPÄISCHE PATENTSCHRIFT**

(45) Veröffentlichungstag und Bekanntmachung des Hinweises auf die Patenterteilung:
**17.09.2025 Patentblatt 2025/38**

(21) Anmeldenummer: **20190855.5**

(22) Anmeldetag: **13.08.2020**

(51) Internationale Patentklassifikation (IPC):
***A61K 6/16*** $^{(2020.01)}$ ***A61K 6/17*** $^{(2020.01)}$
***A61K 6/60*** $^{(2020.01)}$ ***A61K 6/61*** $^{(2020.01)}$
***A61K 6/62*** $^{(2020.01)}$ ***A61K 6/69*** $^{(2020.01)}$
***A61K 6/75*** $^{(2020.01)}$ ***A61K 6/76*** $^{(2020.01)}$
***A61K 6/77*** $^{(2020.01)}$ ***A61K 6/78*** $^{(2020.01)}$
***A61K 6/887*** $^{(2020.01)}$ ***C08F 230/08*** $^{(2006.01)}$
***C08G 77/04*** $^{(2006.01)}$ ***C08G 77/20*** $^{(2006.01)}$
***C08L 83/04*** $^{(2006.01)}$ ***C08L 83/06*** $^{(2006.01)}$

(52) Gemeinsame Patentklassifikation (CPC):
(C-Sets verfügbar)
**C08G 77/20; A61K 6/16; A61K 6/17; A61K 6/60; A61K 6/61; A61K 6/62; A61K 6/69; A61K 6/75; A61K 6/76; A61K 6/77; A61K 6/78; C08F 230/08; C08F 230/085; C08L 83/04; C08L 83/06** (Forts.)

(54) **DENTALE POLYMERISIERBARE ZUSAMMENSETZUNG AUF DER BASIS KONDENSIERTER SILANE**

DENTAL POLYMERIZABLE COMPOSITION BASED ON CONDENSED SILANES

COMPOSITION DENTAIRE POLYMÉRISABLE À BASE DE SILANES CONDENSÉS

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **19.08.2019 DE 102019122174**

(43) Veröffentlichungstag der Anmeldung:
**24.02.2021 Patentblatt 2021/08**

(73) Patentinhaber: **VOCO GmbH**
**27472 Cuxhaven (DE)**

(72) Erfinder:
- **Riedel, Silke**
  **27478 Cuxhaven (DE)**
- **Lübbe, Gerrit**
  **27476 Cuxhaven (DE)**
- **Plaumann, Manfred Thomas**
  **27472 Cuxhaven (DE)**

(56) Entgegenhaltungen:
**DE-A1- 102006 016 474** **DE-A1- 19 903 177**
**JP-A- H08 311 115**

- **MEGAN A. COLE ET AL: "Thiol-ene functionalized siloxanes for use as elastomeric dental impression materials", DENTAL MATERIALS, vol. 30, no. 4, 1 April 2014 (2014-04-01), AMSTERDAM, NL, pages 449 - 455, XP055743888, ISSN: 0109-5641, DOI: 10.1016/ j.dental.2014.01.011**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

(52) Gemeinsame Patentklassifikation (CPC): (Forts.)

C-Sets
**A61K 6/887, C08L 33/08;**
**A61K 6/887, C08L 33/10;**
**A61K 6/887, C08L 33/26;**
**A61K 6/887, C08L 43/04;**
**A61K 6/887, C08L 51/085;**
**A61K 6/887, C08L 83/10;**
**C08F 230/085, C08F 222/102, C08F 222/102**

## Beschreibung

Die vorliegende Erfindung betrifft neue dentale polymerisierbare Zusammensetzungen, umfassend

(A) Polysiloxane, wobei die Polysiloxane umfassen

eine Mischung der Kondensate der drei Silane (a1), (a2) und (a3) und/oder ein Cokondensat aus einer Mischung der drei Silane (a1), (a2) und (a3) und/oder eine Mischung von mindestens zwei der Cokondensate (a1)/(a2), (a1)/(a3) und (a2)/(a3) und/oder eine Mischung aus dem Kondensat eines der drei Silane (a1), (a2) oder (a3) mit dem Cokondesat der anderen beiden Silane,
wobei das Silan (a1) der Formel $(R^1O)_aR^2_bSi[-A(-Y\{-B[-PG]_f\}_e)_d]_c$ entspricht, wobei Y = -C(=O)NH-, -NHC(=O)-, -OC(=O)NH-, -NHC(=O)O-, -SC(=O)NH-, -NHC(=O)S-, -NHC(=O)NH-, -OC(=O)N(-C(=O)NH-)-, -SC(=O)N(-C(=O)NH-)-, -NHC(=O)N(-C(=O)NH-)-, -C(=O)NHC(=O)NH-, -NHC(=O)NHC(=O)-,
wobei die jeweils im Formelbild links angeordnete Bindung dem Strukturelement A und die rechts angeordnete Bindung dem Strukturelement B näher ist,
PG = polymerisierbare Gruppe,
wobei die Gruppe PG ausgewählt ist aus der Gruppe bestehend aus $-Z-C(=O)-CH=CH_2$ und $-Z-C(=O)-C(CH_3)=CH_2$ wobei Z ausgewählt ist aus der Gruppe bestehend aus O und NH,
A = eine organische Verbindungsgruppe, die Si mit Y verbindet und 1 bis 20 C-Atome aufweist,
B = eine organische Verbindungsgruppe, die Y mit PG verbindet und 1 bis 20 C-Atome aufweist,
$R^1$ = H oder C1- bis C4-Alkyl,
$R^2$ = C1- bis C4-Alkyl,
a = 2 oder 3,
b = 0 oder 1,
c = 1 oder 2,
d = 1 bis 3,
e = 1 oder 2,
f = 1 bis 5,

$$a + b + c = 4,$$

und wobei das Silan (a2) der Formel $(R^1O)_aR^2_bSi[-A'(-PG)_f]_c$ entspricht, wobei PG = polymerisierbare Gruppe, wobei die polymerisierbare Gruppe PG ausgewählt ist aus der Gruppe bestehend aus $-Z-C(=O)-CH=CH_2$ und $-Z-C(=O)-C(CH_3)=CH_2$ wobei Z ausgewählt ist aus der Gruppe bestehend aus O und NH,
A' = eine organische Verbindungsgruppe, die Si mit PG verbindet und 1 bis 20 C-Atome aufweist und keine der Gruppen -C(=O)NH-, -NHC(=O)-, -OC(=O)NH-, - NHC(=O)O-, -SC(=O)NH-, -NHC(=O)S-, -NHC(=O)NH-, -OC(=O)N(-C(=O)NH-)-, -SC(=O)N(-C(=O)NH-)-, -NHC(=O)N(-C(=O)NH-)-, -C(=O)NHC(=O)NH-, oder -NHC(=O)NHC(=O)-,enthält,
$R^1$ = H oder C1- bis C4-Alkyl,
$R^2$ = C1- bis C4-Alkyl,
a = 2 oder 3
b = 0 oder 1
c = 1 oder 2
f = 1 bis 5,

$$a + b + c = 4,$$

und wobei das Silan (a3) der Formel $(R^1O)_aR^2_bSiAr_c$ entspricht, wobei
$R^1$ = H oder C1- bis C4-Alkyl,
$R^2$ = C1- bis C4-Alkyl,
Ar = Aryl wobei unterschiedliche Gruppen Ar gleich oder verschieden sein können,
a = 2 oder 3,
b = 0 oder 1,
c = 1 oder 2

$$a + b + c = 4,$$

(B) Füllstoffe und
(C) Initiatoren und/oder Katalysatoren und/oder Aktivatoren für die Polymerisation,

wobei der Anteil der Silane (a1), (a2) und (a3) im Cokondensat oder in der Mischung der Kondensate oder Cokondensate

(a1) in einer Menge von 10 bis 70 Gew.-%,
(a2) in einer Menge von 10 bis 70 Gew.-%, und
(a3) in einer Menge von 5 bis 60 Gew.-%,

beträgt, jeweils bezogen auf die Gesamt Menge der Silane (a1), (a2) und (a3),

die aus den erfindungsgemäßen dentalen polymerisierbaren Zusammensetzungen gehärteten dentalen Produkte, sowie deren jeweilige Verwendung als Dentalmaterial, insbesondere als fließfähiges oder stopfbares, permanentes oder temporäres Füllungskomposit, als sogenanntes "Bulk Fill" Material, als Stumpfaufbaumaterial, als dentaler Befestigungszement, als dentales Versiegelungsmaterial, als dentaler Lack, als dentales Unterfüllungsmaterial, als Kronen- oder Brückenmaterial, als dentales Adhäsiv (Bonding), als dentaler Primer, als weiches oder hartes Unterfütterungsmaterial, als Inlay, Onlay und/oder Overlay, als künstlicher Zahn, als KFO-Material, als Zahnfertigteil, als dentales Gerüst, als dentales Provisorium, als dentales Blockmaterial, als Teilprothese oder als Vollprothese. Darüber hinaus sind sie für einen Einsatz in generativen dentalen Fertigungsverfahren, auch als "Rapid Prototyping" bekannt, vorzugsweise für die Stereolithographie, vorzugsweise das Digital Light Processing (DLP), Selective Laser Assembling (SLA), die Mikrostereolithographie, das 3D-Printing, das Laminated Object Manufacturing oder das Film Transfer Imaging geeignet.

Die Erfindung betrifft ferner ein Verfahren zur Herstellung der dentalen Zusammensetzungen und ein Verfahren zur Herstellung eines jeweiligen dentalen Erzeugnisses. Weiterhin werden erfindungsgemäße Kits, enthaltend die neuen polymerisierbaren, dentalen Zusammensetzungen, beansprucht.

Die vorliegende Erfindung betrifft auch polymerisierbare dentale Zusammensetzungen, umfassend Bestandteile (A), (B) und (C) wie vorstehend definiert und eine oder mehr als eine Verbindung, die keine erfindungsgemäße Polysiloxanverbindung (ist) sind. Optional können erfindungsgemäße polymerisierbare dentale Zusammensetzungen somit auch

- (D) organische, polymerisierbare Monomere, die keine erfindungsgemäßen Polysiloxane sind, vorzugsweise zur Umsetzung mit den erfindungsgemäßen Polysiloxanen, enthalten.

Die vorliegende Erfindung betrifft auch polymerisierbare dentale Zusammensetzungen, umfassend Bestandteile (A), (B), (C) und optional (D) wie vorstehend definiert und eine oder mehr als eine Verbindung, die keine erfindungsgemäßen Polysiloxane sind, sondern polymerisierbare Verbindungen, die Säuregruppen enthalten, die kein Si-Atom aufweisen. Optional können erfindungsgemäße polymerisierbare dentale Zusammensetzungen somit auch

- (E) organische, säuregruppenhaltige Monomeren, die kein Si-Atom aufweisen, enthalten.

Erfindungsgemäße Ausführungsformen sind ebenfalls polymerisierbare Dentalmaterialien wie vorstehend definiert, weiter umfassend ein, zwei, mehr als zwei oder sämtliche Substanzen aus der Gruppe der Additive (F), bestehend aus:

- rheologische Hilfsmittel,
- Farbmittel, vorzugsweise Farbpigmente,
- Aromen
- Stabilisatoren, insbesondere Tageslichtstabilisatoren,
- Inhibitoren,
- Molekulargewichtsregler,
- Konservierungsmittel, vorzugsweise Parabene
- grenzflächenaktive Substanzen, vorzugsweise Tenside
- Mikrobizide, vorzugsweise Bakterizide,
- organische Polymere und Oligomere und Verbindungen mit hohen Molekulargewichten,
- Verdickungsmittel,
- dentale Arzneimittel und

- Weichmacher.

Erfindungsgemäße Ausführungsformen sind ebenfalls polymerisierbare Dentalmaterialien wie vorstehend definiert, weiter umfassend ein, zwei, oder mehr als zwei Lösungsmittel (G).

Ganz besonders bevorzugt betrifft die vorliegende Erfindung polymerisierbare, dentale Zusammensetzungen und polymerisierte dentale Zusammensetzungen, wie vorstehend definiert, zur spezifischen Anwendung in einem therapeutischen Verfahren (einem Verfahren zur therapeutischen Behandlung des menschlichen oder tierischen Körpers, vorzugsweise des menschlichen Körpers).

Der Begriff "umfassen" wird im Rahmen dieser Anmeldung in seiner üblichen und allgemein akzeptierten Bedeutung verwendet.

So sind natürlich auch polymerisierbare, dentale Zusammensetzungen erfindungsgemäß beansprucht, die (A) Polysiloxane enthalten, wobei die Polysiloxane wenigstens eine Mischung der Kondensate der drei Silane (a1), (a2) und (a3) und/oder wenigstens ein Cokondensat aus einer Mischung der drei Silane (a1), (a2) und (a3) und/oder wenigstens eine Mischung von mindestens zwei der Cokondensate (a1)/(a2), (a1)/(a3) und (a2)/(a3) und/oder wenigstens eine Mischung aus dem Kondensat eines der drei Silane (a1), (a2) oder (a3) mit dem Cokondesat der anderen beiden Silane enthalten.

Ebenfalls sind erfindungsgemäß polymerisierbare, dentale Zusammensetzungen beansprucht, die (A) Polysiloxane enthalten, wobei die Polysiloxane jegliche Mischungen von Kondensaten und Cokondensaten enthalten, die (a1), (a2) und (a3) aufweisen, beispielsweise die Mischung zweier oder dreier unterschiedlicher Cokondensate (a1)/(a2)/(a3) oder die Mischung eines Cokondensats (a1)/(a2)/(a3) mit dem Kondensat von (a1), usw.

In anderen Worten: Eine erfindungsgemäße polymerisierbare, dentale Zusammensetzung umfasst Homo- und/oder Cokondensate von jeweils mindestens einem der drei Silane (a1), (a2) und (a3).

Weitere Gesichtspunkte der vorliegenden Erfindung und deren bevorzugte Ausgestaltungen ergeben sich aus der folgenden Beschreibung, den Ausführungsbeispielen und den Ansprüchen.

Polysiloxanverbindungen sind seit langem bekannt und z.B. erhältlich durch Hydrolyse und Kondensation von Silanen mit hydrolysierbaren Gruppen (siehe z.B. DE 27 58 414 A1) oder durch Hydrosilylierung von Allyl- oder Vinylverbindungen mit SiH-haltigen Verbindungen. Polysiloxanverbindungen können zu einer Vielzahl von Produkten weiterverarbeitet werden, wie z.B. Überzüge, Beschichtungen, Membranen oder Bulkmaterialien. Diese Weiterverarbeitung beruht dabei häufig auf einer Vernetzungsreaktion organisch polymerisierbarer Gruppen in den Polysiloxanverbindungen (z.B. (Meth)acrylatgruppen) und der daraus resultierenden Bildung vernetzter Polysiloxanverbindungen.

Unter dem Begriff "(Meth)acryl" ist im Rahmen des vorliegenden Textes sowohl "Acryl" als auch "Methacryl" zu verstehen.

Unter dem Begriff "polymerisierbare Gruppen" sind im Rahmen des vorliegenden Textes funktionelle Gruppen zu verstehen, die als Bestandteile von Molekülen durch Aushärtung feste Polymerisate ergeben, die also zur Umwandlung von einer flüssig-pastösen Phase in eine feste Phase befähigt sind. Hierbei reagieren Monomeren zu Polymeren. Die Reaktionen umfassen dabei sowohl Kettenwachstumsreaktionen als auch Stufenwachstumsreaktionen, sie schließen somit die radikalische Kettenpolymerisation, die koordinative Kettenpolymerisation, die kationische Kettenreaktion und die anionische Kettenpolymerisation sowie die Polyaddition mit ein.

Vorzugsweise polymerisieren (härten oder vernetzen) die Gruppen in einer Kettenwachstumsreaktion entweder radikalisch oder kationisch, besonders bevorzugt polymerisieren sie radikalisch.

Mit polymerisierbaren Gruppen substituierte kettenförmige und/oder zyklische und/oder in Käfigform vorliegende Polysiloxane werden in der Regel über den Sol-Gel Prozess durch gezielte Hydrolyse und Kondensation von entsprechend funktionalisierten Derivaten von Alkoxiden des Siliziums oder von Halogensilanen synthetisiert. Diese Herstellverfahren sind in der Literatur vielfach beschrieben. Im Allgemeinen geht man bei einer solchen Synthese von einem Standardsilan; wie beispielsweise dem Isocyanatopropyldiethoxymethylsilan, aus, das in einem ersten Schritt, ebenfalls in einer Standardreaktion, beispielsweise in einer Isocyanat-Alkohol Addition, beispielsweise mit dem Glycerin-1,3-dimethacrylat zum entsprechenden Urethan umgesetzt wird. Die hierbei erhaltene Verbindung besteht auf der einen Seite aus dem Siliziumatom, das mit hydrolysier- und kondensierbaren Gruppen bestückt ist und das über einen sogenannten Abstandshalter, bestehend aus einer Alkylgruppe (hier eine Propylgruppe) und einer Urethangruppe als strukturelles Verbindungselement zu einem weiteren funktionellen Struktursegment, in diesem Fall zu zwei radikalisch polymerisierbare Methacrylatgruppen übergeht. Ein solch einfaches Syntheseverfahren kann in vielfacher Weise modifiziert werden, da die Reaktionsmöglichkeiten zwischen entsprechend funktionalisierten Silanen und geeigneten Reaktanden unbegrenzt erscheinen. Entsprechend groß sind die Synthesevorschläge in der Literatur. Die Ausgangsverbindung umfasst somit ein anorganisch kondensierbares Strukturelement, ein variabel gestaltbares Verbindungselement sowie ein polymerisierbares organisches Grundgerüst. In einer katalytisch gesteuerten Hydrolyse und Kondensation wird das Polysiloxan als ein anorganisches Kondensat, substituiert beispielsweise mit radikalisch oder kationisch polymerisierbaren Gruppen, gewonnen. Ob das Polykondensat in Form von Ketten, Ringen oder dreidimensionalen Käfigformen, bzw. in den entsprechenden Mischformen vorliegt, hängt von den genauen Bedingungen der Kondensation

ab. Dazu zählen neben den Reaktionsbedingungen (pH-Wert, Menge an Lösungsmittel und Wasser, Art und Menge des Katalysators, Reaktionstemperatur, Art der Aufbereitung, etc.) auch die Strukturformen des Ausgangssilans, wobei die Anzahl der Alkoxygruppen, die Anzahl der polymerisierbaren Gruppen, die chemische Art des Verbindungselements sowie die Kettenlänge des Abstandshalters von Bedeutung sind. Angaben hierzu finden sich sowohl in der wissenschaftlichen Literatur wie in der Patentliteratur.

Nachfolgend sind beispielhaft mögliche Strukturtypen angegeben, wobei die polymerisierbaren Gruppen hier radikalisch härtbare (Meth)acrylatgruppen aufweisen. Abgebildet sind ringförmige, kettenförmige, sowie käfigförmige Strukturen und eine mögliche Mischform von ring- und kettenförmigen Kondensaten. Abhängig von den Reaktionsbedingungen und den Strukturelementen der Edukte ist die Synthese sowohl von Reinformkondensaten als auch von entsprechenden Mischformkondensaten möglich.

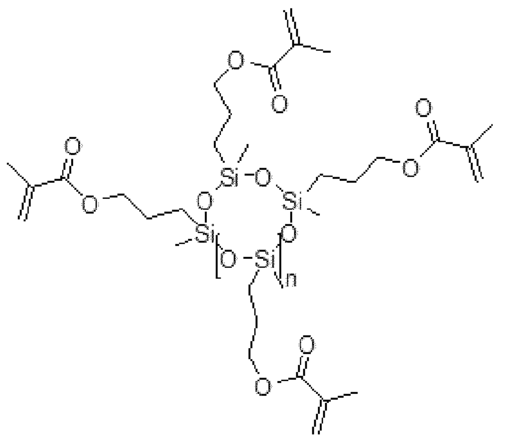

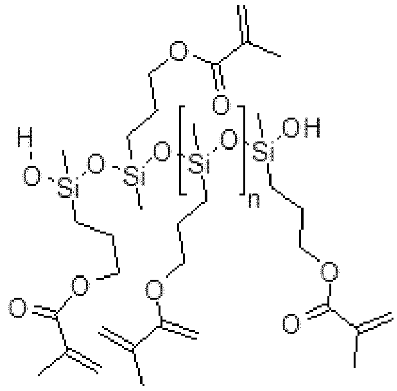

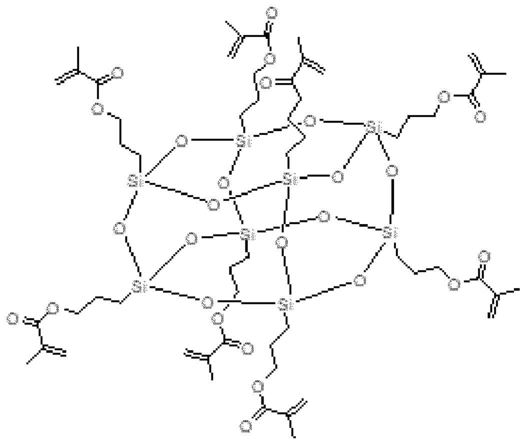

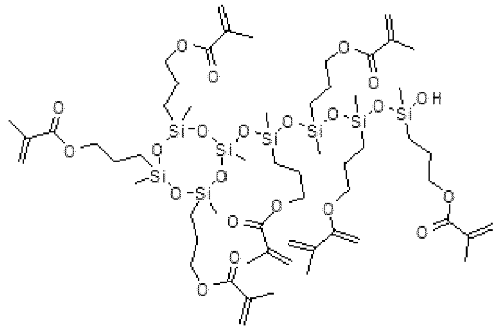

Die Polysiloxane verfügen als Bindeglied zwischen anorganischer und organischer Chemie über besondere Materialeigenschaften. Da sie zudem physiologisch inert sind, also keine nennenswerte Toxizität besitzen, sind sie speziell für Anwendungen in der Medizin bedeutsam. Grundlage für die kaum vorhandene Toxizität der Polysiloxane ist die geringe biologische Angreifbarkeit der Silizium-Kohlenstoffbindungen und die eingeschränkte Diffusionsfähigkeit der stark hydrophoben Polymerketten durch Zellmembranen, weshalb sie sich besonders zur Implantation (in Zähne) eignen sollten.

Eine spezifische Gruppe von Polysiloxanverbindungen enthält in den organischen Gruppen (Seitenketten) neben einer organisch polymerisierbaren Gruppe zusätzliche freie polare funktionelle Gruppen wie z.B. Hydroxy- oder Carboxygruppen.

So betrifft DE 44 16 857 C1 hydrolysierbare und polymerisierbare Silane, Verfahren zu deren Herstellung sowie deren Verwendung zur Herstellung von Kieselsäure(hetero)polykondensaten und (Hetero)polymerisaten. Hydrolysierbare, organisch modifizierte Silane finden eine breite Anwendung bei der Herstellung kratzfester Beschichtungen für die unterschiedlichsten Substrate, für die Herstellung von Füllstoffen, von Klebe- und Dichtungsmassen oder von Formkörpern.

Die DE 44 16 857 C1 offenbart den Einsatz von Kieselsäure(hetero)-polykondensaten (Polysiloxanverbindungen) in härtbaren Dentalmaterialien. Die hier beschriebenen Polysiloxanverbindungen umfassen freie polare funktionelle

Gruppen (z.B. Carboxy- oder Hydroxygruppen), die in der Lage sind, geeignete Metallionen / Übergangsmetallionen zu komplexieren (z.B. Ionen des Titans, Zirkoniums oder Zinns). In härtbaren dentalen Zusammensetzungen kann sich dies positiv auf die Röntgenopazität, die Kontakttoxizität und auf den Brechungsindex eines entsprechenden härtbaren bzw. gehärteten Dentalmaterials auswirken.

DE 198 60 364 C2 betrifft polymerisierbare Dentalmassen auf der Basis von zur Aushärtung befähigten Siloxanverbindungen, deren Verwendung und Herstellung. In dieser Druckschrift wird die Herstellung von cyclischen Polysiloxanen und deren Verwendung als Basis für polymerisierbare Dentalmassen beschrieben. Sie sollen trotz hoher Dichte an zur Polymerisation befähigten Gruppen eine niedrige Viskosität aufweisen, die eine hohe Füllstoffaufnahme ermöglicht, welche zu Massen mit geringem Polymerisationsschrumpf führen. Auch hier befinden sich in den organischen Seitenketten der beschriebenen Polysiloxane neben den polymerisierbaren Einheiten, freie polare Funktionen.

Die freien polaren funktionellen Gruppen, z.B. in den vorstehend genannten Polysiloxanverbindungen führen jedoch regelmäßig auch zu unerwünschten Eigenschaften. So hat sich gezeigt, dass die durch die (freien) polaren funktionellen Gruppen hervorgerufene Hydrophilie der Polysiloxanverbindungen zu einer erhöhten Wasseraufnahme in Gegenwart von Feuchtigkeit führt, wodurch die Nassfestigkeit des härtbaren Dentalmaterials in nachteiliger Weise verringert wird. Vermutlich durch Aufbau interner Wasserstoffbrückenbindungen kommt es zu einer Erhöhung der Viskosität. Dies wirkt sich dann negativ auf die Handhabbarkeit bei der Herstellung der härtbaren Dentalzusammensetzungen aus.

Es besteht ein beträchtliches Bedürfnis seitens Dentalmedizinern und der Dentalindustrie, Polysiloxanverbindungen weiter an die Anforderungen an ein modernes (härtbares bzw. gehärtetes) Dentalmaterial anzupassen und die vorstehend genannten Nachteile zu minimieren. Derart angepasste Polysiloxanverbindungen sollen dabei dentalmedizinisch verbesserte physikalische Eigenschaften besitzen (bzw. zu dentalmedizinisch verbesserten physikalischen Eigenschaften entsprechender härtbarer/gehärteter Dentalmaterialien führen), z.B. einen geringeren Polymerisationsschrumpf beim Polymerisieren/Vernetzen der Polysiloxanverbindungen (d.h. beim Aushärten), eine erhöhte Festigkeit und/oder eine begrenzte Wasseraufnahme bei gleichzeitig komfortabler Konsistenz des härtbaren Dentalmaterials.

Erste Erfolge bei der Verbesserung der Polysiloxane konnten durch Addition bzw. Substitution unterschiedlicher Substrate an die freien polaren Funktionalitäten der oben beschrieben speziellen Polysiloxane erreicht werden.

EP 1 874 847 B1 betrifft ein Verfahren zur Herstellung von Silanen mit zwei, drei oder sogar mehr Struktureinheiten, die über eine urethan-, säureamid- und/oder carbonsäureestergruppenhaltige Brücke miteinander verknüpft sind und von denen jede mindestens einen organisch polymerisierbaren Rest und mindestens einen Silylrest enthält. Diese Silane sollen sich insbesondere zur Modifizierung der Eigenschaften von Kieselsäure(hetero)polykondensaten und silylgruppenhaltigen, organischen Polymeren eignen. Das offenbarte Verfahren soll sich auch zur Verbrückung bereits vorkondensierter Kieselsäure(hetero)-polykondensate eignen.

Die in EP 1 874 847 B1 offenbarten Kieselsäure(hetero)polykondensate (Polysiloxanverbindungen) verfügen über eine freie Hydroxygruppe (d.h. eine freie polare funktionelle Gruppe). Diese freien Hydroxygruppen können mit einem Dicarbonsäurederivat oder Diisocyanat so reagieren, dass Hydroxylgruppen mit einem Dicarbonsäurederivat bzw. Diisocyanat eine Verknüpfung (Verbrückung) bilden. Solche verknüpften Polysiloxanverbindungen besitzen ein deutlich höheres Molekulargewicht, ohne dabei die Doppelbindungsdichte (bedingt durch die organisch polymerisierbaren (Meth) acrylatgruppen) wesentlich zu verringern. Unter Doppelbindungsdichte wird hier der Quotient aus der Anzahl der polymerisierbaren Doppelbindungen in einer Verbindung und dem Molekulargewicht dieser Verbindung verstanden. Das höhere Molekulargewicht wirkt sich positiv auf die Biokompatibilität und den Polymerisationsschrumpf beim Vernetzen der verknüpften Polysiloxanverbindungen aus. Gleichzeitig konnte die Hydrophobizität der Polysiloxanverbindungen gesteigert werden. Es konnte jedoch gezeigt werden, dass sich das höhere Molekulargewicht nachteilig auf die Viskosität der verknüpften Polysiloxanverbindungen (und damit auf die Verarbeitbarkeit bei der Herstellung des härtbaren Dentalmaterials) auswirkt. Die Viskosität steigt mit dem Grad der Verknüpfung, d.h. mit dem Molekulargewicht deutlich an, so dass eine tolerierbare Verarbeitbarkeit bei der Herstellung eines entsprechenden härtbaren Dentalmaterials, das solche verknüpften Polysiloxanverbindungen umfasst, schon bei recht geringem Verknüpfungsgrad nicht mehr zufriedenstellend gegeben ist.

EP 1 685 182 B1 betrifft Silane und daraus gebildete Kieselsäurepolykondensate und -teilkondensate, in denen ein an einem Silizium gebundener, organischer Rest vorhanden ist, der verzweigt ist und an jedem der beiden Zweige mindestens eine eigenständig organisch polymerisierbare Gruppe trägt oder an einem der beiden Zweige eine solche Gruppe, am anderen einen Rest mit einem weiteren Siliziumatom aufweist.

Auch die in EP 1 685 182 B1 offenbarten Polysiloxanverbindungen umfassen freie polare funktionelle Gruppen in Form von Hydroxygruppen. Durch Reaktion von Carbonsäure- oder Isocyanatderivate, die ihrerseits ebenfalls polymerisierbare Doppelbindungen umfassen (z.B. (Meth)acrylatgruppen), können so organisch polymerisierbare Gruppen an freie polare funktionelle Gruppen geknüpft werden. Diese Reaktionsprodukte besitzen regelmäßig eine erhöhte Festigkeit bei gleichzeitig gesteigerter Hydrophobizität und verbesserter Biokompatibilität durch das erhöhte Molekulargewicht.

Es konnte jedoch auch in diesen Fällen gezeigt werden, dass das Einführen zusätzlicher polymerisierbarer Doppelbindungen zu einem erhöhten Polymerisationsschrumpf beim Vernetzen der Polysiloxanverbindungen führt, da

sich die Doppelbindungsdichte deutlich erhöht, die Erhöhung des Molekulargewichtes aber nur vergleichsweise gering ist.

WO 2013/041723 A1 offenbart hydrolysierbare und polymerisierbare Silane (einschließlich Kieselsäurepolykondensate, d.h. Siloxane) mit einstellbarer räumlicher Verteilung der funktionellen Gruppen sowie deren Verwendung. Die in WO 2013/041723 A1 offenbarte Lehre betrifft ein Verfahren zur Kettenverlängerung von über Kohlenstoff an Silizium gebundenen Resten in Silanen oder Siloxanen.

WO 2013/053693 A1 offenbart Kieselsäurepolykondensate (Siloxane) mit cyclischen olefinhaltigen Strukturen und Verfahren zu deren Herstellung sowie deren Verwendung. WO 2013/053693 A1 offenbart, dass sich Polymerwerkstoffe mit in weiten Grenzen einstellbaren E-Moduln bei hoher elastischer Dehnung (d.h. ohne Sprödverhalten) und damit einer hohen Bruchzähigkeit aus Kieselsäure(hetero)polykondensaten mit cyclischen olefinhaltigen Strukturen herstellen lassen.

In der DE 10 2014 210 432 A1 sind Polysiloxanverbindungen beschrieben, die die vorstehend genannten Nachteile aus dem Stand der Technik in einer härtbaren bzw. gehärteten dentalen Zusammensetzung nicht oder zumindest nur noch abgeschwächt aufweisen. Der konzeptuelle Ansatz dieser Systeme sieht vor, die freie funktionelle Gruppe im Silan so umzusetzen, dass keine zusätzlich polymerisierbaren Doppelbindungen ins System eingeführt werden. Stattdessen werden Kohlenwasserstoffreste hohen Molekulargewichts mit wenigstens 11 C-Atomen in das System eingebaut. Überraschenderweise zeigten sich bei diesen härtbaren dentalen Zusammensetzungen

- eine gute Viskosität der Polysiloxanverbindungen (die Viskosität sollte 50 Pa*s oder weniger bei einer Temperatur von 25°C betragen) und eine damit einhergehende hervorragende Verarbeitbarkeit bei der Herstellung eines die Polysiloxanverbindungen enthaltenden härtbaren Dentalmaterials,
- eine gute Hydrophobizität,
- eine gute Festigkeit, insbesondere eine gute Biegefestigkeit,
- einen sehr geringen Polymerisationsschrumpf beim Vernetzen der Polysiloxanverbindungen, d.h. beim Aushärten des härtbaren Dentalmaterials,
- eine gute Biokompatibilität,
- einen Brechungsindex, der nahezu identisch ist mit dem Brechungsindex üblicher dentaler Gläser.

Durch die in der DE 10 2014 210 432 A1 ergriffenen Maßnahmen wurden somit gleich mehrere Probleme gelöst:

- durch Eliminierung polarer funktioneller Gruppen wurde die Ausbildung intermolekularer Wechselwirkungen unterbunden. Es gelang somit, die Viskosität des Systems trotz eines beachtlichen Molekulargewichtszuwachses auf einem vergleichsweise niedrigen Niveau zu halten.
- durch Einbau von Kohlenwasserstoffresten relativ hohen Molekulargewichts wurde das Polysiloxanstrukturgerüst räumlich intramolekular aufgeweitet, so dass die Zugänglichkeit der radikalisch polymerisierbaren Gruppen während der Härtung erhöht wurde und damit die Umsetzungsrate optimiert werden konnte. Wie sonst könnte man den Umstand erklären, dass in diesen Systemen bei vergleichsweise reduzierter Doppelbindungsdichte die Festigkeit der Materialien, beispielsweise die Biegefestigkeit der gehärteten dentalen Zusammensetzungen auf einem sehr guten Niveau verbleiben und in vielen Fällen sogar erhöht sind im Vergleich zu den nicht weiter umgesetzten Polysiloxanen.
- durch die Molekulargewichtserhöhung bei Belassen der Funktionalität, also bei einer effektiven Absenkung der Doppelbindungsdichte konnte insbesondere die klinisch vielleicht wichtigste technische Kenngröße einer härtbaren dentalen Zusammensetzung, nämlich der Wert des Volumenschrumpfs während der Aushärtung, auf einen extrem niedrigen Wert eingestellt werden. In der klinischen Praxis ist der therapeutische Erfolg in erster Linie auch davon abhängig, ob das Dentalmaterial beispielsweise eine vom Zahnarzt präparierte Kavität randdicht abschließt. Durch Schrumpfung des Materials bei der Polymerisation können sich Randspalte bilden, durch die Bakterien in den Zahn eindringen und die Therapie dann scheitern lassen.
- Durch Einbau von Kohlenwasserstoffresten relativ hohen Molekulargewichts wurde das Polysiloxanstrukturgerüst auch vergleichsweise hydrophober gemacht, so dass die nicht erwünschte Wasseraufnahme nunmehr extrem geringe Werte annimmt.

Die in der DE 10 2014 210 432 A1 beschriebenen radikalisch härtbaren Zusammensetzungen eignen sich speziell zur Anwendung in einem therapeutischen Verfahren zum temporären oder permanenten Füllen einer dentalen Kavität. Weiterhin geeignet sind die Systeme zur Anwendung in einem therapeutischen Verfahren als Unterfüllungsmaterial, als Adhäsiv (Bonding), als fließfähiges Kompositmaterial (Flow Material), als Fissurenversiegler, als Kronen- und Brückenmaterial, als Inlay/Onlay und/oder als Stumpfaufbaumaterial.

Die DE 10 2014 116 389 A1 offenbart Kombinationen von Polysiloxanen mit Disiloxanen, die sich besonders gut zur Herstellung fließfähiger, härtbarer Dentalmaterialien eignen, die durch eine Applikationskanüle oder einem statischen

Mischer appliziert werden. Neben guten Fließeigenschaften in Kanüle bzw. Mischer und einem optimalen Anfließen auf der Zahnsubstanz weisen diese Materialien eine ausreichend hohe Standfestigkeit auf, so dass die durch die Polysiloxane eingebrachten guten physikalischen Eigenschaften voll zu Tragen kommen. Durch die Kombination von Polysiloxanen mit Disiloxanen wurde darüber hinaus überraschenderweise auch noch der E-Modul erhöht. Das gehärtete Dentalmaterial kann somit seiner Verformung einen größeren Widerstand entgegensetzen und so den stetigen Kaubelastungen besser widerstehen.

Die DE 10 2014 116 402 A1 lehrt die Verwendung der Kombinationen von Polysiloxanen mit Disiloxanen in generativen Fertigungsverfahren, wobei die Verfahren die Stereolithographie, das digital light processing, die Polyjettechnologie, die Galvanometer type scanning method, die Mikrostereolithographie, das Multi-Jet Modelling, das selektive Lasersintern, das 3D-printing, das Fused Deposition Modelling, das 3D-Plotting, das Laminated Object Manufacturing oder das Film Transfer Imaging umfassen.

MEGAN A. COLE ET AL: "Thiol-ene functionalized siloxanes for use as elastomeric dental impression materials" (DENTAL MATERIALS, Bd. 30, Nr. 4, 1. April 2014 (2014-04-01), Seiten 449-455, XP055743888, AMSTERDAM, NL ISSN: 0109-5641, DOI: 10.1016/j.dental.2014.01.011) offenbart dentale Abformmaterialien, die phenylsubstituierte Polysiloxane enthalten. Neben den Phenylgruppen enthalten die Polysiloxane alternativ Thiol- oder Allylgruppen und härten durch eine Thiol-En-Reaktion aus.

JP H08 311115 A (MITSUBISHI RAYON CO) offenbart die Verwendung von methacryl- und phenylsubstituierten Silanen wie $\gamma$-Methacryloyloxypropyltrimethoxysilan und Phenyltrimethoxysilan zur Hydrophobierung der Oberfläche von kolloidaler Kieselsäure.

Der Einsatz von Polysiloxanen zur Herstellung dentaler härtbarer Zusammensetzungen hat im Laufe der Zeit einige unterschiedliche Entwicklungsfortschritte erzielt, doch allein die Tatsache, dass es heute insgesamt nur drei Produktfamilien auf Basis von Polysiloxanen gibt, die im Markt kommerziell erhältlich sind, zeigt, dass der große Durchbruch dieser interessanten und bioverträglichen Substanzklasse noch nicht vollzogen ist. Hauptgrund für die derzeit noch fehlende allgemeine Akzeptanz dieser Substanzklasse ist der Umstand, dass die polymerisierten Polysiloxane in der Spitze bisher nicht die hohen mechanischen Werte der Biegefestigkeit und des E-Moduls erreichen, die herkömmliche Systeme auf der Basis klassischer Dentalmonomeren (Bis-GMA, UDMA und TEGDMA) aufweisen. Neben der Biegefestigkeit ist der E-Modul ein entscheidender Parameter und eine Schlüsseleigenschaft bei der Auswahl eines Restaurationsmaterials, da ein hoher E-Modul einen hohen Widerstand des Materials zu seiner elastischen Deformation bedeutet, was insbesondere bei der Kompensation okklusaler Kräfte zum Tragen kommt und die hohe klinische Relevanz dieses Parameters begründet.

Laut aktuell gültiger ISO Norm DIN EN ISO 4049, betitelt "Polymerbasierende Restaurationsmaterialien", beträgt der Grenzwert der Biegefestigkeit für diese Materialklasse für den Fall, dass das Material auch für die Restauration von Okklusalflächen geeignet ist, 80 MPa. Ein solches Werteniveau weisen Restaurationsmaterialien auf Basis von Polysiloxanen allemal auf, allerdings werden die Spitzenwerte herkömmlicher Kompositrestaurationsmaterialien verfehlt.

In einer großangelegten, älteren Untersuchung von N. Ilie und R. Hickel (Clin. Oral Invest. (2009), 13, 427 - 438), betitelt "Investigations on mechanical behaviour of dental composites" wurden 61 kommerziell erhältliche Kompositmaterialien untersucht und gemäß Norm geprüft. Dabei gab es folgende Ergebnisse:

Biegefestigkeit = BF in MPa, E-Modul = EM in GPa, angegeben werden die Durchschnittswerte, siehe Publikation, Tabelle 2, Seite 433 unten
Hybrid Komposite: 29 untersuchte Materialien, BF 116.9 , EM 7.3
Stopfbare Komposite: 9 untersuchte Materialien, BF 105.9, EM 8.4
Polysiloxan basierte Komposite: 2 untersuchte Materialien, BF 104.3, EM 7.5
Nano-hybrid Komposite: 7 untersuchte Materialien, BF 103.1, EM 5.0
Fließfähige Komposite: 10 untersuchte Materialien, BF 99.8, EM 4.4
Mikrogefüllte Komposite: 4 untersuchte Materialien, BF 73.5, EM 3.8

Die Werte belegen ein akzeptables Niveau der mechanischen Eigenschaften von polysiloxanbasierten Restaurationsmaterialien, wobei zu berücksichtigen ist, dass diese frühen polysiloxanbasierten Restaurationsmaterialien neben Polysiloxanen zusätzlich noch einen größeren Anteil an herkömmlichen Dentalmonomeren ohne Si-Atomen aufweisen.

In einer ganz aktuellen Zusammenstellung, die dem Lehrbuch "Werkstoffkunde in der Zahnmedizin, Moderne Materialien und Technologien", herausgegeben von M. Rosentritt, N. Illie, U. Lohbauer, 2018, Georg Thieme Verlag, Stuttgart, Seite 208, Tabelle 7.3, "Mechanische Eigenschaften verschiedener Kompositkategorien", entnommen ist, finden sich die folgenden Werte:

faserverstärktes Komposit: BF 132.9, EM 8.2
Mikrohybrid: BF 127.8, EM 6.8

Bulk-fill (fließfähig): BF 127.6, EM 5.0
Nanohybrid: BF 125.7, EM 5,9
Bulk-fill (hochviskos): BF 123.4, EM 7.1
fließfähig: BF 119.3, EM 4.2

Diese Zusammenstellung verdeutlicht, dass innerhalb der letzten 10 Jahre die mechanischen Werte der Biegefestigkeit aller Restaurationsmaterialien im Durchschnitt um jeweils ca. 20 Punkte angehoben werden konnten und dass neue Kompositzusammensetzungen entstanden sind (Bulk-fill Materialien).

Im Gegensatz zu den klassischen lichthärtenden Füllungskompositen, die in einzelnen, dünnen Schichten von ca. 2 mm appliziert werden, um eine ausreichende Durchhärtung des Materials sicherzustellen und um den Volumenschrumpf kompensieren zu können, sind Bulk-Fill Materialien in der Lage, in Schichtstärken von bis zu 5 mm photopolymerisiert zu werden. Diese Fähigkeit wurde durch eine höhere Transparenz der Materialien erreicht. Bulk-Fill Komposite werden - wie aus der obigen Tabelle hervorgeht - in zwei Gruppen unterteilt: die niedrigviskosen, fließfähigen Materialien und die hochviskosen, modellierbaren Typen.

Aufgabe der Erfindung war es somit, die mechanischen Werte von Biegefestigkeit und E-Modul bei den bioverträglichen Polysiloxanen deutlich zu verbessern und sie den herausragenden Werten herkömmlicher dentaler Kompositsysteme in der Spitze anzugleichen, bzw. die mechanischen Werte konventioneller Restaurationsmaterialien zu übertreffen. Zugleich sollten die klinisch relevanten Werte des Volumenschrumpfs reduziert werden. Durch die Polymerisation werden Bindungen zwischen den Molekülen geknüpft, so dass sich die Edukte enger aneinanderlegen und die resultierenden festen Polymerisate eine dichtere Struktur gegenüber den flüssigen Ausgangsmaterialien aufweisen. Diese Volumenkontraktion kann innerhalb des Restaurationsmaterials zu Spannungen führen und ist klinisch unerwünscht. Randspaltbildungen und das Entstehen von Sekundärkaries werden mit dem Phänomen des Schrumpfes in Zusammenhang gebracht.

Überraschenderweise wurde jetzt gefunden, dass dentale polysiloxanbasierte Kompositmaterialien zur Restauration mit extrem guten mechanischen Eigenschaften erhältlich sind - auch ohne auf herkömmliche Dentalmonomeren zurückgreifen zu müssen - wenn die polymerisierbare Matrix Polysiloxane umfasst, wobei die Polysiloxane (A) eine Mischung der Kondensate der drei Silane (a1), (a2) und (a3) und/oder ein Cokondensat aus einer Mischung der drei Silane (a1), (a2) und (a3) und/oder eine Mischung von mindestens zwei der Kondensate (a1)/(a2), (a1)/(a3) und (a2)/(a3) und/oder eine Mischung aus dem Kondensat eines der drei Silane (a1), (a2) und (a3) mit dem Cokondensat der anderen beiden Silane umfassen.

Erfindungsgemäß beträgt der Anteil der Silane (a1), (a2) und (a3) im Cokondensat oder in der Mischung der Kondensate oder Cokondensate (a1) in einer Menge von 10 bis 70 Gew.-%, bevorzugt von 20 bis 60 Gew.-%, (a2) in einer Menge von 10 bis 70 Gew.-%, bevorzugt von 20 bis 50 Gew.-% und (a3) in einer Menge von 5 bis 60 Gew.-%, bevorzugt von 10 bis 40 Gew.-%, jeweils bezogen auf die Gesamt Menge der Silane (a1), (a2) und (a3).

Solche Mischungen sind neu und werden aus dem Stand der Technik nicht nahegelegt.

<u>Bestandteil (A) - unterschiedliche Siloxane (a1), (a2) und (a3)</u>

Im Folgenden werden die Strukturen der Silane (a1), (a2) und (a3) näher erläutert und die Vorschriften zu ihren Synthesen detailliert angegeben. Außerdem werden kommerziell erhältliche Ausgangssubstanzen aufgeführt. Die in diesem Zusammenhang genannten CAS-Nummern sind beispielhaft zu verstehen. So können gleiche Substanzen eventuell auch unterschiedliche CAS-Nummern haben. Dies kann z.B. der Fall sein, wenn es unterschiedliche Isomere (Konstitutionsisomere, Stereoisomere, Konformationsisomere, Konfigurationsisomere, Enantiomere, Diastereomere) gibt oder wenn die Substanz als Reaktionsprodukt ihrer Ausgangangssubstanzen gekennzeichnet ist. Auch diese Varianten sind geeignet und können eingesetzt werden.

<u>Silan $(R^1O)_aR^2_bSi[-A(-Y\{-B[-PG]_f\}_e)_d]_c$</u> (a1):

Das Silan (a1) entspricht der Formel $(R^1O)_aR^2_bSi[-A(-Y\{-B[-PG]_f\}_e)_d]_c$ mit

Y = -C(=O)NH-, -NHC(=O)-, -OC(=O)NH-, -NHC(=O)O-, -SC(=O)NH-, -NHC(=O)S-, -NHC(=O)NH-, -OC(=O)N(-C(=O)NH-)-, -SC(=O)N(-C(=O)NH-)-, -NHC(=O)N(-C(=O)NH-)-, -C(=O)NHC(=O)NH-, -NHC(=O)NHC(=O)-
wobei die jeweils im Formelbild links angeordnete Bindung dem Strukturelement A und die rechts angeordnete Bindung dem Strukturelement B näher ist,
PG = polymerisierbare Gruppe,
wobei die polymerisierbare Gruppe PG ausgewählt ist aus der Gruppe bestehend aus $-Z-C(=O)-CH=CH_2$ und $-Z-C(=O)-C(CH_3)=CH_2$ wobei Z ausgewählt ist aus der Gruppe bestehend aus O und NH,
A = eine organische Verbindungsgruppe, die Si mit Y verbindet und 1 bis 20 C-Atome aufweist,

B = eine organische Verbindungsgruppe, die Y mit PG verbindet und 1 bis 20 C-Atome aufweist,
$R^1$ = H oder C1- bis C4-Alkyl,
$R^2$ = C1- bis C4-Alkyl,
a = 2 oder 3,
b = 0 oder 1,
c = 1 oder 2,
d = 1 bis 3,
e = 1 oder 2,
f = 1 bis 5 und

$$a + b + c = 4.$$

In einer bevorzugten Ausführungsform ist die Gruppe Y ausgewählt aus der Gruppe bestehend aus $-(X)_x-C(=O)NH-$, $-NHC(=O)-(X)_x-$ und $-(X)_xC(=O)N(-C(=O)NH-)-$ wobei X ausgewählt ist aus der Gruppe bestehend aus O, S und NH, vorzugsweise aus O und S, und wobei der Index x entweder 0 oder 1 ist, vorzugsweise 1 ist.

In einer bevorzugten Ausführungsform ist die polymerisierbare Gruppe PG ausgewählt aus der Gruppe bestehend aus $-Z-C(=O)-CH=CH_2$ und $-Z-C(=O)-C(CH_3)=CH_2$ wobei Z = O.

Die organische Verbindungsgruppe A, die das Si-Atom mit der Gruppe Y verbindet, ist eine (d+1)-valente, geradkettige, verzweigte oder cyclische Gruppe, die 1 bis 20 C-Atome aufweist sowie optional O-Atome, S-Atome, NR-Gruppen, Estergruppen oder Thioestergruppen aufweisen kann.

In einer bevorzugten Ausführungsform ist die Verbindungsgruppe A ausgewählt aus der Gruppe bestehend aus $-(CH_2)_n-$ mit n = 1 bis 12, $-(CH_2)_n-N(CH_3)-(CH_2)_n-$mit n = 1 bis 4, $-(CH_2)_nN[(CH_2)_n-]_2$ mit n = 1 bis 4 und $-(CH_2)_n-CH(R)-(CH_2)_n-$ mit n = 0 bis 4 und R = Methyl, Ethyl, Phenyl, wobei jeweils die links angeordnete Bindung mit dem Si-Atom verbunden ist und die rechts angeordnete Bindung mit der Gruppe Y verbunden ist.

In einer ganz bevorzugten Ausführungsform ist die Verbindungsgruppe A $-(CH_2)_n-$mit n = 1 bis 6.

Die organische Verbindungsgruppe B, die die Gruppe Y mit der polymerisierbaren Gruppe PG verbindet, ist eine (f+1)-valente, geradkettige, verzweigte oder cyclische Gruppe, die 1 bis 20 C-Atome aufweist sowie optional O-Atome, S-Atome, NR-Gruppen, Estergruppen oder Thioestergruppen aufweisen kann.

In einer bevorzugten Ausführungsform ist die Verbindungsgruppe B ausgewählt aus der Gruppe bestehend aus $-(CH_2)_n-$ mit n = 1 bis 12, $-CH_2CH(CH_3)-$, $-CH(CH_3)CH_2-$, $-(CH_2)_2(OCH_2CH_2)_n-$ mit n = 1 bis 6, $-(CH_2)_nOC(=O)(CH_2)_m-$ mit n,m = 1 bis 6, $-(CH_2)_nC(=O)O(CH_2)_m-$ mit n,m = 1 bis 6, $-CH(CH_2OPh)CH_2-$,

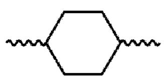 , 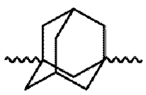 , 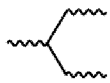 , 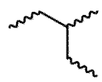 , 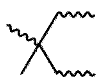 , 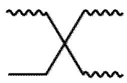 , 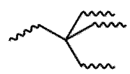 ,

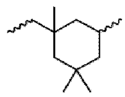 und 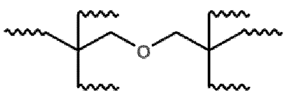 ,

wobei jeweils die links angeordnete Bindung mit der Gruppe Y verbunden ist und die nach rechts angeordnete(n) Bindung(en) mit der/den Gruppe(n) PG verbunden ist/sind.

In einer ganz bevorzugten Ausführungsform ist die Verbindungsgruppe B $-(CH_2)_n-$mit n = 1 bis 6.

In einer bevorzugten Ausführungsform ist der Rest $R^1$ ausgewählt aus der Gruppe bestehend aus H, Methyl und Ethyl.

In einer bevorzugten Ausführungsform ist der Index d = 1 bis 2.

In einer bevorzugten Ausführungsform ist der Index f = 1 bis 2.

Bevorzugte Silane (a1) sind

$$(R^1O)_aR^2_bSi[-A(-C(=O)-NH-B[-Z-C(=O)C(=CH_2)R^3]_f)_d]_c,$$

$$(R^1O)_aR^2_bSi[-A(-NH-C(=O)-B[-Z-C(=O)C(=CH_2)R^3]_f)_d]_c,$$

$$(R^1O)_aR^2_bSi[-A(-X-C(=O)-NH-B[-Z-C(=O)C(=CH_2)R^3]_f)_d]_c$$

und

$$(R^1O)_aR^2_bSi[-A(-NH-C(=O)-X-B[-Z-C(=O)C(=CH_2)R^3]_f)_d]_c$$

mit

A = eine organische Verbindungsgruppe, die Si mit Y verbindet ausgewählt aus der Gruppe bestehend aus $-(CH_2)_n-$ mit n = 1 bis 12, $-(CH_2)_n-N(CH_3)-(CH_2)_n-$ mit n = 1 bis 4, $-(CH_2)_nN[(CH_2)_n-]_2$ mit n = 1 bis 4 und $-(CH_2)_n-CH(R)-(CH_2)_n-$ mit n = 0 bis 4 und R = Methyl, Ethyl, Phenyl, bevorzugt $-(CH_2)_n-$ mit n = 1 bis 6,

B = eine organische Verbindungsgruppe, die Y mit Z verbindet ausgewählt aus der Gruppe bestehend aus $-(CH_2)_n-$ mit n = 1 bis 12, $-CH_2CH(CH_3)-$, $-CH(CH_3)CH_2-$, $-(CH_2)_2(OCH_2CH_2)_n-$ mit n = 1 bis 6, $-(CH_2)_nOC(=O)(CH_2)_m-$ mit n,m = 1 bis 6, $-(CH_2)_nC(=O)O(CH_2)_m-$ mit n,m = 1 bis 6, $-CH(CH_2OPh)CH_2-$,

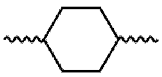 , 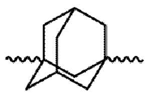 , 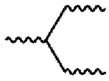 , 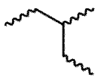 , 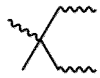 , 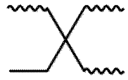 , 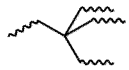 ,

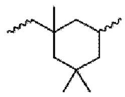 und 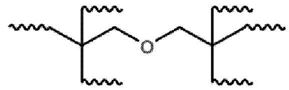 ,

bevorzugt $-(CH_2)_n-$ mit n = 1 bis 6,

X = O, S, NH; bevorzugt O

Z = O, NH; bevorzugt O

$R^1$ = H oder C1- bis C4-Alkyl, bevorzugt H, Methyl oder Ethyl

$R^2$ = C1- bis C4-Alkyl,

$R^3$ = H, Methyl; bevorzugt Methyl

a = 2 oder 3,

b = 0 oder 1,

c = 1 oder 2,

d = 1 bis 3, bevorzugt 1 bis 2,

e = 1 oder 2,

f = 1 bis 5, bevorzugt 1 bis 2 und

$$a + b + c = 4.$$

Synthese von Silanen mit Urethan-, Thiourethan-, Harnstoff- oder Amidgruppen ausgehend von Isocyanaten

Silane mit Urethan-, Thiourethan- oder Harnstoffgruppen lassen sich einfach durch Sn- oder Bi-katalysierte Umsetzung der entsprechenden Silane mit OH-, SH- oder $NH_2$-Gruppen mit (meth)acrylsubstituierten Isocyanaten herstellen.

Alternativ ist auch die Sn- oder Bi-katalysierte Umsetzung von isocyanatsubstituierten Silanen mit den entsprechenden OH-, SH- oder $NH_2$-substituierten (Meth)acryl-Verbindungen möglich.

Durch die Umsetzung von isocyanatsubstituierten Silanen mit carboxylsubstituierten (Meth)acryl-Verbindungen lassen sich unter $CO_2$-Abspaltung die entsprechenden Amide herstellen.

Synthese von $(R^1O)_aR^2_bSi[-A(-X-C(=O)-NH-B[-Z-C(=O)C(=CH_2)R^3]_f)_d]_c$

Die Synthese der Silane $(R^1O)_aR^2_bSi[-A(-X-C(=O)-NH-B[-Z-C(=O)C(=CH_2)R^3]_f)_d]_c$ erfolgt unter Zinn- oder Bismuth-Katalyse aus einem Silan mit XH-Gruppe und einer (Meth)acryl-Verbindung mit Isocyanatgruppe. Bevorzugte Katalysatoren sind Dibutylzinndilaurat und Bismuthneodecanoat. Unter leichter Erwärmung wird im äquimolaren Verhältnis jeweils eine XH-Gruppe mit einer Isocyanatgruppe umgesetzt. Die Reaktion verläuft in der Regel vollständig und kann leicht im IR-Spektrum verfolgt werden, bis die Isocyanat-Bande (2270 $cm^{-1}$) vollständig verschwunden ist.

$$(R^1O)_aR^2_bSi[-A(-XH)_d]_c + (cxd)\ [R^3-C(=CH_2)C(=O)-Z-]_fB-NCO \rightarrow (R^1O)_aR^2_bSi[-A(-X-C(=O)-NH-B[-Z-C(=O)C(=CH_2)R^3]_f)_d]_c$$

In den folgenden beiden Tabellen 1 und 2 sind eine Reihe kommerziell erhältlicher Ausgangsverbindungen aufgeführt.

Tabelle 1:

| | $(R^1O)_aR^2_bSi[-A(-XH)_d]_c$ | | | | | | | |
|---|---|---|---|---|---|---|---|---|
| **CAS-Nr.** | $R^1$ | $R^2$ | **XH** | **a** | **b** | **c** | **d** | **-A-** |
| 53764-54-8 | Me | - | OH | 3 | 0 | 1 | 1 | $-(CH_2)_3-$ |
| 53394-61-9 | Et | - | OH | 3 | 0 | 1 | 1 | $-(CH_2)_3-$ |
| 99697-20-8 | Me | Me | OH | 2 | 1 | 1 | 1 | $-(CH_2)_3-$ |
| 162781-70-6 | Et | - | OH | 3 | 0 | 1 | 1 | $-CH_2-$ |
| 4420-74-0 | Me | - | SH | 3 | 0 | 1 | 1 | $-(CH_2)_3-$ |
| 14814-09-6 | Et | - | SH | 3 | 0 | 1 | 1 | $-(CH_2)_3-$ |
| 31001-77-1 | Me | Me | SH | 2 | 1 | 1 | 1 | $-(CH_2)_3-$ |
| 30817-94-8 | Me | - | SH | 3 | 0 | 1 | 1 | $-CH_2-$ |
| 60764-83-2 | Et | - | SH | 3 | 0 | 1 | 1 | $-CH_2-$ |
| 877593-17-4 | Me | - | SH | 3 | 0 | 1 | 1 | $-(CH_2)_{11}-$ |
| 57765-40-9 | Me | - | SH | 2 | 0 | 2 | 1 | $-(CH_2)_3-$ |
| 13822-56-5 | Me | - | $NH_2$ | 3 | 0 | 1 | 1 | $-(CH_2)_3-$ |
| 3663-44-3 | Me | Me | $NH_2$ | 2 | 1 | 1 | 1 | $-(CH_2)_3-$ |
| 3179-76-8 | Et | Me | $NH_2$ | 2 | 1 | 1 | 1 | $-(CH_2)_3-$ |
| 71408-48-5 | Me | - | $NH_2$ | 3 | 0 | 1 | 1 | $-CH_2-$ |
| 18306-83-7 | Et | - | $NH_2$ | 3 | 0 | 1 | 1 | $-CH_2-$ |
| 51749-36-1 | Me | - | $NH_2$ | 2 | 0 | 2 | 1 | $-(CH_2)_3-$ |
| 53746-12-6 | Et | - | $NH_2$ | 2 | 0 | 2 | 1 | $-(CH_2)_3-$ |
| 330457-46-0 | Me | - | OH | 3 | 0 | 1 | 1 | $-(CH_2)_3-N(CH_3)-(CH_2)_2-$ |
| 24801-87-4 | Me | - | OH | 3 | 0 | 1 | 2 | $-(CH_2)_3N[(CH_2)_2-]_2$ |
| 7538-44-5 | Et | - | OH | 3 | 0 | 1 | 2 | $-(CH_2)_3N[(CH_2)_2-]_2$ |

Tabelle 2:

| | $[R^3-C(=CH_2)C(=O)-Z-]_fB-NCO$ | | | |
|---|---|---|---|---|
| **CAS-Nr.** | $R^3$ | **Z** | **f** | **-B-** |
| 30674-80-7 | Me | O | 1 | $-(CH_2)_2-$ |
| 130025-29-5 | Me | O | 1 | $-CH_2CH(CH_3)-$ |
| 86241-25-0 | Me | O | 1 | $-(CH_2)_3-$ |
| 93956-19-5 | Me | O | 1 | $-CH_2-$ |
| 13641-96-8 | H | O | 1 | $-(CH_2)_2-$ |
| 223909-47-5 | H | O | 1 | $-CH_2CH(CH_3)-$ |
| 119096-71-8 | H | O | 1 | $-(CH_2)_3-$ |
| 886577-76-0 | H | O | 2 | 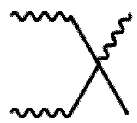 |
| 953028-96-1 | H | NH | 1 | $-(CH_2)_3-$ |

(fortgesetzt)

| | $[R^3\text{-}C(=CH_2)C(=O)\text{-}Z\text{-}]_fB\text{-}NCO$ | | | |
|---|---|---|---|---|
| CAS-Nr. | $R^3$ | Z | f | -B- |
| 61994-33-0 | H | NH | 1 | 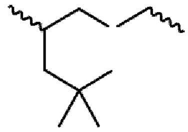 |

Im Folgenden werden beispielhaft einige Synthesen der Silane $(R^1O)_aR^2_bSi[\text{-}A(\text{-}X\text{-}C(=O)\text{-}NH\text{-}B[\text{-}Z\text{-}C(=O)C(=CH_2)R^3]_f)_d]_c$ dargestellt.

Durch die Umsetzung von 3-(Trimethoxysilyl)-1-propanol mit 2-Isocyanatoethylmethacrylat erhält man das Silan (1).

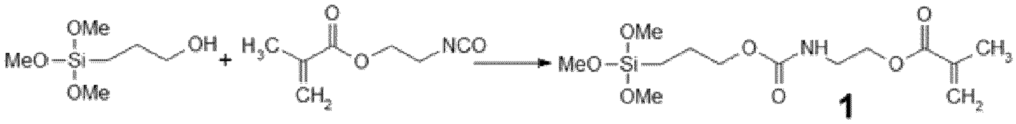

1

Durch die Umsetzung von 3-(Methyldimethoxysilyl)-1-propanol mit 2-Isocyanatoethylmethacrylat erhält man das Silan (2).

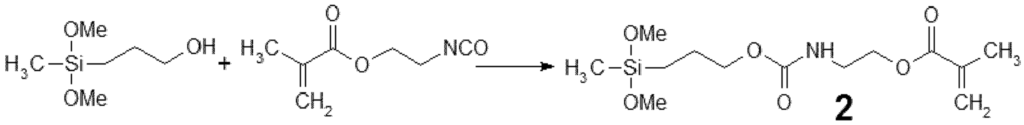

2

Durch die Umsetzung von 1-(Triethoxysilyl)methanol mit 2-Isocyanatoethylmethacrylat erhält man das Silan (3).

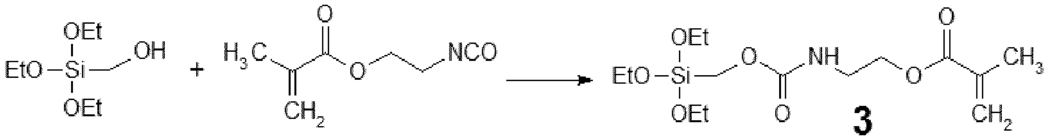

3

Durch die Umsetzung von 3-(Trimethoxysilyl)-1-propanol mit 3-Isocyanatopropylmethacrylat erhält man das Silan (4).

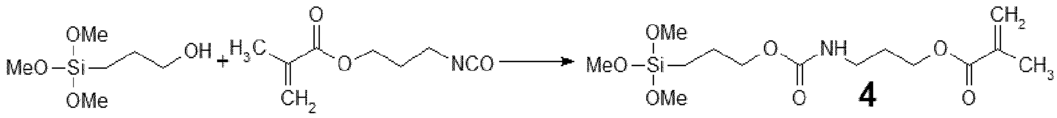

4

Durch die Umsetzung von 3-(Trimethoxysilyl)-1-propanol mit 1,1-Bis(acryloyloxy-methyl)ethylisocyanat erhält man das Silan (5).

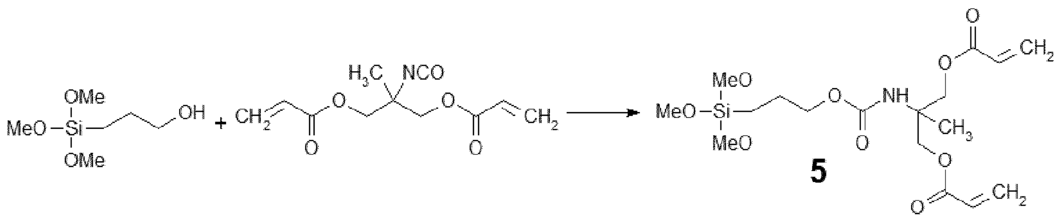

5

Durch die Umsetzung von 1-(Trimethoxysilyl)methanthiol mit 2-Isocyanatoethylmethacrylat erhält man das Silan (6).

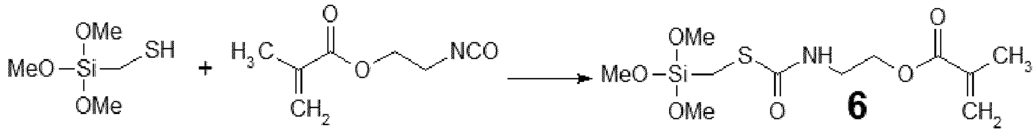

6

Durch die Umsetzung von 3-(Trimethoxysilyl)-1-propanthiol mit 2-Isocyanatoethylmethacrylat erhält man das Silan (7).

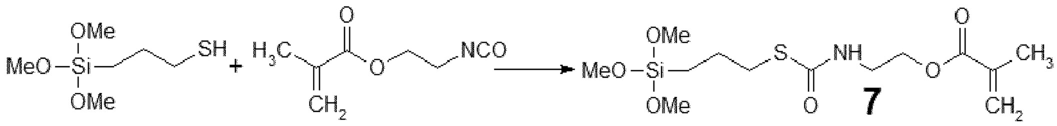

7

Durch die Umsetzung von 11-(Trimethoxysilyl)-1-undecanthiol mit 2-Isocyanatoethylmethacrylat erhält man das Silan (8).

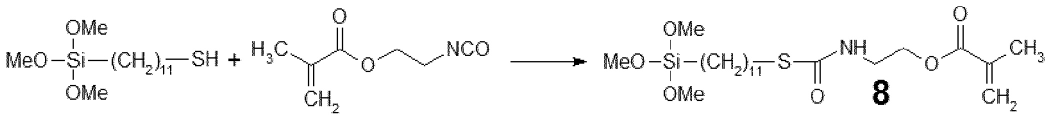

8

Durch die Umsetzung von 3-(Methyldimethoxysilyl)-1-propanthiol mit 2-Isocyanatoethylmethacrylat erhält man das Silan (9).

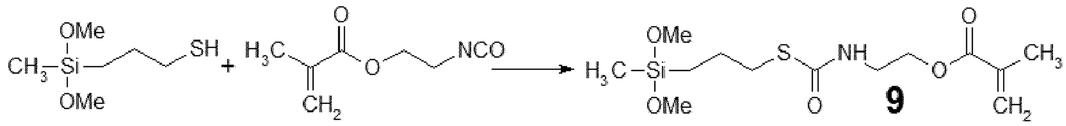

9

Durch die Umsetzung von 1-(Trimethoxysilyl)-methanamin mit 2-Isocyanatoethylmethacrylat erhält man das Silan (10).

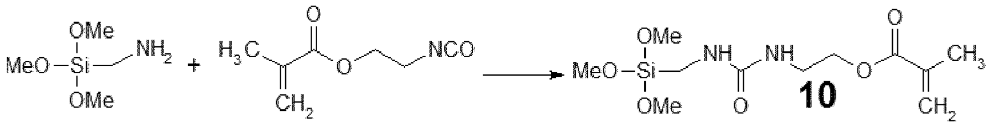

10

Durch die Umsetzung von 3-(Trimethoxysilyl)-1-propanamin mit 2-Isocyanatoethylmethacrylat erhält man das Silan (11).

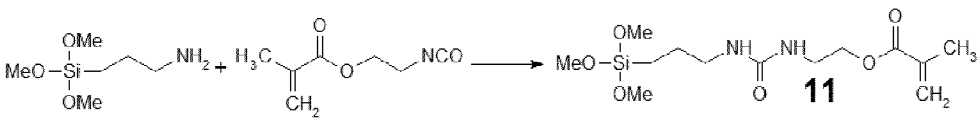

11

Durch die Umsetzung von 3-(Methyldimethoxysilyl)-1-propanamin mit 2-Isocyanatoethylmethacrylat erhält man das Silan (12).

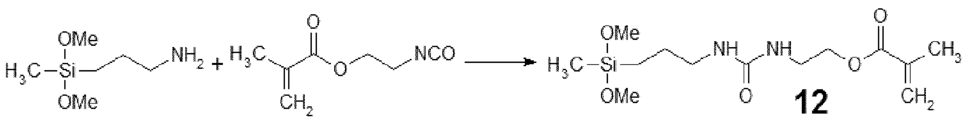

12

Durch die Umsetzung von 3,3'-(Diethoxysilylene)bis[1-propanamin] mit zwei Äquivalenten 2-Isocyanatoethylmethacrylat erhält man das Silan (13).

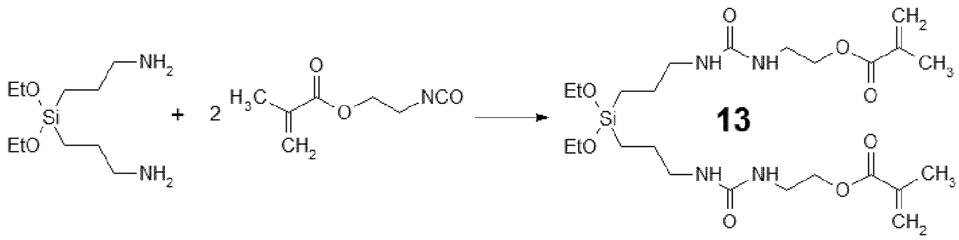

13

Durch die Umsetzung von 2-[Methyl[3-(trimethoxysilyl)propyl]amino]ethanol mit 2-Isocyanatoethylmethacrylat erhält man das Silan (14).

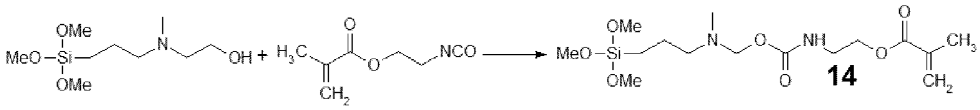

14

Durch die Umsetzung von 2,2'-[[3-(Triethoxysilyl)propyl]imino]bis[ethanol] mit 2 Äquivalenten 2-Isocyanatoethylmethacrylat erhält man das Silan (15).

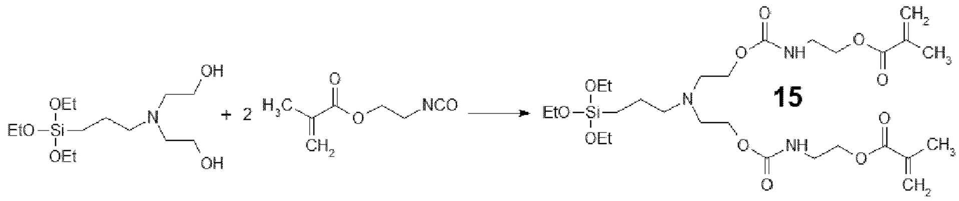

15

<u>Synthese von $(R^1O)_aR^2_bSi[-A(-X-C(=O)-N(-B[-Z-C(=O)C(=CH_2)R^3]_f)-C(=O)-NH-B[-Z-C(=O)C(=CH_2)R^3]_f)_d]_c$</u>

Die Synthese der Silane $(R^1O)_aR^2_bSi[-A(-X-C(=O)-N(-B[-Z-C(=O)C(=CH_2)R^3]_f)-C(=O)-NH-B[-Z-C(=O)C(=CH_2)R^3]_f)_d]_c$ erfolgt analog zu den oben beschriebenen Synthesen der Silane $(R^1O)_aR^2_bSi[-A(-X-C(=O)-NH-B[-Z-C(=O)C(=CH_2)R^3]_f)_d]_c$. Dabei wird ein Äquivalent an XH-Gruppe hier mit zwei Äquivalenten Isocyanat-Verbindung umgesetzt, wodurch die entsprechenden Allophanate, Thioallophanate und Biurete erhalten werden. Die Reaktionen verlaufen ebenfalls unter Zinn- oder Bismuth-Katalyse. Bevorzugte Katalysatoren sind ebenfalls Dibutylzinndilaurat und Bismuthneodecanoat. Unter leichter Erwärmung wird jeweils eine XH-Gruppe mit zwei Äquivalenten Isocyanat-Verbindung umgesetzt. Die Reaktion verläuft in der Regel vollständig und kann leicht im IR-Spektrum verfolgt werden, bis die Isocyanat-Bande (2270 $cm^{-1}$) vollständig verschwunden ist.

Es ist aber genauso möglich; die XH-Gruppen mit den Isocyanat-Gruppen in einer Stöchiometrie zwischen 1:1 und 1:2 umzusetzen. Die zweite Umsetzungsstufe kann dann nicht mehr vollständig ablaufen, so dass ein Gemisch aus Urethan und Allophanat oder aus Thiourethan und Thioallophanat oder aus Harnstoff und Biuret vorliegt. Erfindungsgemäße polymerisierbare, dentale Zusammensetzungen können somit auch entsprechende Mischformen der Spezies (a1) enthalten.

$$(R^1O)_aR^2_bSi[-A(-XH)_d]_c + (2 \times c \times d)\,[R^3-C(=CH_2)C(=O)-Z-]_fB-NCO \rightarrow (R^1O)_aR^2_6Si[-A(-X-C(=O)-N(-B[-Z-C(=O)C(=CH_2)R^3]_f)-C(=O)-NH-B[-Z-C(=O)C(=CH_2)R^3]_f)_d]_c$$

Als Ausgangsverbindungen können ebenfalls die in den Tabellen 1 und 2 aufgeführten kommerziell erhältlichen Verbindungen eingesetzt werden.

Im Folgenden werden beispielhaft einige Synthesen der Silane $(R^1O)_aR^2_bSi[-A(-X-C(=O)-N(-B[-Z-C(=O)C(=CH_2)R^3]_f)-C(=O)-NH-B[-Z-C(=O)C(=CH_2)R^3]_f)_d]_c$ dargestellt.

Durch die Umsetzung von 3-(Trimethoxysilyl)-1-propanol mit zwei Äquivalenten 2-Isocyanatoethylmethacrylat erhält man das Allophanat-Silan (16).

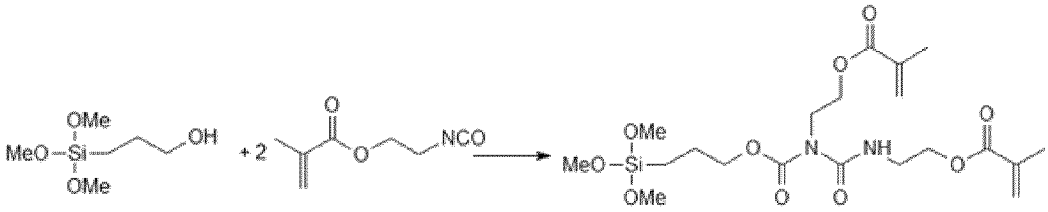

16

Durch die Umsetzung von 3-(Methyldimethoxysilyl)-1-propanol mit zwei Äquivalenten 2-Isocyanatoethylmethacrylat erhält man das Allophanat-Silan (17).

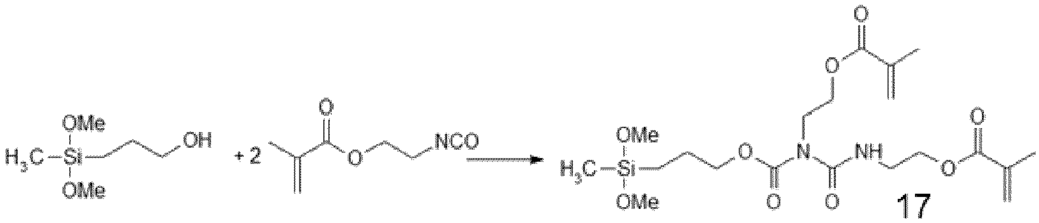

17

Durch die Umsetzung von 1-(Triethoxysilyl)methanol mit zwei Äquivalenten 2-Isocyanatoethylmethacrylat erhält man das Allophanat-Silan (18).

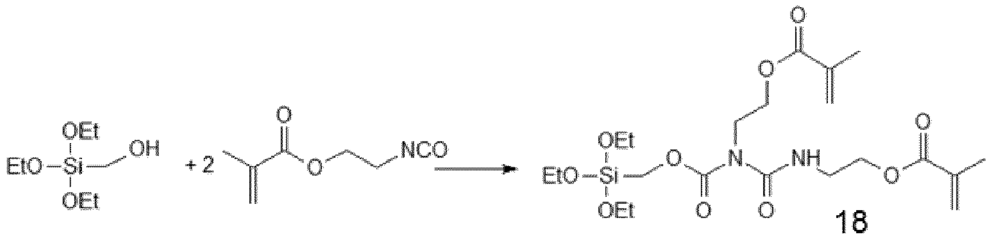

18

Durch die Umsetzung von 3-(Trimethoxysilyl)-1-propanthiol mit zwei Äquivalenten 2-Isocyanatoethylmethacrylat erhält man das Thioallophanat-Silan (19).

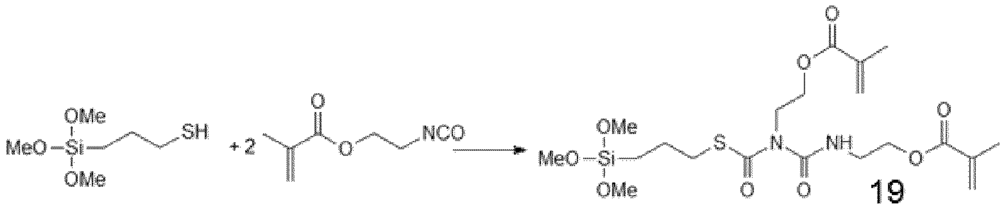

19

Durch die Umsetzung von 3-(Trimethoxysilyl)-1-propanamin mit zwei Äquivalenten 2-Isocyanatoethylmethacrylat erhält man das Biuret-Silan (20).

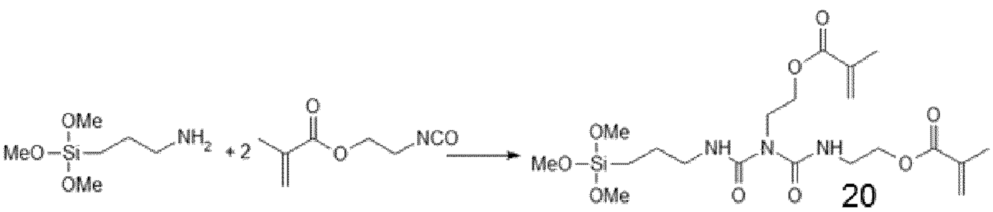

20

Synthese von $(R^1O)_aR^2_bSi[-A(-NH-C(=O)-X-B[-Z-C(=O)C(=CH_2)R^3]_f)_d]_c$

Die Synthese der Silane $(R^1O)_aR^2_bSi[-A(-NH-C(=O)-X-B[-Z-C(=O)C(=CH_2)R^3]_f)_d]_c$ erfolgt analog Zinn- oder Bismuth-katalysiert, wobei sich hier umgekehrt die Isocyanatgruppe am Silan und die XH-Gruppe an der (Meth)acryl-Verbindung befindet. Bevorzugte Katalysatoren sind auch hier Dibutylzinndilaurat und Bismuthneodecanoat. Unter leichter Erwärmung wird im äquimolaren Verhältnis jeweils eine XH-Gruppe mit einer Isocyanatgruppe umgesetzt. Die Reaktion verläuft in der Regel vollständig und kann leicht im IR-Spektrum verfolgt werden, bis die Isocyanat-Bande (2270 $cm^{-1}$) vollständig verschwunden ist.

$$(R^1O)_aR^2_bSi[-A(-NCO)_d]_c + (cxd)\ [R^3\text{-}C(=CH_2)C(=O)\text{-}Z\text{-}]_fB\text{-}XH \rightarrow (R^1O)_aR^2_bSi[-A(-NH\text{-}C(=O)\text{-}X\text{-}B[\text{-}Z\text{-}C(=O)C(=CH_2)R^3]_f)_d]_c$$

In den folgenden beiden Tabellen 3 und 4 sind eine Reihe kommerziell erhältlicher Ausgangsverbindungen aufgeführt.

Tabelle 3:

| | **$(R^1O)_aR^2_bSi[-A(-NCO)_d]_c$** | | | | | | |
|---|---|---|---|---|---|---|---|
| **CAS-Nr.** | **$R^1$** | **$R^2$** | **a** | **b** | **c** | **d** | **-A-** |
| 15396-00-6 | Me | - | 3 | 0 | 1 | 1 | $-(CH_2)_3-$ |
| 24801-88-5 | Et | - | 3 | 0 | 1 | 1 | $-(CH_2)_3-$ |
| 26115-72-0 | Me | Me | 2 | 1 | 1 | 1 | $-(CH_2)_3-$ |
| 33491-28-0 | Et | Me | 2 | 1 | 1 | 1 | $-(CH_2)_3-$ |
| 78450-75-6 | Me | - | 3 | 0 | 1 | 1 | $-CH_2-$ |
| 132112-76-6 | Et | - | 3 | 0 | 1 | 1 | $-CH_2-$ |
| 406679-89-8 | Me | Me | 2 | 1 | 1 | 1 | $-CH_2-$ |
| 20160-30-9 | Et | Me | 2 | 1 | 1 | 1 | $-CH_2-$ |
| 862546-89-2 | Et | - | 3 | 0 | 1 | 1 | $-(CH_2)_{10}-$ |

Tabelle 4:

| | | **$[R^3\text{-}C(=CH_2)C(=O)\text{-}Z\text{-}]_fB\text{-}XH$** | | | |
|---|---|---|---|---|---|
| **CAS-Nr.** | **$R^3$** | **Z** | **f** | **XH** | **-B-** |
| 868-77-9 | Me | O | 1 | OH | $-(CH_2)_2-$ |
| 923-26-2 | Me | O | 1 | OH | $-CH_2CH(CH_3)-$ |
| 2761-09-3 | Me | O | 1 | OH | $-(CH_2)_3-$ |
| 997-46-6 | Me | O | 1 | OH | $-(CH_2)_4-$ |
| 13092-57-4 | Me | O | 1 | OH | $-(CH_2)_6-$ |
| 203245-10-7 | Me | O | 1 | OH | 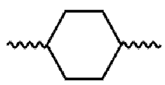 |
| 4855-07-6 | Me | O | 1 | OH | $-(CH_2)_8-$ |
| 56927-66-3 | Me | O | 1 | OH | $-(CH_2)_{10}-$ |
| 115372-36-6 | Me | O | 1 | OH | 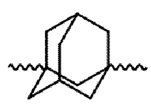 |
| 86282-42-0 | Me | O | 1 | OH | $-(CH_2)_{12}-$ |
| 2351-43-1 | Me | O | 1 | OH | $-(CH_2)_2O(CH_2)_2-$ |
| 2351-42-0 | Me | O | 1 | OH | $-(CH_2)_2O(CH_2)_2O(CH_2)_2-$ |
| 16926-87-7 | Me | O | 1 | OH | $-CH_2CH(CH_2OPh)-$ |
| 818-61-1 | H | O | 1 | OH | $-(CH_2)_2-$ |
| 999-61-1 | H | O | 1 | OH | $-CH_2CH(CH_3)-$ |

(fortgesetzt)

| | | | | $[R^3\text{-}C(=CH_2)C(=O)\text{-}Z\text{-}]_fB\text{-}XH$ | |
|---|---|---|---|---|---|
| **CAS-Nr.** | **$R^3$** | **Z** | **f** | **XH** | **-B-** |
| 2761-08-2 | H | O | 1 | OH | $-(CH_2)_3-$ |
| 2478-10-6 | H | O | 1 | OH | $-(CH_2)_4-$ |
| 57198-94-4 | H | O | 1 | OH | $-(CH_2)_5-$ |
| 10095-14-4 | H | O | 1 | OH | $-(CH_2)_6-$ |
| 118915-15-4 | H | O | 1 | OH | $-(CH_2)_3-$ |
| 23117-38-6 | H | O | 1 | OH | $-(CH_2)_{10}-$ |
| 216581-76-9 | H | O | 1 | OH | 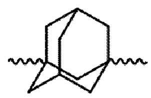 |
| 13533-05-6 | H | O | 1 | OH | $-(CH_2)_2O(CH_2)_2-$ |
| 16695-45-7 | H | O | 1 | OH | $-(CH_2)_2O(CH_2)_2O(CH_2)_2-$ |
| 16969-10-1 | H | O | 1 | OH | $-CH_2CH(CH_2OPh)-$ |
| 1830-78-0 | Me | O | 2 | OH | 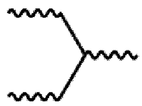 |
| 101525-90-0 | Me | O | 2 | OH | 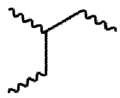 |
| 1709-71-3 | H / Me | O | 2 | OH | 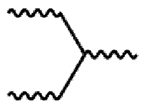 |
| 433937-38-3 | H / Me | O | 2 | OH | 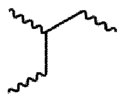 |
| 1709-72-4 | H | O | 2 | OH | 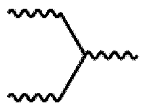 |
| 53151-63-6 | Me | O | 2 | OH | 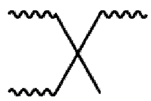 |
| 19727-16-3 | Me | O | 2 | OH | 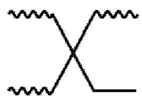 |
| 37275-47-1 | H | O | 2 | OH | 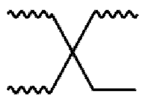 |
| 3524-66-1 | Me | O | 3 | OH | 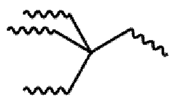 |

(fortgesetzt)

| | | | $[R^3\text{-}C(=CH_2)C(=O)\text{-}Z\text{-}]_fB\text{-}XH$ | | |
|---|---|---|---|---|---|
| **CAS-Nr.** | **$R^3$** | **Z** | **f** | **XH** | **-B-** |
| 3524-68-3 | H | O | 3 | OH | 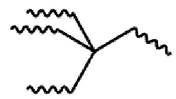 |
| 60506-81-2 | H | O | 5 | OH | 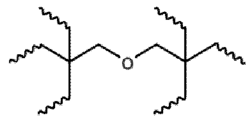 |
| 7659-36-1 | Me | O | 1 | $NH_2$ | $-(CH_2)_2-$ |
| 7659-38-3 | H | O | 1 | $NH_2$ | $-(CH_2)_2-$ |
| 5238-56-2 | Me | NH | 1 | OH | $-(CH_2)_2-$ |
| 21442-01-3 | Me | NH | 1 | OH | $-CH_2CH(CH_3)-$ |
| 89911-51-3 | Me | NH | 1 | OH | $-(CH_2)_2O(CH_2)_2-$ |
| 89911-50-2 | H | NH | 1 | OH | $-(CH_2)_2O(CH_2)_2-$ |
| 96189-83-2 | Me | NH | 1 | OH | $-(CH_2)_2O(CH_2)_2O(CH_2)_2-$ |
| 63298-57-7 | Me | NH | 1 | $NH_2$ | $-(CH_2)_2-$ |
| 23918-29-8 | H | NH | 1 | $NH_2$ | $-(CH_2)_2-$ |

Im Folgenden werden beispielhaft einige Synthesen der Silane $(R^1O)_aR^2_bSi[-A(-NH-C(=O)-X-B[-Z-C(=O)C(=CH_2)R^3]_f)_d]_c$ dargestellt.

Durch die Umsetzung von 1-(Trimethoxysilyl)methylisocyanat mit 2-Hydroxyethylmethacrylat (HEMA) erhält man das Silan (21).

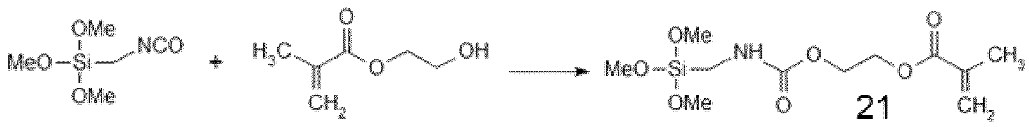

21

Durch die Umsetzung von 3-(Trimethoxysilyl)propylisocyanat mit 2-Hydroxyethylmethacrylat (HEMA) erhält man das Silan (22).

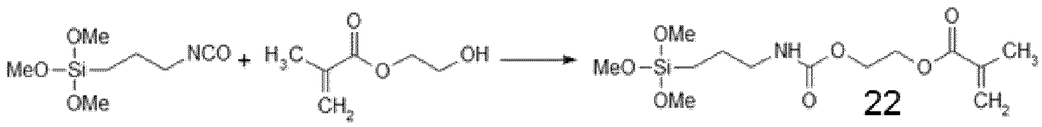

22

Durch die Umsetzung von 10-(Triethoxysilyl)decylisocyanat mit 2-Hydroxyethylmethacrylat (HEMA) erhält man das Silan (23).

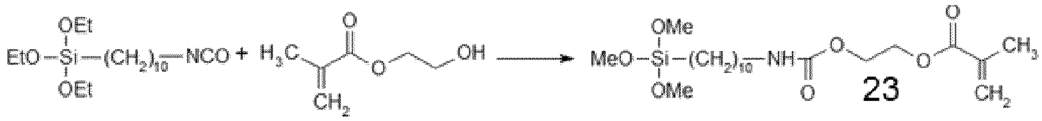

23

Durch die Umsetzung von 3-(Methyldimethoxysilyl)propylisocyanat mit 2-Hydroxyethylmethacrylat (HEMA) erhält man das Silan (24).

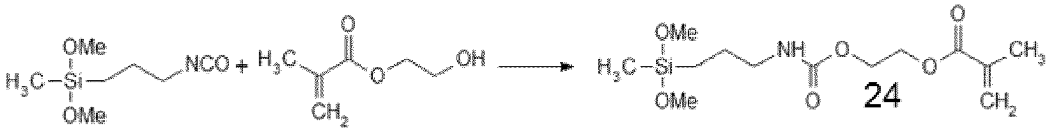

24

Durch die Umsetzung von 1-(Trimethoxysilyl)methylisocyanat mit 4-Hydroxybutylmethacrylat erhält man das Silan (25).

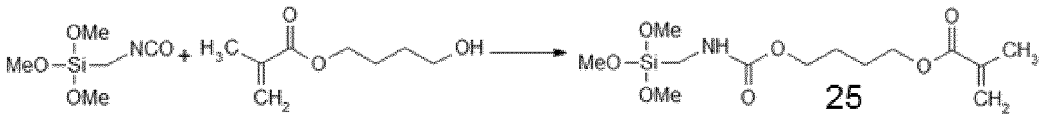

25

Durch die Umsetzung von 1-(Trimethoxysilyl)methylisocyanat mit 6-Hydroxyhexylmethacrylat erhält man das Silan (26).

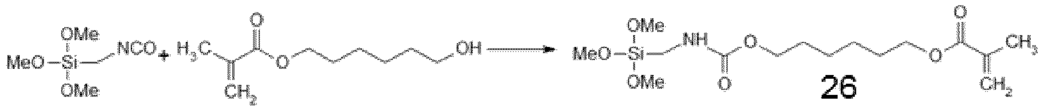

26

Durch die Umsetzung von 1-(Trimethoxysilyl)methylisocyanat mit 4-Hydroxycyclohexylmethacrylat erhält man das Silan (27).

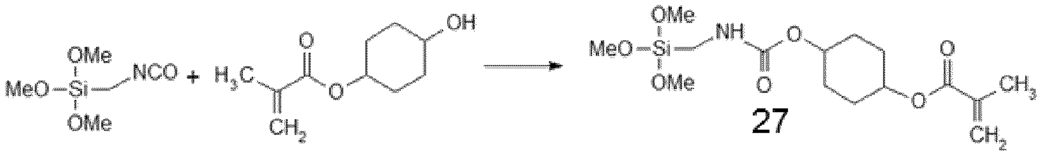

27

Durch die Umsetzung von 1-(Trimethoxysilyl)methylisocyanat mit 3-Hydroxy-adamantan-1-yl-methacrylat erhält man das Silan (28).

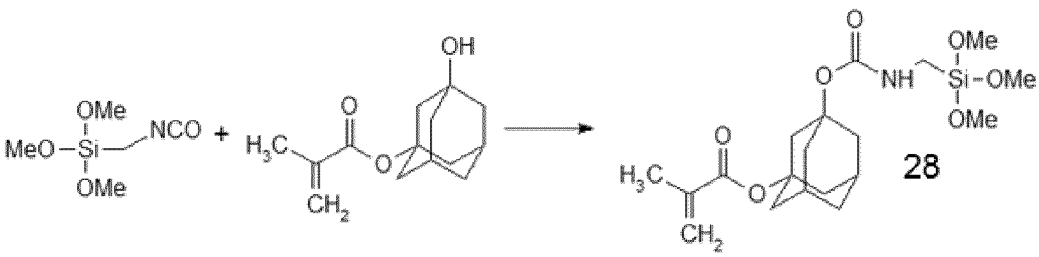

28

Durch die Umsetzung von 1-(Trimethoxysilyl)methylisocyanat mit 2-(2-Hydroxy-ethoxy)ethylmethacrylat erhält man das Silan (29).

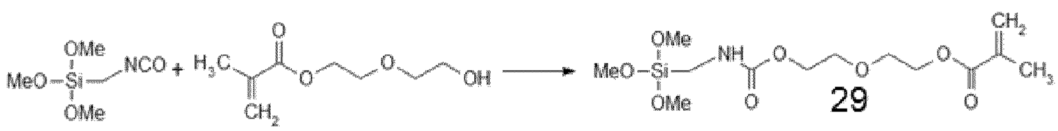

29

Durch die Umsetzung von 1-(Trimethoxysilyl)methylisocyanat mit 2-[2-(2-Hydroxyethoxy)ethoxy]ethylmethacrylat erhält man das Silan (30).

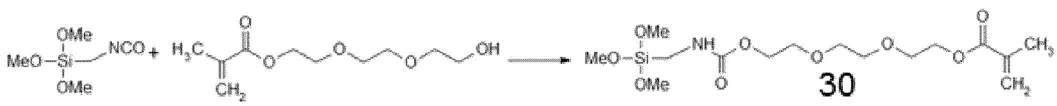

30

Durch die Umsetzung von 1-(Trimethoxysilyl)methylisocyanat mit 2-Hydroxy-3-phenoxy-1-propylmethacrylat erhält man das Silan (31).

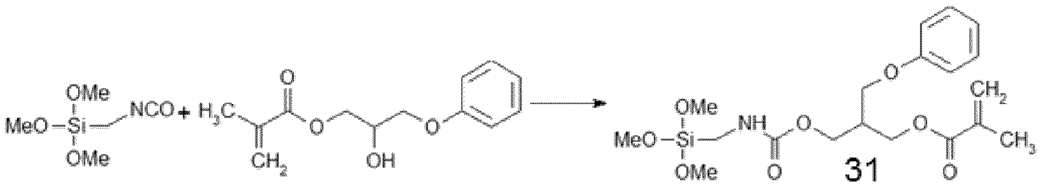

31

Durch die Umsetzung von 1-(Trimethoxysilyl)methylisocyanat mit Glycerin-1,3-dimethacrylat erhält man das Silan (32).

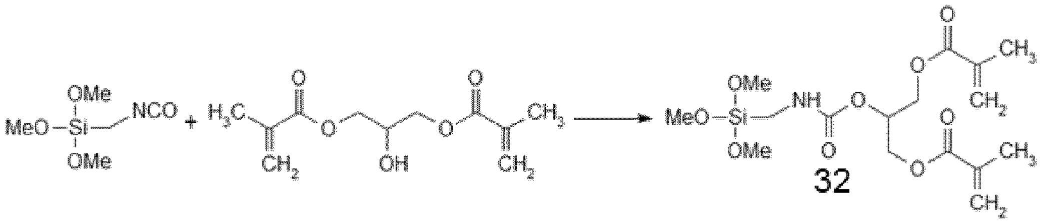

32

Durch die Umsetzung von 1-(Trimethoxysilyl)methylisocyanat mit Pentaerythritoltrimethacrylat erhält man das Silan (33).

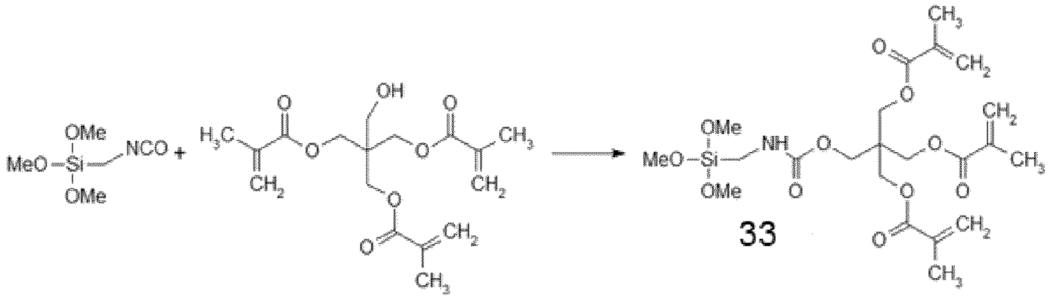

33

Durch die Umsetzung von 1-(Trimethoxysilyl)methylisocyanat mit 2-Aminoethylmethacrylat erhält man das Silan (34).

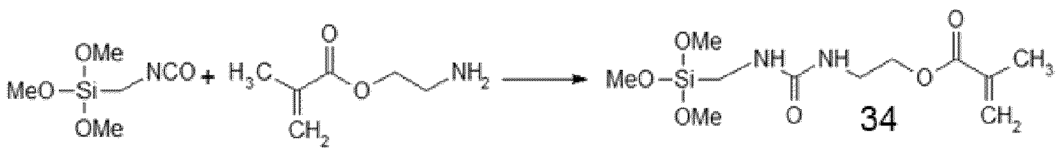

34

Durch die Umsetzung von 1-(Trimethoxysilyl)methylisocyanat mit (2-Hydroxyethyl)methacrylamid erhält man das Silan (35).

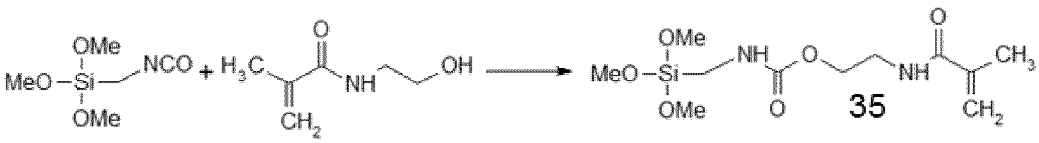

35

Synthese von $(R^1O)_aR^2_bSi[-A(-NH-C(=O)-B[-Z-C(=O)C(=CH_2)R^3]_f)_d]_c$

Die Synthese der Silane $(R^1O)_aR^2_bSi[-A(-NH-C(=O)-B[-Z-C(=O)C(=CH_2)R^3]_f)_d]_c$ erfolgt Zinn- oder Bismuth-katalysiert unter Kohlendioxidabspaltung aus einem Silan mit Isocyanatgruppe und einer carboxylsubstituierten (Meth) acryl-Verbindung. Bevorzugte Katalysatoren sind Dibutylzinndilaurat und Bismuthneodecanoat. Unter leichter Erwärmung wird im äquimolaren Verhältnis jeweils eine Carboxylgruppe mit einer Isocyanatgruppe umgesetzt. Die Reaktion verläuft in der Regel vollständig und kann leicht im IR-Spektrum verfolgt werden, bis die Isocyanat-Bande (2270 $cm^{-1}$) vollständig verschwunden ist.

$$(R^1O)_aR^2_bSi[-A(-NCO)_d]_c + (cxd)[R^3\text{-}C(=CH_2)C(=O)\text{-}Z\text{-}]_fB\text{-}COOH \rightarrow (R^1O)_aR^2_bSi[-A(-NH\text{-}C(=O)\text{-}B[-Z\text{-}C(=O)C(=CH_2)R^3]_f)_d]_c$$

In der folgenden Tabelle 5 sind eine Reihe kommerziell erhältlicher Carboxyl-(Meth)acryl-Verbindungen aufgeführt. Als isocyanatsubstituierte Silane sind die weiter oben in Tabelle 3 aufgeführten, kommerziell erhältlichen Verbindungen geeignet.

Tabelle 5:

| | $[R^3\text{-}C(=CH_2)C(=O)\text{-}Z\text{-}]_fB\text{-}COOH$ | | | |
|---|---|---|---|---|
| **CAS-Nr.** | **$R^3$** | **Z** | **f** | **-B-** |
| 141681-03-0 | H | O | 1 | $-(CH_2)_3-$ |
| 59178-90-4 | Me | NH | 1 | $-(CH_2)_2-$ |
| 16753-07-4 | H | NH | 1 | $-(CH_2)_2-$ |
| 59178-91-5 | Me | NH | 1 | $-(CH_2)_3-$ |
| 59178-91-5 | H | NH | 1 | $-(CH_2)_3-$ |
| 20882-04-6 | Me | O | 1 | $-(CH_2)_2OC(=O)-(CH_2)_2-$ |
| 112241-32-4 | Me | O | 1 | $-(CH_2)_3OC(=O)-(CH_2)_2-$ |
| 51252-88-1 | Me | O | 1 | 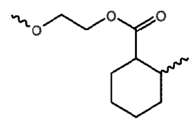 |
| 27697-00-3 | Me | O | 1 | 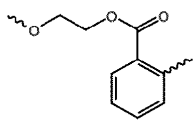 |
| 65859-45-2 | Me | O | 1 | 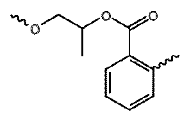 |

Im Folgenden werden beispielhaft einige Synthesen der Silane $(R^1O)_aR^2_bSi[-A(-NH\text{-}C(=O)\text{-}B[-Z\text{-}C(=O)C(=CH_2)R^3]_f)_d]_c$ dargestellt.

Durch die Umsetzung von 3-(Trimethoxysilyl)propylisocyanat mit Mono[2-(methacryloyloxy)ethyl]succinat erhält man das Silan (36).

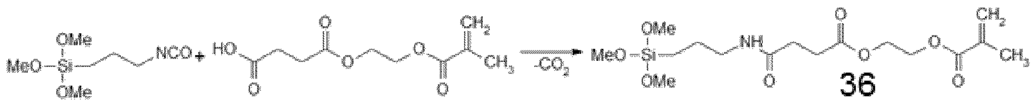

36

Durch die Umsetzung von 1-(Trimethoxysilyl)methylisocyanat mit Mono[2-(methacryloyloxy)ethyl]succinat erhält man das Silan (37).

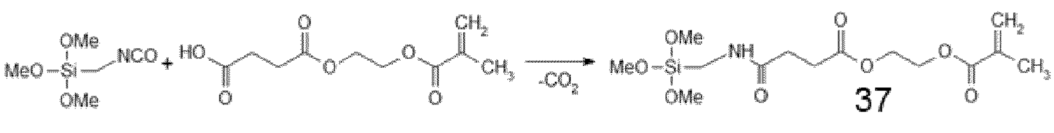

37

Durch die Umsetzung von 1-(Trimethoxysilyl)methylisocyanat mit Butandisäure-1-[3-[(2-methyl-1-oxo-2-propen-1-yl)oxy]propyl]ester erhält man das Silan (38).

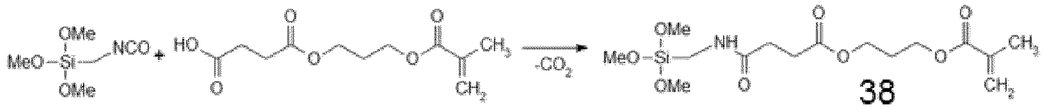

38

Durch die Umsetzung von 1-(Trimethoxysilyl)methylisocyanat mit 1,2-Cyclohexandicarbonsäure-1-[2-[(2-methyl-1-oxo-2-propen-1-yl)oxy]ethyl]ester erhält man das Silan (39).

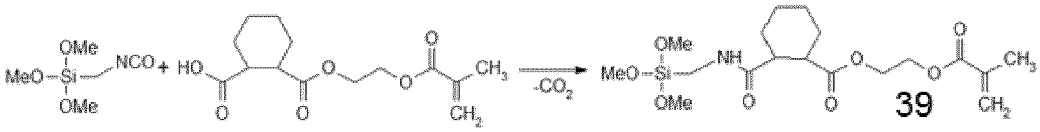

39

Durch die Umsetzung von 1-(Trimethoxysilyl)methylisocyanat mit Mono[2-(methacryloyloxy)ethyl]phthalat erhält man das Silan (40).

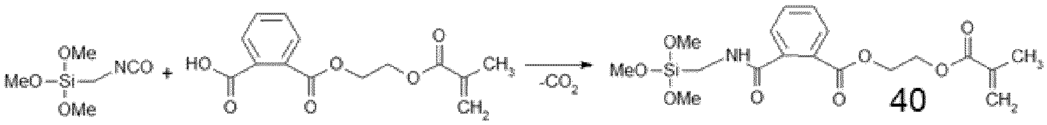

40

Synthese von Silanen mit Urethan-, Thiourethan-, Harnstoff- oder Amidgruppen ausgehend von Hydrosilanen

Silanen mit Urethan-, Thiourethan-, Harnstoff- oder Amidgruppen lassen sich in einer mehrstufigen Synthese durch Platin-katalysierte Umsetzung von Vinyl-Verbindungen mit Hydrosilanen herstellen. Entsprechende Synthesen sind in US 2015/0299469 A1 offenbart.

Synthese von $(R^1O)_aR^2_bSi[-(CH_2)_2-CH(R^4)-NH-C(=O)-B[-Z-C(=O)C(=CH_2)R^3]_f]_c$

In einer zweistufigen Synthese wird zunächst eine Carboxyl-(meth)acryl-Verbindung mit einem Allylamin umgesetzt.

$$[R^3\text{-}C(=CH_2)C(=O)\text{-}Z\text{-}]_fB\text{-}COOH + H_2C{=}CHCH(R^4)\text{-}NH_2 \rightarrow [R^3\text{-}C(=CH_2)C(=O)\text{-}Z\text{-}]_fB\text{-}C(=O)\text{-}NH\text{-}CH(R^4)\text{-}CH{=}CH_2$$

Kommerziell erhältliche Carboxyl-(meth)acryl-Verbindungen sind weiter oben in Tabelle 5 aufgeführt. Kommerziell erhältliche Allylamine sind in der folgenden Tabelle 6 aufgeführt.

Tabelle 6:

| | **$H_2C=CHCH(R^4)-NH_2$** |
|---|---|
| **CAS-Nr.** | **$R^4$** |
| 107-11-9 | H |
| 34375-90-1 | Me |
| 70267-50-4 | Et |
| 4181-11-7 | *n*-Pr |
| 127209-34-1 | $^i$Pr |
| 5963-71-3 | *n*-Bu |
| 36024-39-2 | $^t$Bu |
| 4393-21-9 | Ph |

(fortgesetzt)

| | $H_2C=CHCH(R^4)-NH_2$ |
|---|---|
| CAS-Nr. | $R^4$ |
| 1186139-06-9 | 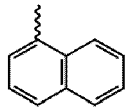 |

In der zweiten Synthesestufe wird die erhaltene Vinyl-Verbindung Platin-katalysiert mit einem Hydrosilan $(R^1O)_aR^2_bSiH_c$ umgesetzt. Kommerziell erhältliche Hydrosilane sind in Tabelle 7 aufgeführt. Weitere Trialkoxysilane $HSi(OR^1)_3$ lassen sich entsprechend EP 0 285 133 A2 durch Kupfer(II)hydroxid-katalysierte Umsetzung von Silicium mit Alkoholen herstellen.

$$c\ [R^3\text{-}C(=CH_2)C(=O)\text{-}Z\text{-}]_fB\text{-}C(=O)\text{-}NH\text{-}CH(R^4)\text{-}CH=CH_2 + (R^1O)_aR^2_bSiH_c \rightarrow (R^1O)_aR^2_bSi[\text{-}(CH_2)_2\text{-}CH(R^4)\text{-}NH\text{-}C(=O)\text{-}B[\text{-}Z\text{-}C(=O)C(=CH_2)R^3]_f]_c$$

Tabelle 7:

| | $(R^1O)_aR^2_bSiH_c$ | | | | |
|---|---|---|---|---|---|
| CAS-Nr. | $R^1$ | $R^2$ | a | b | c |
| 2487-90-3 | Me | - | 3 | 0 | 1 |
| 998-30-1 | Et | - | 3 | 0 | 1 |
| 6485-85-4 | *n*-Pr | - | 3 | 0 | 1 |
| 6675-79-2 | $^i$Pr | - | 3 | 0 | 1 |
| 6485-86-5 | *n*-Bu | - | 3 | 0 | 1 |
| 16881-77-9 | Me | Me | 2 | 1 | 1 |
| 2031-62-1 | Et | Me | 2 | 1 | 1 |
| 54010-11-6 | $^i$Pr | Me | 2 | 1 | 1 |
| 2487-91-4 | *n*-Bu | Me | 2 | 1 | 1 |
| 19753-84-5 | Me | Et | 2 | 1 | 1 |
| 13175-88-7 | Et | Et | 2 | 1 | 1 |
| 18132-62-2 | *n*-Bu | Et | 2 | 1 | 1 |
| 163215-58-5 | Me | $^i$Pr | 2 | 1 | 1 |
| 5314-52-3 | Me | - | 2 | 0 | 2 |
| 18165-68-9 | Et | - | 2 | 0 | 2 |

Im Folgenden werden beispielhaft einige Synthesen der Silane $(R^1O)_aR^2_bSi[\text{-}(CH_2)_2\text{-}CH(R^4)\text{-}NH\text{-}C(=O)\text{-}B[\text{-}Z\text{-}C(=O)C(=CH_2)R^3]_f]_c$ dargestellt.

Durch Umsetzung von Mono[2-(methacryloyloxy)ethyl]succinat mit Allylamin erhält man zunächst die Vinyl-Verbindung (41). Durch weitere Platin-katalysierte Umsetzung mit Triethoxysilan erhält man schließlich das Silan (42).

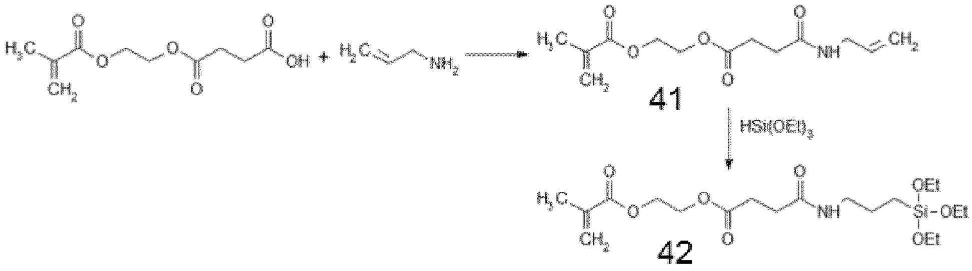


Durch Umsetzung von 3-Methacryloylaminopropansäure mit Allylamin erhält man zunächst die Vinyl-Verbindung (43). Durch weitere Platin-katalysierte Umsetzung mit Triethoxysilan erhält man schließlich das Silan (44).

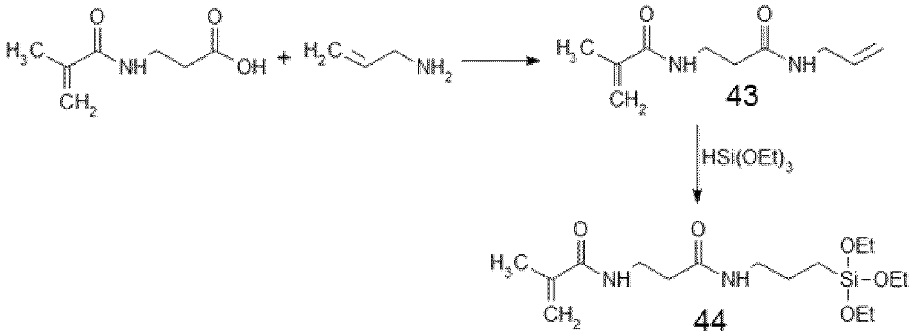


Durch Umsetzung von 3-Methacryloylaminopropansäure mit Allylamin erhält man zunächst die Vinyl-Verbindung (45). Durch weitere Platin-katalysierte Umsetzung mit Methyldimethoxysilan erhält man schließlich das Silan (46).

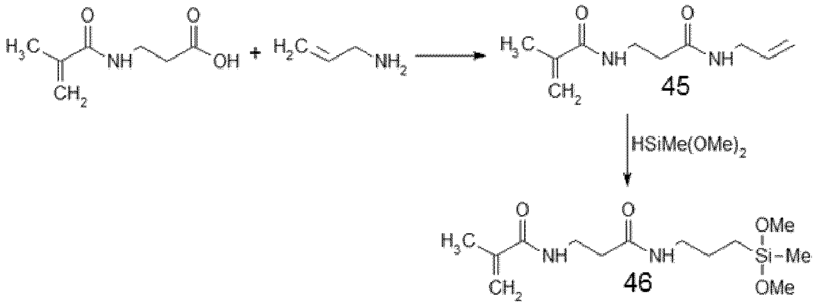


<u>Synthese von $(R^1O)_aR^2_bSi[-(CH_2)_2-C(=O)-NH-B[-Z-C(=O)C(=CH_2)R^3]_f]_c$</u>

In einer zweistufigen Synthese wird zunächst eine Amino-(meth)acryl-Verbindung mit Acryloylchlorid (CAS-Nr. 814-68-6) umgesetzt. Bevorzugt ist $R^3$ hier eine Methylgruppe, da in diesem Fall die Folgereaktion mit $(R^1O)_aR^2_bSiH_c$ selektiv an der Acrylamid-Funktion erfolgt. Kommerziell erhältliche Amino-(meth)acrylVerbindungen sind in Tabelle 4 (für XH = $NH_2$) aufgeführt.

$$[R^3\text{-}C(=CH_2)C(=O)\text{-}Z\text{-}]_fB\text{-}NH_2 + H_2C{=}CHCOCl \rightarrow [R^3\text{-}C(=CH_2)C(=O)\text{-}Z\text{-}]_fB\text{-}NH\text{-}C(=O)\text{-}CH{=}CH_2$$

In der zweiten Synthesestufe wird die erhaltene Vinyl-Verbindung Platin-katalysiert mit einem Hydrosilan $(R^1O)_aR^2_bSiH_c$ umgesetzt. Kommerziell erhältliche Hydrosilane sind in Tabelle 7 aufgeführt.

$$c\,[R^3\text{-}C(=CH_2)C(=O)\text{-}Z\text{-}]_fB\text{-}NH\text{-}C(=O)\text{-}CH{=}CH_2 + (R^1O)_aR^2_bSiH_c \rightarrow (R^1O)_aR^2_bSi[\text{-}(CH_2)_2\text{-}C(=O)\text{-}NH\text{-}B[\text{-}Z\text{-}C(=O)C(=CH_2)R^3]_f]_c$$

Durch Umsetzung von 2-Aminoethylmethacrylat mit Acryloylchlorid erhält man die Vinyl-Verbindung (47). Durch weitere Platin-katalysierte Umsetzung mit Triethoxysilan erhält man schließlich das Silan (48).

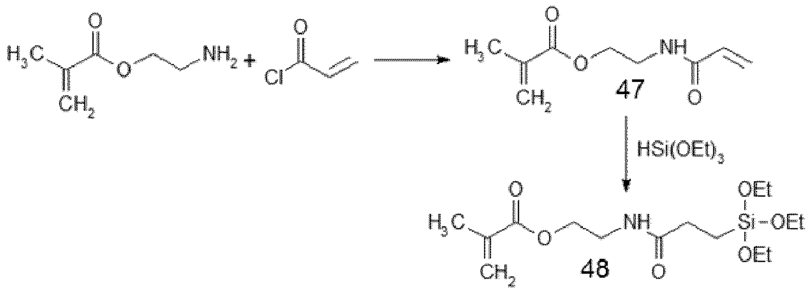

47
48

Synthese von $(R^1O)_aR^2_bSi[-(CH_2)_2-CH(R^4)-X-C(=O)-NH-B[-Z-C(=O)C(=CH_2)R^3]_f]_c$

In einer zweistufigen Synthese wird zunächst die isocyanatsubstituierte (Meth)acryl-Verbindung Zinn- oder Bismuth-katalysiert mit einer XH-Verbindung umgesetzt. Bevorzugte Katalysatoren sind auch hier Dibutylzinndilaurat und Bismuthneodecanoat. Unter leichter Erwärmung wird im äquimolaren Verhältnis jeweils eine XH-Gruppe mit einer Isocyanatgruppe umgesetzt. Die Reaktion verläuft in der Regel vollständig und kann leicht im IR-Spektrum verfolgt werden, bis die Isocyanat-Bande (2270 $cm^{-1}$) vollständig verschwunden ist. Kommerziell erhältliche isocyanatsubstituierte (Meth)acryl-Verbindungen sind weiter oben in Tabelle 2 aufgeführt. Kommerziell erhältliche XH-Verbindungen sind in der folgenden Tabelle 8 aufgeführt.

$$[R^3-C(=CH_2)C(=O)-Z-]_fB-NCO + H_2C=CHCH(R^4)-XH \rightarrow [R^3-C(=CH_2)C(=O)-Z-]_fB-NH-C(=O)-X-CH(R^4)-CH=CH_2$$

Tabelle 8:

| | **$H_2C=CHCH(R^4)-XH$** | |
|---|---|---|
| **CAS-Nr.** | **XH** | **$R^4$** |
| 107-18-6 | OH | H |
| 598-32-3 | OH | Me |
| 616-25-1 | OH | Et |
| 4798-44-1 | OH | *n*-Pr |
| 4798-45-2 | OH | $^i$Pr |
| 4938-52-7 | OH | *n*-Bu |
| 24580-44-7 | OH | $^t$Bu |
| 3391-86-4 | OH | *n*-Pentyl |
| 21964-44-3 | OH | *n*-Hexyl |
| 51100-54-0 | OH | *n*-Heptyl |
| 35329-42-1 | OH | *n*-Octyl |
| 4393-06-0 | OH | Ph |
| 61619-02-1 | OH | 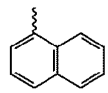 |
| 870-23-5 | SH | H |
| 5937-82-6 | SH | Me |
| 61758-08-5 | SH | *n*-Pentyl |
| 39707-48-7 | SH | Ph |
| 107-11-9 | $NH_2$ | H |
| 34375-90-1 | $NH_2$ | Me |

(fortgesetzt)

| | $H_2C{=}CHCH(R^4)\text{-}XH$ | |
|---|---|---|
| **CAS-Nr.** | **XH** | **$R^4$** |
| 70267-50-4 | $NH_2$ | Et |
| 4181-11-7 | $NH_2$ | *n*-Pr |
| 127209-34-1 | $NH_2$ | $^i$Pr |
| 5963-71-3 | $NH_2$ | *n*-Bu |
| 36024-39-2 | $NH_2$ | $^t$Bu |
| 4393-21-9 | $NH_2$ | Ph |
| 1186139-06-9 | $NH_2$ | 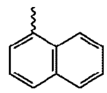 |

In der zweiten Synthesestufe wird die erhaltene Vinyl-Verbindung Platin-katalysiert mit einem Hydrosilan $(R^1O)_aR^2_bSiH_c$ umgesetzt. Kommerziell erhältliche Hydrosilane sind in Tabelle 7 aufgeführt.

$$c\ [R^3\text{-}C(=CH_2)C(=O)\text{-}Z\text{-}]_fB\text{-}NH\text{-}C(=O)\text{-}X\text{-}CH(R^4)\text{-}CH=CH_2 + (R^1O)_aR^2_bSiH_c \rightarrow (R^1O)_aR^2_bSi[\text{-}(CH_2)_2\text{-}CH(R^4)\text{-}X\text{-}C(=O)\text{-}NH\text{-}B[\text{-}Z\text{-}C(=O)C(=CH_2)R^3]_f]_c$$

Im Folgenden werden beispielhaft einige Synthesen der Silane $(R^1O)_aR^2_bSi[\text{-}(CH_2)_2\text{-}CH(R^4)\text{-}X\text{-}C(=O)\text{-}NH\text{-}B[\text{-}Z\text{-}C(=O)C(=CH_2)R^3]_f]_c$ dargestellt.

Durch Umsetzung von 2-Isocyanatoethylmethacrylat mit 2-Propen-1-ol erhält man die Vinyl-Verbindung (49). Durch weitere Platin-katalysierte Umsetzung mit Triethoxysilan erhält man schließlich das Silan (50).

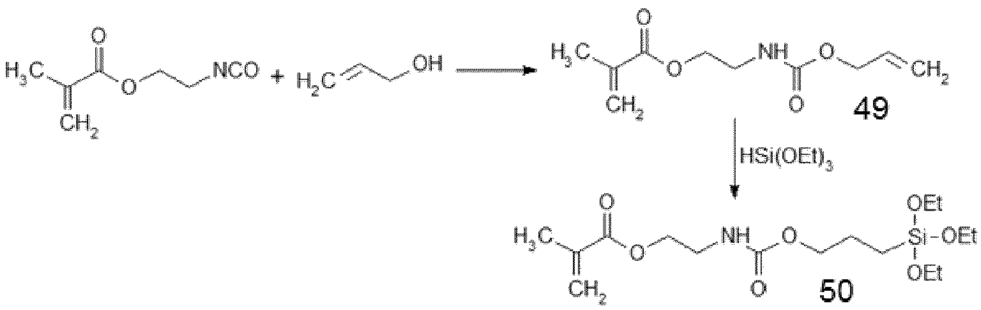

49
50

Durch Umsetzung von 2-Isocyanatoethylmethacrylat mit 2-Propen-1-thiol erhält man die Vinyl-Verbindung (51). Durch weitere Platin-katalysierte Umsetzung mit Triethoxysilan erhält man schließlich das Silan (52).

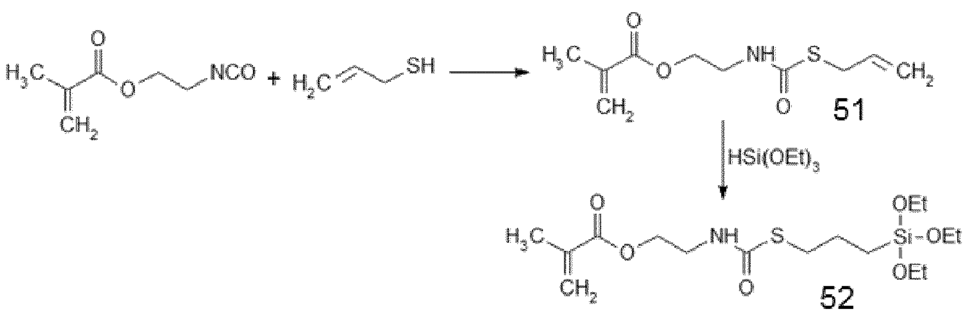

51
52

Durch Umsetzung von 2-Isocyanatoethylmethacrylat mit 3-Amino-1-propen erhält man die Vinyl-Verbindung (53). Durch weitere Platin-katalysierte Umsetzung mit Triethoxysilan erhält man schließlich das Silan (54).

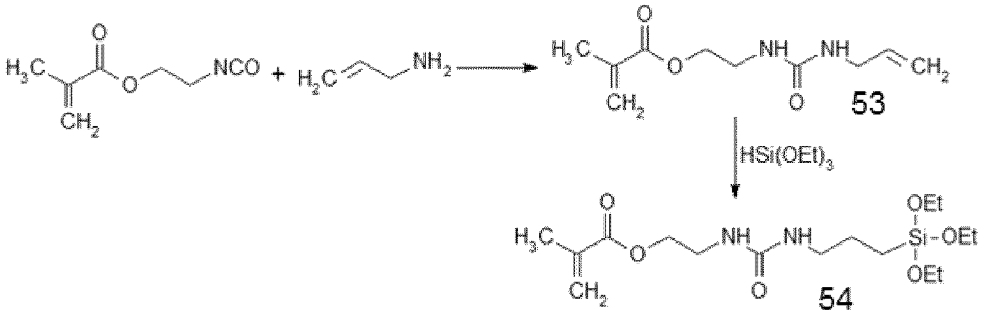


53

54

Synthese von $(R^1O)_aR^2_bSi[-(CH_2)_2-CH(R^4)-X-C(=O)-N(-B[-Z-C(=O)C(=CH_2)R^3]_f)-C(=O)-NH-B[-Z-C(=O)C(=CH_2)R^3]_f]_c$

Analog zur Synthese von $[R^3-C(=CH_2)C(=O)-Z-]_fB-NH-C(=O)-X-CH(R^4)-CH=CH_2$ lassen sich auch die Silane $(R^1O)_aR^2_bSi[-(CH_2)_2-CH(R^4)-X-C(=O)-N(-B[-Z-C(=O)C(=CH_2)R^3]_f)-C(=O)-NH-B[-Z-C(=O)C(=CH_2)R^3]_f]_c$ in einer zweistufigen Synthese aus XH-Verbindung und isocyanatsubstituierter (Meth)acryl-Verbindung herstellen. Dabei werden zunächst zwei Äquivalente der isocyanatsubstituierten (Meth)acryl-Verbindung Zinn- oder Bismuth-katalysiert mit einem Äquivalent einer XH-Verbindung umgesetzt, wobei die entsprechenden Allophanate, Thioallophanate bzw. Biurete erhalten werden. Bevorzugte Katalysatoren sind auch hier Dibutylzinndilaurat und Bismuthneodecanoat. Die Reaktion verläuft unter leichter Erwärmung, in der Regel vollständig und kann leicht im IR-Spektrum verfolgt werden, bis die Isocyanat-Bande (2270 $cm^{-1}$) vollständig verschwunden ist. Kommerziell erhältliche isocyanatsubstituierte (Meth)acryl-Verbindungen sind weiter oben in Tabelle 2 aufgeführt. Kommerziell erhältliche XH-Verbindungen sind in der folgenden Tabelle 8 aufgeführt. Auch hier ist es möglich die XH-Gruppen mit den Isocyanat-Gruppen in einer Stöchiometrie zwischen 1:1 und 1:2 umzusetzen, so dass die zweite Umsetzungsstufe dann nicht mehr vollständig abläuft und ein Gemisch aus Urethan und Allophanat oder aus Thiourethan und Thioallophanat oder aus Harnstoff und Biuret vorliegt. In der zweiten Synthesestufe werden die erhaltenen Vinyl-Verbindung Platin-katalysiert wiederum mit einem Hydrosilan $(R^1O)_aR^2_bSiH_c$ umgesetzt. Kommerziell erhältliche Hydrosilane sind in Tabelle 7 aufgeführt.

Durch Umsetzung von zwei Äquivalenten 2-Isocyanatoethylmethacrylat mit 2-Propen-1-ol erhält man die Vinyl-Verbindung (55). Durch weitere Platin-katalysierte Umsetzung mit Triethoxysilan erhält man schließlich das Allophanat-Silan (56).

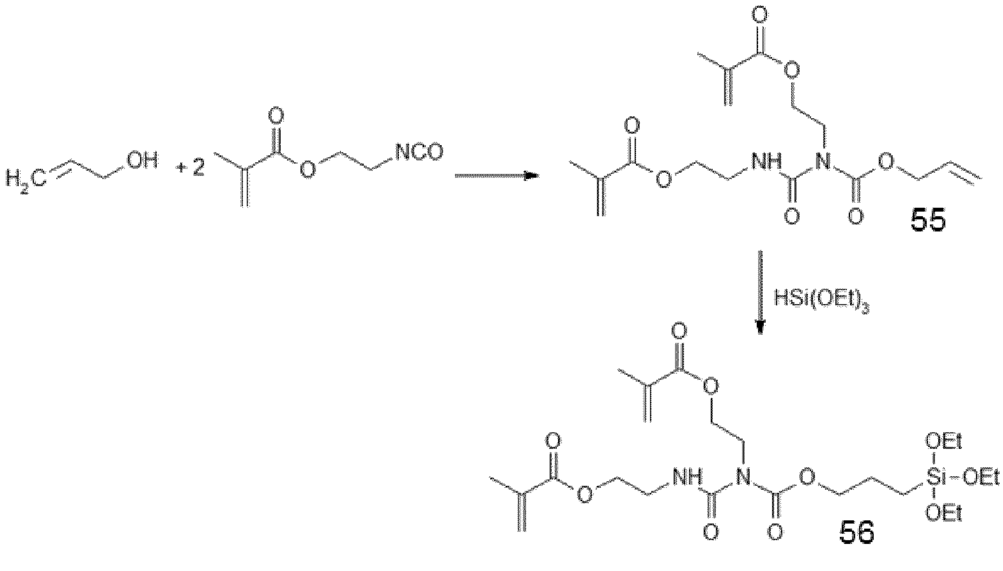


55

56

Durch Umsetzung von zwei Äquivalenten 2-Isocyanatoethylmethacrylat mit 2-Propen-1-thiol erhält man die Vinyl-Verbindung (57). Durch weitere Platin-katalysierte Umsetzung mit Triethoxysilan erhält man schließlich das Thioallophanat-Silan (58).

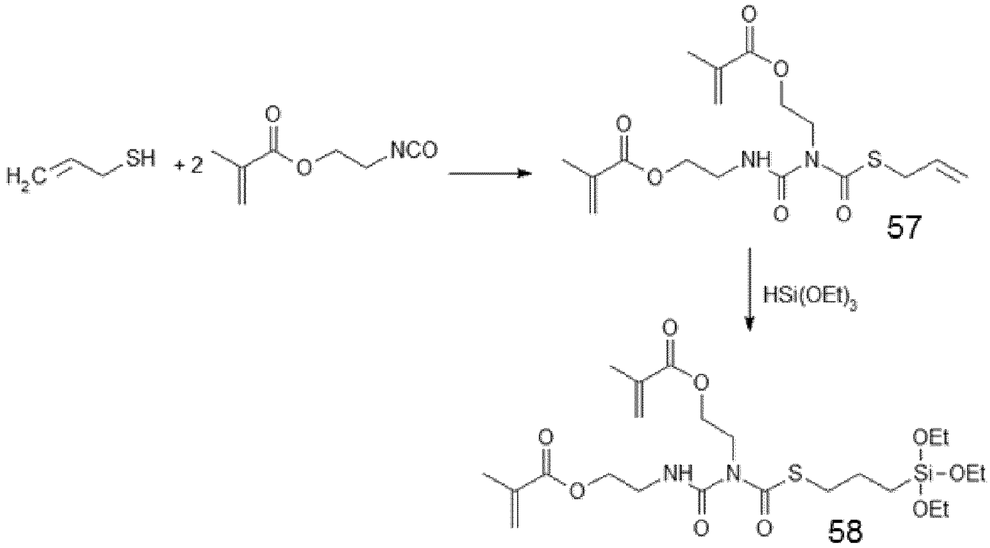


57

58

Durch Umsetzung von zwei Äquivalenten 2-Isocyanatoethylmethacrylat mit 3-Amino-1-propen erhält man die Vinyl-Verbindung (59). Durch weitere Platin-katalysierte Umsetzung mit Triethoxysilan erhält man schließlich das Biuret-Silan (60).

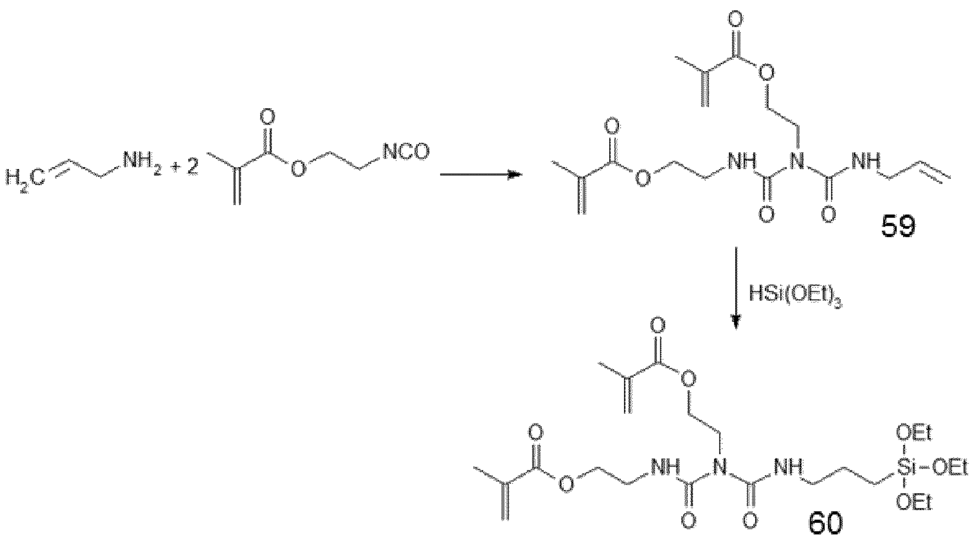


59

60

Synthese von $(R^1O)_aR^2_bSi[-(CH_2)_2-CH(R^4)-NH-C(=O)-X-B[-Z-C(=O)C(=CH_2)R^3]_f]_c$

In einer zweistufigen Synthese wird zunächst eine OH-, SH- oder $NH_2$-substituierte (Meth)acryl-Verbindung Zinn- oder Bismuth-katalysiert mit einer isocyanatsubstituierten Vinyl-Verbindung umgesetzt. Bevorzugte Katalysatoren sind auch hier Dibutylzinndilaurat und Bismuthneodecanoat. Unter leichter Erwärmung wird im äquimolaren Verhältnis jeweils eine XH-Gruppe mit einer Isocyanatgruppe umgesetzt. Die Reaktion verläuft in der Regel vollständig und kann leicht im IR-Spektrum verfolgt werden, bis die Isocyanat-Bande (2270 $cm^{-1}$) vollständig verschwunden ist. Kommerziell erhältliche XH-substituierte (Meth)acryl-Verbindungen sind weiter oben in Tabelle 4 aufgeführt. Kommerziell erhältliche isocyanatsubstituierten Vinyl-Verbindungen sind in der folgenden Tabelle 9 aufgeführt.

$$[R^3-C(=CH_2)C(=O)-Z-]_fB-XH \ + \ H_2C=CHCH(R^4)-NCO \ \rightarrow \ [R^3-C(=CH_2)C(=O)-Z-]_fB-X-C(=O)-NH-CH(R^4)CH=CH_2$$

Tabelle 9:

| | $H_2C=CHCH(R^4)$-NCO |
|---|---|
| **CAS-Nr.** | **$R^4$** |
| 1476-23-9 | H |
| 155469-99-1 | Me |
| 55887-59-7 | Ph |

In der zweiten Synthesestufe wird die erhaltene Vinyl-Verbindung Platin-katalysiert mit einem Hydrosilan $(R^1O)_aR^2_bSiH_c$ umgesetzt. Kommerziell erhältliche Hydrosilane sind in Tabelle 7 aufgeführt.

$$c\ [R^3\text{-}C(=CH_2)C(=O)\text{-}Z\text{-}]_fB\text{-}X\text{-}C(=O)\text{-}NH\text{-}CH(R^4)\text{-}CH=CH_2 + (R^1O)_aR^2_bSiH_c \rightarrow (R^1O)_aR^2_bSi[\text{-}(CH_2)_2\text{-}CH(R^4)\text{-}NH\text{-}C(=O)\text{-}X\text{-}B[\text{-}Z\text{-}C(=O)C(=CH_2)R^3]_f]_c$$

Im Folgenden werden beispielhaft einige Synthesen der Silane $(R^1O)_aR^2_bSi[\text{-}(CH_2)_2\text{-}CH(R^4)\text{-}NH\text{-}C(=O)\text{-}X\text{-}B[\text{-}Z\text{-}C(=O)C(=CH_2)R^3]_f]_c$ dargestellt.

Durch Umsetzung von 2-Hydroxyethylmethacrylat (HEMA) mit 3-Isocyanato-1-propen erhält man die Vinyl-Verbindung (61). Durch weitere Platin-katalysierte Umsetzung mit Triethoxysilan erhält man schließlich das Silan (62).

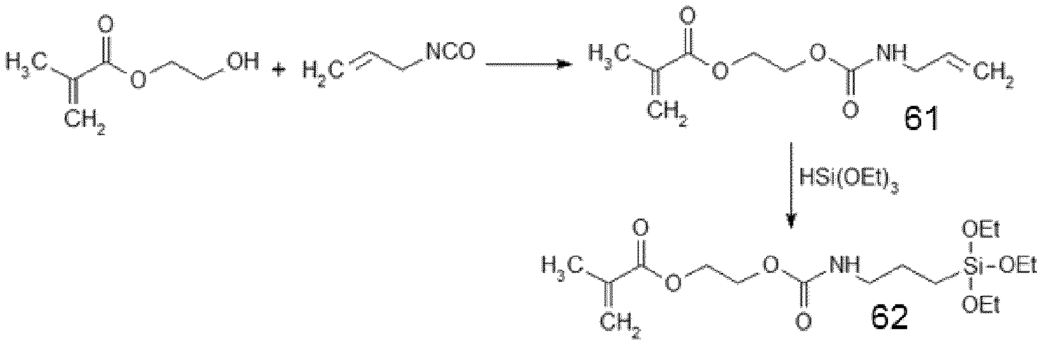

61
62

Durch Umsetzung von 1,3-Glycerindimethacrylat mit 3-Isocyanato-1-propen erhält man die Vinyl-Verbindung (63). Durch weitere Platin-katalysierte Umsetzung mit Triethoxysilan erhält man schließlich das Silan (64).

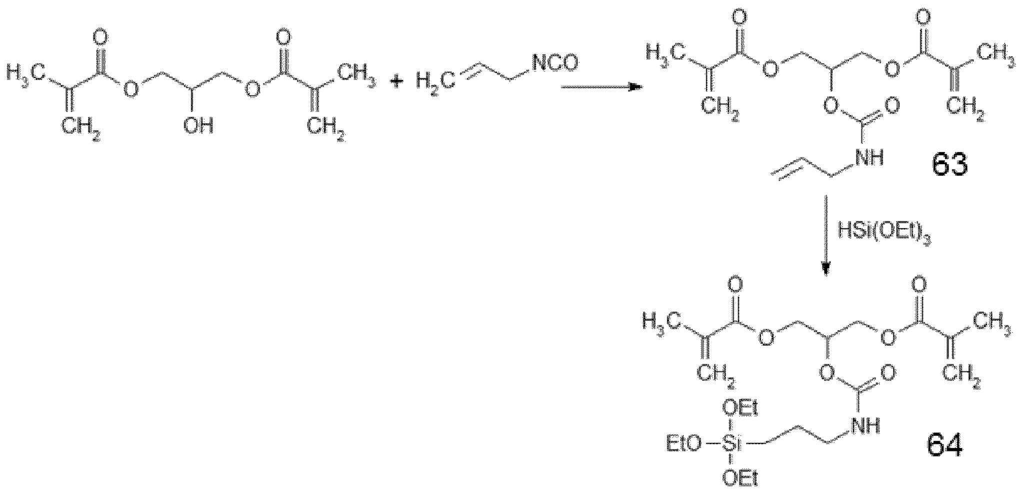

63
64

Durch Umsetzung von 2-Hydroxypropylmethacrylat mit 3-Isocyanato-3-phenyl-1-propen erhält man die Vinyl-Verbindung (65). Durch weitere Platin-katalysierte Umsetzung mit Triethoxysilan erhält man schließlich das Silan (66).

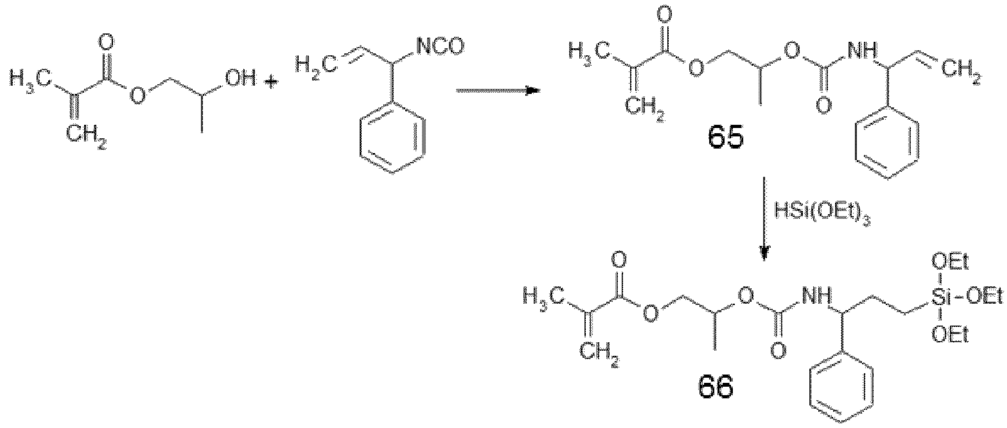

65
66

Durch Umsetzung von 2-Aminoethylmethacrylat mit 3-Isocyanato-1-propen erhält man die Vinyl-Verbindung (67). Durch weitere Platin-katalysierte Umsetzung mit Triethoxysilan erhält man schließlich das Silan (68).

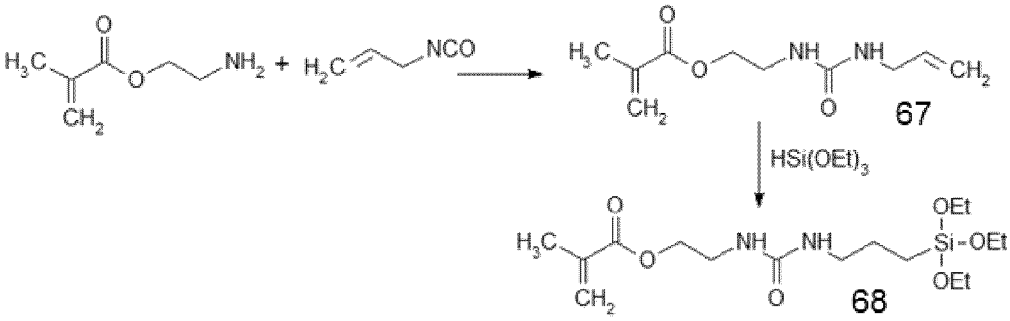

67
68

Synthese von $(R^1O)_aR^2_bSi[-A(-C(=O)NHC(=O)NH\{-B[-Z-C(=O)C(=CH_2)R^3]_f\}_e)_d]_c$:

Acylharnstoff-Silane lassen sich z.B. in einer zweistufigen Synthese ausgehend von vinylfunktionalisierten Amiden durch Umsetzung mit isocyanatsubstituierten (Meth)acrylaten herstellen. Dabei wird zunächst das Amid Zinn- oder Bismuth-katalysiert mit einer isocyanatsubstituierten Vinyl-Verbindung umgesetzt. Bevorzugte Katalysatoren sind auch hier Dibutylzinndilaurat und Bismuthneodecanoat. Unter leichter Erwärmung wird im äquimolaren Verhältnis jeweils eine Amid-Gruppe mit einer Isocyanatgruppe umgesetzt. Die Reaktion verläuft in der Regel vollständig und kann leicht im IR-Spektrum verfolgt werden, bis die Isocyanat-Bande (2270 $cm^{-1}$) vollständig verschwunden ist. Kommerziell erhältliche isocyanatsubstituierten Vinyl-Verbindungen sind in Tabelle 2 aufgeführt. Anschließend erfolgt eine Hydrosilylierung. Kommerziell erhältliche Hydrosilane sind in Tabelle 7 aufgeführt.

So ergibt die Umsetzung von 3-Butenamid (CAS-Nr. 28446-58-4) mit 2-Isocyanatoethylmethacrylat und anschließende Hydrosilylierung mit Triethoxysilan das entsprechende Acylharnstoff-Silan.

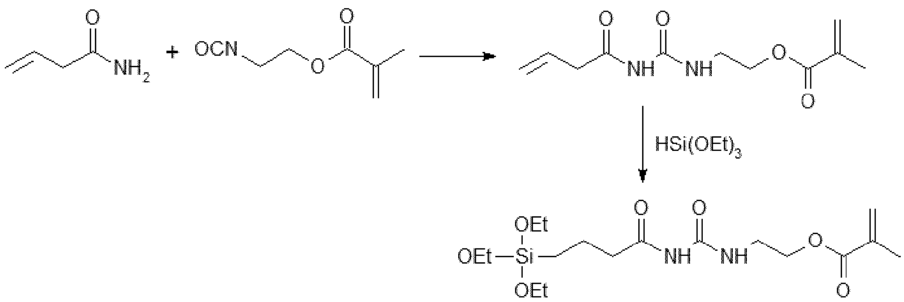


Synthese von $(R^1O)_aR^2_bSi[-A(-NHC(=O)NHC(=O)\{-B[-Z-C(=O)C(=CH_2)R^3]_f\}_e)_d]_c$:

Acylharnstoff-Silane lassen sich z.B. in einer zweistufigen Synthese ausgehend von amidfunktionalisierten (Meth)acrylaten durch Umsetzung mit vinylsubstituierten Isocyanaten herstellen. Dabei wird zunächst das Amid Zinn- oder Bismuth-katalysiert mit einer vinylsubstituierten Isocyanat-Verbindung umgesetzt. Bevorzugte Katalysatoren sind

auch hier Dibutylzinndilaurat und Bismuthneodecanoat. Unter leichter Erwärmung wird im äquimolaren Verhältnis jeweils eine Amidgruppe mit einer Isocyanatgruppe umgesetzt. Die Reaktion verläuft in der Regel vollständig und kann leicht im IR-Spektrum verfolgt werden, bis die Isocyanat-Bande (2270 $cm^{-1}$) vollständig verschwunden ist. Kommerziell erhältliche vinylsubstituierte Isocyanate sind in Tabelle 9 aufgeführt. Anschließend erfolgt eine Hydrosilylierung. Kommerziell erhältliche Hydrosilane sind in Tabelle 7 aufgeführt.

So ergibt die Umsetzung von 2-Carbamoylethylmethacrylat (CAS-Nr. 160031-60-7) mit 3-Isocyanato-1-propen und anschließende Hydrosilylierung mit Triethoxysilan das entsprechende Acylharnstoff-Silan.

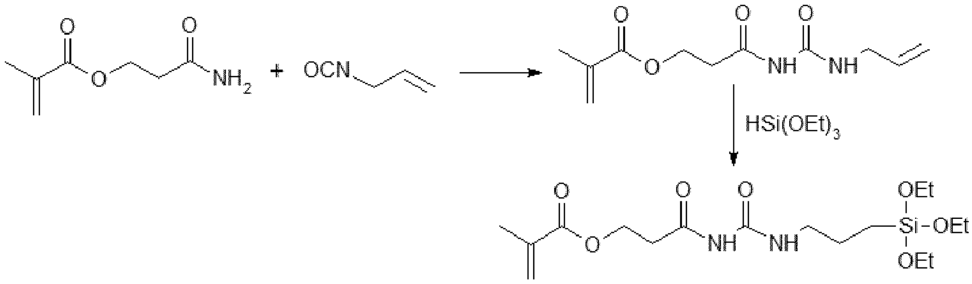


<u>Silan $(R^1O)_aR^2_bSi[-A'(-PG)_f]_c$ (a2):</u>

Das Silan (a2) entspricht der Formel $(R^1O)_aR^2_bSi[-A'(-PG)_f]_c$ mit

PG = polymerisierbare Gruppe,
wobei die polymerisierbare Gruppe PG ausgewählt ist aus der Gruppe bestehend aus $-Z-C(=O)-CH=CH_2$ und $-Z-C(=O)-C(CH_3)=CH_2$ wobei Z ausgewählt ist aus der Gruppe bestehend aus O und NH,
A' = eine organische Verbindungsgruppe, die Si mit PG verbindet und 1 bis 20 C-Atome aufweist und keine der Gruppen -C(=O)NH-, -NHC(=O)-, -OC(=O)NH-, -NHC(=O)O-, -SC(=O)NH-, -NHC(=O)S-, -NHC(=O)NH-, -OC(=O)N(-C(=O)NH-)-, -SC(=O)N(-C(=O)NH-)-, -NHC(=O)N(-C(=O)NH-)- -C(=O)NHC(=O)NH-, oder -NHC(=O)NHC(=O)- enthält,
$R^1$ = H oder C1- bis C4-Alkyl,
$R^2$ = C1- bis C4-Alkyl,
a = 2 oder 3
b = 0 oder 1
c = 1 oder 2
f = 1 bis 5 und

$$a + b + c = 4.$$

In einer bevorzugten Ausführungsform ist die polymerisierbare Gruppe PG ausgewählt aus der Gruppe bestehend aus $-Z-C(=O)-CH=CH_2$ und $-Z-C(=O)-C(CH_3)=CH_2$ wobei Z = O.

Die organische Verbindungsgruppe A', die das Si-Atom mit der polymerisierbaren Gruppe PG verbindet, ist eine (f+1)-valente, geradkettige, verzweigte oder cyclische Gruppe, die 1 bis 20 C-Atome aufweist sowie optional O-Atome, S-Atome, NR-Gruppen, Estergruppen oder Thioestergruppen aufweisen kann.

In einer bevorzugten Ausführungsform ist die Verbindungsgruppe A' ausgewählt aus der Gruppe bestehend aus $-(CH_2)_n-$ mit n = 1 bis 12, $-(CH_2)_n-N(CH_3)-(CH_2)_n-$mit n = 1 bis 4, $-(CH_2)_nN[(CH_2)_n-]_2$ mit n = 1 bis 4 und $-(CH_2)_n-CH(R)-(CH_2)_n-$ mit n = 0 bis 4 und R = Methyl, Ethyl, Phenyl sowie

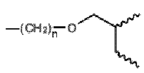

mit n = 1 bis 6, wobei jeweils die links angeordnete Bindung mit dem Si-Atom verbunden ist und die rechts angeordnete(n) Bindung(en) mit der/den Gruppe(n) PG verbunden ist/sind.

In einer ganz bevorzugten Ausführungsform ist die Verbindungsgruppe A' $-(CH_2)_n-$ mit n = 1 bis 6.

In einer bevorzugten Ausführungsform ist der Rest $R^1$ ausgewählt aus der Gruppe bestehend aus H, Methyl und Ethyl.

In einer bevorzugten Ausführungsform ist der Index f = 1 oder 2.

Eine Reihe geeigneter Silane $(R^1O)_aR^2_bSi[-A'(-Z-C(=O)C(=CH_2)R^3)_f]_c$ sind kommerziell erhältlich (siehe Tabelle

10). Darüber hinaus lassen sich die Silane nach gängigen Synthesemethoden herstellen. Einige Syntheserouten sind z.B. in US 2015/0299469 A1 offenbart.

Tabelle 10:

| | $(R^1O)_aR^2_bSi$ | | | | $[A'(-Z-C(=O)C(=CH_2)R^3)_f]_c$ | | | | |
|---|---|---|---|---|---|---|---|---|---|
| **CAS-Nr.** | **$R^1$** | **$R^2$** | **$R^3$** | **Z** | **a** | **b** | **c** | **f** | **-A'-** |
| 21134-38-3 | Me | - | H | O | 3 | 0 | 1 | 1 | $-(CH_2)-$ |
| 54586-78-6 | Me | - | Me | O | 3 | 0 | 1 | 1 | $-(CH_2)-$ |
| 78884-71-6 | Et | - | H | O | 3 | 0 | 1 | 1 | $-(CH_2)-$ |
| 5577-72-0 | Et | - | Me | O | 3 | 0 | 1 | 1 | $-(CH_2)-$ |
| 121177-93-3 | Me | Me | Me | O | 2 | 1 | 1 | 1 | $-(CH_2)-$ |
| 3978-58-3 | Et | Me | Me | O | 2 | 1 | 1 | 1 | $-(CH_2)-$ |
| 4369-14-6 | Me | - | H | O | 3 | 0 | 1 | 1 | $-(CH_2)_3-$ |
| 2530-85-0 | Me | - | Me | O | 3 | 0 | 1 | 1 | $-(CH_2)_3-$ |
| 57577-96-5 | Me | - | H | NH | 3 | 0 | 1 | 1 | $-(CH_2)_3-$ |
| 10310-41-5 | Me | - | Me | NH | 3 | 0 | 1 | 1 | $-(CH_2)_3-$ |
| 20208-39-3 | Et | - | H | O | 3 | 0 | 1 | 1 | $-(CH_2)_3-$ |
| 21142-29-0 | Et | - | Me | O | 3 | 0 | 1 | 1 | $-(CH_2)_3-$ |
| 80750-05-6 | $^iPr$ | - | Me | O | 3 | 0 | 1 | 1 | $-(CH_2)_3-$ |
| 13732-00-8 | Me | Me | H | O | 2 | 1 | 1 | 1 | $-(CH_2)_3-$ |
| 14513-34-9 | Me | Me | Me | O | 2 | 1 | 1 | 1 | $-(CH_2)_3-$ |
| 146666-71-9 | Et | Me | H | O | 2 | 1 | 1 | 1 | $-(CH_2)_3-$ |
| 65100-04-1 | Et | Me | Me | O | 2 | 1 | 1 | 1 | $-(CH_2)_3-$ |

Synthese von $(R^1O)_aR^2_bSi[-A'(-Z-C(=O)C(=CH_2)R^3)_f]_c$

Durch Umsetzung von OH-, SH- oder $NH_2$-funktionalisierten Silanen mit (Meth)acryloylchlorid werden geeignete Silane erhalten. Die Synthese verläuft in der Regel einfach und quantitativ. Alternativ können auch (Meth) acrylsäure oder andere (Meth)acrylsäurederivate in den üblichen Verfahren eingesetzt werden. Geeignete kommerzielle ZH-funktionalisierte Silane sind in Tabelle 11 aufgeführt.

$$(R^1O)_aR^2_bSi[-A'(-ZH)_f]_c + (cxe)\ R^3C(=CH_2)C(=O)Cl \rightarrow (R^1O)_aR^2_bSi[-A'(-Z-C(=O)C(=CH_2)R^3)_f]_c$$

Tabelle 11:

| | $(R^1O)_aR^2_bSi[-A'(-ZH)_f]_c$ | | | | | | | |
|---|---|---|---|---|---|---|---|---|
| **CAS-Nr.** | **$R^1$** | **$R^2$** | **ZH** | **a** | **b** | **c** | **f** | **-A'-** |
| 53764-54-8 | Me | - | OH | 3 | 0 | 1 | 1 | $-(CH_2)_3-$ |
| 53394-61-9 | Et | - | OH | 3 | 0 | 1 | 1 | $-(CH_2)_3-$ |
| 99697-20-8 | Me | Me | OH | 2 | 1 | 1 | 1 | $-(CH_2)_3-$ |
| 162781-70-6 | Et | - | OH | 3 | 0 | 1 | 1 | $-(CH2)_1-$ |
| 13822-56-5 | Me | - | $NH_2$ | 3 | 0 | 1 | 1 | $-(CH_2)_3-$ |
| 3663-44-3 | Me | Me | $NH_2$ | 2 | 1 | 1 | 1 | $-(CH_2)_3-$ |
| 3179-76-8 | Et | Me | $NH_2$ | 2 | 1 | 1 | 1 | $-(CH_2)_3-$ |

(fortgesetzt)

| | $(R^1O)_aR^2_bSi[-A'(-ZH)_f]_c$ | | | | | | | |
|---|---|---|---|---|---|---|---|---|
| CAS-Nr. | $R^1$ | $R^2$ | ZH | a | b | c | f | -A'- |
| 71408-48-5 | Me | - | $NH_2$ | 3 | 0 | 1 | 1 | $-(CH2)_1-$ |
| 18306-83-7 | Et | - | $NH_2$ | 3 | 0 | 1 | 1 | $-(CH_2)_1-$ |
| 51749-36-1 | Me | - | $NH_2$ | 2 | 0 | 2 | 1 | $-(CH_2)_3-$ |
| 53746-12-6 | Et | - | $NH_2$ | 2 | 0 | 2 | 1 | $-(CH_2)_3-$ |
| 330457-46-0 | Me | - | OH | 3 | 0 | 1 | 1 | $-(CH_2)_3-N(CH_3)-(CH_2)_2-$ |
| 24801-87-4 | Me | - | OH | 3 | 0 | 1 | 2 | $-(CH_2)_3N[(CH_2)_2-]_2$ |
| 7538-44-5 | Et | - | OH | 3 | 0 | 1 | 2 | $-(CH_2)_3N[(CH_2)_2-]_2$ |

Im Folgenden werden beispielhaft einige Synthesen der Silane $(R^1O)_aR^2_bSi[-A'(-Z-C(=O)C(=CH_2)R^3)_f]_c$ dargestellt.

Durch Umsetzung von 3-(Trimethoxysilyl)-1-propanol mit Methacryloylchlorid erhält man das Silan (69).

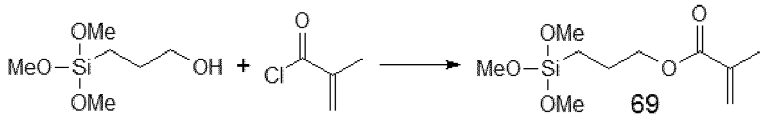

69

Durch Umsetzung von 3-(Methyldimethoxysilyl)-1-propanol mit Methacryloylchlorid erhält man das Silan (70).

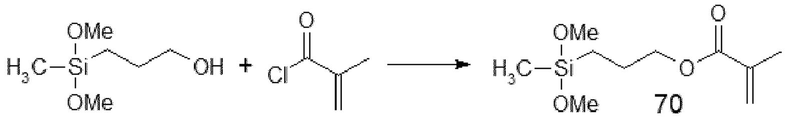

70

Durch Umsetzung von 1-(Trimethoxysilyl)-methanol mit Methacryloylchlorid erhält man das Silan (71).

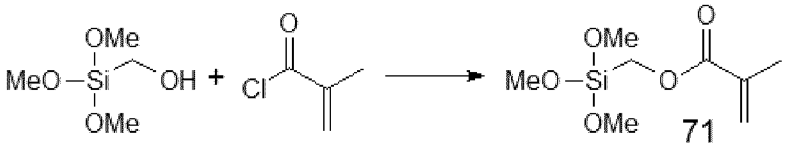

71

Durch Umsetzung von 1-(Methyldimethoxysilyl)-methanol mit Methacryloylchlorid erhält man das Silan (72).

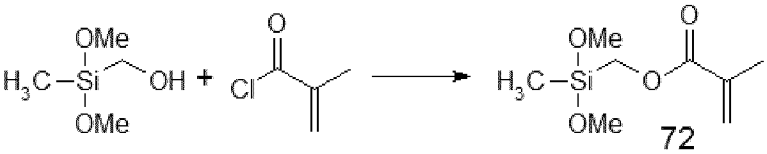

72

Durch die Umsetzung von 2,2'-[[3-(Triethoxysilyl)propyl]imino]bis[ethanol] mit 2 Äquivalenten Methacryloylchlorid erhält man das Silan (73).

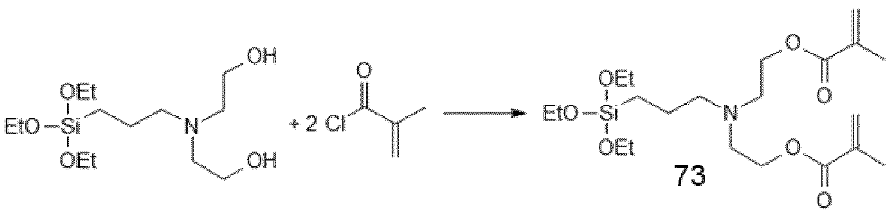

73

Synthese von Silanen ausgehend von Hydrosilanen

Geeignete Silane lassen sich in einer mehrstufigen Synthese durch Platin-katalysierte Umsetzung von Vinyl-Verbindungen mit Hydrosilanen herstellen. Entsprechende Synthesen sind in US 2015/0299469 A1 offenbart.

Synthese von $(R^1O)_aR^2_bSi[-(CH_2)_{(n+2)}-Z-C(=O)C(=CH_2)R^3]_c$

In einer zweistufigen Synthese wird zunächst (Meth)acryloylchlorid mit einer OH- oder $NH_2$-funktionalisierten terminalen Vinylverbindung umgesetzt. Alternativ können auch hier (Meth)acrylsäure oder andere (Meth)acrylsäurederivate in den üblichen Verfahren eingesetzt werden.

$$R^3C(=CH_2)C(=O)Cl + H_2C=CH(CH_2)_n-ZH \rightarrow R^3C(=CH_2)C(=O)-Z-(CH_2)_nCH=CH_2$$

Tabelle 12:

| | $H_2C=CH(CH_2)_n$-ZH | |
|---|---|---|
| **CAS-Nr.** | **n** | **ZH** |
| 107-18-6 | 1 | OH |
| 627-27-0 | 2 | OH |
| 821-09-0 | 3 | OH |
| 821-41-0 | 4 | OH |
| 4117-10-6 | 5 | OH |
| 13175-44-5 | 6 | OH |
| 107-11-9 | 1 | $NH_2$ |
| 2524-49-4 | 2 | $NH_2$ |
| 22537-07-1 | 3 | $NH_2$ |
| 34825-70-2 | 4 | $NH_2$ |
| 151626-26-5 | 5 | $NH_2$ |
| 82223-49-2 | 6 | $NH_2$ |

In der zweiten Synthesestufe wird die erhaltene Vinyl-Verbindung Platin-katalysiert mit einem Hydrosilan $(R^1O)_aR^2_bSiH_c$ umgesetzt. Kommerziell erhältliche Hydrosilane sind weiter oben in Tabelle 7 aufgeführt.

$$c\ R^3C(=CH_2)C(=O)-Z-(CH_2)_nCH=CH_2 + (R^1O)_aR^2_bSiH_c \rightarrow (R^1O)_aR^2_bSi[-(CH_2)_{(n+2)}-Z-C(=O)C(=CH_2)R^3]_c$$

Im Folgenden werden beispielhaft einige Synthesen der Silane $(R^1O)_aR^2_bSi[-(CH_2)_{(n+2)}-Z-C(=O)C(=CH_2)R^3]_c$ dargestellt.

Durch Umsetzung von Methacryloylchlorid mit 2-Propen-1-ol erhält man zunächst die Vinyl-Verbindung (74). Durch weitere Platin-katalysierte Umsetzung mit Triethoxysilan erhält man schließlich das Silan (75).

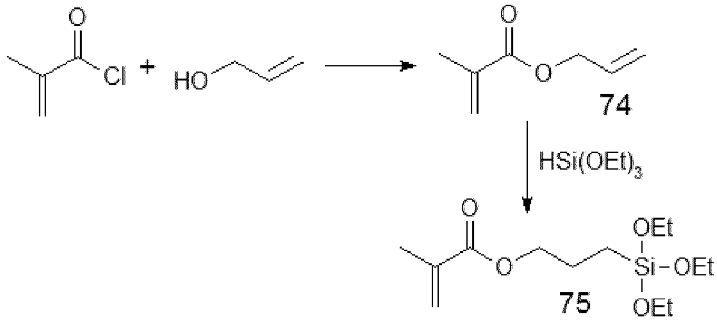


Durch Umsetzung von Methacryloylchlorid mit 2-Propen-1-ol erhält man zunächst die Vinyl-Verbindung (74 s.o.). Durch weitere Platin-katalysierte Umsetzung mit Methyldimethoxysilan erhält man schließlich das Silan (76).

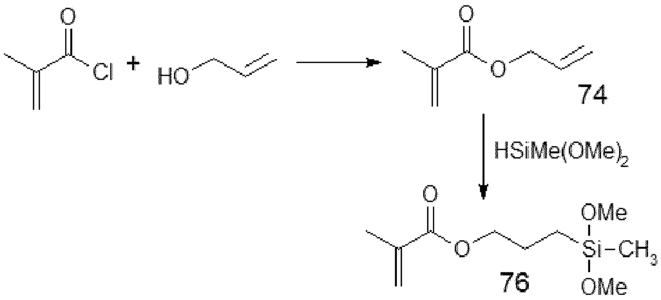


Durch Umsetzung von Methacryloylchlorid mit 4-Penten-1-ol erhält man zunächst die Vinyl-Verbindung (77). Durch weitere Platin-katalysierte Umsetzung mit Triethoxysilan erhält man schließlich das Silan (78).

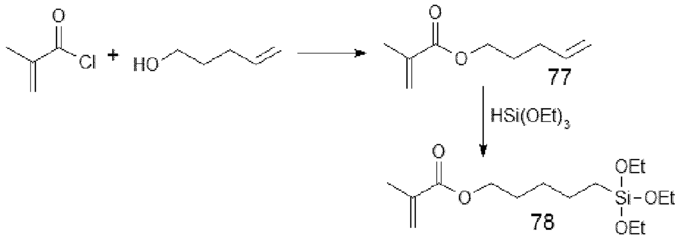


Durch Umsetzung von Methacryloylchlorid mit Allylamin erhält man zunächst die Vinyl-Verbindung (79). Durch weitere Platin-katalysierte Umsetzung mit Triethoxysilan erhält man schließlich das Silan (80).

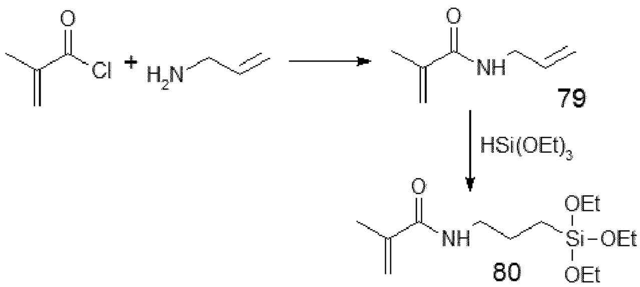


Durch Umsetzung von Methacryloylchlorid mit Allylamin erhält man zunächst die Vinyl-Verbindung (79 s.o.). Durch weitere Platin-katalysierte Umsetzung mit Methyldimethoxysilan erhält man schließlich das Silan (81).

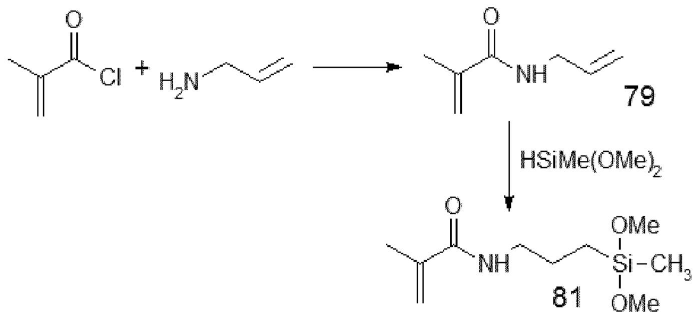


79

81

Durch Umsetzung von Methacryloylchlorid mit 4-Penten-1-amin erhält man zunächst die Vinyl-Verbindung (82). Durch weitere Platin-katalysierte Umsetzung mit Triethoxysilan erhält man schließlich das Silan (83).

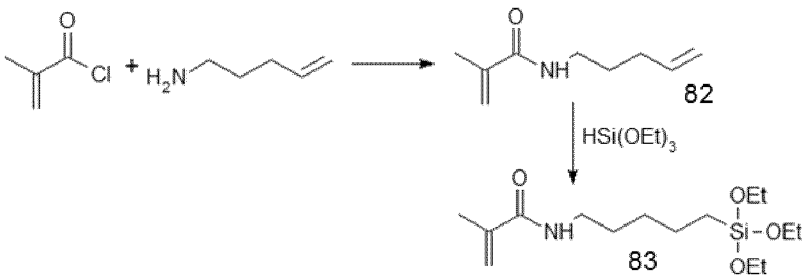


82

83

<u>Synthese von $(R^1O)_aR^2{}_bSi[-A'(-Z-C(=O)C(=CH_2)R^3)_f]_c$</u>

Durch Umsetzung von Glycidoxy-funktionalisierten Silanen mit (Meth)acrylsäure werden geeignete OH-funktionalisierte Silane erhalten. Die Synthese verläuft in der Regel einfach und quantitativ. Durch weitere Umsetzung mit (Meth)acryloylchlorid oder einem anderen (Meth)acrylsäurederivat können geeignete Silane mit zwei (Meth)acryl-Gruppe erhalten werden. Geeignete kommerzielle Glycidoxy-funktionalisierte Silane sind in Tabelle 13 aufgeführt.

Tabelle 13:

| | 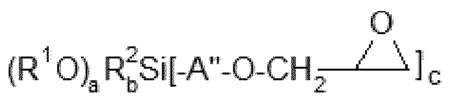 | | | | | |
|---|---|---|---|---|---|---|
| **CAS-Nr.** | **$R^1$** | **$R^2$** | **a** | **b** | **c** | **-A"-** |
| 215301-24-9 | Et | Me | 2 | 1 | 1 | $-CH_2-$ |
| 2530-83-8 | Me | - | 3 | 0 | 1 | $-(CH_2)_3-$ |
| 2602-34-8 | Et | - | 3 | 0 | 1 | $-(CH_2)_3-$ |
| 98899-94-6 | n-Pr | - | 3 | 0 | 1 | $-(CH_2)_3-$ |
| 252255-95-1 | $^i$Pr | - | 3 | 0 | 1 | $-(CH_2)_3-$ |
| 65799-47-5 | Me | Me | 2 | 1 | 1 | $-(CH_2)_3-$ |
| 2897-60-1 | Et | Me | 2 | 1 | 1 | $-(CH_2)_3-$ |
| 131535-64-3 | *n*-Pr | *n*-Pr | 2 | 1 | 1 | $-(CH_2)_2-$ |
| 233765-90-7 | Me | - | 3 | 0 | 1 | $-CH(CH_3)CH_2-$ |
| 139485-54-4 | Et | - | 3 | 0 | 1 | $-CH(CH_3)CH_2-$ |
| 70187-33-6 | Me | - | 3 | 0 | 1 | $-CH_2CH(CH_3)-$ |
| 20411-24-1 | Me | - | 2 | 0 | 2 | $-(CH_2)_3-$ |

Darüber hinaus lassen sich geeignete Glycidoxy-funktionalisierte Silane erhalten, indem man Glycidoxy-funktionalisierte Vinylverbindungen Platin-katalysiert mit $(R^1O)_aR^2{}_bSiH_c$ (siehe Tabelle 7) umsetzt. Als Glycidoxy-funktionalisierte Vinylverbindung wird bevorzugt 1,2-Epoxy-3-allyloxypropan (CAS-Nr. 106-92-3) eingesetzt. Geeignete Glycidoxy-funktionalisierte Vinylverbindung können aber auch durch bekannte Synthesen hergestellt werden. Beispielsweise kann Glycidol (CAS-Nr. 556-52-5) mit 3-Butenoylchlorid (CAS-Nr. 1470-91-3), 4-Pentenoylchlorid (CAS-Nr. 39716-58-0) oder 5-Hexenoylchlorid (CAS-Nr. 36394-07-7) umgesetzt werden. Statt Glycidol können auch substituierte Glycidolderivate eingesetzt werden. Kommerziell erhältliche Glycidolderivate sind in Tabelle 14 aufgeführt.

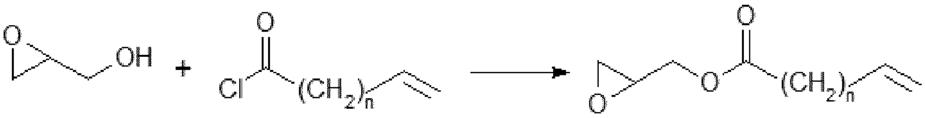

Tabelle 14:

| | 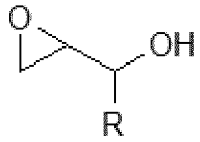 |
|---|---|
| **CAS-Nr.** | **R** |
| 556-52-5 | H |
| 765-44-6 | Me |
| 4798-48-5 | Et |
| 33143-44-1 | Ph |

Durch Umsetzung von (3-Glycidoxypropyl)trimethoxysilan mit Methacrylsäure erhält man das Silan (84). Die weitere Umsetzung mit Methacryloylchlorid liefert das Silan (85).

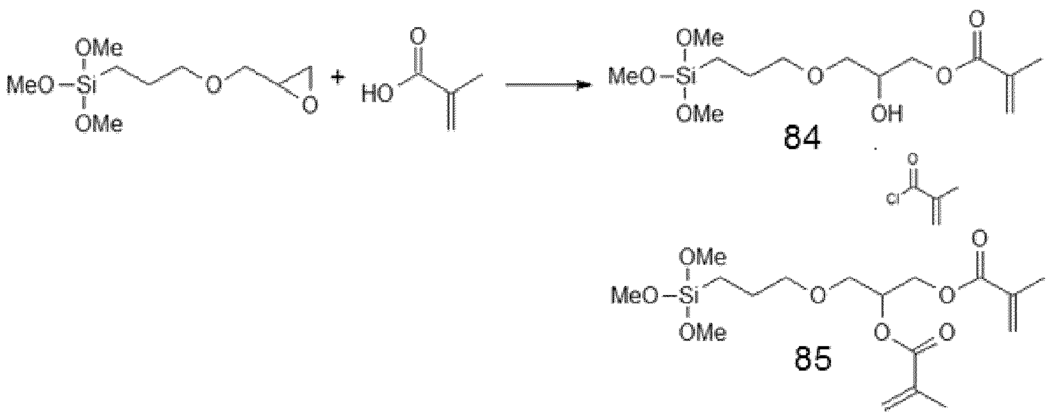

84

85

Synthese von $(R^1O)_aR^2_bSi[-(CH_2)_{(n+2)}-X'-C(=O)-B'[-Z-C(=O)C(=CH_2)R^3]_f]_c$

In einer zweistufigen Synthese wird zunächst eine Carboxyl-(meth)acryl-Verbindung mit einer OH- oder SH-funktionalisierten terminalen Vinylverbindung umgesetzt. Geeignete kommerzielle Carboxyl-(meth)acryl-Verbindungen sind in Tabelle 15 aufgeführt. Geeignete OH- oder SH-funktionalisierte terminale Vinylverbindungen sind in Tabelle 16 aufgeführt. Bevorzugt ist die Verwendung von Allylalkohol oder Allylmercaptan.

$$[R^3C(=CH_2)C(=O)-Z-]_fB'-COOH \quad + \quad H_2C=CH(CH_2)_n-X'H \quad \rightarrow \quad [R^3C(=CH_2)C(=O)-Z-]_rB'-C(=O)-X'-(CH_2)_nCH=CH_2$$

Tabelle 15:

| | $[R^3\text{-}C(=CH_2)C(=O)\text{-}Z\text{-}]_fB'\text{-}COOH$ | | | |
|---|---|---|---|---|
| **CAS-Nr.** | **$R^3$** | **Z** | **f** | **-B'-** |
| 141681-03-0 | H | O | 1 | $-(CH_2)_3-$ |
| 59178-90-4 | Me | NH | 1 | $-(CH_2)_2-$ |
| 16753-07-4 | H | NH | 1 | $-(CH_2)_2-$ |
| 59178-91-5 | Me | NH | 1 | $-(CH_2)_3-$ |
| 59178-91-5 | H | NH | 1 | $-(CH_2)_3-$ |
| 20882-04-6 | Me | O | 1 | $-(CH_2)_2OC(=O)-(CH_2)_2-$ |
| 112241-32-4 | Me | O | 1 | $-(CH_2)_3OC(=O)-(CH_2)_2-$ |
| 51252-88-1 | Me | O | 1 | 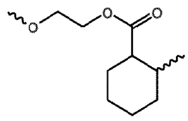 |
| 27697-00-3 | Me | O | 1 | 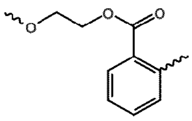 |
| 65859-45-2 | Me | O | 1 | 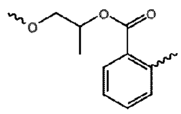 |

Tabelle 16:

| | $H_2C=CH(CH_2)_n$-X'H | |
|---|---|---|
| **CAS-Nr.** | **n** | **X'H** |
| 107-18-6 | 1 | OH |
| 627-27-0 | 2 | OH |
| 821-09-0 | 3 | OH |
| 821-41-0 | 4 | OH |
| 4117-10-6 | 5 | OH |
| 13175-44-5 | 6 | OH |
| 870-23-5 | 1 | SH |
| 5954-70-1 | 2 | SH |
| 17651-37-5 | 3 | SH |
| 17651-39-7 | 4 | SH |
| 173777-16-7 | 5 | SH |
| 178561-30-3 | 9 | SH |

In der zweiten Synthesestufe wird die erhaltene Vinyl-Verbindung Platin-katalysiert mit einem Hydrosilan $(R^1O)_aR^2_bSiH_c$ umgesetzt. Kommerziell erhältliche Hydrosilane sind weiter oben in Tabelle 7 aufgeführt.

$$c\,[R^3C(=CH_2)C(=O)\text{-}Z\text{-}]_fB'\text{-}C(=O)\text{-}X'\text{-}(CH2)_nCH=CH_2 + (R^1O)_aR^2_sSiH_c \rightarrow (R^1O)_aR^2_bSi[\text{-}(CH_2)_{(n+2)}\text{-}X'\text{-}C(=O)\text{-}B'[\text{-}Z\text{-}C(=O)C(=CH_2)R^3]_r]_c$$

Im Folgenden werden beispielhaft einige Synthesen der Silane $(R^1O)_aR^2_bSi[-(CH_2)_{(n+2)}$-X'-C(=O)-B'[-Z-C(=O)

$C(=CH_2)R^3]_f]_c$ dargestellt.

Durch Umsetzung von 3-[(2-methyl-1-oxo-2-propen-1-yl)amino]-propansäure mit 2-Propen-1-ol erhält man zunächst die Vinyl-Verbindung (86). Durch weitere Platin-katalysierte Umsetzung mit Triethoxysilan erhält man schließlich das Silan (87).

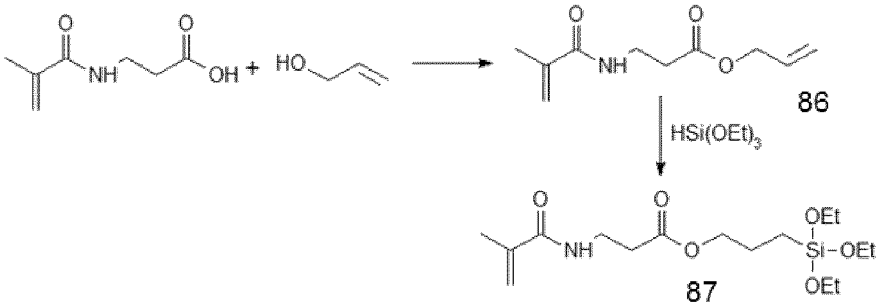


Durch Umsetzung von 4-[(2-methyl-1-oxo-2-propen-1-yl)amino]-butansäure mit 2-Propen-1-ol erhält man zunächst die Vinyl-Verbindung (88). Durch weitere Platin-katalysierte Umsetzung mit Triethoxysilan erhält man schließlich das Silan (89).

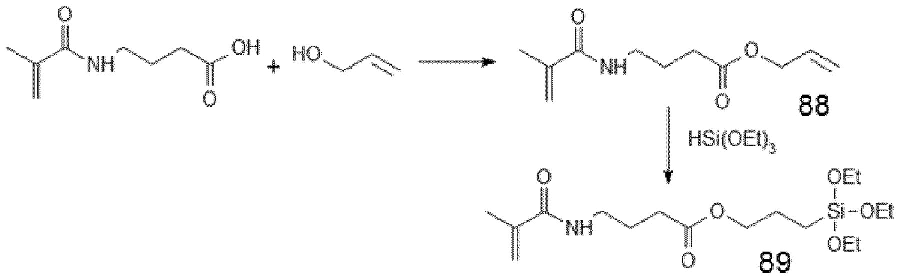


Durch Umsetzung von 3-[(2-methyl-1-oxo-2-propen-1-yl)amino]-propansäure mit 2-Propen-1-thiol erhält man zunächst die Vinyl-Verbindung (90). Durch weitere Platin-katalysierte Umsetzung mit Triethoxysilan erhält man schließlich das Silan (91).

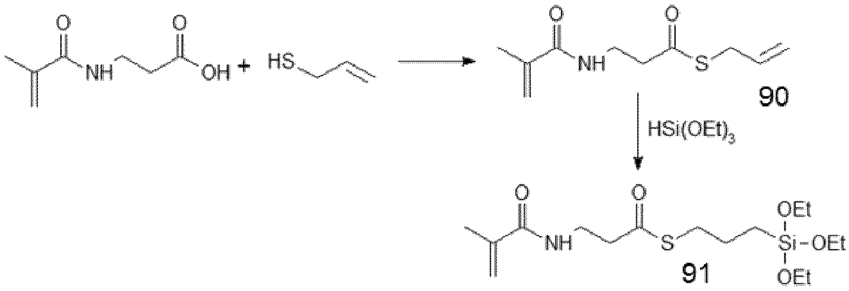


Durch Umsetzung von 4-[(2-methyl-1-oxo-2-propen-1-yl)amino]-butansäure mit 2-Propen-1-thiol erhält man zunächst die Vinyl-Verbindung (92). Durch weitere Platin-katalysierte Umsetzung mit Methyldimethoxysilan erhält man schließlich das Silan (93).

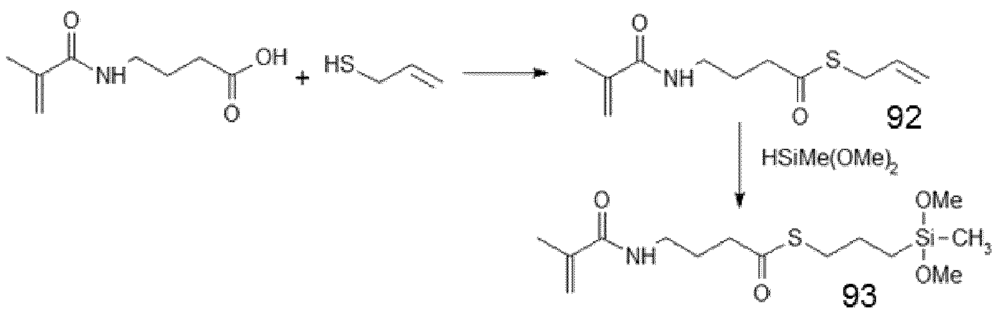

Durch Umsetzung von Mono[2-(methacryloyloxy)ethyl]succinat mit 2-Propen-1-ol erhält man zunächst die Vinyl-Verbindung (94). Durch weitere Platin-katalysierte Umsetzung mit Triethoxysilan erhält man schließlich das Silan (95).

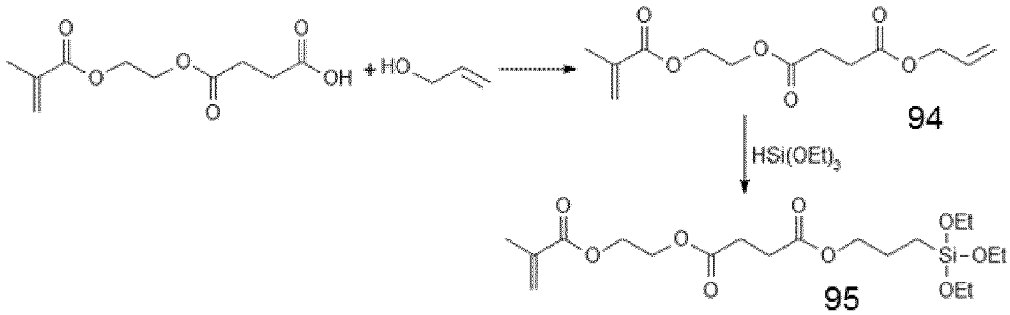

94
95

Durch Umsetzung von Mono[2-(methacryloyloxy)ethyl]succinat mit 2-Propen-1-ol erhält man zunächst die Vinyl-Verbindung (94 s.o.). Durch weitere Platin-katalysierte Umsetzung mit Methyldimethoxysilan erhält man schließlich das Silan (96).

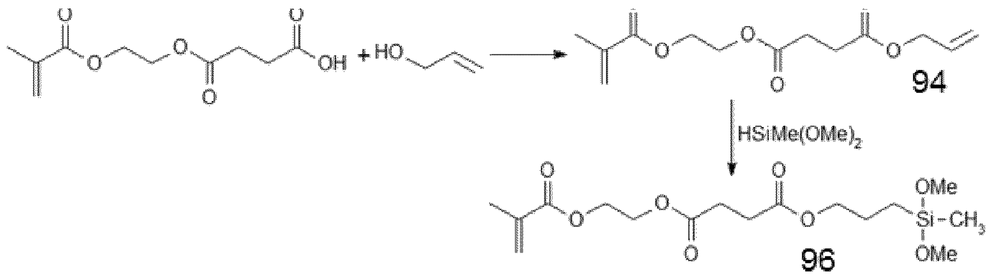

94
96

Durch Umsetzung von Mono[2-(methacryloyloxy)ethyl]succinat mit 2-Propen-1-thiol erhält man zunächst die Vinyl-Verbindung (97). Durch weitere Platin-katalysierte Umsetzung mit Triethoxysilan erhält man schließlich das Silan (98).

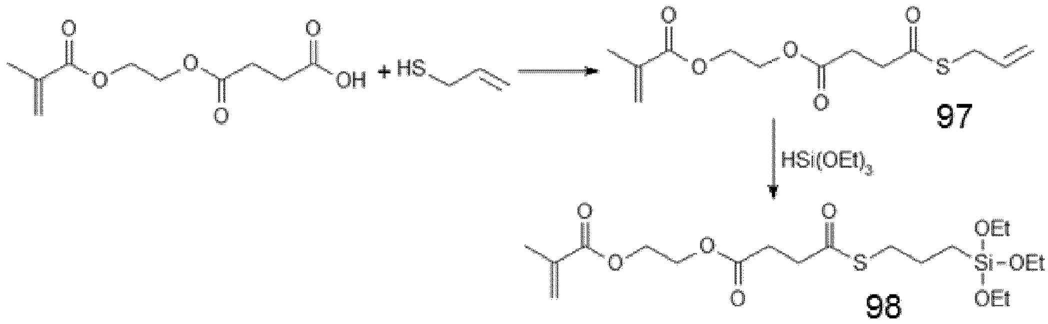

97
98

<u>Silan $(R^1O)_aR^2_bSiAr_c$ (a3):</u>

Das Silan (a3) entspricht der Formel $(R^1O)_aR^2_bSiAr_c$ mit

$R^1$ = H oder C1- bis C4-Alkyl,
$R^2$ = C1- bis C4-Alkyl,
Ar = Aryl wobei unterschiedliche Gruppen Ar gleich oder verschieden sein können,
a = 2 oder 3,
b = 0 oder 1,
c = 1 oder2 und

$$a + b + c = 4.$$

In einer bevorzugten Ausführungsform ist der Rest $R^1$ ausgewählt aus der Gruppe bestehend aus H, Methyl und Ethyl.

In einer weiteren bevorzugten Ausführungsform ist der Rest Ar eine - gegebenenfalls auch substituierte - Phenylgruppe.

Eine Reihe geeigneter aromatischer Silane sind kommerziell erhältlich. In Tabelle 17 sind Silane $(R^1O)_aR^2_bSiAr$ mit einem Arylrest und in Tabelle 18 sind Silane $(R^1O)_2SiAr_2$ mit zwei Arylresten aufgeführt.

Tabelle 17:

| | $(R^1O)_aR^2_bSiAr$ | | | | |
|---|---|---|---|---|---|
| **CAS-Nr.** | **$R^1$** | **$R^2$** | **a** | **b** | **Ar** |
| 2996-92-1 | Me | - | 3 | 0 | Ph |
| 780-69-8 | Et | - | 3 | 0 | Ph |
| 17903-00-3 | $^i$Pr | - | 3 | 0 | Ph |
| 10581-02-9 | *n*-Bu | - | 3 | 0 | Ph |
| 3027-21-2 | Me | Me | 2 | 1 | Ph |
| 775-56-4 | Et | Me | 2 | 1 | Ph |
| 223668-64-2 | Me | - | 3 | 0 | 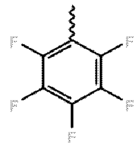 |
| 17043-05-9 | Me | - | 3 | 0 | 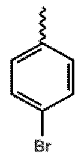 |
| 35692-33-2 | Me | - | 3 | 0 | 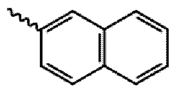 |
| 17995-18-5 | Et | - | 3 | 0 | 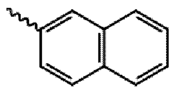 |
| 17938-34-0 | Et | Me | 2 | 1 | 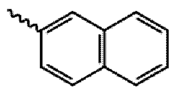 |
| 18052-76-1 | Me | - | 3 | 0 | 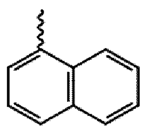 |
| 17938-06-6 | Et | - | 3 | 0 | 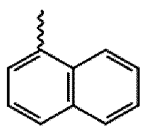 |
| 17938-33-9 | Et | Me | 2 | 1 | 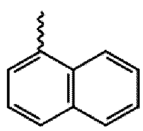 |

(fortgesetzt)

| | $(R^1O)_aR^2_bSiAr$ | | | | |
|---|---|---|---|---|---|
| **CAS-Nr.** | **$R^1$** | **$R^2$** | **a** | **b** | **Ar** |
| 912576-47-7 | Et | - | 3 | 0 | 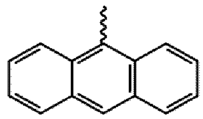 |
| 21591-53-7 | Et | - | 3 | 0 | 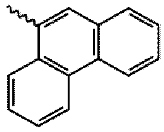 |
| 212609-47-7 | Et | - | 3 | 0 | 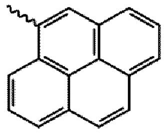 |
| 21591-51-5 | Et | - | 3 | 0 | 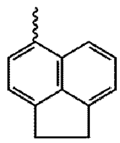 |
| 135251-76-2 | Me | - | 3 | 0 | 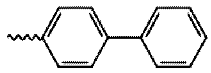 |
| 18056-97-8 | Et | - | 3 | 0 | 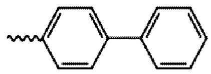 |
| 91309-02-3 | Me | Me | 2 | 1 | 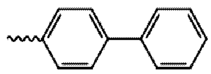 |

Tabelle 18:

| | $(R^1O)_aSiAr^1Ar^2$ | | | |
|---|---|---|---|---|
| **CAS-Nr.** | **$R^1$** | **a** | **$Ar^1$** | **$Ar^2$** |
| 6843-66-9 | Me | 2 | Ph | Ph |
| 2553-19-7 | Et | 2 | Ph | Ph |
| 92779-72-1 | Me | 2 | 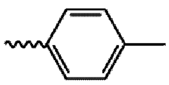 | 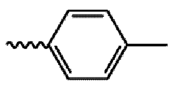 |
| 52897-51-5 | Et | 2 | 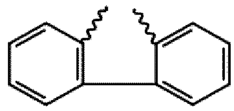 | |
| 223668-68-6 | Me | 2 | 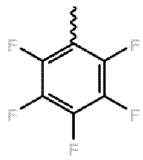 | 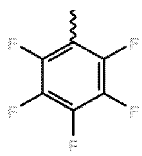 |

(fortgesetzt)

| | $(R^1O)_aSiAr^1Ar^2$ | | | |
|---|---|---|---|---|
| CAS-Nr. | $R^1$ | a | $Ar^1$ | $Ar^2$ |
| 36147-17-8 | Me | 2 | Ph | 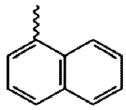 |
| 21591-48-0 | Me | 2 | 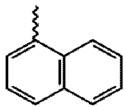 | 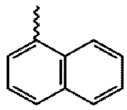 |
| 144677-99-6 | Me | 2 | 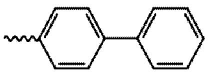 | 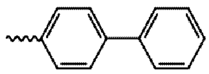 |

Darüber hinaus lassen sich die Silane $(R^1O)_aR^2_bSiAr_c$ auch durch katalysierte Umsetzung von Arylchlorsilanen oder Arylsilanen mit Alkoholen darstellen. Beispielhaft ist hier die Synthese von Trimethoxyphenylsilan (99) durch Umsetzung von Trichlorphenylsilan oder von Phenylsilan mit Methanol dargestellt.

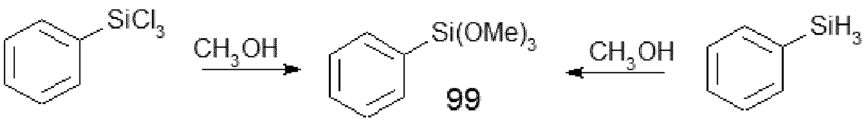

99

WO 2012/091154 A1 offenbart die Lewissäure-katalysierte Synthese von Arylalkoxysilanen durch Umsetzung von Arylchlorsilanen mit Ethern. Als Katalysator wird z.B. Bismuth(III)chlorid eingesetzt.

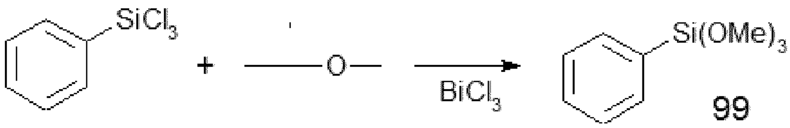

99

Geeignete kommerziell erhältliche Chlorsilane sind in Tabellen 19 und 20 aufgeführt.

Tabelle 19:

| | $Cl_aR^2_bSiAr$ | | | |
|---|---|---|---|---|
| CAS-Nr. | $R^2$ | a | b | Ar |
| 98-13-5 | - | 3 | 0 | Ph |
| 149-74-6 | Me | 2 | 1 | Ph |
| 1125-27-5 | Et | 2 | 1 | Ph |
| 790234-74-1 | $^i$Pr | 2 | 1 | Ph |
| 17887-41-1 | $^t$Bu | 2 | 1 | Ph |
| 701-35-9 | - | 3 | 0 | 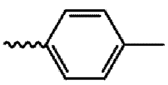 |
| 18236-57-2 | Me | 2 | 1 | 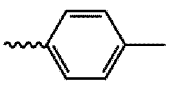 |

(fortgesetzt)

| | $Cl_aR^2_bSiAr$ | | | |
|---|---|---|---|---|
| CAS-Nr. | $R^2$ | a | b | Ar |
| 20083-38-9 | - | 3 | 0 | 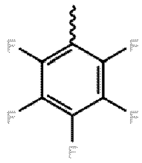 |
| 21980-43-8 | Me | 2 | 1 | 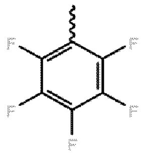 |
| 18164-08-4 | - | 3 | 0 | 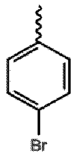 |
| 18141-19-0 | Me | 2 | 1 | 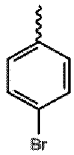 |
| 1521-07-9 | - | 3 | 0 | 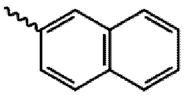 |
| 17998-61-7 | Me | 2 | 1 | 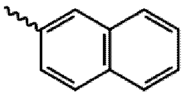 |
| 17995-31-2 | Et | 2 | 1 | 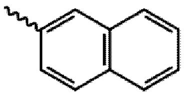 |
| 1521-08-0 | - | 3 | 0 | 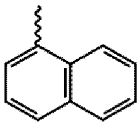 |
| 17998-62-8 | Me | 2 | 1 | 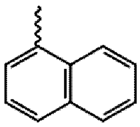 |
| 17950-78-6 | Et | 2 | 1 | 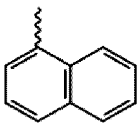 |
| 1187327-65-6 | - | 3 | 0 | 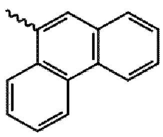 |

(fortgesetzt)

| | $Cl_aR^2_bSiAr$ | | | |
|---|---|---|---|---|
| **CAS-Nr.** | **$R^2$** | **a** | **b** | **Ar** |
| 136687-79-1 | - | 3 | 0 | 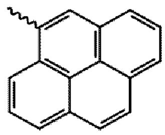 |
| 18030-61-0 | - | 3 | 0 | 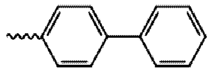 |
| 51840-45-0 | Me | 2 | 1 | 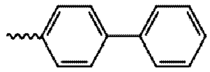 |
| 18557-48-7 | Et | 2 | 1 | 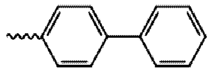 |

Tabelle 20:

| | $Cl_aSiA^1Ar^2$ | | |
|---|---|---|---|
| **CAS-Nr.** | **a** | **$Ar^1$** | **$Ar^2$** |
| 80-10-4 | 2 | Ph | Ph |
| 13788-41-5 | 2 | Ph | 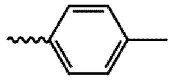 |
| 18414-38-5 | 2 | 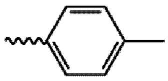 | 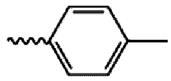 |
| 20160-53-6 | 2 | Ph | 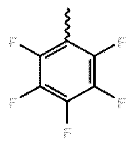 |
| 20160-45-6 | 2 | 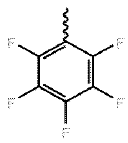 | 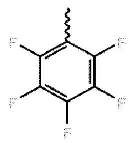 |
| 18030-58-5 | 2 | 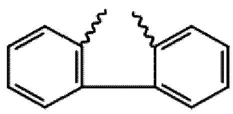 | |
| 7751-39-5 | 2 | Ph | 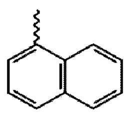 |
| 18557-48-7 | 2 | Ph | 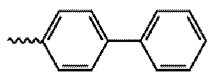 |

Im Folgenden werden beispielhaft einige Synthesen der Silane $(R^1O)_3SiAr$ und $(R^1O)_2SiAr^1Ar^2$ und dargestellt.

Die Bismuth(III)chlorid-katalysierte Umsetzung von 2-(Trichlorosilyl)naphthalen in tert-Butylmethylether liefert das 2-(Trimethoxysilyl)naphthalen (100).

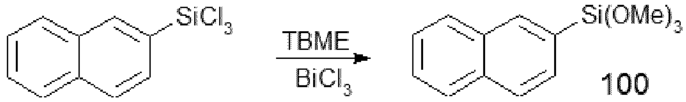


100

Die Bismuth(III)chlorid-katalysierte Umsetzung von 1-(Trichlorosilyl)naphthalen in tert-Butylmethylether liefert das 1-(Trimethoxysilyl)naphthalen (101).

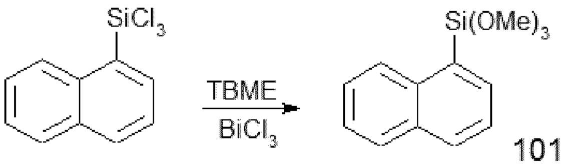


101

Die Bismuth(III)chlorid-katalysierte Umsetzung von 4-(Trichlorosilyl)-1,1'-biphenyl in tert-Butylmethylether liefert das 4-(Trimethoxysilyl)-1,1'-biphenyl (102).

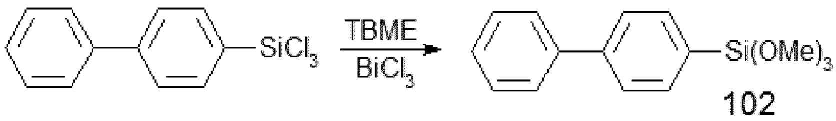


102

Die Bismuth(III)chlorid-katalysierte Umsetzung von 9,9-Dichloro-9*H*-9-silafluoren in tert-Butylmethylether liefert das 9,9-Dimethoxy-9*H*-9-silafluoren (103).

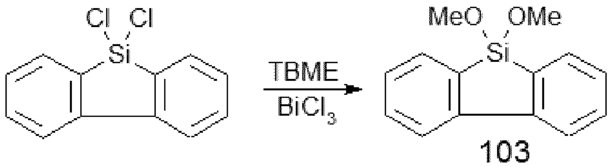


103

Die Bismuth(III)chlorid-katalysierte Umsetzung von 4-(Dichlorophenylsilyl)-1,1'-biphenyl in *tert*-Butylmethylether liefert das 4-(Dimethoxyphenylsilyl)-1,1'-biphenyl (104).

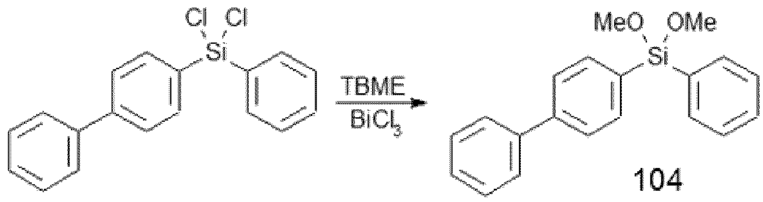


104

JP 2016-34912 A offenbart Synthesen von $(R^1O)_aR^2_bSiAr_c$ durch Umsetzung von Tetraalkoxysilanen mit Diarylmangan-Verbindungen. Durch geeignete Wahl der Reaktionsbedingungen lassen sich dabei entweder überwiegend die Monoarylsilane $(R^1O)_3SiAr$ oder die Diarylsilane $(R^1O)_2SiAr_2$ erhalten.

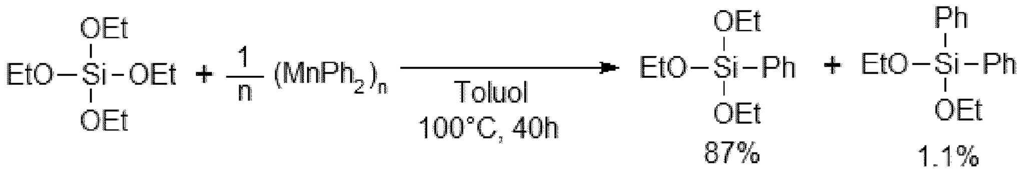


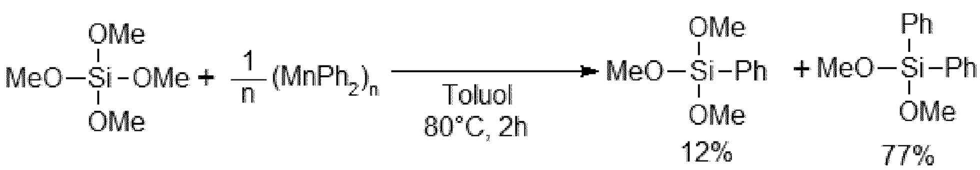

Insbesondere sind auch Arylreste bevorzugt, die ein oder mehrere Halogenatome tragen. Durch solche halogensubstituierten Arylreste lässt sich der Brechungsindex $n_A$ der Polysiloxane noch genauer im gewünschten Bereich einstellen.

Eine erfindungsgemäße polymerisierbare dentale Zusammensetzung enthält neben dem Bestandteil (A) die Komponente (B), einen Anteil an Füllstoffpartikeln von 0,3 bis 92 Gew.-%, bezogen auf die Gesamtmasse der erfindungsgemäßen polymerisierbaren Dentalzusammensetzung.

Je nach Indikation eines Dentalproduktes wird die Menge der Füllstofffraktion festgelegt. So werden für standfeste Füllungskomposite, für Dentalzusammensetzungen zur Herstellung von Inlays, Onlays oder Overlays sowie für Zusammensetzungen zur Herstellung von dentalen Blockmaterialien möglichst hohe Füllstoffmengen verwendet, in der Regel weisen diese Zusammensetzungen Füllstoffgehalte 80 Gew.-% bis zu 92 Gew.-%, bezogen auf die Gesamtzusammensetzung, auf. Fließfähige Dentalkomposite, Befestigungskomposite, Stumpfaufbaumaterialien, Kronen- und Brückenmaterialien weisen im Allgemeinen einen mittleren Füllstoffbereich von 50 bis 80 Gew.-%, bezogen auf die Gesamtzusammensetzung, auf, während dentale Lacke, dentale Versiegelungsmaterialien oder dentale Adhäsive Füllstoffe im Bereich von 0,3 bis 50 Gew.-%, bezogen auf die Gesamtzusammensetzung, enthalten.

Die oben angegebenen Füllstoffbereiche sind immer nur als Richtwerte anzusehen, denn es gibt auch spezielle polymerisierbare Zusammensetzungen, die beispielsweise größere Mengen an nanoskaligen Füllstoffen (20 Gew-%) verwenden, so dass sie mit einem Füllstoffgehalt von 70 Gew.-% bezogen auf die Gesamtzusammensetzung als Fissurenversiegler eingesetzt werden. Diese Versiegelungsmaterialien könnten auch als fließfähige Kompositmaterialien (Flow-Materialien) Verwendung finden.

<u>Bestandteil (B) - organische und/oder anorganische Füllstoffe.</u>

Organische Füllstoffpartikel umfassen oder bestehen aus beispielsweise ein oder mehr Verbindungen ausgewählt aus der Gruppe bestehend aus Polyvinylacetat und Copolymere des Polyvinylacetats mit einer oder mehreren polymerisierbaren Verbindungen, Polystyrol, Polyethylen, Polypropylen, Wachse wie Polyethylenwachs, Polybutylen, Polybutadien, Copolymere des Butadiens und des Styrols, Polyacrylnitril, Harze wie Kolophoniumharz oder Kohlenwasserstoffharze, Poly(meth)acrylatester, d.h. Umsetzungsprodukte von Poly(meth)-acrylsäure mit linearen oder verzweigten aliphatischen, aromatischen oder cycloaliphatischen Alkoholen wie Methanol, Ethanol, Propanol, Isopropanol, den isomeren Butanolen und höheren Homologen der genannten Alkohole mit bis zu 22 Kohlenstoffatomen, Cyclohexanol, Benzylalkohol und dergleichen, Polydialkylmaleinate wie Dibutylmaleinat und deren Copolymere und silylgruppenhaltige Polymere wie Polyvinylsilane oder Copolymere von Vinylsilan mit einem oder mehreren der genannten Monomeren. Die organischen Füllstoffe können alleine oder als Mischungen eingesetzt werden.

Die anorganischen Füllstoffe können ebenfalls allein oder als Mischungen eingesetzt werden. Zur Optimierung der Produkteigenschaften können die anorganischen Füllstoffe in unterschiedlichen Korngrößen in die Rezepturen eingebracht werden. Die Füllstoffe können eine unimodale oder polymodale, beispielsweise eine bimodale Verteilung aufweisen.

Als anorganische Füllstoffe können kompakte Gläser und unterschiedliche Kieselsäuren in verschiedenen Größen und Zuständen (monodispers, polydispers) zum Einsatz kommen.

Geeignete anorganische Bestandteile sind beispielsweise amorphe Materialien auf der Basis von Mischoxiden aus $SiO_2$, $ZrO_2$ und/oder $TiO_2$ sowie Füllstoffe wie Quarz-Glaskeramik oder Glaspulver, Bariumsilikatgläser, Bariumfluorsilikatgläser, Strontiumsilikatgläser, Strontiumborsilikat, Li/Al-Silikatgläser, Bariumgläser, Calciumsilikate, Natriumaluminiumsilikate, Fluoraluminiumsilikatgläser, Oxide von Aluminium oder Silicium, Zeolithe, Apatit, Zirkonsilikate, schwerlösliche Metallsalze wie Bariumsulfat oder Calciumfluorid sowie röntgenopake Füllstoffe wie Ytterbiumfluorid.

Zum besseren Einbau in die Polymermatrix können die Füllstoffe organisch oberflächenmodifiziert sein. Beispielhaft genannt sei die Oberflächenbehandlung der Füllstoffe mit einem Silan. Als Haftvermittler eignet sich besonders das Methacryloxypropyltrimethoxysilan.

Zur Einstellung der Rheologie können die polymerisierbaren dentalen Zusammensetzungen unterschiedliche Kieselsäuren, bevorzugt pyrogene Kieselsäuren, enthalten.

Bevorzugt enthalten die erfindungsgemäßen polymerisierbaren Zusammensetzungen nanoskalige Feststoffteilchen. Bei den nanoskaligen Feststoffteilchen handelt es sich um Partikel mit einer mittleren Teilchengröße von nicht mehr als 200 nm, bevorzugt nicht mehr als 100 nm und insbesondere nicht mehr als 70 nm. Die nanoskaligen anorganischen Feststoffteilchen sind bevorzugt solche von Oxiden, Sulfiden, Seleniden und Telluriden von Metallen, Mischmetallen und deren Mischungen. Besonders bevorzugt sind nanoskalige Teilchen von $SiO_2$, $TiO_2$, $ZrO_2$, ZnO, $SnO_2$ und $Al_2O_3$ und deren Mischungen. Die Herstellung der nanoskaligen Feststoffteilchen erfolgt auf bekannte Weise, z.B. durch Flammpyrolyse, Plasmaverfahren, Gasphasenkondensation, Kolloidtechniken, Präzipitationsverfahren, Sol-Gel Verfahren, etc.

In einer bevorzugten Ausgestaltung liegen die nanoskaligen Teilchen in nicht agglomerierter und/oder nicht aggregierter Form vor, beispielsweise dispergiert in einem Medium, vorzugsweise in monodisperser Form.

Um eine gute Einbindung der Nanopartikel in die Polymermatrix einer erfindungsgemäßen polymerisierbaren dentalen Zusammensetzung zu ermöglichen, sind die Oberflächen der Nanopartikel ebenfalls organisch oberflächenmodifiziert, d.h. ihre Oberflächen weisen organische Strukturelemente auf. Beispielhaft genannt sei die Oberflächenbehandlung der Füllstoffe mit einem Silan. Als Haftvermittler eignet sich auch hier besonders das Methacryloxypropyltrimethoxysilan.

In einer weiteren bevorzugten Ausgestaltung sind die nanoskaligen Teilchen somit nicht agglomerierte und/oder nicht aggregierte, organisch oberflächenmodifizierte Nanopartikel mit einer mittleren Partikelgröße kleiner 200 nm, vorzugsweise kleiner 100 nm, besonders bevorzugt kleiner 70 nm, die wiederum bevorzugt silanisiert sind.

Kommerziell erhältliche nanoskalige, nicht-agglomerierte und nicht aggregierte Kieselsole, die erfindungsgemäß eingesetzt werden können, sind beispielsweise unter der Bezeichnung "NALCO COLLOIDAL SILICAS" (Nalco Chemical Co.), "Ludox colloidal silica" (Grace) oder "Highlink OG" (Clariant) im Handel.

In einer bevorzugten Ausgestaltung umfasst der Füllstoffanteil einer erfindungsgemäßen polymerisierbaren dentalen Zusammensetzung eine Mischung eines ersten Füllstoffs (b1) in Form nicht agglomerierter, nicht aggregierter organisch oberflächenmodifizierter Nanopartikel mit einer mittleren Partikelgröße kleiner 200 nm und eines zweiten Füllstoffs (b2) in Form von makroskopischen Mikropartikeln mit einer mittleren Partikelgröße im Bereich von 0,4 $\mu$m bis 10 $\mu$m. Durch die Kombination von (b1) Nanopartikeln und (b2) Mikropartikeln in einer erfindungsgemäßen polymerisierbaren dentalen Zusammensetzung wird eine vollständige und gleichmäßige Volumenfüllung des Kompositmaterials erzielt. Dadurch wird sowohl die Schrumpfung der polymerisierbaren Zusammensetzung beim Aushärten der Polymermatrix als auch die Empfindlichkeit der erfindungsgemäßen Zusammensetzung gegen Abrasion vermindert.

Der Anteil organisch oberflächenmodifizierter Nanopartikel in einer bevorzugten erfindungsgemäßen polymerisierbaren dentalen Zusammensetzung mit einer mittleren Partikelgröße kleiner 200 nm ist größer als 1 Gew.-%, vorzugsweise größer als 2 Gew.-% und besonders bevorzugt größer als 3 Gew.-%. In eigenen Untersuchungen hat sich gezeigt, dass bei einem Gehalt von 1 Gew.- % oder weniger an nicht agglomerierten und/oder nicht aggregierten, organisch oberflächenmodifizierten Nanopartikeln mit einer mittleren Partikelgröße kleiner 200 nm die radikalisch härtbare dentale Zusammensetzung im Einzelfall nicht mehr ausreichend abriebfest ist. Dies ist wahrscheinlich u.a. darauf zurückzuführen, dass bei einem Gehalt von 1 Gew.- % oder weniger an den besagten Nanopartikeln die Bereiche zwischen den Mikropartikeln mit einer mittleren Partikelgröße von 0,4 $\mu$m bis 10 $\mu$m nicht mehr ausreichend gefüllt sind. Andererseits hat sich gezeigt, dass bei einem Gehalt von mehr als 20 Gew.- % an nicht agglomerierten und/oder aggregierten, organisch oberflächenmodifizierten Nanopartikeln mit einer mittleren Partikelgröße kleiner 200 nm die Verarbeitbarkeit der Zusammensetzung nicht mehr ausreichend ist. Aufgrund des hohen Feststoffgehalts wird dann seine Viskosität zu hoch.

Die Materialien für die erfindungsgemäß einzusetzenden Nanopartikel sind vorzugsweise Oxide oder Mischoxide und bevorzugt ausgewählt aus der Gruppe bestehend aus Oxiden und Mischoxiden der Elemente Silizium, Titan, Yttrium, Strontium, Barium, Zirkonium, Hafnium, Niob, Tantal, Wolfram, Bismut, Molybdän, Zinn, Zink, Ytterbium, Lanthan, Cer, Aluminium und deren Mischungen. Die bevorzugten oxidischen Nanopartikel sind dabei, wie dargelegt, nicht agglomeriert und/oder nicht aggregiert sowie organisch oberflächenbehandelt.

Innerhalb einer erfindungsgemäßen polymerisierbaren dentalen Zusammensetzung bewirken die Mikropartikel eine weitgehende gleichmäßige Füllung des Volumens, wobei die verbleibenden Hohlräume zwischen den Mikropartikeln durch die oben beschriebenen Nanopartikel (Komponente (b1)) zumindest teilweise gefüllt werden. Unter Mikropartikeln werden in Zusammenhang mit der vorliegenden Erfindung Partikel mit einer mittleren Partikelgröße von 400 nm bis 10 $\mu$m verstanden. Vorzugsweise ist die mittlere Partikelgröße kleiner als 5 $\mu$m. Es hat sich gezeigt, dass die mit den Mikropartikeln bereits erreichbare Volumenfüllung der polymerisierbaren dentalen Zusammensetzung umso vollständiger und gleichmäßiger ist, je kleiner die Mikropartikel sind.

Die Mikropartikel der Komponente (b2) können eine monomodale oder polymodale, beispielsweise eine bimodale Partikelgrößenverteilung aufweisen. Mikropartikel mit einer bimodalen oder multimodalen Partikelgrößenverteilung sind erfindungsgemäß bevorzugt, da mit ihnen eine vollständigere Volumenfüllung erreichbar ist als bei allgemeiner Verwendung von Mikropartikeln mit monomodaler Partikelgrößenverteilung. Bei Vorliegen einer bi- oder multimodalen Partikelgrößenverteilung bewirken die Partikel der Fraktionen mit der größeren Partikelgröße eine grobe Ausfüllung des Volumens, während die Partikel der Fraktion mit der kleineren Partikelgröße soweit möglich die Bereiche zwischen den Partikeln der Fraktionen mit der größeren Partikelgröße ausfüllen werden. Die noch verbleibenden Hohlräume werden wie oben beschrieben durch Nanopartikel gefüllt.

Ganz besonders bevorzugt wird somit in einer erfindungsgemäßen polymerisierbaren dentalen Zusammensetzung eine Komponente (b2) eingesetzt, welche zwei oder mehr Fraktionen von Mikropartikeln enthält, wobei sich die mittleren Partikelgrößen der Fraktionen unterscheiden.

Vorzugsweise enthält Komponente (b2) zumindest zwei Mikropartikel-Fraktionen, wobei deren mittlere Partikelgrößen um mindestens 0,5 $\mu$m, bevorzugt um mindestens 0,7 $\mu$m, voneinander abweichen. In manchen Ausgestaltungen beträgt die Differenz der mittleren Partikelgrößen der Mikropartikel-Fraktionen mindestens 1,0 $\mu$m.

Die Mikropartikel verschiedener Fraktionen können aus dem gleichen oder aus verschiedenen Materialien

bestehen; es können dabei auch mehrere Fraktionen von Mikropartikeln vorliegen, deren mittlere Partikelgröße annähernd gleich ist oder in einem bestimmten Bereich liegt, wobei die Materialien der Partikel sich zwischen den Fraktionen unterscheiden.

Besonders bevorzugt umfasst eine erfindungsgemäße polymerisierbare dentale Zusammensetzung eine Komponente (b2), welche eine oder mehrere erste Mikropartikelfraktionen, die jeweils eine mittlere Partikelgröße im Bereich von 1 µm bis 10 µm, vorzugsweise 1 µm bis 5 µm, besitzen, und eine oder mehrere zweite Mikropartikelfraktionen, die jeweils eine mittlere Partikelgröße im Bereich von > 0,4 µm bis < 1 µm (d.h. größer als 0,4 µm, aber kleiner als 1 µm), vorzugsweise 0,5 µm bis 0,8 µm, besitzen.

Bevorzugt liegt das Verhältnis der Gesamtmasse der ersten Mikropartikelfraktionen zur Gesamtmasse der zweiten Mikropartikelfraktionen im Bereich von 1 : 1 bis 12 : 1, vorzugsweise im Bereich von 1,5 : 1 bis 8 : 1.

Bevorzugt liegt das Verhältnis der mittleren Korngröße der oder einer ersten Mikropartikelfraktion zur mittleren Korngröße der oder einer zweiten Mikropartikelfraktion der Komponente (b2) im Bereich von 1,5 : 1 bis 10 : 1, vorzugsweise im Bereich von 2 : 1 bis 5 : 1.

In einer besonders bevorzugten erfindungsgemäßen polymerisierbaren dentalen Zusammensetzung umfasst die Komponente (b2) eine oder mehrere erste Mikropartikelfraktionen, die jeweils eine mittlere Partikelgröße im Bereich von 1 µm bis 10 µm, vorzugsweise 1 µm bis 5 µm, besitzen, und eine oder mehrere zweite Mikropartikelfraktionen, die jeweils eine mittlere Partikelgröße im Bereich von > 0,4 µm bis < 1 µm, vorzugsweise 0,5 µm bis 0,8 µm, besitzen; wobei das Verhältnis der Gesamtmasse der ersten Mikropartikelfraktionen zur Gesamtmasse der zweiten Mikropartikelfraktionen im Bereich von 1 : 1 bis 12 : 1, vorzugsweise 1,5 : 1 bis 8 : 1 und/oder das Verhältnis der mittleren Korngröße der oder einer ersten Mikropartikelfraktion zur mittleren Korngröße der oder einer zweiten Mikropartikelfraktion der Komponente (b2) im Bereich von 1,5 : 1 bis 10 : 1, vorzugsweise 2 : 1 bis 5 : 1 liegt.

In einer besonders bevorzugten erfindungsgemäßen polymerisierbaren dentalen Zusammensetzung wird zumindest ein Teil der Mikropartikel der Komponente (b2) durch organisch oberflächenmodifizierte Partikel, vorzugsweise silanisierte Partikel gebildet und/oder es wird zumindest ein Teil der Mikropartikel der Komponente (b2) durch Dentalglas-Partikel gebildet; vorzugsweise sind zumindest ein Teil der Mikropartikel der Komponente (b2) organisch oberflächenmodifizierte Dentalglas-Partikel, vorzugsweise silanisierte Dentalglas-Partikel.

Bevorzugt zeichnet sich in diesen Fällen Komponente (b2) durch eine bi- oder multimodale Partikelgrößenverteilung aus, insbesondere eine bi- oder multimodale Partikelgrößenverteilung mit den oben beschriebenen bevorzugten Merkmalen.

Neben den Komponenten (b1) und (b2) kann die polymerisierbare dentale Zusammensetzung zu der Mischung von Füllstoffpartikeln zusätzlich weitere Füllstoffe als Komponente (b3) umfassen.

So können z.B. verstärkend wirkende Füllstoff-Materialien, wie Glasfasern, Polyamid- oder Kohlenstofffasern eingesetzt werden. Eine erfindungsgemäße polymerisierbare dentale Zusammensetzung kann auch feinteilige Splitter- oder Perlpolymerisate enthalten, wobei die Perlpolymerisate Homo- oder Copolymere organischer härtbarer Monomerer sein können.

In einer besonders bevorzugten Ausführungsform enthält eine erfindungsgemäße polymerisierbare dentale Zusammensetzung einen röntgenopaken Füllstoff. Ganz besonders bevorzugt enthält die erfindungsgemäße Zusammensetzung nanoskaliges $YbF_3$ und/oder $BaSO_4$.

Es hat sich in eigenen Untersuchungen gezeigt, dass ein Brechungsindex $n_A$ der Gesamtmenge aus den polymerisierbaren Monomeren (A) und (D) im Bereich von 1,45 bis 1,55 sehr häufig zu sehr guten Transluzenzwerten führt, da die Brechungsindices der ein, zwei, drei oder mehr als drei Füllstoffe, welche die Gesamtmenge (B) der Füllstoffe im härtbaren Dentalmaterial bilden, vergleichsweise einfach auf einen solchen Brechungsindex $n_A$ abgestimmt werden können. Diese gute Abstimmung hat zur Folge, dass bei lichtinduzierter Polymerisation von polymerisierbaren Monomeren im härtbaren Dentalmaterial das eingesetzte Licht eine hohe Durchdringungstiefe erreicht und somit eine gleichmäßige Polymerisation bewirkt. Dies führt zu einem qualitativ hochwertigen, gehärteten Dentalmaterial, mit sehr guten mechanischen Eigenschaften.

Sofern der Brechungsindex $n_A$ kleiner 1,45 oder größer 1,55 ist, wird die vorstehend genannte Abstimmung in vielen Fällen komplexer (insbesondere bei einem Brechungsindex kleiner 1,45) und die Durchdringungstiefe des Lichtes ist nur noch in wenigen Fällen akzeptabel bzw. kaum noch akzeptabel (da in den meisten Fällen eine besonders intensive Lichtstreuung zu erwarten ist). Dies erhöht das Risiko, dass aus einem (nur teil-)gehärteten Dentalmaterial nichtpolymerisierte polymerisierbare Monomere austreten und in den Mundraum wandern. Zusätzlich besitzen derartige (teil-) gehärtete Dentalmaterialien eine erheblich reduzierte Festigkeit. Solche härtbaren Dentalmaterialien müssen dann aufwendig in sehr dünnen Schichten aufgetragen und jede Schicht einzeln gehärtet werden, um die vorstehend genannten Nachteile zu minimieren. Sofern der Brechungsindex größer 1,45 aber kleiner 1,48 ist, wird in den meisten Fällen eine ausreichend gute Abstimmung erreicht, so dass eine ausreichend gute Durchdringungstiefe des Lichtes resultiert. Eine Applikation eines solchen härtbaren Dentalmaterials (und die anschließende Härtung) kann dann entsprechend ohne großen Aufwand erfolgen. Liegt der Brechungsindex in einem Bereich von 1,48 bis 1,55 werden regelmäßig sehr gute Ergebnisse bei der üblichen Verwendung von röntgenopaken Dentalgläsern erreicht.

Besonders bevorzugt ist ein erfindungsgemäßes härtbares Dentalmaterial (wie vorstehend beschrieben, vorzugsweise wie vorstehend als bevorzugt definiert), wobei die Gesamtmenge der partikulären Füllstoffe (B) einen Brechungsindex $n_B$ im Bereich von 1,50 bis 1,55 besitzt.

Insbesondere bevorzugt ist ein erfindungsgemäßes härtbares Dentalmaterial (wie vorstehend beschrieben, vorzugsweise wie vorstehend als bevorzugt definiert), wobei der Betrag der Differenz $|n_A - n_B|$ zwischen dem Brechungsindex der Gesamtmenge der polymerisierbaren Monomere ( (A) und (D) ) $n_A$ und dem Brechungsindex der Gesamtmenge der Füllstoffe $n_B$ kleiner als 0,05, bevorzugt kleiner 0,03, besonders bevorzugt kleiner als 0,02 und ganz besonders bevorzugt kleiner als 0,01 ist. Solche Dentalmaterialien weisen in besonderem Maße die beschriebene hohe Transluzenz und damit verbunden gute Durchhärtungseigenschaften auf.

Während der Polymerisation erhöht sich die Dichte der Harzmatrix durch die auftretende Schrumpfung. Diese Erhöhung der Dichte führt auch zu einer Erhöhung des Brechungsindexes während der Polymerisation. Demgegenüber ändert sich der Brechungsindex der Füllstoffe während der Polymerisation nicht. Dies kann dazu führen, dass Transluzenz und Durchhärtungseigenschaften während der Polymerisation abnehmen. Die erfindungsgemäßen Zusammensetzungen zeichnen sich durch eine sehr geringe Schrumpfung aus, so dass nur eine geringe Änderung der Dichte und des Brechungsindexes während der Polymerisation erfolgt und damit auch Transluzenz und Durchhärtungseigenschaften sich während der Polymerisation nur geringfügig ändern, so dass eine zuverlässige Aushärtung gewährleistet ist.

Bevorzugt ist daher ein erfindungsgemäßes härtbares Dentalmaterial (wie vorstehend beschrieben, vorzugsweise wie vorstehend als bevorzugt definiert), bei dem die Differenz $n_P - n_A$ des Brechungsindexes $n_P$ der polymerisierten Harzmatrix (aus (A) und (D)) und des Brechungsindexes $n_A$ der Gesamtmenge der polymerisierbaren Monomere (A) und (D) vor der Polymerisation kleiner als 0,03 und bevorzugt kleiner als 0,02 ist.

Qualitative und quantitative Charakterisierung der Füllstoffpartikel:
Die nachfolgend beschriebenen Schritte in der qualitativen und quantitativen Charakterisierung der Füllstoffpartikel (insbesondere von nanoskaligen Füllstoffpartikeln) sind dem Fachmann gut bekannt und in der Literatur umfassend beschrieben.

Harz-/Füllstofftrennung:
In einem ersten Schritt werden 1 g einer erfindungsgemäßen polymerisierbaren dentalen Zusammensetzung (nachfolgend auch Kompositmaterial genannt) in 10 ml Aceton suspendiert und die erhaltene Suspension anschließend mit einer Zentrifuge für 10 min bei 5000 U/min zentrifugiert. Der Überstand (nachfolgend Harzphase genannt) wird in ein Sammelglas abdekantiert und der Rückstand in 5 ml Aceton aufgeschlämmt. Es wird erneut für 10 min bei 5000 U/min zentrifugiert, dekantiert und der Rückstand in 5 ml Aceton erneut aufgeschlämmt. Die Schritte Zentrifugieren, Dekantieren und Aufschlämmen werden noch zweimal unter identischen Bedingungen wiederholt. Die von den Harzphasen getrennte Gesamtmenge an Rückständen wird getrocknet und die Gesamtmenge an Harzphasen am Rotationsverdampfer von Aceton befreit.

Nach Durchführung des ersten Schrittes umfasst die getrocknete Gesamtmenge an Rückständen regelmäßig solche Füllstoffpartikel, die eine Partikelgröße von ca. 400 nm oder größer 400 nm besitzen (nachfolgend makroskopische Füllstoffpartikel genannt). Die von Aceton befreite Gesamtmenge an Harzphasen (nachfolgend Harzanteil genannt) umfasst neben polymerisierbaren Monomeren außerdem regelmäßig Füllstoffpartikel mit einer Partikelgröße von ca. 400 nm oder insbesondere kleiner 400 nm (nachfolgend nanoskalige Teilchen genannt). Dieses Verfahren stellt somit sicher, dass das dentale Kompositmaterial durch die Zentrifugation in (i) einen Anteil makroskopischer Füllstoffpartikel, wobei speziell die dentalen Gläser im Größenordnungsbereich von größer 400 nm bis in den hohen Mikrometerbereich betroffen sind, und (ii) einen Harzanteil umfassend nanoskaligen Teilchen vollständig aufgetrennt wird.

Die mittlere Partikelgröße $d_{50}$ der erfindungsgemäß einzusetzenden makroskopischen Füllstoffpartikel der Füllstoffkomponente (b2) einer erfindungsgemäßen Zusammensetzung wird mittels Lichtstreuung (Laserbeugung) bestimmt, vorzugsweise mit einem Partikelgrößenmessgerät Beckman Coulter LS 13320.

Die nanoskaligen Teilchen, die sich in dem Harzanteil befinden, können beispielsweise sowohl nicht-aggregierte und/oder nicht agglomerierte, beispielsweise auch röntgenopake Partikel sein, beispielsweise $YbF_3$ oder $BaSO_4$ mit Partikelgrößen in einem Bereich von ca. 3 nm bis 200 nm aufweisen, als auch nicht-röntgenopake Kieselsäuren, die beispielsweise als pyrogene Kieselsäuren in Form von Aggregaten und/oder Agglomeraten mit einer Partikelgröße in einem Bereich von ca. 150 nm bis ca. 300 nm vorliegen oder auch Kieselsäuren, die nach dem Sol-Gel Verfahren (oder auch aus Wasserglas) synthetisiert werden und die ebenfalls nicht-aggregiert und/oder nichtagglomeriert vorliegen und Partikelgrößen in einem Bereich von ca. 3 nm bis 200 nm aufweisen.

Der Gesamtmassenanteil anorganischer Partikel in dem Harzanteil wird durch Differenzwiegung nach Veraschung eines entsprechenden Harzanteils gravimetrisch bestimmt.

TEM in Kombination mit EELS:
In einem zweiten Schritt werden die Füllstoffpartikel in dem Harzanteil einer qualitativen und quantitativen Charakterisierung unterzogen. Hierzu wird TEM (Transmissionselektronenmikroskopie) in Verbindung mit EELS (Elektronenenergieverlustspektroskopie) eingesetzt.

Mittels TEM werden die Partikelgrößen der einzelnen Partikel sowie deren Anzahl ermittelt; eine Elementarbestimmung einzelner Partikel erfolgt mittels EELS.

Zur Durchführung der kombinierten TEM/EELS-Charakterisierung wird in einem ersten Schritt durch Verdünnung mit polymerisierbarem Harz die Konzentration der nanoskaligen Teilchen im Harzanteil zunächst reduziert. Dadurch wird weitestgehend ausgeschlossen, dass eine "Überlagerung" von nanoskaligen Teilchen in den späteren Bildern beobachtet wird. Eine solche "Überlagerung" würde die Partikelcharakterisierung verfälschen. Eigene Untersuchungen haben gezeigt, dass die optimale Partikelkonzentration (d.h. der Volumenanteil der Füllstoffpartikel) für solche Untersuchungen bei 1 Vol. % liegt, bezogen auf die Gesamtmasse der verdünnten Probe.

Aus den durch Verdünnung mit polymerisierbarem Harz gewonnenen verdünnten Harzanteilen werden in einem zweiten Schritt durch Aushärtung Stäbchen hergestellt. Aus diesen Stäbchen werden anschließend mit einem Ultradiamantmesser (beispielsweise Ultramikrotom ULTRCAT UCT, LEICA, Wetzlar) mehrere 300 nm dünne Ultradünnschnitte angefertigt. Die Ultradünnschnitte werden zur Stabilisierung auf Kupfer-TEM-Grids transferiert. Es resultieren Dünnschnittpräparate. Diese Dünnschnittpräparate werden dann mit 120 kV Beschleunigungsspannung in einem TEM mittels Hellfeld-Abbildungen untersucht.

Eine TEM-Untersuchung an den vorstehend beschriebenen Dünnschichtpräparaten erlaubt es, nicht aggregierte und nicht agglomerierte nanoskalige Partikel von aggregierten und/oder agglomerierten Partikeln (z.B. Kieselsäuren, wie beispielsweise Aerosilen), zu unterscheiden (zur Identifizierung der chemischen Zusammensetzung siehe die nachfolgenden Ausführungen).

Sofern hochauflösende Abbildungen untersucht werden sollen, können Ultradünnschnitte mit Schichtdicken kleiner 100 nm hergestellt und untersucht werden.

In einem dritten Schritt werden die Füllstoffpartikel in den Ultradünnschnitten bzw. Dünnschnittpräparaten mittels EELS-Punktanalysen chemisch charakterisiert, so dass die chemische Zusammensetzung einzelner Partikel bekannt wird (zur Bestimmung der Oberflächenmodifikation von Partikeln siehe nachfolgende Punkte).

Die volumen- oder gewichtsbezogenen Anteile von (ggf. auch mehrerer) Partikelfraktionen werden in einem vierten Schritt wie folgt aus einer TEM Aufnahme ermittelt: Der im Mikroskop betrachtete Bildausschnitt einer TEM-Aufnahme stellt eine Fläche dar, deren Kantenlängen a und b mittels der Legende bestimmt werden. Multipliziert mit der Dicke c des Ultradünnschnittes ergibt sich ein Gesamtvolumen $V_{Gesamt}$ für den in der TEM betrachteten Bereich. Dieses Gesamtvolumen $V_{Gesamt}$ ist die Summe aus dem Harzvolumen $V_{Harz}$ und dem Volumen aller Partikel $V_{Partikel}$ innerhalb dieses Volumens (das Volumen aller Partikel umfasst ggf. mehrere Gruppen von Partikeln, z.B. sortiert nach verschiedenen Kriterien wie z.B. der Größe). Es gilt $V_{Gesamt} = a * b * c = V_{Harz} + V_{Partikel}$.

Das Volumen einzelner Partikel (und damit das Volumen aller Partikel in dem betrachteten Volumen) ist rechnerisch zugänglich über das Kugelvolumen der einzelnen Partikel. Hierzu wird in der TEM-Aufnahme der Durchmesser bzw. Radius eines entsprechenden Partikels bestimmt. Das daraus errechnete Kugelvolumen, multipliziert mit der Dichte des entsprechenden Materials, aus dem das Partikel besteht (Material identifizierbar mittels EELS), ergibt die Masse des Partikels. Das Harzvolumen, zugänglich aus dem Gesamtvolumen minus dem Partikelvolumen, multipliziert mit der Harzdichte, ergibt die Harzmasse. Die Harzdichte ergibt sich weitestgehend aus der Dichte des zur Verdünnung eingesetzten Harzes und ggf. der Dichte des verdünnten Harzanteils (letztere kann ggf. bei der Berechnung der Harzdichte vernachlässigt werden, wenn der Anteil des verdünnten Harzes vernachlässigbar ist). Der Anteil der Partikel (oder einer Gruppe von Partikeln) in Gewichtsprozent errechnet sich aus $m_P*100/(m_{Partikel}+m_{Harz})$, wobei $m_P$ die Masse der betrachteten Partikelfraktion in dem betrachteten Volumen, $m_{Partikel}$ die Masse aller Partikel im betrachteten Volumen und $m_{Harz}$ die Masse des Harzes in dem betrachteten Volumen bedeutet. Bei der abschließenden Berechnung des Gewichtsanteils der zu betrachtenden Partikelfraktion wird der Verdünnungsfaktor entsprechend berücksichtigt.

Bestimmung organischer Oberflächenmodifikationen:

Vorabbetrachtung:

Viele bekannte röntgenopake Füllstoffmaterialien (wie z.B. Ytterbiumfluorid oder Bariumsulfat) weisen den Nachteil auf, dass sie nur schwer in die Matrix (Harzmatrix) aus polymerisierbaren Monomeren (die sogenannte organische Harzphase) einzuarbeiten sind, weil sie keine hinreichenden chemischen Bindungen (Anbindungsmöglichkeiten) mit den hydrophoben Gruppen des Mediums eingehen. Glasartige Füllstoffe lassen sich beispielsweise mit Hilfe der Silanisierung über Si-OH Gruppen hervorragend in die Harzmatrix dentaler Kompositmaterialien einarbeiten. Bei Ytterbiumfluorid und Bariumsulfat sind solche Gruppen auf den Oberflächen nicht vorhanden; sie sind somit nicht silanisierbar und führen in einem gehärteten Dentalmaterial zu einer unzureichenden physikalischen und chemischen Resistenz (siehe hierzu WO 2005/011621 A1, Seite 2 unten).

Die in einem erfindungsgemäßen härtbaren Dentalmaterial eingesetzten röntgenopaken nanoskaligen Teilchen werden somit auf ihren Oberflächen keine Silane aufweisen. Vielmehr erfolgt die Verknüpfung über Stickstoff-, Sauerstoff-, Schwefel- und/oder Phosphoratomen (siehe hierzu wiederum WO 2005/011621 A1 sowie unsere Ausführungen weiter oben im Text).

Abtrennung polymerisierbarer Monomere von nanoskaligen Teilchen:

"Cross-Flow"-Verfahren:

Die Abtrennung polymerisierbarer Monomere von nanoskaligen Teilchen erfolgt beispielsweise in einem dem Fachmann

bekannten "Cross-Flow"-Verfahren mittels Ultrafiltrationsmembranen.

Hierbei wird ein Harzanteil enthaltend nanoskalige Teilchen, polymerisierbare Monomere und ggf. ein geeignetes Verdünnungsmittel aus einem Behältnis mittels einer Pumpe in einen Kreislauf aus bestimmten Membranen gepumpt, wobei die polymerisierbaren Monomere die Poren der Membranen passieren und als Filtrat separiert werden, während die nanoskaligen Teilchen innerhalb des Kreislaufs (und damit im Behältnis) verbleiben.

Für diesen Trennungsgang ist beispielsweise das System "Vivaflow 50" von "Sartorius Stedim Biotech GmbH, Göttingen" geeignet. Pumpenantrieb (7554-95) und Pumpenkopf entstammen der Reihe "Masterflex L/S" von "Cole-Palmer Instrument Co.", Illinois, USA. Der Betrieb der Pumpe wird während der Filtration auf 2,5 bar eingestellt. Zwei Trennmembranen des Typs "50,000 MWCO (PES)" werden hintereinander geschaltet. Der MWCO (Molecular Weight Cut Off) gibt hierbei die Trenngrenze an, d.h. die Größe der Moleküle, die noch effizient die Membran passieren können. Dieser Wert wird in Dalton angegeben. Die erhaltenen Fraktionen werden anschließend, wie unten beschrieben, untersucht.

Sedimentations-Feld-Fluss-Fraktionierung (SF3):
Besser noch als das "Cross-Flow"-Verfahren ist die Durchführung einer Sedimentations-Feld-Fluss-Fraktionierung (SF3). Dabei lassen sich insbesondere unterschiedliche Partikelfraktionen voneinander und zusätzlich vom Harzanteil trennen. Voraussetzung hierbei ist, dass sich die unterschiedlichen Partikelfraktionen ausreichend in Größe und/oder Dichte voneinander unterscheiden.

Entsprechende Geräte, die eine hierfür notwendige Trennsäule enthalten, sind bei der Firma Postnova Analytics GmbH, Landsberg, erhältlich. Das die Trennsäule enthaltende Modul trägt die Bezeichnung CF2000 Centrifugal FFF und wird durch die weiteren Module PN7140 (Eluent Organizer), PN1130 (Isocratic Pump), PN5300 (Autosampler), PN3621 MALS (21-Multi-Angle Light Scattering Detector) und PN8050 (Fraction Collector) ergänzt. In dieser Kombination erlaubt die Centrifugal FFF-Anlage nicht nur die analytische, sondern auch die präparative Trennung von Partikelfraktionen. Die erhaltenen Fraktionen werden anschließen, wie unten beschrieben, untersucht.

Charakterisierung der Oberflächenmodifikation:
Eine wie oben hergestellte und anschließend von Lösungsmitteln befreite Probe, die nanoskalige Teilchen in Form eines Pulvers enthält, wird anschließend mittels spektroskopischer Verfahren untersucht (z.B. mittels $^{1}$H-NMR, $^{13}$C-NMR, $^{15}$N-NMR, $^{29}$Si-NMR und $^{31}$P-NMR sowie IR).

Signale, die sich nicht einem Silan, beispielsweise dem gamma-Methacryloxypropylsilylrest, zuordnen lassen, werden organischen Oberflächenmodifikationen zugeordnet, die nicht auf Silanen basieren, z.B. Oberflächenmodifikationen mittels organischer Verbindungen auf Oberflächen von Ytterbiumfluorid bzw. Bariumsulfatpartikeln.

Die Anteile organisch oberflächenmodifizierter Partikel bzw. nicht organisch oberflächenmodifizierter Partikel können regelmäßig auch durch Auswertung der Intensitäten entsprechender Schwingungsbanden im IR-Spektrum bestimmt werden. Hierzu werden üblicherweise Referenz-Schwingungsbanden (Referenzkurven) organisch oberflächenmodifizierter bzw. nicht organisch oberflächenmodifizierter Partikel mit den entsprechenden chemischen Zusammensetzungen herangezogen.

Charakterisierung mittels Bildanalyse und Ramanspektroskopie:
Dem Fachmann sind zusätzliche Verfahren bzw. Kopplungen von Verfahren bekannt, die eine qualitative und quantitative Charakterisierung der Füllstoffpartikel erlauben. Insoweit sei beispielsweise verwiesen auf den Artikel "Chemische Identität einzelner Partikel" von Deborah Huck-Jones und Renate Hessemann in "Nachrichten aus der Chemie", Volume 62, September 2014, Seiten 886 und 887. Die darin offenbarte Kombination von Bildanalyse und Ramanspektroskopie eignet sich regelmäßig auch zur Charakterisierung der Füllstoffpartikel im Rahmen der vorliegenden Erfindung. Dies gilt insbesondere für Proben, die nach der oben beschriebenen Harzfüllstofftrennung erhalten werden. Eine geeignete Bildanalyse ist z.B. auch die oben im Text beschriebene TEM-Analyse.

Bestandteil (C) - Initiatoren und/oder Katalysatoren und/oder Aktivatoren für die Polymerisation

Eine erfindungsgemäße polymerisierbare dentale Zusammensetzung ist vorzugsweise radikalisch lichthärtbar und/oder radikalisch chemisch härtbar oder kationisch härtbar.

Bevorzugt ist eine erfindungsgemäße polymerisierbare dentale Zusammensetzung, wobei Bestandteil (C) einen oder mehrere Lichthärtungsinitiatoren und/oder einen oder mehrere Initiatoren zur chemischen oder zur kationischen Polymerisation umfasst oder aus diesen besteht.

Bevorzugte erfindungsgemäße polymerisierbare dentale Zusammensetzungen sind lichthärtbar (photohärtbar) und umfassen Lichthärtungsinitiatoren. Beispiele für einen Lichthärtungsinitiator schließen Substanzen ein, die nur photosensibilisierend wirken sowie Kombinationen aus Sensibilisator und Beschleuniger.

Beispiele für Photosensibilisatoren sind alpha-Diketone, Benzoinalkylether, Thioxanthone, Benzophenone, Acylphosphinoxide, Acylgermanium-Verbindungen, Acetophenone, Ketale, Titanocene, sensibilisierende Farbstoffe, etc. Die Sensibilisatoren können alleine oder in Kombination angewendet werden. Konkrete Substanzbeispiele der unterschiedlichen Klassen finden sich beispielsweise in der DE 10 2006 019 092 A1, oder in der DE 39 41 629 C2.

Beispiele für Beschleuniger, die zusammen mit den Sensibilisatoren eingesetzt werden, sind tertiäre Amine, sekundäre Amine, Barbitursäuren, Zinnverbindungen, Aldehyde und Schwefelverbindungen. Konkrete Substanzbeispiele der unterschiedlichen Klassen finden sich in der DE 10 2006 019 092 A1 oder in der DE 39 41 629 C2.

Weitere geeignete Initiatoren sowie Initiatorkombinationen sind in der DE 601 16 142 T2 beschrieben.

Die im Rahmen der vorliegenden Erfindung verwendbaren Photoinitiatoren sind dadurch charakterisiert, dass sie durch Absorption von Licht im Wellenlängenbereich von 300 nm bis 700 nm, bevorzugt von 350 nm bis 600 nm und besonders bevorzugt von 380 nm bis 500 nm, gegebenenfalls in Kombination mit einem oder mehreren Coinitiatoren, die Aushärtung einer erfindungsgemäßen polymerisierbaren dentalen Zusammensetzung bewirken können.

Das Absorptionsmaximum von Campherchinon (CQ) liegt bei ca. 470 nm und somit im Bereich des blauen Lichts. Campherchinon (CQ) zählt zu den $PI_2$-Initiatoren und wird regelmäßig zusammen mit einem Coinitiator eingesetzt.

Vorzugsweise enthält ein erfindungsgemäßes Kompositmaterial die Kombination eines alpha-Diketons und eines aromatischen tertiären Amins, bevorzugt ist die Kombination von Campherchinon (CQ) und Ethyl-*p*-*N,N*-dimethylaminobenzoat (DABE).

Ebenfalls bevorzugt ist die weitere Kombination des Systems "alpha-Diketon/aromatisches tertiäres Amin" mit einem Phosphinoxid, insbesondere mit dem Phenyl-bis(2,4,6-trimethylbenzoyl)phosphinoxid und/oder dem 2,4,6-Trimethylbenzoyldiphenylphosphinoxid. Bezüglich der Strukturen geeigneter Phosphinoxide zum Einsatz in einer erfindungsgemäßen radikalisch härtbaren dentalen Zusammensetzung wird auf die Druckschriften DE 38 01 511 C2, DE 10 2006 050 153 A1, EP 0 184 095 B1, DE 42 31 579 C2, EP 0 366 977 B1, US 7,081,485 B2, DE 32 36 026 A1, US 2007/0027229 A1, EP 0 262 629 B1, EP 0 073 413, US 7,148,382 B2, US 5,761,169, DE 197 08 294 A1, EP 0 057 474, EP 0 047 902 A, EP 0 007 508, DE 600 29 481 T2, EP 0 980 682 B1, EP 0 948 955 B1, EP 1 236 459 B1 und EP 0 173 567 A2 verwiesen.

Die in diesen Druckschriften angegebenen Phosphinoxide eigenen sich besonders allein oder in Kombination mit dem System "alpha-Diketon/Amin" als Photopolymerisationsinitiatorsystem in einer erfindungsgemäßen polymerisierbaren dentalen Zusammensetzung.

Die EP 1 905 415 A1 beschreibt polymerisierbare Dentalzusammensetzungen mit Acylgermanium-Verbindungen als Initiatoren, die auch hier für die erfindungsgemäßen polymerisierbaren dentalen Zusammensetzungen geeignet sind.

Alternativ können auch Boratsalze, wie sie beispielsweise in US 4,772,530, US 4,954,414, US 4,874,450, US 5,055,372 und US 5,057,393 beschrieben sind, als Photoinitiatoren Verwendung finden.

Weitere geeignete Photoinitiatoren sind in J.-P. Fouassier, Photoinitiation, Photopolymerization and Photocuring, Hanser Publishers, Munich, Vienna, New York 1995 sowie in J.F. Rabek (Hrsg.), Radiation Curing in Polymer Science and Technology, Vol. II, Elsevier Applied Science, London, New York 1993 beschrieben.

Dem Fachmann sind diverse Initiatoren für eine chemische Aushärtung bekannt. Es sei insoweit exemplarisch auf die EP 1 720 506 verwiesen. Initiatoren zur chemischen Aushärtung werden auch in den oben bereits erwähnten Druckschriften DE 10 2006 019 092 sowie in der DE 39 41 629 beschrieben.

Bevorzugte Initiatoren zur chemischen Härtung sind Benzoylperoxid, Lauroylperoxid insbesondere Dibenzoylperoxid in Kombination mit Aminen wie *N,N*-Dimethyl-*p*-toluidin, *N,N*-Dihydroxyethyl-*p*-toluidin sowie strukturverwandten Aminen.

Die Peroxide und die Amine werden dabei auf zwei unterschiedliche Komponenten des Dentalmaterials aufgeteilt. Beim Mischen der aminhaltigen Komponente (sogenannte Basispaste) mit der peroxidhaltigen Komponente (sogenannte Initiator- oder Katalysatorpaste) wird durch die Reaktion von Amin und Peroxid (Redoxreaktion) die Polymerisation initiiert.

Neben Peroxiden und Aminen können auch Hydroperoxide in Kombination mit Thioharnstoffen verwendet werden.

Dualhärtende Systeme umfassen eine Kombination aus Photoinitiatoren und Initiatoren zur chemischen Aushärtung.

Beispielsweise kann die Basispaste zusätzlich einen Photoinitiator enthalten, so dass die Basispaste entweder allein als lichthärtender oder zusammen mit der Initiatorpaste als licht- und selbsthärtende dentale Zusammensetzung eingesetzt werden kann.

Neben den oxidativ wirksamen organischen Peroxidverbindungen können als Redoxsysteme auch Barbitursäuren bzw. Barbitursäurederivate sowie Malonylsulfamide verwendet werden.

Von den Barbitursäuresystemen sind die sogenannten "Bredereck-Systeme" von hoher Bedeutung. Beispiele geeigneter "Bredereck-Systeme" sowie Verweise auf die entsprechende Patentliteratur findet man in der EP 1 839 640 sowie in DE 1495520, WO 02/092021 oder in WO 02/092023.

Anstelle der Barbitursäuren können auch deren Salze verwendet werden. Beispiele hierzu finden sich in den folgenden Dokumenten: EP 1 872 767, EP 2 070 506, EP 1 881 010, DE 10 2007 050 763, US 6,288,138, DE 11 2006 001 049, US 7,214,726 sowie EP 2 070 935.

Geeignete Malonylsulfamide sind in der EP 0 059 451 beschrieben. Bevorzugte Verbindungen sind dabei 2,6-Dimethyl-4-isobutylmalonylsulfamid, 2,6-Diisobutyl-4-propylmalonylsulfamid, 2,6-Dibutyl-4-propylmalonylsulfamid, 2,6-Dimethyl-4-ethylmalonylsulfamid sowie 2,6-Diocytyl-4-isobutylmalonylsulfamid.

Ferner können Schwefelverbindungen in der Oxidationsstufe +2 oder +4 wie Natriumbenzolsulfinat oder Natriumparatoluolsulfinat eingesetzt werden.

Zur Beschleunigung der Aushärtung kann die Polymerisation in Gegenwart von Aktivatoren in Form von Schwermetallverbindungen wie Ce, Fe, Cu, Mn, Co, Sn oder Zn durchgeführt werden, wobei Kupferverbindungen besonders bevorzugt sind. Die Schwermetallverbindungen werden bevorzugt in Form löslicher organischer Verbindungen eingesetzt. Bevorzugte Kupferverbindungen sind dabei Kupferbenzoat, Kupferacetat, Kupferethylhexanoat, Kupferdi(methacrylat), Kupferacetylacetonat und Kupfernaphthenat.

Die kationische Polymerisation kann sowohl photoinduziert als auch thermisch erfolgen. Bevorzugt wird die kationische Polymerisation photoinduziert ausgeführt, da in dieser Anwendungsform ein höherer Umsatz der Vernetzung erreicht wird, da die thermische Polymerisation zu einem erheblichen Maß an Nebenreaktionen führen kann. Weiter bevorzugt ist eine kationisch ringöffnende Polymerisation von Epoxiden. Hierbei reagiert das epoxidhaltige Monomer mit der aus dem Zerfall eines Photoinitiators gebildeten Protonensäure zu einem offenkettigen Intermediat, das als reaktives Kation ein weiteres Epoxid angreift, öffnet und die Polymerisation fortführt. Triebkraft der ringöffnenden Polymerisation ist der Gewinn von Ringspannungsenergie, die bei den hierbei verwendeten bifunktionellen Monomeren durch die größere Ringverzerrung weiter erhöht wird. Kationische Photoinitiatoren sind beispielsweise ionische Iodonium- oder Sulfoniumderivate. Beispiele kommerziell erhältliche kationischer Photoinitiatoren sind das (4-Phenylthiophenyl)diphenylsulfoniumtriflat, das [4(2-Hydroxytetradecyl)oxy]phenyliodonium Hexafluoroantimonat oder das p-Octyloxyphenylphenyliodonium Hexafluoroantimonat. Durch Bestrahlung beispielsweise eines Oniumsalzes wird die Onium-Benzol Bindung homolytisch unter Bildung eines Phenylradikals und eines Radikalkations gespalten, wobei Letzteres mit R-H zu einem Radikal, dem Oniumbenzol und einer Säure reagiert. Analog verlaufen die Mechanismen für andere phenacylbasierte Photoinitiationssysteme.

Ein bevorzugtes System zur kationischen Vernetzung umfasst drei Komponenten: das ebenfalls zur klassischen radikalischen Polymerisation von Dentalmaterialien verwendete Campherchinon als lichtabsorbierendes Mittel, einen ebenfalls zur klassischen radikalischen Polymerisation von Dentalmaterialien eingesetzten Elektronendonor wie ein Amin, beispielsweise das oben angegebene Ethyl-*p-N,N*-dimethylaminobenzoat (DABE), sowie zusätzlich noch ein Iodoniumsalz. Hierbei wird das Campherchinon angeregt und reagiert mit dem Amin, das in einem Redoxprozess das Iodoniumsalz in ein saures Kation umwandelt. Dieses startet den ringöffnenden Prozess der Oxirane.

In der Patentliteratur ist eine Fülle an Initiatorsystemen für die kationisch ringöffnende Polymerisation, speziell auch für die Verwendung in dentalen Kompositmaterialien beschrieben. Geeignete Systeme für die erfindungsgemäßen dentalen Kompositmaterialien befinden sich in den Druckschriften EP 2 133 064 A1, betitelt "Initiator system containing a diarylalkylamine derivate, hardenable composition and use thereof", EP 0 897 710 A2, betitelt "Lichtinduziert kationisch härtende Zusammensetzungen und deren Verwendung", WO 2005/051332 A1, betitelt "Photoinitiator systems with anthracene-based electron donors for curing cationically polymerizable resins", US 9,770,528 B2, betitelt "Biomaterial compositions", sowie die Offenlegungsschrift DE 196 48 283 A1, betitelt "Polymerisierbare Massen auf der Basis von Epoxiden".

Auch in der wissenschaftlichen Literatur findet der Fachmann eine Vielzahl weiterer geeigneter Initiatoren. Insofern verweisen wir auf die Publikation "Photoinduced Electron Transfer Reactions for macromolecular Synthesis" von S.D. Silab, S. Doran und Y. Yagci, Chem. Rev. 116, 10212 - 10275, 2016.

<u>Bestandteil (D) - organische, polymerisierbare Monomere, die keine erfindungsgemäßen Polysiloxane sind, vorzugsweise zur Umsetzung mit den erfindungsgemäßen Polysiloxanen</u>

Die polymerisierbaren Monomeren sind Monomeren, die - für den Fall einer radikalischen Polymerisation - bevorzugt eine, zwei oder mehr ethylenische Gruppen aufweisende Substanzen sind, wie beispielsweise, ohne darauf beschränkt zu sein, die in der Dentalchemie üblicherweise eingesetzten (Meth)acrylatmonomeren.

Die (Meth)acrylatmonomeren können monofunktionell sowie polyfunktionell sein.

Vorzugsweise eingesetzte monofunktionelle (Meth)acrylatmonomeren stellen die Ester der (Meth)acrylsäure mit Alkylgruppen von 1 bis 12 C-Atomen sowie Ester der (Meth)acrylsäure, die aromatische Gruppen mit 6 bis 12 C-Atomen enthalten, wobei die Alkylgruppen und aromatischen Gruppen, die die Ester bilden, Substituenten wie Hydroxylgruppen und Etherbindungen enthalten können, dar.

In der Patentliteratur ist eine Vielzahl weiterer Verbindungen genannt (beispielsweise auch in der DE 39 41 629 A1), die allesamt Ester der Acryl- oder Methacrylsäure sind und sich zum Einsatz in einem härtbaren Gemisch eignen.

Die polymerisierbaren Monomeren können auch mindestens eine ethylenische Doppelbindung aufweisende Hydroxylverbindungen sein. Dabei können vorzugsweise die in der Dentalchemie üblicherweise eingesetzten Hydroxylverbindungen von (Meth)acrylaten eingesetzt werden.

Auch können als Beispiele polyfunktioneller (Meth)acrylatmonomeren Di(meth)-acrylate von Alkylenglycol mit 2

bis 20 C-Atomen, Di(meth)acrylate von Oligomeren des Alkylenglycols, Polyalkylenglycoldi(meth)acrylat, Di(meth) acrylate von Bisphenol A bzw. vom Diglycidylether von Bisphenol A genannt werden.

Besonders bevorzugt sind weiterhin polymerisierbare Verbindungen, die auf ein zentrales polyalicyclisches Strukturelement beruhen, wie beispielsweise 3(4), 8(9)-Bis((meth)acryloyloxymethyl)tricyclo[5.2.1.0$^{2,6}$]decan, alkoxyliertes 3(4), 8(9)-Bis((meth)acryloyloxymethyl)tricyclo[5.2.1.0$^{2,6}$]decan, 2,3-Bis((meth)acryloyloxy-methyl)bicyclo[2.2.1] heptan, alkoxyliertes 2,3-Bis((meth)acryloyloxymethyl)-bicyclo[2.2.1]heptan, 1,3,5-Tri(meth)acryloyloxytricyclo [3.3.1.1$^{3,7}$]decan, alkoxyliertes Tri(meth)acryloyloxytricyclo-[3.3.1.1$^{3,7}$]decan sowie (Meth)acrylsäureester von Tricyclo[5.2.1.0$^{2,6}$]decan-3(4),8(9)-dimethanol, alkoxyliertem Tricyclo-[5.2.1.0$^{2,6}$]decan-3(4),8(9)-dimethanol, Bicyclo[2.2.1] heptan-2,3-dimethanol, alkoxyliertem Bicyclo[2.2.1]heptan-2,3-dimethanol, 1,3,5-Adamantantriol, alkoxyliertem 1,3,5-Adamantantriol, wobei zwischen dem polyalicyclischen Strukturelement und den (Meth)acrylsäureestern Urethan-, Harnstoff-, Amid-, Allophanat-, Acylharnstoff- oder Biuretgruppen angeordnet sind.

Angaben zur Herstellung dieser substituierten (Meth)acrylsäureester finden sich in den Patentanmeldungen EP 11 183 333, EP 11 183 328, EP 11 183 345, EP 11 183 338, EP 11 183 342 und EP 11 188 086 sowie in den in diesen Dokumenten zitierten Schriftstücken.

Bevorzugt sind ebenfalls Urethan(meth)acrylatester, Reaktionsprodukte aus 2 Mol eines (Meth)acrylats mit einer Hydroxylgruppe und einem Mol eines Diisocyanats.

Weiter bevorzugt sind die klassischen Dentalmonomeren Ethylenglycoldi(meth)acrylat, Diethylenglycoldi(meth)acrylat, 1,6-Hexandiol-di(meth)acrylat (HEDMA), Triethylenglycoldi(meth)acrylat (TEGDMA), 1,12-Dodecandioldi(meth)acrylat, Bisphenol-A-di(meth)acrylat, alkoxyliertes Bisphenol-A-di(meth)acrylat, Bisphenol-B-di(meth)acrylat, alkoxyliertes Bisphenol-B-di(meth)acrylat, Bisphenol-C-di(meth)acrylat, alkoxyliertes Bisphenol-C-di(meth)acrylat, Bisphenol-F-di(meth)acrylat, alkoxyliertes Bisphenol-F-di(meth)acrylat, Polyethylenglycoldi(meth)acrylat, 7,7,9-Trimethyl-3,14-dioxa-4,13-dioxo-5,12-diazahexadecan-1,16-dioxydi(meth)acrylat (UDMA), Butandioldi(meth)acrylat, Tetraethylenglycoldi(meth)acrylat, Neopentylglycol-di(meth)acrylat, 2-Hydroxypropyl-1,3-di(meth)acrylat, 3-Hydroxypropyl-1,2-di(meth)acrylat, Pentaerythritoldi(meth)acrylat, Di(meth)acrylaten des Dihydroxy-methyltricyclo[5.2.1.0$^{2,6}$]decans, 2-Hydroxyethyl(meth)acrylat, 2-Hydroxypropyl-(meth)acrylat, 3-Hydroxypropyl(meth)acrylat, 1,2-Dihydroxypropyl(meth)acrylat, 1,3-Dihydroxypropyl(meth)acrylat, 2,3-Dihydroxypropyl(meth)acrylat, 2,2-Bis[4-[3-(meth)acryloyloxy-2-hydroxypropoxy]phenyl]propan (Bis-GMA), Trimethylol-propantri(meth)acrylat, Trimethylolethantri(meth)acrylat, Pentaerythritoltri(meth)-acrylat, Trimethylolmethantri(meth)acrylat, Pentaerythritoltetra(meth)acrylat, Ditrimethylolpropantetra(meth)acrylat, Pentaerythritolhexa(meth)acrylat, Butylenglycoldi(meth)acrylat, Propylenglycoldi(meth)acrylat, Nonandioldi(meth)-acrylat, Decandioldi(meth)acrylat, Glycerolmono(meth)acrylat, Glycerol-di(meth)acrylat, Trimethylolpropanmono(meth)acrylat, Trimethylolpropan-di(meth)acrylat, Sorbitolmono-, di-, tri-, tetra- oder penta(meth)acrylat, Methyl(meth)acrylat, Ethyl(meth)acrylat, Propyl(meth)acrylat, Butyl(meth)acrylat, Hexyl(meth)acrylat, Tetrahydrofurfuryl(meth)acrylat, Lauryl(meth)acrylat, Cyclohexyl(meth)- acrylat, Allyl(meth)acrylat, Glycidyl(meth)acrylat, 2-Ethoxyethyl(meth)acrylat, Methoxypolyethylenglycol(meth)acrylat, Isobornyl-(meth)acrylat, 2-(N, N-Dimethylamino)ethyl(meth) acrylat, N-Methylol(meth)-acrylamid, Diaceton(meth)acrylamide, 2,2-Bis[4-(meth)acryloyloxyphenyl]-propan, 2,2-Bis [4-(meth)acryloyloxyethoxyphenyl]propan, 2,2-Bis[4-(meth)-acryloyloxydiethoxyphenyl]propan, 2,2-Bis[4-(meth)acryloyloxytriethoxyphenyl]-propan 2,2-Bis[4-(meth)acryloyloxytetraethoxyphenyl]propan, 2,2-Bis[4-(meth)-acryloyloxypentaethoxyphenyl]-propan, 2,2-Bis[4-(meth)acryloyloxydipropoxy-phenyl]propan, 2,2-Bis[4-(meth)acryloyloxyethoxyphenyl]-2-[4-(meth)acryloyl-oxydiethoxyphenyl]propan, 2-[4-(Meth)acryloyloxydiethoxyphenyl]-2-[4-(meth)-acryloyloxytriethoxyphenyl]propan, 2-[4-(Meth)acryloyloxdipropoxyphenyl]-2-[4-(meth)acryloyloxytriethoxyphenyl]propan, 2,2-Bis [4-(meth)acryloyloxyisopropoxy phenyl]propan, Hydroxypivalinsäureneopentylglycoldi(meth)acrylat, Aceto-acetoxyethyl(meth)acrylat, Polypropylenglycoldi(meth)acrylat, Glycerolalkoxylatdimethacrylat, Neopentylglycol(meth)acrylat, N,N-(1,2-Dihydroxyethylen)bis-acrylamid, 2,2-Bis[4-(meth)acryloyloxypentaethoxyphenyl]propan, 2,2-Bis[4-(meth)acryloyloxypolyethoxyphenyl]-propan, Diethylenglycoldi(meth)acrylat, Dipentaerythritoltetra(meth)acrylat, Dipentaerythritolhexa(meth)acrylat, N,N-(2,2,4-Trimethylhexamethylen)bis[2-(aminocarboxy)propan-1,3-diol]-tetra(meth)-acrylat, das Kondensationsprodukt von 3,(4)-(Meth)acryloxymethyl-8,(9)-hydroxymethyltricyclo[5.2.1.0$^{2,6}$]decan mit Dicarbonsäuren, 2-Ethylhexyl-(meth)acrylat, Tridecyl(meth)acrylat, Stearyl(meth)acrylat, Benzyl(meth)acrylat, Methoxydiethylenglycol(meth)acrylat, Dicyclopentenyl(meth)acrylat, Phenyl-(meth)acrylat, Pentaerythritolmono(meth)acrylat, Dipentaerythritolmono(meth)-acrylat, sowie Caprolacton modifiziertes Tetrahydrofurfuryl(meth)acrylat.

Die polymerisierbaren Monomeren sind Monomeren, die - für den Fall einer kationischen Polymerisation - bevorzugt eine, zwei oder mehr Oxirangruppen aufweisende Substanzen sind, wie beispielsweise, ohne darauf beschränkt zu sein, die in der Dentalchemie üblicherweise eingesetzten Monomerderivate mit einer Epoxycycloyhexaneinheit oder einer Epoxynorbornaneinheit.

In der Patentliteratur ist eine Vielzahl an geeigneten Verbindungen beschrieben. Beispiele mit detaillierten Synthesenangaben finden sich in der DE 196 48 283 A1.

Bestandteil (E) - säuregruppenhaltige Monomeren, die kein Si-Atom aufweisen

Geeignete säuregruppenhaltige Monomere sind 10-(Meth)acryloyloxydecyl-dihydrogenphosphat (10-MDP), 2-(Meth)acryloyloxyethyldihydrogenphosphat, 6-(Meth)acryloyloxyhexyldihydrogenphosphat, 4-(Meth)acryloyloxybutyldi-hydrogenphosphat, 8-(Meth)acryloyloxyoctyldihydrogenphosphat, 2-(Meth)-acryloyloxynonyldihydrogenphosphat, 11-(Meth)acryloyloxyundecyldihydrogen-phosphat, 20-(Meth)acryloyloxyeicosyldihydrogenphosphat, 1,3-Di(meth)acyloyl-oxypropyl-2-dihydrogenphosphat, 2-(Meth)acryloyloxyethylphenyldihydrogen-phosphat, Di(2-(meth)acyloyloxyethyl)pyrophosphat, Di(2-(meth)acyloyl-oxypropyl)pyrophosphat, Di(2-(meth)acyloyloxybutyl)pyrophosphat, Di(2-(meth)-acyloyloxypentyl)pyrophosphat, das Di(2-(meth)acyloyloxyhexyl)pyrophosphat, Di(2-(meth)acyloyloxydecyl)pyrophosphat, Mono-, Di- und/oder Triester der Phosphorsäure, welche durch Umsetzung von Hydroxy-C2-C8-alkylmethacrylat (dabei vorzugsweise Hydroxyethylmethacrylat) oder Glyceryldimethacrylat mit Phosphoroxychlorid erhalten werden, Glyceryldimethacrylatphosphat, Pentaerythritoltrimethacrylatphosphat, Dipentaerythritolpentaacrylatphosphat, Tetramethacryloxyethylpyrophosphat, Trimellitsäure-4-methacryloyloxyethylester (4-MET), Trimellitsäureanhydrid-4-methacryloyloxyethylester (4-META), Pyromellitsäuredimethacrylat, Pyromellitsäureglyce-roldimethacrylat, Methacryloyloxyethylphthalat, Methacryloyloxyethylmaleat, Methacryloyloxyethylsuccinat, 1,3-Glyceroldimethacrylatmaleat und Di-Oxyethoxymethacrylsäureethylendiamintetraessigsäureester.

Dabei bevorzugte säuregruppenhaltige Monomere sind 10-(Meth)acryloyloxy-decyldihydrogenphosphat (10-MDP), Glyceryldimethacrylatphosphat, Pentaerythritoltrimethacrylatphosphat, Dipentaerythritolpentaacrylatphosphat, Tetramethacryloxyethylpyrophosphat, Trimellitsäure-4-methacryloyloxyethylester (4-MET), Trimellitsäureanhydrid-4-methacryloyloxyethylester (4-META), Pyromellitsäuredimethacrylat, Pyromellitsäureglyceroldimethacrylat.

Weitere geeignete säuregruppentragende Monomere sind beispielsweise in EP 0980682 B1 und EP 0948955 A1 genannt. In der Literatur sind weitere geeignete säuregruppenhaltige Monomeren, die haftfördernd wirken, genannt.

Bestandteil (F) - weitere übliche Additive

Eine erfindungsgemäße polymerisierbare dentale Zusammensetzung kann ein, zwei, mehr als zwei oder sämtliche weitere übliche Additive für dentale Zusammensetzungen aus der Gruppe umfassend die folgenden Additive enthalten.

Diese Additive können verschiedene Funktionen haben. Übliche Additive für den Einsatz in dentalen Werkstoffen sind dem Fachmann bekannt, je nach gewünschter Funktion wird er das oder die geeigneten Additive auswählen. Beispielhaft sind im Folgenden typische Additive und ihre Funktionen beschrieben.

Polymerisierbare dentale Zusammensetzungen enthalten vorzugsweise einen oder mehrere Inhibitor(en), auch Stabilisator(en) genannt. Diese werden üblicherweise zugesetzt, um eine Spontanpolymerisation zu vermeiden. Sie reagieren mit vorzeitig entstehenden Radikalen, die abgefangen werden, verhindern eine vorzeitige Polymerisation und erhöhen die Lagerstabilität der polymerisierbaren, dentalen Zusammensetzung. Gängige Inhibitoren sind Phenolderivate wie Hydrochinonmonomethylether (HQME) oder 2,6-Di-tert.-butyl-4-methylphenol (BHT). Weitere Inhibitoren wie tert.-Butylhydroxyanisol (BHA), 2,2-Diphenyl-1-picryl-hydrazyl-, Galvinoxyl-, Triphenylmethyl-Radikale, 2,3,6,6,-Tetramethylpiperidinyl-1-oxylradikale (TEMPO) sowie Derivate von TEMPO oder Phenothiazin sowie Phenothiazin-Derivate werden in der EP 0 783 880 B1 beschrieben. Alternative Inhibitoren sind in der DE 101 19 831 A1 oder in der EP 1 563 821 A1 angegeben.

Eine erfindungsgemäß bevorzugte polymerisierbare dentale Zusammensetzung umfasst somit als Additiv einen oder mehrere Polymerisationsinhibitor(en) zur Erhöhung der Lagerstabilität der Zusammensetzung, vorzugsweise ausgewählt aus der Gruppe bestehend aus Hydrochinonmonomethylether (HQME), Phenolen, dabei vorzugsweise 2,6-Di-tert.-butyl-4-methylphenol (BHT) und tert.-Butylhydroxyanisol (BHA), 2,2-Diphenyl-1-picrylhydrazyl-Radikalen, Galvinoxyl-Radikalen, Triphenylmethyl-Radikalen, 2,3,6,6,-Tetramethylpiperidinyl-1-oxylradikal (TEMPO) sowie dessen Derivaten und Phenothiazin sowie dessen Derivaten.

Eine erfindungsgemäße polymerisierbare Zusammensetzung kann als Additiv eine oder mehrere fluoridabgebende Substanzen, dabei vorzugsweise Natriumfluorid und/oder Aminfluoride, umfassen.

UV-Absorber werden ebenfalls zu den Stabilisatoren oder Inhibitoren gezählt, die beispielsweise durch ihre konjugierten Doppelbindungssysteme und aromatischen Ringe befähigt sind, UV-Strahlung zu absorbieren, sind in manchen Fällen Bestandteil einer erfindungsgemäßen polymerisierbare dentalen Zusammensetzung. Beispiele an UV-Absorbern sind 2-Hydroxy-4-methoxybenzo-phenon, Salicylsäurephenylester 3-(2'-Hydroxy-5'-methylphenyl)benzotriazol oder Diethyl-2,5-dihydroxyterephthalat.

Eine erfindungsgemäß bevorzugte polymerisierbare dentale Zusammensetzung kann als Additiv auch einen oder mehrere Molekulargewichtsregler umfassen. Molekulargewichtsregler sind aus dem Stand der Technik bekannt und kommerziell erhältlich. Man benutzt sie beispielsweise bei der Lösungspolymerisation von Olefinen, bei der Emulsionspolymerisation von Methacrylaten oder zur Herstellung von Formkörpern aus PMMA-Formmassen (PMMA = Polymethylmethacrylat) durch Formpressen oder Spritzgießen.

Molekulargewichtsregler stellen sogenannte Übertragungsreagentien dar, die in einer freien radikalischen Reaktion Transferreaktionen eingehen und mechanistisch H-Abstraktion und Übertragung der Radikalfunktion auf den Regler beinhaltet. Der Regler findet sich dann aufgrund seiner Funktion in Form von Endgruppen im vernetzten Polymerisat wieder.

Übliche Regler sind beispielsweise Aldehyde und Ketone, wie Formaldehyd, Acetaldehyd, Propionaldehyd, n-Butyraldehyd, Isobutyraldehyd, Methylethylketon, Aceton, Methylisobutylketon, Ameisensäure, Ammoniumformiat, Hydroxylammoniumsulfat und Hydroxylammoniumphosphat, Verbindungen, die Schwefel in organisch gebundener Form enthalten, wie Di-n-butylsulfid, Di-n-octylsulfid, Diphenylsulfid, Diisopropyldisulfid, Di-n-butyldisulfid, Di-n-hexyldisulfid, Diacetyldisulfid und Di-tert.-butyltrisulfid, Verbindungen, die Schwefel in Form von SH-Gruppen aufweisen, wie n-Butylmercaptan, n-Hexylmercaptan und n-Dodecylmercaptan, Octadecylmercaptan, weitere Schwefelverbindungen wie Hydrogensulfite, Disulfite, Verbindungen wie Mercaptoethanol, Mercaptobutanol, Mercaptoessigsäure, 3-Mercaptopropionsäure, Mercaptobernsteinsäure, Thioglycerin, Thioglycolsäure, Diethanolsulfid, Thiodiglycol, Ethylthioethanol, 2,2,4,6,6-Pentamethylheptan-4-thiol, 2,2,4,6,6,8,8-Heptamethylnonan-4-thiol, Thioharnstoff, Dimethylsulfoxid, Ethylhexylthioglycolat, Pentaerythrittetrathioglycolat, Mercaptopropyltrimethoxysilan, dann Allylverbindungen wie Allylalkohol, Allylbromid, oder Benzylverbindungen wie Benzylchlorid oder Alkylhalogenide wie Chloroform, Bromtrichlormethan oder Tetrachlormethan, Tetrabrommethan, Methylenchlorid, weiterhin niedere und höhermolekulare einwertige oder mehrwertige Alkohole wie Methanol, Ethanol, n-Propanol, Isopropanol, tert.-Butanol, sec.-Butanol, n-Butanol, Amylalkohol, Cyclohexanol, Octanol, Dodecanol, 1-Ethylhexanol, Glycerin, Stearylalkohol, Oleylalkohol, Hydroxyethylmethacrylat oder Amine wie Triethylamin sowie Toluol oder Ethylbenzol.

Bevorzugte Molekulargewichtsregler in erfindungsgemäßen polymerisierbaren dentalen Zusammensetzungen umfassen diverse Terpene, insbesondere Terpinene ($\alpha$-Terpinen, $\beta$-Terpinen, $\gamma$-Terpinen), Phellandrene ($\alpha$-Phellandren, $\beta$-Phellandren) und Terpinolen (auch $\delta$-Terpinen genannt), 1,4-Cyclohexadien (gegebenenfalls substituiert), 1,3-Cyclohexadien (gegebenenfalls substituiert), 1,4-Dihydronaphtalin, 1,4,5,8-Tetrahydronaphthalin, 2,5-Dihydrofuran oder dimeres $\alpha$-Styrol (2,4-Diphenyl-4-methyl-1-penten) sowie Linolsäure und α-Linolensäure. Diese Molekulargewichtsregler sind beschrieben in den Druckschriften EP 2 374 444 B1 und EP 2 374 445 B1.

Ebenfalls bevorzugte Molekulargewichtsregler in erfindungsgemäßen polymerisierbaren dentalen Zusammensetzungen sind doppelbindungshaltige Substanzen, die nach einem radikalischen Addition-Fragmentierungs-Kettenübertragungsmechanismus reagieren. Diese Mittel werden nach dem Ausdruck "Addition-Fragmentation Chain Transfer Agents" AFCT-Reagenzien genannt. Bekannte AFCT-Reagenzien umfassen spezielle Schwefelverbindungen wie Allylsulfide, Allylsulfone, Dithioester, Dithiocarbamate, Xanthate und Trithiocarbonate. Weitere bekannte Kettenüberträger sind reversible AFCT-Reagenzien, sogenannte RAFT-Reagenzien. Auch diese Substanzen werden bevorzugt in erfindungsgemäßen polymerisierbaren Dentalzusammensetzungen eingesetzt.

Weitere bevorzugte Übertragungsmittel für die erfindungsgemäßen polymerisierbaren Zusammensetzungen sind in den Druckschriften EP 3 090 722 A1, EP 2 916 801 B1, EP 2 748 206 B1, EP 2 965 742 A1, EP 3 058 014 B1, EP 3 166 569 B1, EP 3 046 962 B1 und EP 2 931 756 B1 offenbart.

In einigen Ausführungsformen kann eine erfindungsgemäß bevorzugte polymerisierbare dentale Zusammensetzung als Additiv auch einen oder mehrere Weichmacher erhalten. Diese Substanzklasse ist für eine Verwendung in polymerisierbaren Dentalzusammensetzungen umfassend beschrieben. In der Literatur sind explizit benannt: Säureester, ausgewählt aus der Gruppe bestehend aus Trimellitsäureestern, Fettsäureestern, Essigsäureestern, Maleinsäureestern, Fumarsäureestern und Zitronensäureestern, weiter Tri-2-ethylhexyl-trimellitat, Dimethyladipat, Dibutyladipat, Diisobutyladipat, Diisonorbornyladipat, Di-2-ethylhexyladipat, Diisodecyladipat, Diethylenglycoladipat, Dibutyldiglycoladipat, Di-2-ethylhexylazelat, Dimethylsebacat, Dibutylsebacat, Di-2-ethylhexyl-sebacat, Methylacetylricinolat, epoxydiertes Sojaöl, Glyceryltriacetat, 2-Ethylhexylacetat, Dimethylmaleat, Dibutylmaleat, Di-2-ethylhexylmaleat, Dibutylfumarat, Di-2-ethylhexylfumarat, Trimethylcitrat, Triethylcitrat, Tripropylcitrat und Triisobutylcitrat, sowie Polyethylenglycolderivate, Polypropylenglycole, niedermolekulare Polyester, Dibutyl-, Dioctyl-, Dinonyl-, Diphenylphthalat, Di(iso-nonyl) adipat, Tricresylphosphat und Siliconöle, Dibenzyltoluol oder polyethoxylierte Sorbitanester, Phthalsäureester längerer verzweigter Alkohole wie Bis(2-ethylhexyl)phthalat oder Phthalsäurepolyester, $C_2$ bis $C_{18}$-Dialkylester von $C_2$- bis $C_6$-Dicarbonsäuren, wie Dioctylmalat, Diisopropyladipat, aromatische und aliphatische Sulfonsäureester, wie $C_2$- bis $C_{20}$-Alkylsulfonsäureester des Phenols oder von $C_1$ bis $C_{18}$- Alkanolen und typische aromatische Weichmacher wie Polyphenyle, sowie Isomerengemische von $C_{20}$- bis $C_{30}$-Aromaten, Biphenyl, 1,2-Diphenylethan, Decanol, 2,4,6-Trimethylnaphthalin, Hexamethylbenzol, Diphenylmethan, 1,1-Diphenylethan, Pentadecan, 2,3-Dimethylbiphenyl, Zimtalkohol, Dibenzylether, Hexaethylbenzol, raffinierte Mineralöle, Ölsäure, Castoröl (Rizinusöl), Kornöl, Kampher, und Zuckeralkohole, sowie Tributylphosphat, Tri-2-ethylhexylphosphat, Triphenylphosphat und Tricresylphosphat und Weichmacher umfassend ein zentrales polyalicyclisches Strukturelement. Zu den genannten Weichmachern sei auf die Patentliteratur EP 2 623 087 B1, EP 2 623 086 B1, DE 101 47 125 A1, EP 1 194 110 B1, DE 32 46 654 A1, DE 101 26 476 A1, DE 197 11 514 B4, DE 39 02 417 A1, DE 199 61 341 C2, DE 197 54 029 A1, DE 60 2004 009 552 T2, DE 10 2008 283 306, DE 699 21 231 T2, DE 692 31 737 T2, DE 690 17 484 T2, DE 697 25 380 T2, DE 698 01 010 T2, DE 20 2010 014 676 U1, DE 199 41 738 B4, DE 10 2009 046 251 A1, DE 2420351 C3 verwiesen.

Da die Zähne möglichst naturgetreu wiederherzustellen sind, ist es notwendig, erfindungsgemäße dentale Zusammensetzungen in den unterschiedlichsten Farbtönen bereitzustellen. Bevorzugte erfindungsgemäße härtbare Dentalmaterialien besitzen charakteristische Farben, vorzugsweise eine Farbfarbe, die von der "VITA classical A1 - D4 Farbskala" umfasst ist; solche Farben werden mit A1 - A4 (rötlichbräunlich), B1 - B4 (rötlichgelblich), C1 - C4 (Grautöne), D2 - D4 (rötlichgrau) bezeichnet. Zur Farbeinstellung benutzt man in der Regel anorganische Farbstoffe sowie organische Pigmente in sehr geringen Mengen, die somit in bevorzugten Ausgestaltungen als Additiv eingesetzt werden.

Weitere optionale Additive sind Aromastoffe, dentale Arzneimittel, organische Polymere und Oligomere, Mikrobizide, vorzugsweise Bakterizide, grenzflächenaktive Substanzen, vorzugsweise Tenside und Konservierungsmittel, vorzugsweise Parabene, rheologische Hilfsmittel und Verdickungsmittel.

Bestandteil (G) - Lösungsmittel

Eine erfindungsgemäße polymerisierbare, dentale Zusammensetzung, beispielsweise ein dentales Adhäsiv, kann auch ein Lösungsmittel bis zu 70 Gew.-%, bezogen auf die Gesamtzusammensetzung, enthalten. Als Lösungsmittel kann die Zusammensetzung auch Wasser enthalten. Weiterhin geeignet sind die üblicherweise verwendeten organischen Lösungsmittel wie beispielsweise Kohlenwasserstoffe, Ketone und Ester wie beispielsweise Toluol, Xylol, Isooctan, Aceton, Butanon, methylisobutylketon, Ethylacetat, Butylacetat, Tetrahydrofuran, N-Methylpyrrolidon, Dimethylacetamin und Dimethylformamid. Es können auch Alkohole wie Ethanol, Propanole, Butanole, Pentanole, Hexanole, Cyclohexanol, Heptanole, Octanole, Nonanole, Decanole, etc., eingesetzt werden. Ebenfalls geeignet sind cycloaliphatische und arylaliphatische Alkohole.

In einer bevorzugten Ausgestaltung enthält eine erfindungsgemäße polymerisierbare, dentale Zusammensetzung, beispielsweise ein dentales Adhäsiv, ein organisches Lösungsmittel, vorzugsweise ausgewählt aus der Gruppe bestehend aus mit Wasser mischbaren organischen Lösungsmitteln, vorzugsweise Aceton, Ethanol, n-Propanol und Isopropanol sowie deren Mischungen.

Besonders bevorzugt enthält eine erfindungsgemäße polymerisierbare dentale Zusammensetzung, beispielsweise ein dentales Adhäsiv, Wasser und ein mit Wasser mischbares organisches Lösungsmittel/gemisch. Dabei liegt das Verhältnis des organischen Lösungsmittel(s)/gemisches zu Wasser vorzugsweise im Bereich von 1:1 bis 10:1, bevorzugt im Bereich von 2:1 bis 8:1 und weiter bevorzugt im Bereich von 3:1 bis 5:1.

Eine erfindungsgemäße polymerisierbare, dentale Zusammensetzung enthält die folgenden Komponenten:

(A) in einer Menge von 5 bis 99,99 Gew.-%,
(B) in einer Menge von 0,3 bis 92 Gew.-%,
(C) in einer Menge von 0,01 bis 5 Gew.-%,
(D) in einer Menge von 0 bis 94 Gew.-%,
(E) in einer Menge von 0 bis 20 Gew.-%
(F) in einer Menge von 0 bis 20 Gew.-% und
(G) in einer Menge von 0 bis 70 Gew.-%,

wobei die Gewichtsangaben jeweils auf die Gesamtzusammensetzung bezogen sind.

Bevorzugte erfindungsgemäße polymerisierbare, dentale Zusammensetzungen enthalten die folgenden Komponenten:

(A) in einer Menge von 5 bis 19,99 Gew.-%,
(B) in einer Menge von 80 bis 92 Gew.-%,
(C) in einer Menge von 0,01 bis 5 Gew.-%,
(D) in einer Menge von 0 bis 14 Gew.-% und
(F) in einer Menge von 0 bis 8 Gew.-%

oder

(A) in einer Menge von 5 bis 49,99 Gew.-%,
(B) in einer Menge von 50 bis 80 Gew.-%,
(C) in einer Menge von 0,01 bis 5 Gew.-%,
(D) in einer Menge von 0 bis 35 Gew.-%,
(E) in einer Menge von 0 bis 20 Gew.-% und
(F) in einer Menge von 0 bis 20 Gew.-%

oder

(A) in einer Menge von 5 bis 99,99 Gew.-%,
(B) in einer Menge von 0,3 bis 50 Gew.-%,
(C) in einer Menge von 0,01 bis 5 Gew.-%,
(D) in einer Menge von 0 bis 94 Gew.-%,
(E) in einer Menge von 0 bis 20 Gew.-%
(F) in einer Menge von 0 bis 20 Gew.-% und
(G) in einer Menge von 0 bis 70 Gew.-%,

wobei die Gewichtsangaben jeweils auf die Gesamtzusammensetzung bezogen sind.

In besonders bevorzugten Fällen enthält eine erfindungsgemäße polymerisierbare dentale Zusammensetzung keinen Bestandteil (D).

Wie oben beschrieben, kann die erfindungsgemäße polymerisierbare dentale Zusammensetzung gehärtet werden. Die vorliegende Erfindung betrifft somit auch eine polymerisierte Dentalzusammensetzung, erhältlich aus einer erfindungsgemäßen polymerisierbaren, dentalen Zusammensetzung (wie vorstehend definiert, vorzugsweise eine polymerisierbare Zusammensetzung wie vorstehend als bevorzugt definiert) mittels Polymerisation der in der Zusammensetzung enthaltenen Polysiloxanverbindungen sowie gegebenenfalls weiterer in der dentalen Zusammensetzung enthaltener polymerisierbarer Bestandteile. Die Polymerisation erfolgt vorzugsweise entweder radikalisch mittels der in den (Meth)acrylatgruppen enthaltenen organisch polymerisierbaren Doppelbindungen oder kationisch über einen ringöffnenden Mechanismus über eine Oxirangruppe.

Das zu bevorzugten Ausführungsformen erfindungsgemäßer polymerisierbarer dentaler Zusammensetzungen Gesagte gilt auch für die erfindungsgemäßen polymerisierten dentalen Zusammensetzungen entsprechend.

Ein wesentlicher Aspekt der vorliegenden Erfindung betrifft eine erfindungsgemäße polymerisierbare, dentale Zusammensetzung (wie vorstehend definiert, vorzugsweise wie vorstehend als bevorzugt definiert), bzw. eine erfindungsgemäße polymerisierte, dentale Zusammensetzung (wie vorstehend definiert, vorzugsweise wie vorstehend als bevorzugt definiert) zur Anwendung in einem therapeutischen Verfahren (einem Verfahren zur therapeutischen Behandlung des menschlichen oder tierischen Körpers, vorzugsweise des menschlichen Körpers).

Besonders bevorzugt ist insoweit die spezifische Anwendung der erfindungsgemäßen polymerisierbaren, dentalen Zusammensetzungen, bzw. der erfindungsgemäßen polymerisierten dentalen Zusammensetzungen in einem therapeutischen Verfahren zum temporären oder permanenten Füllen einer dentalen Kavität oder

in einem therapeutischen Verfahren als dentales Füllungsmaterial, dentales Bulk-Fill Material, dentales Unterfüllungsmaterial, dentales Stumpfaufbaumaterial, dentaler Befestigungszement, dentales Kronenmaterial, dentales Brückenmaterial, Unterfütterungsmaterial, dentales Adhäsiv (Bonding), dentaler Lack, dentales Versiegelungsmaterial, fließfähiges dentales Kompositmaterial, dentales Inlay, dentales Onlay, dentales Overlay, künstlicher Zahn, KFO-Material, dentales Gerüst, dentale Prothese, dentales Provisorium oder dentales Blockmaterial.

Ganz besonders bevorzugt ist eine erfindungsgemäße dentale, polymerisierbare Zusammensetzung oder ein erfindungsgemäßes polymerisiertes Dentalmaterial zur spezifischen Anwendung

in einem therapeutischen Verfahren zum temporären oder permanenten Füllen einer dentalen Kavität oder
in einem therapeutischen Verfahren als

a.) standfestes Füllungskomposit, standfestes Bulk-Fill Material, Inlay, Onlay, Overlay, dentales Blockmaterial, künstlicher Zahn, dentales Gerüst, wobei

(A) in einer Menge von 5 bis 19,99 Gew.-%,
(B) in einer Menge von 80 bis 92 Gew.-%,
(C) in einer Menge von 0,01 bis 5 Gew.-%,
(D) in einer Menge von 0 bis 14 Gew.-% und
(F) in einer Menge von 0 bis 8 Gew.-% enthalten ist,

b.) fließfähiges Füllungskomposit, fließfähiges Bulk-Fill Material, Stumpfaufbaumaterial, dentaler Befestigungszement, Kronen- und Brückenmaterial, KFO-Material, dentales Unterfüllungsmaterial oder dentales Unterfütterungsmaterial, wobei

(A) in einer Menge von 5 bis 49,99 Gew.-%,
(B) in einer Menge von 50 bis 80 Gew.-%,
(C) in einer Menge von 0,01 bis 5 Gew.-%,
(D) in einer Menge von 0 bis 35 Gew.-%,
(E) in einer Menge von 0 bis 20 Gew.-% und

(F) in einer Menge von 0 bis 20 Gew.-% enthalten ist,

c.) dentales Adhäsiv, dentales Versiegelungsmaterial, dentaler Lack, dentaler Primer oder dentale Druckzusammensetzung in generativen dentalen Fertigungsverfahren, auch als "Rapid Prototyping" bekannt, vorzugsweise für die Stereolithographie und hier vorzugsweise für das Digital Light Processing (DLP), das Selective Laser Assembling (SLA), für die Mikrostereolithographie, für das 3D-Printing, für das Laminated Object Manufacturing oder für das Film Transfer Imaging, wobei

(A) in einer Menge von 5 bis 99,99 Gew.-%,
(B) in einer Menge von 0,3 bis 50 Gew.-%,
(C) in einer Menge von 0,01 bis 5 Gew.-%,
(D) in einer Menge von 0 bis 94 Gew.-%,
(E) in einer Menge von 0 bis 20 Gew.-%
(F) in einer Menge von 0 bis 20 Gew.-% und
(G) in einer Menge von 0 bis 70 Gew.-% enthalten ist und

wobei die Gewichtsangaben jeweils auf die Gesamtzusammensetzung bezogen sind.

Die vorliegende Erfindung betrifft außerdem ein Verfahren zur Herstellung einer polymerisierbaren dentalen Zusammensetzung mit den folgenden Schritten:

- Bereitstellen der Bestandteile (A), (B), (C), sowie optional der Bestandteile (D), (E), (F), (G) und
- Mischen der Bestandteile

Beschrieben wird darüber hinaus auch ein Verfahren zur Herstellung einer polymerisierten, vorzugsweise radikalisch polymerisierten, dentalen Zusammensetzung mit den folgenden Schritten:

- Bereitstellen der Bestandteile (A), (B), (C), sowie optional der Bestandteile (D), (E), (F), (G),

- Mischen der Bestandteile und

- Polymerisation, vorzugsweise radikalische Polymerisation des Gemischs Vorzugsweise werden die polymerisierbaren, dentalen Zusammensetzungen als Bestandteil eines erfindungsgemäßen Kits eingesetzt. Die vorliegende Erfindung betrifft somit auch ein Kit, umfassend

- Ein, zwei oder mehr als zwei erfindungsgemäße polymerisierbare, dentale Zusammensetzungen in einer Spritze und/oder Compule,

- optional ein, zwei oder mehr als zwei Bondings,

- optional ein, zwei oder mehr als zwei Ätzgele,

- optional eine oder mehr als eine Farbskala,

- optional einen oder mehr als einen Pinsel.

Beispiele:
Abkürzungen:

| | |
|---|---|
| CQ: | DL-Campherchinon |
| DABE: | 4-(Dimethylamino)benzoesäureethylester |
| BHT: | 2,6-Di-tert.-butyl-4-methylphenol |
| BPO: | Dibenzoylperoxid |
| DEPT: | *N,N*-Bis(2-hydroxyethyl)-p-toluidin |
| NTPB: | Natriumtetraphenylborat |
| BisGMA: | 2,2-Bis[4-(2-hydroxy-3-methacryloxypropyloxy)phenyl]propan) |
| TEGDMA: | Triethylenglycoldimethacrylat |
| MDP: | 10-Methacryloyloxydecylphosphat |

Dentalglas 1: Barium-Aluminium-Borosilikat-Glas (D50 0,8 μm / D25 0,5 μm / D75 1,0 μm), silanisiert mit γ-Methacyloxypropyltrimethoxysilan

Dentalglas 2: Barium-Aluminium-Borosilikat-Glas (D50 2,7 μm / D25 1,4 μm / D75 6,1 μm), silanisiert mit γ-Methacryloxypropyltrimethoxysilan

Pyrogen.-$SiO_2$: pyrogene Kieselsäure (D50 40 nm), silanisiert mit γ-Methacryloxypropyltrimethoxysilan

Nano-$SiO_2$: nicht agglomerierte, nicht aggregierte Kieselsäure (D50 40 nm), silanisiert mit γ-Methacryloxypropyltrimethoxysilan

Synthese der Silane (a1):

Synthese von 2-Methyl-2-propensäure-9,9-dimethoxy-4-oxo-5,10-dioxa-3-aza-9-silaundec-1-yl-ester (Beispiel 1a)

18.03 g (0.1 mol) 3-(Trimethoxysilyl)-1-propanol werden mit 15.52 g (0.1 mol) 2-Isocyanatoethylmethacrylat, 0.25 Gew.-% Dibutylzinndilaurat und 200 ppm BHT unter Rühren für 6 Stunden auf 55 °C erwärmt. Als Reaktionskontrolle wird das Verschwinden der NCO-Bande bei ca. 2270 $cm^{-1}$ im IR-Spektrum beobachtet. Es wird eine schwach gelbe Flüssigkeit erhalten. Viskosität (25°C): 20 mPa*s. $n^D_{20}$:1.448.

IR (Film): $\tilde{\nu}$ ($cm^{-1}$) 3361 (w, NH), 2972 (w), 1715 (vs, C=O), 1639 (w), 1525 (m), 1447 (w), 1244 (m), 1166 (m), 1073 (vs), 946 (s), 771 (s).

$^1H$ NMR (60 MHz, $CDCl_3$): $\delta$ (ppm) 0.7 - 1.0 (m, 2H, $SiCH_2$), 1.9 - 2.1 (m, 2H, $SiCH_2CH_2$), 2.05 (s, 3H, $CH_3$), 3.2 - 3.5 (m, 2H, $SiCH_2CH_2CH_2$), 3.60 (s, 9H, $OCH_3$), 4.07 (t, 2H, $NCH_2$), 4.42 (t, 2H, $OCH_2$), 5.05 (s, 1H, NH), 5.59 (1H, C=CH), 6.17 (1H, C=C*H*).

Synthese von 2-Methyl-2-propensäure-9,9-dimethoxy-4-oxo-5-oxa-3-aza-9-siladec-1-yl-ester (Beispiel 1b)

16.43 g (0.1 mol) 3-(Methyldimethoxysilyl)-1-propanol werden mit 15.52 g (0.1 mol) 2-Isocyanatoethylmethacrylat, 0.25 Gew.-% Dibutylzinndilaurat und 200 ppm BHT unter Rühren für 6 Stunden auf 55 °C erwärmt. Als Reaktionskontrolle wird das Verschwinden der NCO-Bande bei ca. 2270 $cm^{-1}$ im IR-Spektrum beobachtet. Es wird eine schwach gelbe Flüssigkeit erhalten. Viskosität (25°C): 20 mPa*s. $n^D_{20}$:1.460.

IR (Film): $\tilde{\nu}$ ($cm^{-1}$) 3353 (w, NH), 2946 (w), 1713 (vs, C=O), 1637 (w), 1529 (m), 1451 (w), 1247 (m), 1166 (m), 1077 (vs), 945 (s), 767 (s).

$^1H$ NMR (60 MHz, $CDCl_3$): $\delta$ (ppm) 0.12 (s, 3H $SiCH_3$), 0.9 - 1.2 (m, 2H, $SiCH_2$), 1.9 - 2.1 (m, 2H, $SiCH_2CH_2$), 2.05 (s, 3H, $CH_3$), 3.2 - 3.5 (m, 2H, $SiCH_2CH_2CH_2$), 3.62 (s, 6H, $OCH_3$), 4.08 (t, 2H, $NCH_2$), 4.42 (t, 2H, $OCH_2$), 5.05 (s, 1H, NH), 5.59 (1H, C=C*H*), 6.17 (1H, C=C*H*).

Synthese von 2-Methyl-2-propensäure-7,7-diethoxy-4-oxo-5,8-dioxa-3-aza-7-siladec-1-yl-ester (Beispiel 1c)

19.43 g (0.1 mol) 1-(Triethoxysilyl)-methanol werden mit 15.52 g (0.1 mol) 2-Isocyanatoethylmethacrylat, 0.25 Gew.-% Dibutylzinndilaurat und 200 ppm BHT unter Rühren für 6 Stunden auf 55 °C erwärmt. Als Reaktionskontrolle wird das Verschwinden der NCO-Bande bei ca. 2270 $cm^{-1}$ im IR-Spektrum beobachtet. Es wird eine schwach gelbe Flüssigkeit erhalten. Viskosität (25°C): 21 mPa*s. $n^D_{20}$:1.455.

IR (Film): $\tilde{\nu}$ ($cm^{-1}$) 3352 (w, NH), 2970 (w), 1717 (vs, C=O), 1635 (w), 1532 (m), 1451 (w), 1245 (m), 1163 (m), 1072 (vs), 945 (s), 776 (s).

$^1H$ NMR (60 MHz, $CDCl_3$): $\delta$ (ppm) 1.32 (t, 9H, $OCH_2CH_3$), 2.05 (s, 3H, $CH_3$), 2.70 (m, 2H, $SiCH_2$), 3.92 (q, 6H, $OCH_2CH_3$), 4.09 (t, 2H, $NCH_2$), 4.41 (t, 2H, $OCH_2$), 5.07 (s, 1H, NH), 5.57 (1H, C=C*H*), 6.19 (1H, C=C*H*).

Synthese von 2-Methyl-2-propensäure-9,9-dimethoxy-4-oxo-3,10-dioxa-5-aza-9-silaundec-1-yl-ester (Beispiel 1d)

20.53 g (0.1 mol) 3-(Trimethoxysilyl)propylisocyanat werden mit 13.01 g (0.1 mol) 2-Hydroxyethylmethacrylat (HEMA), 0.25 Gew.-% Dibutylzinndilaurat und 200 ppm BHT unter Rühren für 6 Stunden auf 55 °C erwärmt. Als Reaktionskontrolle wird das Verschwinden der NCO-Bande bei ca. 2270 $cm^{-1}$ im IR-Spektrum beobachtet. Es wird eine schwach gelbe Flüssigkeit erhalten. Viskosität (25°C): 21 mPa*s. $n^D_{20}$:1.448.

IR (Film): $\tilde{\nu}$ ($cm^{-1}$) 3356 (w, NH), 2974 (w), 1716 (vs, C=O), 1638 (w), 1527 (m), 1448 (w), 1242 (m), 1164 (m), 1072 (vs), 947 (s), 773 (s).

$^1H$ NMR (60 MHz, $CDCl_3$): $\delta$ (ppm) 0.6 - 0.9 (m, 2H, $SiCH_2$), 1.6 - 1.8 (m, 2H, $SiCH_2CH_2$), 2.05 (s, 3H, $CH_3$), 3.1 - 3.4 (m, 2H, $SiCH_2CH_2CH_2$), 3.61 (s, 9H, $OCH_3$), 4.21 (t, 2H, $OCH_2$), 4.41 (t, 2H, $OCH_2$), 5.17 (s, 1H, NH), 5.68 (1H, C=C*H*), 6.24 (1H, C=C*H*).

Synthese von 2-Methyl-2-propensäure-2-methyl-9,9-dimethoxy-4-oxo-3,10-dioxa-5-aza-9-silaundec-1-yl-ester (Beispiel 1e)

20.53 g (0.1 mol) 3-(Trimethoxysilyl)propylisocyanat werden mit 14.42 g (0.1 mol) 2-Hydroxypropylmethacrylat (HPMA), 0.25 Gew.-% Dibutylzinndilaurat und 200 ppm BHT unter Rühren für 6 Stunden auf 55 °C erwärmt. Als Reaktionskontrolle wird das Verschwinden der NCO-Bande bei ca. 2270 $cm^{-1}$ im IR-Spektrum beobachtet. Es wird eine schwach gelbe Flüssigkeit erhalten. Viskosität (25°C): 27 mPa*s. $n^D_{20}$:1.448.

IR (Film): $\tilde{\nu}$ ($cm^{-1}$) 3360 (w, NH), 2975 (w), 1713 (vs, C=O), 1638 (w), 1526 (m), 1449 (w), 1242 (m), 1164 (m), 1072 (vs), 947 (s), 773 (s).

$^1H$ NMR (60 MHz, $CDCl_3$): $\delta$ (ppm) 0.4 - 0.7 (m, 2H, $SiCH_2$), 1.15 (d, 3H, $CH_3$), 0.4 - 1.7 (m, 2H, $SiCH_2CH_2$), 1.94 (s, 3H, $CH_3$), 3.0 - 3.3 (m, 2H, $SiCH_2CH_2CH_2$), 3.61 (s, 9H, $OCH_3$), 4.15 (d, 4H, $OCH_2$), 4.8 - 5.2 (m, 2H, NH + OC*H*), 5.52 (1H, C=C*H*), 6.12 (1H, C=C*H*).

Synthese von 2-Methyl-2-propensäure-2-[[[[3-(trimethoxysilyl)propyl] amino]carbonyl]oxy]-1,3-propanediyl-ester (Beispiel 1f)

20.53 g (0.1 mol) 3-(Trimethoxysilyl)propylisocyanat werden mit 22.82 g (0.1 mol) Glycerin-1,3-dimethacrylat, 0.25 Gew.-% Dibutylzinndilaurat und 200 ppm BHT unter Rühren für 6 Stunden auf 55 °C erwärmt. Als Reaktionskontrolle wird das Verschwinden der NCO-Bande bei ca. 2270 $cm^{-1}$ im IR-Spektrum beobachtet. Es wird eine schwach gelbe Flüssigkeit erhalten. Viskosität (25°C): 100 mPa*s. $n^D_{20}$: 1.460.

IR (Film): $\tilde{\nu}$ ($cm^{-1}$) 3366 (w, NH), 2974 (w), 1718 (vs, C=O), 1638 (w), 1525 (m), 1450 (w), 1294 (w), 1241 (m), 1157 (m), 1072 (vs), 946 (s), 776 (s).

$^1H$ NMR (60 MHz, $CDCl_3$): $\delta$ (ppm) 0.5 - 0.8 (m, 2H, $SiCH_2$), 1.5 - 1.7 (m, 2H, $SiCH_2CH_2$), 1.97 (s, 3H, $CH_3$), 3.0 - 3.3 (m, 2H, $SiCH_2CH_2CH_2$), 3.61 (s, 9H, $OCH_3$), 4.3 (d, 4H, $OCH_2$), 5.00 (s, 1H, NH), 5.25 1 (m, 1H, OC*H*), 5.62 (1H, C=C*H*), 6.15 (1H, C=C*H*).

Synthese von *N*-(2-Methacryloyloxyethyl)-*N*-[[(2-Methacryloyloxyethyl)amino]-carbonyllcarbamidsäure-3-(trimethoxysilyl)propylester (Beispiel 1g)

12.02 g (0.067 mol) 3-(Trimethoxysilyl)-1-propanol werden mit 20.69 g (0.133 mol, 2 eq.) 2-Isocyanatoethylmethacrylat, 0.25 Gew.-% Dibutylzinndilaurat und 200 ppm BHT unter Rühren für 6 Stunden auf 55 °C erwärmt. Als Reaktionskontrolle wird das Verschwinden der NCO-Bande bei ca. 2270 $cm^{-1}$ im IR-Spektrum beobachtet. Es wird eine schwach gelbe Flüssigkeit erhalten. Viskosität (25°C): 34 mPa*s. $n^D_{20}$:1.454.

IR (Film): $\tilde{\nu}$ ($cm^{-1}$) 3405 (s, NH), 2972 (w), 1712 (vs, C=O), 1635 (w), 1527 (m), 1446 (w), 1245 (s), 1164 (w), 1072 (vs), 945 (s), 770 (s).

$^1H$ NMR (60 MHz, $CDCl_3$): $\delta$ (ppm) 0.7 - 1.0 (m, 2H, $SiCH_2$), 1.9 - 2.1 (m, 2H, $SiCH_2CH_2$), 2.05 (s, 3H, $CH_3$), 2.10 (s, 3H, $CH_3$), 3.2 - 3.5 (m, 2H, $SiCH_2CH_2CH_2$), 3.60 (s, 9H, $OCH_3$), 4.0 - 4.2 (4H, $NCH_2$), 4.4 - 4.6 (4H, $OCH_2$), 7.5 (1H, NH), 5.5 - 5.7 (2H, C=C*H*), 6.1 - 6.3 (2H, C=C*H*).

Synthese von 2-Methyl-2-propensäure-9,9-diethoxy-4-oxo-5-oxa-3-aza-9-silaundec-1-yl-ester (Beispiel 1h)

5.81 g (0.1 mol) 2-Propen-1-ol werden mit 15.52 g (0.1 mol) 2-Isocyanatoethylmethacrylat, 0.25 Gew.-% Dibutylzinndilaurat und 200 ppm BHT unter Rühren für 6 Stunden auf 55 °C erwärmt. Als Reaktionskontrolle wird das Verschwinden der NCO-Bande bei ca. 2270 $cm^{-1}$ im IR-Spektrum beobachtet. Es wird eine schwach gelbe Flüssigkeit erhalten, die direkt weiter umgesetzt wird. Es werden 16.43 g (0.1 mol) Triethoxysilan und 0.2% Karstedt-Katalysator-Lösung (Platin(0)-1,3-divinyl-1,1,3,3-tetramethyldisiloxan in vinyl-terminiertem Poly(dimethylsiloxan, 1% Pt) zugegeben und unter Rühren für 2 Stunden auf 80 °C erwärmt. Es wird eine schwach gelbe Flüssigkeit erhalten. Viskosität (25°C): 20 mPa*s. $n^D_{20}$:1.450.

IR (Film): $\tilde{\nu}$ ($cm^{-1}$) 3357 (w, NH), 2975 (w), 1716 (vs, C=O), 1637 (w), 1525 (m), 1447 (w), 1241 (m), 1164 (m), 1072 (vs), 947 (s), 773 (s).

$^1H$ NMR (60 MHz, $CDCl_3$): $\delta$ (ppm) 0.7 - 1.0 (m, 2H, $SiCH_2$), 1.32 (t, 9H, $OCH_2CH_3$), 1.8 - 2.0 (m, 2H, $SiCH_2CH_2$), 2.05 (s, 3H, $CH_3$), 3.4 - 3.6 (m, 2H, $SiCH_2CH_2CH_2$), 3.85 (q, 6H, $OCH_2CH_3$), 4.07 (t, 2H, $NCH_2$), 4.42 (t, 2H, $OCH_2$), 5.08 (s, 1H, NH), 5.57 (1H, C=C*H*), 6.14 (1H, C=C*H*).

Synthese von 2-Methyl-2-propensäure-9,9-diethoxy-4-oxo-3-oxa-5-aza-9-silaundec-1-yl-ester (Beispiel 1i)

8.31 g (0.1 mol) 3-Isocyanato-1-propen werden mit 13.01 g (0.1 mol) 2-Hydroxyethylmethacrylat (HEMA), 0.25 Gew.-% Dibutylzinndilaurat und 200 ppm BHT unter Rühren für 6 Stunden auf 55 °C erwärmt. Als Reaktionskontrolle wird

das Verschwinden der NCO-Bande bei ca. 2270 $cm^{-1}$ im IR-Spektrum beobachtet. Es wird eine schwach gelbe Flüssigkeit erhalten, die direkt weiter umgesetzt wird.

Es werden 16.43 g (0.1 mol) Triethoxysilan und 0.2% Karstedt-Katalysator-Lösung (Platin(0)-1,3-divinyl-1,1,3,3-tetramethyldisiloxan in vinyl-terminiertem Polydimethylsiloxan, 1% Pt) zugegeben und unter Rühren für 2 Stunden auf 80 °C erwärmt. Es wird eine schwach gelbe Flüssigkeit erhalten. Viskosität (25°C): 21 mPa*s. $n^D_{20}$:1.450.

IR (Film): $\tilde{v}$ ($cm^{-1}$) 3356 (w, NH), 2974 (w), 1716 (vs, C=O), 1638 (w), 1527 (m), 1448 (w), 1242 (m), 1164 (m), 1072 (vs), 947 (s), 773 (s).

$^1H$ NMR (60 MHz, $CDCl_3$): $\delta$ (ppm) 0.6 - 0.9 (m, 2H, $SiCH_2$), 1.32 (t, 9H, $OCH_2CH_3$), 1.6 - 1.8 (m, 2H, $SiCH_2CH_2$), 2.05 (s, 3H, $CH_3$), 3.1 - 3.4 (m, 2H, $SiCH_2CH_2CH_2$), 3.92 (q, 6H, $OCH_2CH_3$), 4.41 (s, 4H, $OCH_2CH_2O$), 5.17 (s, 1H, NH), 5.68 (1H, C=C*H*), 6.24 (1H, C=C*H*).

Synthese der Silane (a2):

Synthese von (3-Methacryloyloxypropyl)methyldimethoxysilan (Beispiel 2a)

Es werden 12.62 g (0.1 mol) Allylmethacrylat, 10.62 g (0.1 mol) Methyldimethoxysilan und 0.2% Karstedt-Katalysator-Lösung (Platin(0)-1,3-divinyl-1,1,3,3-tetramethyldisiloxan in vinyl-terminiertem Polydimethylsiloxan, 1% Pt) unter Rühren für 2 Stunden auf 80 °C erwärmt. Es wird eine schwach gelbe Flüssigkeit erhalten. Viskosität (25°C): 5 mPa*s. $n^D_{20}$:1.436.

IR (Film): $\tilde{v}$ ($cm^{-1}$) 2947 (w), 1718 (vs, C=O), 1638 (w), 1525 (m), 1454 (w), 1296 (m), 1162 (s), 1080 (vs), 814 (s), 765 (s).

$^1H$ NMR (60 MHz, $CDCl_3$): $\delta$ (ppm) 0.12 (s, 3H $SiCH_3$), 0.6 - 0.9 (m, 2H, $SiCH_2$), 1.7 - 1.9 (m, 2H, $SiCH_2CH_2$), 2.00 (s, 3H, $CH_3$), 3.56 (s, 3H $OCH_3$), 4.16 (t, 2H, $SiCH_2CH_2CH_2$), 5.58 (1H, C=C*H*), 6.14 (1H, C=C*H*).

Synthese von N-[3-(Dimethoxymethylsilyl)propyl]-2-methyl-2-propenamid (Beispiel 2b)

Es werden 12.52 g (0.1 mol) *N*-Allylmethacrylamid, 10.62 g (0.1 mol) Methyldimethoxysilan und 0.2% Karstedt-Katalysator-Lösung (Platin(0)-1,3-divinyl-1,1,3,3-tetramethyldisiloxan in vinyl-terminiertem Polydimethylsiloxan, 1% Pt) unter Rühren für 2 Stunden auf 80 °C erwärmt. Es wird eine schwach gelbe Flüssigkeit erhalten. Viskosität (25°C): 9 mPa*s. $n^D_{20}$:1.439.

IR (Film): $\tilde{v}$ ($cm^{-1}$) 3351 (w, NH), 2948 (w), 1716 (vs, C=O), 1636 (w), 1524 (m), 1452 (w), 1295 (m), 1162 (s), 1079 (vs), 812 (s), 766 (s).

$^1H$ NMR (60 MHz, $CDCl_3$): $\delta$ (ppm) 0.12 (s, 3H $SiCH_3$), 0.4 - 0.7 (m, 2H, $SiCH_2$), 1.5 - 1.7 (m, 2H, $SiCH_2CH_2$), 2.05 (s, 3H, $CH_3$), 3.0 - 3.3 (m, 2H, $SiCH_2CH_2CH_2$), 3.50 (s, 3H $OCH_3$), 5.36 (1H, C=C*H*), 5.85 (1H, C=C*H*), 6.94 (1H, NH).

Synthese der Silane (a3):

Synthese von Dimethoxyphenylmethylsilan (Beispiel 3a)

Entsprechend Beispiel 1 aus WO 2012/091154 A1 werden 0.47g (1.5 mmol) Bismuth(III)-chlorid, 8.82 g (0.1 mol) *tert*.-Butylmethylether und 19.11 g (0.1 mol) Dichlorophenylmethylsilan umgesetzt. Es wird ein schwach gelber Feststoff erhalten. Smp.: 73 °C. $n^D_{20}$:1.469.

Synthese von 9,9-Dimethoxy-9*H*-9-silafluoren (Beispiel 3b)

Entsprechend Beispiel 1 aus WO 2012/091154 A1 werden 0.47g (1.5 mmol) Bismuth(III)-chlorid, 8.82 g (0.1 mol) tert.-Butylmethylether und 25.12 g (0.1 mol) 9,9-Dichloro-9*H*-9-silafluoren umgesetzt. Es wird ein langsam kristallisierendes gelbes Öl erhalten. $n^D_{20}$:1.545.

Synthese der Polysiloxane:

Allgemeine Kondensationsvorschrift:

Es werden 0.1 mol Silanverbindung(en) und 0.5 mmol BHT in 100 ml Ethylacetat gelöst. Anschließend werden 2.5 ml 1N HCl-Lösung zugegeben und für 72 h auf 30 °C erwärmt. Es wird mit 2N NaOH-Lösung ausgeschüttelt und die organische Phase mit Wasser gewaschen. Anschließend wird über Magnesiumsulfat getrocknet und das Lösungsmittel im Vakuum entfernt.

Beispiel 4a: Entsprechend der allgemeinen Kondensationsvorschrift werden 0.2 mol 2-Methyl-2-propensäure-9,9-dimethoxy-4-oxo-5,10-dioxa-3-aza-9-silaundec-1-yl-ester (Silan a1), 0.2 mol γ-(Methacyloxypropyl)trimethoxysilan (Silan a2) und 0.2 mol Dimethoxydiphenylsilan (Silan a3) in Gegenwart von 0.3 mmol BHT in 60 ml Ethylacetat kondensiert. Es wird eine schwach gelbe Flüssigkeit erhalten. Viskosität (25°C): 24 Pa*s. $n^D_{20}$: 1.515.

Beispiel 4b: Entsprechend der allgemeinen Kondensationsvorschrift werden 0.4 mol 2-Methyl-2-propensäure-9,9-dimethoxy-4-oxo-5,10-dioxa-3-aza-9-silaundec-1-yl-ester (Silan a1), 0.2 mol γ-(Methacyloxypropyl)trimethoxysilan (Silan a2) und 0.2 mol Dimethoxydiphenylsilan (Silan a3) in Gegenwart von 0.4 mmol BHT in 80 ml Ethylacetat kondensiert. Es wird eine schwach gelbe Flüssigkeit erhalten. Viskosität (25°C): 29 Pa*s. $n^D_{20}$: 1.509.

Beispiel 4c: Entsprechend der allgemeinen Kondensationsvorschrift werden 0.4 mol 2-Methyl-2-propensäure-7,7-diethoxy-4-oxo-5,8-dioxa-3-aza-7-siladec-1-yl-ester (Silan a1), 0.2 mol γ-(Methacyloxypropyl)trimethoxysilan (Silan a2) und 0.2 mol Dimethoxydiphenylsilan (Silan a3) in Gegenwart von 0.4 mmol BHT in 80 ml Ethylacetat kondensiert. Es wird eine schwach gelbe Flüssigkeit erhalten. Viskosität (25°C): 28 Pa*s. $n^D_{20}$:1.510.

Beispiel 4d: Entsprechend der allgemeinen Kondensationsvorschrift werden 0.4 mol *N*-(2-Methacryloyloxyethyl)-N-[[(2-Methacryloyloxyethyl)amino]carbonyl]-carbamidsäure-3-(trimethoxysilyl)propylester (Silan a1), 0.2 mol γ-(Methacyloxy-propyl)trimethoxysilan (Silan a2) und 0.2 mol Dimethoxydiphenylsilan (Silan a3) in Gegenwart von 0.4 mmol BHT in 80 ml Ethylacetat kondensiert. Es wird eine schwach gelbe Flüssigkeit erhalten. Viskosität (25°C): 38 Pa*s. $n^D_{20}$:1.518.

Beispiel 5a: Entsprechend der allgemeinen Kondensationsvorschrift werden 0.8 mol 2-Methyl-2-propensäure-9,9-dimethoxy-4-oxo-5,10-dioxa-3-aza-9-silaundec-1-yl-ester (Silan a1) in Gegenwart von 0.4 mmol BHT in 80 ml Ethylacetat kondensiert. Es wird eine schwach gelbe Flüssigkeit erhalten.

Beispiel 5b: Entsprechend der allgemeinen Kondensationsvorschrift werden 0.8 mol 2-Methyl-2-propensäure-9,9-dimethoxy-4-oxo-5-oxa-3-aza-9-siladec-1-yl-ester (Silan a1) in Gegenwart von 0.4 mmol BHT in 80 ml Ethylacetat kondensiert. Es wird eine schwach gelbe Flüssigkeit erhalten.

Beispiel 5c: Entsprechend der allgemeinen Kondensationsvorschrift werden 0.8 mol 2-Methyl-2-propensäure-7,7-diethoxy-4-oxo-5,8-dioxa-3-aza-7-siladec-1-yl-ester (Silan a1) in Gegenwart von 0.4 mmol BHT in 80 ml Ethylacetat kondensiert. Es wird eine schwach gelbe Flüssigkeit erhalten.

Beispiel 5d: Entsprechend der allgemeinen Kondensationsvorschrift werden 0.8 mol 2-Methyl-2-propensäure-2-[[[[3-(trimethoxysilyl)propyl]amino]carbonyl]oxy]-1,3-propanediyl-ester (Silan a1) in Gegenwart von 0.4 mmol BHT in 80 ml Ethylacetat kondensiert. Es wird eine schwach gelbe Flüssigkeit erhalten.

Beispiel 5e: Entsprechend der allgemeinen Kondensationsvorschrift werden 0.6 mol *N*-(2-Methacryloyloxyethyl)-*N*-[[(2-Methacryloyloxyethyl)amino]carbonyl]-carbamidsäure-3-(trimethoxysilyl)propylester (Silan a1) in Gegenwart von 0.3 mmol BHT in 60 ml Ethylacetat kondensiert. Es wird eine schwach gelbe Flüssigkeit erhalten.

Beispiel 5f: Entsprechend der allgemeinen Kondensationsvorschrift werden 0.8 mol γ-(Methacyloxypropyl) trimethoxysilan (Silan a2) in Gegenwart von 0.4 mmol BHT in 80 ml Ethylacetat kondensiert. Es wird eine schwach gelbe Flüssigkeit erhalten. Viskosität (25°C): 20 Pa*s. $n^D_{20}$: 1.479.

Beispiel 5g: Entsprechend der allgemeinen Kondensationsvorschrift werden 0.8 mol γ-(Methacyloxypropyl) methyldimethoxysilan (Silan a2) in Gegenwart von 0.4 mmol BHT in 80 ml Ethylacetat kondensiert. Es wird eine schwach gelbe Flüssigkeit erhalten. Viskosität (25°C): 3 Pa*s. $n^D_{20}$:1.466.

Beispiel 5h: Entsprechend der allgemeinen Kondensationsvorschrift werden 0.8 mol (Methacryloxymethyl) trimethoxysilan (Silan a2) in Gegenwart von 0.4 mmol BHT in 80 ml Ethylacetat kondensiert. Es wird eine schwach gelbe Flüssigkeit erhalten. Viskosität (25°C): 18 Pa*s. $n^D_{20}$: 1.475.

Beispiel 5i: Entsprechend der allgemeinen Kondensationsvorschrift werden 0.8 mol (Methacryloxymethyl) methyldimethoxysilan (Silan a2) in Gegenwart von 0.4 mmol BHT in 80 ml Ethylacetat kondensiert. Es wird eine schwach gelbe Flüssigkeit erhalten. Viskosität (25°C): 3 Pa*s. $n^D_{20}$:1.462.

Beispiel 5j: Entsprechend der allgemeinen Kondensationsvorschrift werden 0.8 mol 2-Dimethoxydiphenylsilan (Silan a3) in Gegenwart von 0.4 mmol BHT in 80 ml Ethylacetat kondensiert. Es wird eine schwach gelbe Flüssigkeit erhalten.

Beispiel 5k: Entsprechend der allgemeinen Kondensationsvorschrift werden 0.8 mol 2-Dimethoxyphenylmethylsilan (Silan a3) in Gegenwart von 0.4 mmol BHT in 80 ml Ethylacetat kondensiert. Es wird eine schwach gelbe Flüssigkeit erhalten.

Beispiel 5l: Entsprechend der allgemeinen Kondensationsvorschrift werden 0.8 mol 2-9,9-Dimethoxy-9H-9-silafluoren (Silan a3) in Gegenwart von 0.4 mmol BHT in 80 ml Ethylacetat kondensiert. Es wird eine schwach gelbe Flüssigkeit erhalten.

Beispiel 5m: Entsprechend der allgemeinen Kondensationsvorschrift werden 0.8 mol Dimethoxydi-1-naphthalenylsilan (Silan a3) in Gegenwart von 0.4 mmol BHT in 80 ml Ethylacetat kondensiert. Es wird eine schwach gelbe Flüssigkeit erhalten.

Beispiel 6a: Entsprechend der allgemeinen Kondensationsvorschrift werden 0.4 mol γ-(Methacyloxypropyl) trimethoxysilan (Silan a2) und 0.4 mol Dimethoxydiphenylsilan (Silan a3) in Gegenwart von 0.4 mmol BHT in 80 ml Ethylacetat kondensiert. Es wird eine schwach gelbe Flüssigkeit erhalten. Viskosität (25°C): 24 Pa*s. $n^D_{20}$:1.523.

Beispiel 6b: Entsprechend der allgemeinen Kondensationsvorschrift werden 0.4 mol γ-(Methacyloxypropyl)methyldimethoxysilan (Silan a2) und 0.4 mol Dimethoxydiphenylsilan (Silan a3) in Gegenwart von 0.4 mmol BHT in 80 ml Ethylacetat kondensiert. Es wird eine schwach gelbe Flüssigkeit erhalten. Viskosität (25°C): 2 Pa*s. $n^{D}_{20}$:1.534.

Beispiel 6c: Entsprechend der allgemeinen Kondensationsvorschrift werden 0.4 mol (Methacryloxymethyl) trimethoxysilan (Silan a2) und 0.4 mol Dimethoxydiphenylsilan (Silan a3) in Gegenwart von 0.4 mmol BHT in 80 ml Ethylacetat kondensiert. Es wird eine schwach gelbe Flüssigkeit erhalten. Viskosität (25°C): 3 Pa*s. $n^{D}_{20}$:1.546.

Beispiel 6d: Entsprechend der allgemeinen Kondensationsvorschrift werden 0.4 mol (Methacryloxymethyl) trimethoxysilan (Silan a2) und 0.4 mol Dimethoxydi-1-naphthalenylsilan (Silan a3) in Gegenwart von 0.4 mmol BHT in 80 ml Ethylacetat kondensiert. Es wird eine schwach gelbe Flüssigkeit erhalten. Viskosität (25°C): 4 Pa*s. $n^{D}_{20}$: 1.565.

Beispiel 6e: Entsprechend der allgemeinen Kondensationsvorschrift werden 0.4 mol 2-Methyl-2-propensäure-9,9-dimethoxy-4-oxo-5,10-dioxa-3-aza-9-silaundec-1-yl-ester (Silan a1) und 0.4 mol (Methacryloxymethyl)trimethoxysilan (Silan a2) in Gegenwart von 0.4 mmol BHT in 80 ml Ethylacetat kondensiert. Es wird eine schwach gelbe Flüssigkeit erhalten.

Beispiel 7 (stopfbare Füllungskomposite):

<table>
<tr><th>Beispiel</th><th>7a</th><th>7b</th><th colspan="3">7c</th><th>7d</th></tr>
<tr><td>Polysiloxan (a1)</td><td>Beispiel 5a 6.00</td><td rowspan="3">Beispiel 4a 18.00</td><td rowspan="2">Bsp. 6e 9.00</td><td rowspan="3">+</td><td>-</td><td>Beispiel 5a 6.00</td></tr>
<tr><td>Polysiloxan (a2)</td><td>Beispiel 5f 6.00</td><td rowspan="2">Bsp. 6a 9.00</td><td rowspan="2">Beispiel 6a 12.00</td></tr>
<tr><td>Polysiloxan (a3)</td><td>Beispiel 5j 6.00</td><td>-</td></tr>
<tr><td>Dentalglas 1</td><td>14.00</td><td>14.00</td><td colspan="3">14.00</td><td>14.00</td></tr>
<tr><td>Dentalglas 2</td><td>62.00</td><td>62.00</td><td colspan="3">62.00</td><td>62.00</td></tr>
<tr><td>Pyrogen.-$SiO_2$</td><td>5.00</td><td>5.00</td><td colspan="3">5.00</td><td>5.00</td></tr>
<tr><td>Nano-$SiO_2$</td><td>-</td><td>-</td><td colspan="3">-</td><td>-</td></tr>
<tr><td>CQ</td><td>0.40</td><td>0.40</td><td colspan="3">0.40</td><td>0.40</td></tr>
<tr><td>DABE</td><td>0.60</td><td>0.60</td><td colspan="3">0.60</td><td>0.60</td></tr>
<tr><td>BF [MPa]</td><td>120.4</td><td>125.7</td><td colspan="3">122.4</td><td>128.1</td></tr>
<tr><td>E-Modul [GPa]</td><td>9.9</td><td>10.2</td><td colspan="3">10.1</td><td>10.8</td></tr>
<tr><td>Schrumpfung [%]</td><td>1.72%</td><td>1.62%</td><td colspan="3">1.62%</td><td>1.55%</td></tr>
</table>

<table>
<tr><th>Beispiel</th><th>7e</th><th>7f</th><th>7g</th><th>7h</th></tr>
<tr><td>Polysiloxan (a1)</td><td rowspan="3">Beispiel 4a 18.00</td><td>Beispiel 5a 6.00</td><td>Beispiel 5a 6.00</td><td rowspan="3">Beispiel 4b 18.00</td></tr>
<tr><td>Polysiloxan (a2)</td><td>Beispiel 5f 6.00</td><td rowspan="2">Beispiel 6a 12.00</td></tr>
<tr><td>Polysiloxan (a3)</td><td>Beispiel 5j 6.00</td></tr>
<tr><td>Dentalglas 1</td><td>12.00</td><td>12.00</td><td>12.00</td><td>12.00</td></tr>
<tr><td>Dentalglas 2</td><td>56.00</td><td>56.00</td><td>56.00</td><td>56.00</td></tr>
<tr><td>Pyrogen.-$SiO_2$</td><td>-</td><td>-</td><td>-</td><td>-</td></tr>
<tr><td>Nano-$SiO_2$</td><td>13.00</td><td>13.00</td><td>13.00</td><td>13.00</td></tr>
<tr><td>CQ</td><td>0.40</td><td>0.40</td><td>0.40</td><td>0.40</td></tr>
<tr><td>DABE</td><td>0.60</td><td>0.60</td><td>0.60</td><td>0.60</td></tr>
<tr><td>BF [MPa]</td><td>142.5</td><td>130.4</td><td>138.1</td><td>135.6</td></tr>
<tr><td>E-Modul [GPa]</td><td>11.1</td><td>10.9</td><td>10.8</td><td>10.9</td></tr>
</table>

(fortgesetzt)

| Beispiel | 7e | 7f | 7g | 7h |
|---|---|---|---|---|
| Schrumpfung [%] | 1.23% | 1.32% | 1.25% | 1.27% |

<table>
<tr><td>Beispiel</td><td>7i</td><td>7j</td><td>7k</td><td colspan="3">7l</td></tr>
<tr><td>Polysiloxan (a1)</td><td rowspan="3">Beispiel 4a 12.00</td><td>Beispiel 5a 4.00</td><td>Beispiel 5c 8.00</td><td rowspan="3">Bsp. 4a 12.25</td><td rowspan="3">+</td><td>Bsp. 5a 3.25</td></tr>
<tr><td>Polysiloxan (a2)</td><td>Beispiel 5f 4.00</td><td rowspan="2">Beispiel 6d 10.00</td><td rowspan="2">Bsp. 6d 2.25</td></tr>
<tr><td>Polysiloxan (a3)</td><td>Beispiel 5j 4.00</td></tr>
<tr><td>BisGMA</td><td>4.00</td><td>4.00</td><td>-</td><td colspan="3">-</td></tr>
<tr><td>TEGDMA</td><td>2.00</td><td>2.00</td><td>-</td><td colspan="3">-</td></tr>
<tr><td>Dentalglas 1</td><td>14.00</td><td>14.00</td><td>12.00</td><td colspan="3">11.30</td></tr>
<tr><td>Dentalglas 2</td><td>62.00</td><td>62.00</td><td>56.00</td><td colspan="3">56.55</td></tr>
<tr><td>Pyrogen-$SiO_2$</td><td>5.00</td><td>5.00</td><td>-</td><td colspan="3">-</td></tr>
<tr><td>$SiO_2$ 40 nm</td><td>-</td><td>-</td><td>13.00</td><td colspan="3">13.40</td></tr>
<tr><td>CQ</td><td>0.40</td><td>0.40</td><td>0.40</td><td colspan="3">0.40</td></tr>
<tr><td>DABE</td><td>0.60</td><td>0.60</td><td>0.60</td><td colspan="3">0.60</td></tr>
<tr><td>BF [MPa]</td><td>121.3</td><td>118.7</td><td>132.5</td><td colspan="3">142.5</td></tr>
<tr><td>E-Modul [GPa]</td><td>10.4</td><td>10.1</td><td>11.2</td><td colspan="3">11.6</td></tr>
<tr><td>Schrumpfung [%]</td><td>1.72%</td><td>1.83%</td><td>1.24%</td><td colspan="3">1.21%</td></tr>
</table>

<table>
<tr><td>Beispiel</td><td>7m</td><td>7n</td><td>7o</td><td colspan="3">7p</td></tr>
<tr><td>Polysiloxan (a1)</td><td rowspan="2">Beispiel 4d 17.70</td><td>Beispiel 5e 7.60</td><td>Beispiel 5e 6.00</td><td rowspan="3">Bsp. 4d 12.25</td><td rowspan="3">+</td><td rowspan="3">Bsp. 4a 5.45</td></tr>
<tr><td>Polysiloxan (a2)</td><td rowspan="2">Beispiel 6a 9.90</td><td>Beispiel 5f 6.00</td></tr>
<tr><td>Polysiloxan (a3)</td><td></td><td>Beispiel 5j 6.00</td></tr>
<tr><td>Dentalglas 1</td><td>11.30</td><td>11.30</td><td>11.30</td><td colspan="3">11.30</td></tr>
<tr><td>Dentalglas 2</td><td>56.60</td><td>56.80</td><td>56.30</td><td colspan="3">56.60</td></tr>
<tr><td>Pyrogen-$SiO_2$</td><td>0.50</td><td>0.50</td><td>0.50</td><td colspan="3">0.50</td></tr>
<tr><td>$SiO_2$ 40 nm</td><td>13.40</td><td>13.40</td><td>13.40</td><td colspan="3">13.40</td></tr>
<tr><td>CQ</td><td>0.20</td><td>0.20</td><td>0.20</td><td colspan="3">0.20</td></tr>
<tr><td>DABE</td><td>0.30</td><td>0.30</td><td>0.30</td><td colspan="3">0.30</td></tr>
<tr><td>BF [MPa]</td><td>146.3</td><td>145.8</td><td>141.7</td><td colspan="3">147.3</td></tr>
<tr><td>E-Modul [GPa]</td><td>12.2</td><td>12.1</td><td>11.5</td><td colspan="3">12.5</td></tr>
<tr><td>Schrumpfung [%]</td><td>1.13%</td><td>1.16%</td><td>1.26%</td><td colspan="3">1.21%</td></tr>
</table>

Beispiel 8 (fließfähige Füllungskomposite):

<table>
<tr><th>Beispiel</th><th>8a</th><th>8b</th><th>8c</th><th>8d</th></tr>
<tr><td>Polysiloxan (a1)</td><td rowspan="3">Beispiel 4a<br>35.00</td><td rowspan="3">Beispiel 4b<br>35.00</td><td>Beispiel 5a<br>11.00</td><td>Beispiel 5a<br>11.00</td></tr>
<tr><td>Polysiloxan (a2)</td><td>Beispiel 5f<br>14.00</td><td rowspan="2">Beispiel 6a<br>21.00</td></tr>
<tr><td>Polysiloxan (a3)</td><td>Beispiel 5j<br>7.00</td></tr>
<tr><td>Dentalglas 1</td><td>10.00</td><td>10.00</td><td>10.50</td><td>10.50</td></tr>
<tr><td>Dentalglas 2</td><td>43.00</td><td>43.00</td><td>45.50</td><td>45.50</td></tr>
<tr><td>Pyrogen.-$SiO_2$</td><td>-</td><td>-</td><td>-</td><td>-</td></tr>
<tr><td>Nano-$SiO_2$</td><td>11.00</td><td>11.00</td><td>11.00</td><td>11.00</td></tr>
<tr><td>CQ</td><td>0.40</td><td>0.40</td><td>0.40</td><td>0.40</td></tr>
<tr><td>DABE</td><td>0.60</td><td>0.60</td><td>0.60</td><td>0.60</td></tr>
<tr><td>BF [MPa]</td><td>118.3</td><td>116.8</td><td>121.0</td><td>123.1</td></tr>
<tr><td>E-Modul [GPa]</td><td>7.2</td><td>6.9</td><td>7.5</td><td>7.7</td></tr>
<tr><td>Schrumpfung [%]</td><td>2.49%</td><td>2.38%</td><td>2.31%</td><td>2.28%</td></tr>
</table>

Beispiel 9 (dualhärtendes Stumpfaufbaumaterial):

<table>
<tr><th>Beispiel</th><th colspan="2">9a</th><th colspan="2">9b</th></tr>
<tr><td>Polysiloxan (a1)</td><td rowspan="3">Beispiel 4a<br>35.00</td><td rowspan="3">Beispiel 4a<br>35.00</td><td>Beispiel 5a<br>11.00</td><td>Beispiel 5a<br>11.00</td></tr>
<tr><td>Polysiloxan (a2)</td><td>Beispiel 5f<br>14.00</td><td>Beispiel 5f<br>14.00</td></tr>
<tr><td>Polysiloxan (a3)</td><td>Beispiel 5j<br>7.00</td><td>Beispiel 5j<br>7.00</td></tr>
<tr><td>Dentalglas 1</td><td>10.00</td><td>10.00</td><td>10.50</td><td>10.50</td></tr>
<tr><td>Dentalglas 2</td><td>43.00</td><td>43.00</td><td>45.50</td><td>45.50</td></tr>
<tr><td>Pyrogen.-$SiO_2$</td><td>3.00</td><td>3.00</td><td>3.00</td><td>3.00</td></tr>
<tr><td>Nano-$SiO_2$</td><td>8.00</td><td>8.00</td><td>8.00</td><td>8.00</td></tr>
<tr><td>DEPT</td><td>0.60</td><td>-</td><td>0.60</td><td>-</td></tr>
<tr><td>BPO</td><td>-</td><td>1.00</td><td>-</td><td>1.00</td></tr>
<tr><td>BHT</td><td>-</td><td>0.05</td><td>-</td><td>0.05</td></tr>
<tr><td>CQ</td><td>0.15</td><td>-</td><td>0.15</td><td>-</td></tr>
<tr><td>DABE</td><td>0.25</td><td>-</td><td>0.25</td><td>-</td></tr>
<tr><td>BF [MPa]</td><td colspan="2">112.3</td><td colspan="2">118.6</td></tr>
<tr><td>E-Modul [GPa]</td><td colspan="2">6.5</td><td colspan="2">6.9</td></tr>
<tr><td>Schrumpfung [%]</td><td colspan="2">2.53%</td><td colspan="2">2.41%</td></tr>
</table>

Beispiel 10 (dualhärtendes Befestigungsmaterial):

<table>
<tr><td>Beispiel</td><td colspan="2">10a</td><td colspan="2">10b</td></tr>
<tr><td>Polysiloxan (a1)</td><td rowspan="3">Beispiel 4a<br>35.00</td><td rowspan="3">Beispiel 4a<br>27.00</td><td>Beispiel 5a<br>11.00</td><td>Beispiel 5a<br>8.25</td></tr>
<tr><td>Polysiloxan (a2)</td><td>Beispiel 5f<br>14.00</td><td>Beispiel 5f<br>10.50</td></tr>
<tr><td>Polysiloxan (a3)</td><td>Beispiel 5j<br>7.00</td><td>Beispiel 5j<br>5.25</td></tr>
<tr><td>MDP</td><td></td><td>9.00</td><td></td><td>9.00</td></tr>
<tr><td>Dentalglas 1</td><td>9.60</td><td>9.50</td><td>10.10</td><td>10.00</td></tr>
<tr><td>Dentalglas 2</td><td>43.00</td><td>42.50</td><td>45.50</td><td>45.00</td></tr>
<tr><td>Pyrogen.-$SiO_2$</td><td>3.00</td><td>3.00</td><td>3.00</td><td>3.00</td></tr>
<tr><td>Nano-$SiO_2$</td><td>8.00</td><td>8.00</td><td>8.00</td><td>8.00</td></tr>
<tr><td>DEPT</td><td>0.60</td><td>-</td><td>0.60</td><td>-</td></tr>
<tr><td>NTPB</td><td>0.40</td><td></td><td>0.40</td><td></td></tr>
<tr><td>BPO</td><td>-</td><td>0.95</td><td>-</td><td>0.95</td></tr>
<tr><td>BHT</td><td>-</td><td>0.05</td><td>-</td><td>0.05</td></tr>
<tr><td>CQ</td><td>0.15</td><td>-</td><td>0.15</td><td>-</td></tr>
<tr><td>DABE</td><td>0.25</td><td>-</td><td>0.25</td><td>-</td></tr>
<tr><td>BF [MPa]</td><td colspan="2">111.5</td><td colspan="2">117.7</td></tr>
<tr><td>E-Modul [GPa]</td><td colspan="2">6.3</td><td colspan="2">6.8</td></tr>
<tr><td>Schrumpfung [%]</td><td colspan="2">2.58%</td><td colspan="2">2.43%</td></tr>
</table>

Biegefestigkeit (BF): Die Biegefestigkeit wird entsprechend ISO 4049 bestimmt. Alle Materialien werden mit einer Celalux 2-Lampe (VOCO GmbH) abschnittsweise für 40 Sekunden lichtgehärtet. Die Bestimmung der Biegefestigkeit erfolgt bei einer Vorschubgeschwindigkeit von 0,75 mm/min an einer Zwick-Universalprüfmaschine (Zwick GmbH & Co. KG, Ulm).

Elastizitätsmodul (E-Modul): Der Elastizitätsmodul wird aus der Steigung im elastischen Bereich der Kraft-Weg-Kurven der Biegefestigkeitsmessungen ermittelt.

Schrumpfung: Die Schrumpfung wurde nach der Bonded-Disk-Methode von Watts et al. bestimmt (Watts DC, Cash AJ. Determination of polymerization kinetics in visible-light cured materials: Methods development. Dent Mater 1991; 7:281-287). Abweichend wurde mit einer Celalux 2-Lampe (VOCO GmbH) für 40 Sekunden pro Messung belichtet.

Viskosität: Die Bestimmung der Viskosität erfolgt mittels eines Rheometers (Physica MCR 301) der Firma Anton Paar (Graz, Österreich). Die Messung erfolgt bei 25 °C im Rotationsversuch mit Platte/Platte-Anordnung (Durchmesser 25 mm, Spaltabstand 1 mm) in einem Scherratenbereich von $10^{-2}$ bis 10 $s^{-1}$. Es werden pro Messung 16 Messwerte in einem Intervall von 30 Sekunden je Scherrate aufgenommen. In den Tabellen sind jeweils die Viskositäten für die Scherrate 10 $s^{-1}$ angegeben.

Da die Füllstoffphase eines dentalen Kompositmaterials für die mechanischen Eigenschaften von entscheidender Bedeutung ist, ist es für einen aussagekräftigen und verlässlichen Vergleich der mechanischen Eigenschaften unerlässlich, immer nur die Produkte gegenüberzustellen, die über einen ähnlichen Füllstoffgehalt und über eine ähnliche Füllstoffzusammensetzung verfügen. Die oben bezüglich ihrer mechanischen Werte miteinander verglichenen Produktkategorien entsprechen dieser Voraussetzung, da dort beispielsweise zwischen stopfbaren oder hochviskosen (d.h. hochgefüllten) und fließfähigen (d.h. mittelgefüllten) Kompositen unterschieden ist (zu den unterschiedlichen Füllstoffmengen verweisen wir auf unsere Ausführungen auf Seite 62 ff). Weiter werden durch Begriffe wie "Hybrid-Komposit", "Mikrohybrid", Nano-Hybrid", "mikrogefüllte Komposite" oder "faserverstärktes Komposit" Angaben über Art der Füllstoffzusammensetzung und damit auch indirekt Informationen über die Mengen zugesetzter Füllstoffe gegeben. So bestehen mikrogefüllte Komposite in ihrer Füllstoffphase aus (pyrogener) Kieselsäure. Aufgrund ihrer hohen Thixotropie kann diese Produktkategorie nur eine mittlere Füllstoffmenge enthalten. Die Hybridkomposite setzen sich aus unterschiedlichen Gläsern unterschiedlicher Größenfraktionen (von 10 $\mu$m bis < 1 $\mu$m) in Kombination mit Kieselsäure zusammen. Hybridkomposite haben sich als dentale Universalkomposite etabliert, die praktisch für jede Zahnrestauration, von großen Schneidekantenaufbauten bis zu Kaulast tragenden Füllungen im Seitenzahnbereich, verwendet

werden können. Eine weitere Variante eines Hybridkomposits ist der "Nanohybridtyp". Hier werden nanoskalige, größtenteils nicht aggregierte und nicht agglomerierte Füllstoffe in die Füllstoffphase integriert. Das Einarbeiten oberflächenmodifizierter Nanoteilchen ermöglicht so eine Erhöhung des Füllstoffanteils, ohne dass sich die Viskosität des Komposits entsprechend erhöht. Damit sollten die mechanischen Eigenschaften nochmals verbessert werden.

Unsere Beispielkomposite 7a bis 7p entsprechen einem typischen Hybridkomposit, das als makroskopischen Füllstoffbestandteil eine bimodale Glaspartikelverteilung aufweist. Der Füllstoffanteil des Komposits liegt bei ca. 81 Gew.-%.

Um einen verlässlichen Vergleich der mechanischen Werte der erfindungsgemäßen polymerisierbaren dentalen Zusammensetzungen auf der Basis kondensierter Silane mit entsprechenden Werten von Kompositen aus der Literatur zu ziehen, müssen natürlich nicht nur die Systeme miteinander vergleichbar sein, auch die durchgeführten Messungen müssen identisch sein. Insbesondere schreibt die Norm DIN EN ISO 4049 vor, dass die ausgehärteten Prüfkörper nach Entformung für einen Zeitraum von 24 Stunden in deionisiertes Wasser bei 37°C gelagert werden müssen, bevor die mechanischen Eigenschaften ermittelt werden. Dieser Schritt der Wasserlagerung wird in vielen Prüfungen der mechanischen Eigenschaften von dentalen polymerisierbaren Zusammensetzungen auf der Basis kondensierter Silane nicht berücksichtigt. Damit werden systematisch höhere Werte erzielt.

In der DE 198 60 364 C2, betitelt "Polymerisierbare Dentalmassen auf Basis von zur Aushärtung befähigten Siloxanverbindungen, deren Verwendung und Herstellung" wird in den Beispielen 3 und 5 das kondensierte Silan 1,3,5,7-Tetramethyl-1,3,5,7-tetrakis-(3-methacryloxypropyl)-cyclotetrasiloxan als Hauptmonomerkomponente mit dem Initiatorsystem Campherchinon/Amin sowie einem anorganischen Füllstoffanteil von ca. 80 Gew.-% ausgehärtet. In Beispiel 3 komplettiert das klassische Dentalmonomer UDMA (7,7,9-Trimethyl-3,14-dioxa-4,13-dioxo-5,12-diazahexadecan-1,16-dioldimethacrylat) und in Beispiel 5 das klassische Dentalmonomer Bis-GMA (2,2-Bis-4(3-hydroxypropoxyphenyl)-propandimethacrylat) die Monomermatrix.

Diese Komposite sollten insoweit mit den erfindungsgemäßen Kompositen vergleichbar sein, obwohl hier noch die klassischen, bewährten Dentalmonomeren unterstützend zu den Polysiloxanen mit verwendet werden. In den Beispielen 3 und 5 liegt der Anteil des Polysiloxans über dem Anteil der Standardmonomeren.

Komposit 3 weist eine Biegefestigkeit von 119 MPa, einen E-Modul von 8731 MPa und einen Volumenschrumpf von 2,73% auf.

Komposit 5 weist eine Biegefestigkeit von 115 MPa, einen E-Modul von 8713 MPa und einen Volumenschrumpf von 2,67% auf.

In der DE 198 60 361 A1, betitelt "Vernetzbare Monomere auf Cyclosiloxanbasis, deren Herstellung und deren Verwendung in polymerisierbaren Massen" werden ebenfalls polymerisierbare dentale Zusammensetzungen auf der Basis von Co- und Homokondensaten von Silanen bei einem Füllstoffanteil von ca. 80 Gew.-% und mit dem Initiatorsystem Campherchinon/Amin offenbart. Berücksichtigt werden die Zusammensetzungen, bei denen der mengenmäßige Anteil des Polysiloxans zumindest in der Größenordnung der Menge der herkömmlichen Dentalmonomeren liegt.

Beispiel 3 offenbart ein Dentalkomposit, das unterschiedlich kondensierte 1-(Trimethoxysilylethyl)-3,5,7-tris(3-methacryloxypropyl)-1,3,5,7-tetramethyltetra-siloxan, das Cokondensat aus 1-(Trimethoxysilylethyl)-3,5,7-tris(3-methacryloxypropyl)-1,3,5,7-tetramethyltetrasiloxan und Tetramethoxysilan, das klassische Dentalmonomer UDMA (7,7,9-Trimethyl-4,13-dioxo-3,14-dioxa-5,12-diazahexadecan-1,16-dioldimethacrylat) in kleinem Überschuss gegenüber den kondensierten Silanen, Füllstoffe in einer Menge von ca. 82 Gew.-%, sowie das Photoinitiatorsystem Campherchinon/Amin umfasst.

Komposit 3 weist eine Biegefestigkeit von 107 MPa, einen E-Modul von 7317 MPa und einen Volumenschrumpf von 2,57% auf.

Beispiel 5 offenbart ein Dentalkomposit, das das Homokondensat 1-(Trimethoxysilylethyl)-3,5,7-tris(3-methacryloxypropyl)-1,3,5,7-tetramethyltetrasiloxan, das Cokondensat von 1-(Trimethoxysilylethyl)-3,5,7-tris(3-methacryloxypropyl)-1,3,5,7-tetramethyltetrasiloxan mit Bis-(hydroxymethyl)-tricyclo[5.2.1.0$^{2,6}$]-decanmethacrylat(3-trimethoxysilyl-1-carbamat), die klassischen Dentalmonomeren Bis-GMA (2,2-Bis-4(2-hydroxypropoxyphenyl)-propandimethacrylat) und Bisacryloyloxymethyltricyclo[5.2.1.0$^{2,6}$]decan als monomere Matrix, eine Füllstoffbeladung von ca. 80 Gew.-% und das Photoinitiatorsystem Campherchinon/Amin enthält.

Komposit 5 weist eine Biegefestigkeit von 121 MPa, einen E-Modul von 8132 MPa und einen Volumenschrumpf von 2,29% auf.

Beispiel 6 offenbart ein Dentalkomposit, das das Homokondensat 1-(Trimethoxysilylethyl)-3,5,7-tris(3-methacryloxypropyl)-1,3,5,7-tetramethyltetrasiloxan, das Cokondensat aus 1-(Trimethoxysilylethyl)-3,5,7-tris(3-methacryloxypropyl)-1,3,5,7-tetramethyltetrasiloxan und Tetramethoxysilan, das Cokondensat von 1-(Trimethoxysilylethyl)-3,5,7-tris(3-methacryloxypropyl)-1,3,5,7-tetramethyltetrasiloxan mit 2,2-[Bis-(4-hydroxyphenyl)-propan-4-methacrylat-4'-(3-trimethoxysilyl-1-carbamt), die klassischen Dentalmonomeren Bis-GMA (2,2-Bis-4(3-hydroxypropoxyphenyl)-propandimethacrylat), 2,2-Bis-4(2-hydroxyethoxyphenyl)-propandimethacrylat), Bisacryloyloxymethyltricyclo[5.2.1.0$^{2,6}$]decan als monomere Matrix, 80,8 Gew.-% Füllstoff und das Photoinitiatorsystem Campherchinon/Amin enthält.

Komposit 6 weist eine Biegefestigkeit von 117 MPa, einen E-Modul von 8615 MPa und einen Volumenschrumpf

von 2,51% auf.

Beispiel 8 offenbart ein Dentalmaterial, das das Homokondensat 1-(Trimethoxysilylethyl)-3,5,7-tris(3-methacryloxypropyl)-1,3,5,7-tetramethyltetrasiloxan, das Cokondensat von 1-(Trimethoxysilylethyl)-3,5,7-tris(3-methacryloxypropyl)-1,3,5,7-tetramethyltetrasiloxan mit 2,2-[Bis-(4-hydroxyphenyl)-propan-4-methacrylat-4'-(3-trimethoxysilyl-1-carbamt), sowie das klassische Dentalmonomer UDMA (7,7,9-Trimethyl-4,13-dioxo-3,14-dioxa-5,12-diazahexadecan-1,16-dioldimethacrylat) als monomere Matrix, ca. 80 Gew.-% Füllstoff und das Photoinitiatorsystem Campherchinon/Amin enthält.

Komposit 8 weist eine Biegefestigkeit von 116 MPa, einen E-Modul von 7513 MPa und einen Volumenschrumpf von 2,19% auf.

Beispiel 9 offenbart ein Dentalmaterial, das unterschiedlich kondensierte 1-(Trimethoxysilylethyl)-3,5,7-tris(3-methacryloxypropyl)-1,3,5,7-tetramethyltetrasiloxan, das Cokondensat aus 1-(Trimethoxysilylethyl)-3,5,7-tris(3-methacryloxypropyl)-1,3,5,7-tetramethyltetrasiloxan und Tetramethoxysilan, das Cokondensat von 1-(Trimethoxysilylethyl)-3,5,7-tris(3-methacryloxypropyl)-1,3,5,7-tetramethyltetrasiloxan mit 2,2-[Bis-(4-hydroxyphenyl)-propan-4-methacrylat-4'-(3-trimethoxysilyl-1-carbamt), sowie die klassischen Dentalmonomeren Bis-GMA (2,2-Bis-4(2-hydroxypropoxyphenyl)-propandimethacrylat) UDMA (7,7,9-Trimethyl-4,13-dioxo-3, 14-dioxa-5,12-diazahexadecan-1, 16-dioldimethacrylat) und Bisacryloyloxymethyltricyclo[5.2.1.0$^{2,6}$]decan als monomere Matrix, 80 Gew.-% Füllstoff und das Photoinitiatorsystem Campherchinon/Amin enthält.

Komposit 9 weist eine Biegefestigkeit von 117 MPa, einen E-Modul von 7916 MPa und einen Volumenschrumpf von 2,24% auf.

In der DE 41 33 494 A1, betitelt "Dentalharzmassen", werden chemisch, thermisch oder photochemisch härtende Dentalharzmassen auf Basis polymerisierbarer Polysiloxane, Verfahren zu deren Herstellung und deren Verwendung zur Herstellung von pastösen, härtbaren Dentalmaterialien, die erhältlich sind durch hydrolytische Kondensation einer oder mehrerer hydrolytisch kondensierbarer Silane, offenbart. In dieser Druckschrift werden die Systeme sowohl radikalisch als auch kationisch polymerisiert.

Kompositsystem L1 (Beispiel 16, Seite 27) besteht aus einem Harzsystem, das erhalten wird, indem Trimethylolpropantriacrylat (TMPTA) mit (Mercaptomethyl)methyldiethoxysilan in einem molaren Verhältnis von 1,2 zu 1 zu dem entsprechenden Michael-Addukt umgesetzt wird. Die Ethoxygruppen des Addukts werden unter üblichen sauren Bedingungen zu einem transparenten Harz hydrolysiert, dann kondensiert und schließlich mit Trimethylchlorsilan silanisiert, um freie Si-OH-Gruppen umzusetzen und somit die Viskosität der Harzphase zu reduzieren, um einen höheren Füllstoffanteil im Komposit erzielen zu können. Aus dem Harz wird ein dentales Hybridkomposit mit 75 Gew.-% Füllstoff erhalten, das dann mit einem Photoinitiator versetzt und radikalisch einkomponentig ausgehärtet wird.

Komposit L1 weist eine Biegefestigkeit (hier bezeichnet als Bruchfestigkeit, wobei die Prüfstäbe vor der Prüfung nicht wassergelagert wurden) von 120 MPa, einen E-Modul von 8000 MPa und einen Volumenschrumpf von 2.9% auf (siehe Tabelle, Seite 28).

Beispiel 18 offenbart eine dentale Kompositzusammensetzung eines 2-komponentigen dentalen Paste/Paste Systems, das chemisch mit Hilfe des Redoxsystems BPO (Benzoylperoxid)/Amin (*N,N*-Bis-(2-hydroxyethyl)-*p*-toluidin) radikalisch aushärtet. Die Harzmatrix des Hybridkomposits umfasst - wie oben - das Umsetzungsprodukt aus Trimethylolpropantriacrylat (TMPTA) mit (Mercaptomethyl)methyldiethoxysilan in einem molaren Verhältnis von 1,2 zu 1, wobei das resultierende Michael-Addukt in diesem Falle aber nicht silanisiert wird. Entsprechend höher ist die Viskosität des erhaltenen Harzes. Das Hybridkomposit enthält zum Silankondensat noch das klassische Dentalmonomer "2,2-Bis-[4'(2'-methacroylethoxy)phenyl]propan", ein ethoxyliertes Bisphenol-A-dimethacrylat, sowie 76 Gew.-% Füllstoff.

Die Biegefestigkeit der nicht wassergelagerten Prüfkörper beträgt 120 MPa und der Volumenschrumpf weist 2,3% auf.

In der DE 199 03 177 A1, betitelt "Dentalmaterialien auf der Basis von Polysiloxanen" werden Dentalmaterialien offenbart, die mindestens ein Polysiloxan auf der Basis von einem oder mehreren hydrolytisch kondensierbarer Silan(e) einer bestimmten Struktur umfasst. Das Dentalmaterial kann zusätzlich weitere ionisch und/oder radikalisch polymerisierbare Monomeren enthalten.

Beispiel 7 offenbart einen pastösen Dentalzement in Form eines Hybridkomposits, umfassend in einer Menge von 31,6 Gew.-% ein Polysiloxan, das nach hydrolytischer Kondensation und anschließender Silylierung von Bis(methacryloylethoxycarbonylethyl)-[3-(triethoxysilylpropyl)]amin (erhalten aus der Michael-Addition von 3-Amininopropyltriethoxysilan und 2-Acryloyloxyethylmethacrylat) erhalten wurde, UDMA (7,7,9-Trimethyl-3,14-dioxa-4,13-dioxo-5,12-diazahexadecan-1,16-dioldimethacrylat) in einer Menge von 7,8 Gew.-%, silanisierte pyrogene Kieselsäure in einer Menge von 41,4 Gew.-%, Ytterbiumfluorid in einer Menge von 18,7 Gew.-%, sowie das Photoinitiatorgemisch "Campherchinon/Amin" in einer Menge von 0,5 Gew.-%.

Die Biegefestigkeit des Dentalkomposits beträgt 62 MPa, der E-Modul beträgt 3260 MPa und der Volumenschrumpf beträgt 3,6%.

In der DE 101 02 297 A1, betitelt "Dentalmaterialien auf der Basis von Metalloxid-Clustern" werden die oben beschriebenen Dentalmaterialien mechanisch durch den Einsatz von Clustern weiter verbessert.

Wie oben wird in Zusammensetzung K-1 ein Polysiloxan verwendet, das nach hydrolytischer Kondensation von Bis(methacryloylethoxycarbonylethyl)-[3-(triethoxysilylpropyl)]amin erhalten wurde und das nicht silyliert wurde. Dieses photoinitiierte Polysiloxan wird in einem Hybridkomposit in einer Menge von 25 Gew.-% mit 75 Gew.-% Füllstoff (1 Gew.-% pyrogene Kieselsäure, 15 Gew.-% Ytterbiumfluorid, 15 Gew.-% Sphärosil und 44 Gew.-% Glas) zu einer Paste verarbeitet und gemäß Norm DIN EN ISO 4049 ausgehärtet.

Die Biegefestigkeit des Dentalkomposits K-1 beträgt 68 MPa und der E-Modul weist 5500 MPa auf.

In Zusammensetzung K-3 wird ein Polysiloxan verwendet, das nach hydrolytischer Kondensation von photoinitiiertem (3-Triethoxysilylpropylaminocarbonylbuttersäure-(1,3-(2)-bismethacryloyloxypropyl)-ester in einem Hybridkomposit in einer Menge von 25 Gew.-% mit 75 Gew.-% der oben angegebenen Füllstoffmischung zu einer Paste verarbeitet und gemäß Norm DIN EN ISO 4049 ausgehärtet. Das Polysiloxan wurde aus 3-Aminopropyltriethoxysilan und dem Addukt aus Glycerindimethacrylat und Glutarsäureanhydrid unter Amidbindung erhalten.

Als Photoinitiator der Zusammensetzungen wurde eine Mischung aus 0,3 Gew.- % Campherchinon, 0,6 Gew.-% 4-(N,N-Dimethylamino)benzoesäureethylester und 0,4 Gew.-% Acylphosphinoxid verwendet, wobei sich die Gewichtsangaben auf die Gesamtzusammensetzung beziehen.

Die Biegefestigkeit des Dentalkomposits K-3 beträgt 89 MPa und der E-Modul weist 7160 MPa auf.

Aufgrund der höheren Füllstoffbeladung liegen die mechanischen Werte Der dentalen Hybridkomposite in dieser Druckschrift etwas höher als die aus der obigen DE 199 03 177 A1.

In der DE 101 02 297 A1 sollte die mechanische Festigkeit von dentalen polymerisierbaren Zusammensetzungen auf der Basis kondensierter Silane durch die Zugabe bestimmter Cluster vom Typ $Zr_4O_2(OMc)_{12}$ verbessert werden und zu einer Erhöhung des E-Moduls der Materialien führen.

Werden 10 Gew.-% der monomeren Matrix aus K-1 mit dem Clustertyp $Zr_4O_2(OMc)_{12}$ substituiert, dann beträgt die Biegefestigkeit von K-2 100 MPa und der E-Modul weist 9300 MPa auf.

Werden 10 Gew.-% der monomeren Matrix aus K-3 mit dem Clustertyp $Zr_4O_2(OMc)_{12}$ substituiert, dann beträgt die Biegefestigkeit von K-4 110 MPa und der E-Modul weist 9750 MPa auf.

Werden 20 Gew.-% der monomeren Matrix aus K-3 mit dem Clustertyp $Zr_4O_2(OMc)_{12}$ substituiert, dann beträgt die Biegefestigkeit von K-5 113 MPa und der E-Modul weist 10900 MPa auf.

In der DE 10 2006 016 474 A1, betitelt "Dentalmaterialien enthaltend hydrophobe, nanopartikuläre Kieselsäurecokondensate und deren Verwendung" werden dentale Kompositmaterialien auf Basis von Polysiloxanen mit zusätzlichen flüssigen, funktionalisierten Cokondensaten von Kieselsäuretetraalkylestern mit funktionalisierten Trialkoxysilanen offenbart. Durch die Gegenwart dieser Cokondensate, deren Endgruppen mit Trimethylsilylgruppen abreagiert sind, sollen die mechanischen Eigenschaften der Komposite verbessert werden.

In dem dentalen Hybridkompositmaterial A wird das hydrolytische Kondensat des Silans (1,3-Dimethacryloyloxypropyl-[4-(3-triethoxysilyl)propyl-N-methylaminocarbonyl)]butyrats, das durch Umsetzung von Glycerindimethacrylat mit Glutarsäureanhydrid erhalten wird, in einer Menge 29,8 Gew.-% mit dem Photoinitatorsystem "Campherchinon/*p*-Dimethylaminobenzoessäureethylester" in einer Menge von 0,2 Gew.-% und einer Füllstoffphase aus Ytterbiumfluorid (12,7 Gew.-%), pyrogener Kieselsäure (0,9 Gew.-%), Bariumaluminiumborsilikatglasfüllstoff (44,1 Gew.-%) und einem Kieselsäure-Zirkonmischoxid (12,3 Gew.-%) bei einem Gesamtfüllstoffanteil von 70 Gew.-% bezogen auf die Gesamtzusammensetzung zu einer Paste verarbeitet und ausgehärtet.

Die Biegefestigkeit beträgt 51 MPa und der E-Modul weist 5410 MPa auf.

Wird ein Anteil von 5,9 Gew.-% des Polysiloxans aus Komposit A mit dem zusätzlichen niedigviskosen Cokondensat aus Tetraethoxysilan mit 3-Methacryloxypropyltriethoxysilan und Endgruppenumsetzung mit Trimethylchlorsilan, substituiert, so weist Komposit B eine Biegefestigkeit von 75 MPa und einen E-Modul von 5240 MPa auf.

Mechanische Werte (Stand der Technik)

| Patentschrift | Harz/Füllstoff | Biegefestigkeit (MPA) | E-Modul (MPa) | Schrumpf (%) |
|---|---|---|---|---|
| DE 19 860 364 C2 | | | | |
| Beispiel 3 | 20/80 | 119 | 8731 | 2,7 |
| Beispiel 5 | 20/80 | 115 | 8713 | 3,3 |
| | | | | |
| DE 19 860 361 A1 | | | | |
| Beispiel 3 | 20/80 | 107 | 7317 | 2,6 |
| Beispiel 5 | 20/80 | 121 | 8132 | 2,3 |
| Beispiel 6 | 19/81 | 117 | 8615 | 2,5 |
| Beispiel 8 | 20/80 | 116 | 7513 | 2,2 |

(fortgesetzt)

| | | | | |
|---|---|---|---|---|
| DE 19 860 361 A1 | | | | |
| Beispiel 9 | 20/80 | 117 | 7916 | 2,2 |
| | | | | |
| DE 41 33 494 A1 | | | | |
| Beispiel 16 | 25/75 | 120 | 8000 | 2,9 |
| Beispiel 18 | 24/76 | 120 | - | 2,3 |
| | | | | |
| DE 19 903 177 A1 | | | | |
| Beispiel 7 | 40/60 | 62 | 3260 | 3,6 |
| | | | | |
| DE 10102297 A1 | | | | |
| K1 | 25/75 | 68 | 5500 | - |
| K3 | 25/75 | 89 | 7160 | - |
| K2 | 25/75 | 100 | 9300 | - |
| K4 | 25/75 | 110 | 9750 | - |
| K5 | 25/75 | 113 | 10900 | - |
| | | | | |
| DE 10 2006 016 474 A1 | | | | |
| A | 30/70 | 51 | 5410 | - |
| B | 30/70 | 75 | 5240 | - |

Der Vergleich zwischen den klinisch relevanten Werten der erfindungsgemäßen Zusammensetzungen und den entsprechenden Werten aus dem Stand der Literatur zeigt eine deutliche Überlegenheit der neuen Systeme auf der Basis kondensierter erfindungsgemäßer Silane. Betrachtet man die Verbesserung dieser Werte in der Reihenfolge der Literaturdaten aus 2009 und 2018 (siehe oben), so erreichen die Werte vergleichbarer erfindungsgemäßer dentaler Zusammensetzungen vom Typ "Hybridkomposit" neue Höchststände, wobei selbst die Werte faserverstärkter Komposite aus der aktuellen Zusammenstellung von Rosentritt, Illie und Lohbauer aus 2018 übertroffen werden. Zusätzlich wird durch den extrem geringen Schrumpf eine nur äußerst geringe Änderung der Dichteverhältnisse beim Übergang von der flüssig/pastösen Phase in die Festphase induziert, so dass eine besonders vorteilhafte Durchhärtungstiefe erreicht werden kann.

**Patentansprüche**

**1.** Dentale polymerisierbare Zusammensetzung umfassend

(A) Polysiloxane, wobei die Polysiloxane umfassen

eine Mischung der Kondensate der drei Silane (a1), (a2) und (a3) und/oder ein Cokondensat aus einer Mischung der drei Silane (a1), (a2) und (a3) und/oder eine Mischung von mindestens zwei der Cokondensate (a1)/(a2), (a1)/(a3) und (a2)/(a3) und/oder
eine Mischung aus dem Kondensat eines der drei Silane (a1), (a2) oder (a3) mit dem Cokondesat der anderen beiden Silane,
wobei das Silan (a1) der Formel $(R^1O)_aR^2sSi[-A(-Y\{-B[-PG]_f\}_e)_d]_c$ entspricht, wobei Y = -C(=O)NH-, -NHC(=O)-, -OC(=O)NH-, -NHC(=O)O-, -SC(=O)NH-, -NHC(=O)S-, -NHC(=O)NH-, -OC(=O)N(-C(=O)NH-)-, -SC(=O)N(-C(=O)NH-)-, -NHC(=O)N(-C(=O)NH-)-, -C(=O)NHC(=O)NH-, -NHC(=O)NHC(=O)-, wobei die jeweils im Formelbild links angeordnete Bindung dem Strukturelement A und die rechts angeordnete Bindung dem Strukturelement B näher ist,

PG = polymerisierbare Gruppe,
wobei die polymerisierbare Gruppe PG ausgewählt ist aus der Gruppe bestehend aus $-Z-C(=O)-CH=CH_2$ und $-Z-C(=O)-C(CH_3)=CH_2$ wobei Z ausgewählt ist aus der Gruppe bestehend aus O und NH,
A = eine organische Verbindungsgruppe, die Si mit Y verbindet und 1 bis 20 C-Atome aufweist,
B = eine organische Verbindungsgruppe, die Y mit PG verbindet und 1 bis 20 C-Atome aufweist,
$R^1$ = H oder C1- bis C4-Alkyl,
$R^2$ = C1- bis C4-Alkyl,
a = 2 oder 3,
b = 0 oder 1,
c = 1 oder 2,
d = 1 bis 3,
e = 1 oder 2,
f = 1 bis 5,

$$a + b + c = 4,$$

und wobei das Silan (a2) der Formel $(R^1O),R^2_bSi[-A'(-PG)_f]_c$ entspricht, wobei PG = polymerisierbare Gruppe,
wobei die polymerisierbare Gruppe PG ausgewählt ist aus der Gruppe bestehend aus $-Z-C(=O)-CH=CH_2$ und $-Z-C(=O)-C(CH_3)=CH_2$ wobei Z ausgewählt ist aus der Gruppe bestehend aus O und NH,
A' = eine organische Verbindungsgruppe, die Si mit PG verbindet und 1 bis 20 C-Atome aufweist und keine der Gruppen -C(=O)NH-, -NHC(=O)-, -OC(=O)NH-, -NHC(=O)O-, -SC(=O)NH-, -NHC(=O)S-, -NHC(=O)NH-, -OC(=O)N(-C(=O)NH-)-, -SC(=O)N(-C(=O)NH-)-, oder -NHC(=O)N(-C(=O)NH-)-, -C(=O)NHC(=O)NH-, -NHC(=O)NHC(=O)- enthält,
$R^1$ = H oder C1- bis C4-Alkyl,
$R^2$ = C1- bis C4-Alkyl,
a = 2 oder 3
b = 0 oder 1
c = 1 oder 2
f = 1 bis 5,

$$a + b + c = 4,$$

und wobei das Silan (a3) der Formel $(R^1O)_aR^2_bSiAr_c$ entspricht, wobei
$R^1$ = H oder C1- bis C4-Alkyl,
$R^2$ = C1- bis C4-Alkyl,
Ar = Aryl wobei unterschiedliche Gruppen Ar gleich oder verschieden sein können,
a = 2 oder 3,
b = 0 oder 1,
c = 1 oder 2

$$a + b + c = 4,$$

(B) Füllstoffe und
(C) Initiatoren und/oder Katalysatoren und/oder Aktivatoren für die Polymerisation, wobei der Anteil der Silane (a1), (a2) und (a3) im Cokondensat oder in der Mischung der Kondensate oder Cokondensate

(a1) in einer Menge von 10 bis 70 Gew.-%,
(a2) in einer Menge von 10 bis 70 Gew.-%, und
(a3) in einer Menge von 5 bis 60 Gew.-%,

beträgt, jeweils bezogen auf die Gesamt Menge der Silane (a1), (a2) und (a3).

**2.** Dentale polymerisierbare Zusammensetzung nach Anspruch 1, zusätzlich umfassend

(D) organische, polymerisierbare Monomere, die keine erfindungsgemäßen Polysiloxane sind und/oder
(E) organische, säuregruppenhaltige Monomeren, die kein Si-Atom aufweisen, und/oder

(F) Additive und/oder
(G) Lösungsmittel.

3. Dentale polymerisierbare Zusammensetzung nach einem der vorangehenden Ansprüche, wobei die Zusammensetzung die Komponente

(A) in einer Menge von 5 bis 99,99 Gew.-%,
(B) in einer Menge von 0,3 bis 92 Gew.-%,
(C) in einer Menge von 0,01 bis 5 Gew.-%,
(D) in einer Menge von 0 bis 94 Gew.-%,
(E) in einer Menge von 0 bis 20 Gew.-%
(F) in einer Menge von 0 bis 20 Gew.-% und
(G) in einer Menge von 0 bis 70 Gew.-% enthält und

wobei die Gewichtsangaben jeweils auf die Gesamtzusammensetzung bezogen sind.

4. Dentale polymerisierbare Zusammensetzung nach einem der vorangehenden Ansprüche, wobei die Gruppe Y aus Silan (a1) ausgewählt ist aus der Gruppe bestehend aus $-(X)_x-C(=O)NH-$, $-NHC(=O)-(X)_x-$ und $-(X)_xC(=O)N(-C(=O)NH-)-$wobei X ausgewählt ist aus der Gruppe bestehend aus O, S und NH, vorzugsweise aus O und S, und wobei der Index x entweder 0 oder 1 ist, vorzugsweise 1 ist, und wobei die polymerisierbare Gruppe PG ausgewählt ist aus der Gruppe bestehend aus $Z-C(=O)-CH=CH_2$ und $-Z-C(=O)-C(CH_3)=CH_2$, und wobei Z ausgewählt ist aus der Gruppe bestehend aus O und NH, vorzugsweise O ist.

5. Dentale polymerisierbare Zusammensetzung nach einem der vorangehenden Ansprüche, wobei Bestandteil (D) organische, polymerisierbare Monomeren umfasst, die keine erfindungsgemäße Polysiloxane sind, vorzugsweise zur Umsetzung mit den erfindungsgemäßen Polysiloxanen und wobei (D) ausgewählt ist aus der Gruppe bestehend aus

monofunktionellen oder polyfunktionellen (Meth)acrylatmonomeren, vorzugsweise aus Estern der (Meth)acrylsäure mit Alkylgruppen von 1 bis 12 C-Atomen sowie aus Estern der (Meth)acrylsäure, die aromatische Gruppen mit 6 bis 12 C-Atomen enthalten, wobei die Alkylgruppen und aromatischen Gruppen, die die Ester bilden, Substituenten wie Hydroxylgruppen und Etherbindungen enthalten können und
polymerisierbaren Monomeren, die mindestens eine ethylenische Doppelbindung aufweisende Hydroxylverbindungen sind und
polyfunktionellen (Meth)acrylatmonomeren, vorzugsweise aus Di(meth)acrylaten von Alkylenglycol mit 2 bis 20 C-Atomen, Di(meth)acrylaten von Oligomeren des Alkylenglycols, Polyalkylenglycoldi(meth)acrylat, Di(meth)acrylaten von Bisphenol A bzw. vom Diglycidylether von Bisphenol A und
polymerisierbaren Verbindungen, die auf ein zentrales polyalicyclisches Strukturelement beruhen, wie 3(4), 8(9)-Bis((meth)acryloyloxymethyl)-tricyclo[5.2.1.$0^{2,6}$]decan, alkoxyliertes 3(4), 8(9)-Bis((meth)acryloyloxymethyl)-tricyclo[5.2.1.$0^{2,6}$]decan, 2,3-Bis((meth)acryloyloxymethyl)- bicyclo[2.2.1]heptan, alkoxyliertes 2,3-Bis((meth)acryloyloxymethyl)bicyclo[2.2.1]heptan, 1,3,5-Tri-(meth)acryloyloxytricyclo[3.3.1.$1^{3,7}$]decan, alkoxyliertes Tri(meth)acryloyloxytricyclo- [3.3.1.$1^{3,7}$]decan sowie (Meth)acrylsäureester von Tricyclo[5.2.1.$0^{2,6}$]-decan-3(4),8(9)-dimethanol, alkoxyliertem Tricyclo[5.2.1.$0^{2,6}$]decan-3(4),8(9)-dimethanol, Bicyclo[2.2.1]heptan-2,3-dimethanol, alkoxyliertem Bicyclo[2.2.1]-heptan-2,3-dimethanol, 1,3,5-Adamantantriol, alkoxyliertem 1,3,5-Adamantantriol, wobei zwischen dem polyalicyclischen Strukturelement und den (Meth)acrylsäureestern Urethan-, Harnstoff-, Amid-, Allophanat-, Acylharnstoff- oder Biuretgruppen angeordnet sind und
Urethan(meth)acrylatester, Reaktionsprodukte aus 2 Mol eines (Meth)acrylats mit einer Hydroxylgruppe und einem Mol eines Diisocyanats. und
Ethylenglycoldi(meth)acrylat, Diethylenglycoldi(meth)acrylat, 1,6-Hexandiol-di(meth)acrylat (HEDMA), Triethylenglycoldi(meth)acrylat (TEGDMA), 1,12-Dodecandioldi(meth)acrylat, Bisphenol-A-di(meth)acrylat, alkoxyliertes Bisphenol-A-di(meth)acrylat, Bisphenol-B-di(meth)acrylat, alkoxyliertes Bisphenol-B-di(meth)acrylat, Bisphenol-C-di(meth)acrylat, alkoxyliertes Bisphenol-C-di(meth)acrylat, Bisphenol-F-di(meth)acrylat, alkoxyliertes Bisphenol-F-di(meth)acrylat, Polyethylenglycoldi(meth)acrylat, 7,7,9-Trimethyl-3,14-dioxa-4,13-dioxo-5,12-diazahexadecan-1,16-dioxydi(meth)acrylat (UDMA), Butandioldi(meth)acrylat, Tetraethylenglycoldi(meth)acrylat, Neopentylglycoldi(meth)acrylat, 2-Hydroxypropyl-1,3-di(meth)acrylat, 3-Hydroxypropyl-1,2-di(meth)acrylat, Pentaerythritoldi(meth)acrylat, Di(meth)acrylaten des Dihydroxymethyltricyclo[5.2.1.$0^{2,6}$]decans, 2-Hydroxyethyl(meth)acrylat, 2-Hydroxypropyl-(meth)acrylat, 3-Hydroxypropyl(meth)acrylat, 1,2-Dihydroxypropyl(meth)acrylat, 1,3-Dihydroxypropyl(meth)acrylat, 2,3-Dihydroxypropyl(meth)acrylat, 2,2-Bis[4-[3-(meth)acryloyloxy-2-hydroxypropoxy]phenyl]propan (Bis-GMA), Trimethylolpropantri(meth)acrylat, Trimethylolethantri(meth) acrylat, Pentaerythritoltri(meth)-acrylat, Trimethylolmethantri(meth)acrylat, Pentaerythritoltetra(meth)acrylat, Ditrimethylol- propantetra(meth)acrylat, Pentaerythritolhexa(meth)acrylat, Butylenglycoldi(meth)acrylat, Propylenglycoldi(meth)acrylat, Nonandioldi(meth)-acrylat, Decandioldi(meth)acrylat, Glycerolmono(meth)acrylat, Glyceroldi(meth)acrylat, Trimethylolpropanmono(meth)acrylat, Trimethylolpropandi(meth)acrylat, Sorbitolmono-, di-, tri-, tetra- oder penta(meth)acrylat, Methyl(meth)acrylat, Ethyl(meth)acrylat, Propyl(meth)acrylat, Butyl(meth)acrylat, Hexyl(meth)acrylat, Tetrahydrofurfuryl(meth)acrylat, Lauryl(meth)acrylat, Cyclohexyl(meth)-acrylat, Allyl(meth)acrylat, Glycidyl(meth)acrylat, 2-Ethoxyethyl(meth)acrylat, Methoxypoly- ethylenglycol(meth)acrylat, Isobornyl(meth)acrylat, 2-(N,N-Dimethylamino)ethyl(meth)acrylat, N-Methylol-(meth)acrylamid, Diaceton(meth)acrylamide, 2,2-Bis[4-(meth)acryloyloxyphenyl]-propan, 2,2-Bis[4-(meth)acryloyloxyethoxyphenyl]propan, 2,2-Bis[4-(meth)-acryloyloxydiethoxyphenyl]propan, 2,2-Bis[4-(meth)acryloyloxytriethoxyphenyl]-propan 2,2-Bis[4-(meth)acryloyloxytetraethoxyphenyl]propan, 2,2-Bis[4-(meth)-acryloyloxypentaethoxyphenyl]-propan, 2,2-Bis[4-(meth)acryloyloxydipropoxyphenyl]propan, 2,2-Bis[4-(meth)acryloyloxy- ethoxyphenyl]-2-[4-(meth)acryloyloxydiethoxyphenyl]propan, 2-[4-(Meth)acryloyloxydiethoxyphenyl]-2-[4-(meth)-acryloyloxytriethoxyphenyl]propan, 2-[4-(Meth)acryloyloxdipropoxy- phenyl]-2-[4-(meth)acryloyloxytriethoxyphenyl]propan, 2,2-Bis[4-(meth)acryloyloxyisopropoxy phenyl]propan, Hydroxypivalinsäureneopentylglycoldi(meth)acrylat, Acetoacetoxyethyl-(meth)acrylat, Polypropylenglycoldi(meth)acrylat, Glycerolalkoxylatdimethacrylat, Neopentylglycol(meth)acrylat, N,N-(1,2-Dihydroxyethylen)-bisacrylamid, 2,2-Bis[4-(meth)acryloyloxypentaethoxyphenyl]propan, 2,2-Bis[4-(meth)acryloyloxypolyethoxyphenyl]-propan, Diethylenglycoldi(meth)acrylat, Dipentaerythritoltetra(meth)acrylat, Dipentaerythritol- hexa(meth)acrylat, N,N-(2,2,4-Trimethylhexamethylen)bis[2-(aminocarboxy)propan-1,3-diol]-tetra(meth)-acrylat, das Kondensationsprodukt von 3,(4)-(Meth)acryloxymethyl-8,(9)-hydroxymethyltricyclo[5.2.1.$0^{2,6}$]decan mit Dicarbonsäuren, 2-Ethylhexyl-(meth)acrylat, Tridecyl(meth)acrylat, Stearyl(meth)acrylat, Benzyl(meth)acrylat, Methoxydiethylenglycol(meth)acrylat, Dicyclopentenyl(meth)acrylat, Phenyl-(meth)acrylat, Pentaerythritolmono(meth)acrylat, Dipentaerythritolmono(meth)-acrylat, sowie Caprolacton modifiziertes Tetrahydrofurfuryl(meth)acrylat.

**6.** Dentale polymerisierbare Zusammensetzung nach einem der vorangehenden Ansprüche, wobei Bestandteil (E) säuregruppenhaltige Monomeren, die kein Si-Atom aufweisen, ausgewählt ist aus der Gruppe bestehend aus 10-(Meth)acryloyloxydecyldihydrogenphosphat (10-MDP), 2-(Meth)acryloyloxyethyldihydrogenphosphat, 6-(Meth)acryloyloxyhexyldihydrogenphosphat, 4-(Meth)acryloyloxybutyldihydrogenphosphat, 8-(Meth)acryloyloxyoctyldihydrogenphosphat, 2-(Meth)acryloyloxynonyldihydrogenphosphat, 11-(Meth)acryloyloxyundecyldihydrogenphosphat, 20-(Meth)acryloyloxyeicosyldihydrogenphosphat, 1,3-Di(meth)acyloyloxypropyl-2-dihydrogenphosphat, 2-(Meth)-acryloyloxyethylphenyldihydrogenphosphat, Di(2-(meth)acyloyloxyethyl)pyrophosphat, Di(2-(meth) acyloyloxypropyl)pyrophosphat, Di(2-(meth)acyloyloxybutyl)pyrophosphat, Di(2-(meth)acyloyloxypentyl)pyrophosphat, das Di(2-(meth)acyloyloxyhexyl)pyrophosphat, Di(2-(meth)acyloyloxydecyl)pyrophosphat, Mono-, Di- und/oder Triester der Phosphor-säure mit Hydroxy-C2-C8-alkylmethacrylat, Glyceryldimethacrylatphosphat, Pentaerythritoltrimethacrylatphosphat, Dipentaerythritolpentaacrylatphosphat, Tetramethacryloxyethylpyrophosphat, Trimellitsäure-4-methacryloyloxyethylester (4-MET), Trimellitsäureanhydrid-4-methacryloyloxyethylester (4-META), Pyromellitsäuredimethacrylat, Pyromellitsäureglyce-roldimethacrylat, Methacryloyloxyethylphthalat, Methacryloyloxyethylmaleat, Methacryloyloxyethylsuccinat, 1,3-Glyceroldimethacrylatmaleat und Di-Oxyethoxymethacrylsäure-ethylendiamintetraessigsäureester, wpbei (E) bevorzugt ausgewählt ist aus der Gruppe bestehend aus 10-(Meth) acryloyloxydecyldihydrogenphosphat (10-MDP), Glyceryldimethacrylatphosphat, Pentaerythritoltrimethacrylatphosphat, Dipentaerythritolpentaacrylatphosphat, Tetramethacryloxyethylpyrophosphat, Trimellitsäure-4-methacryloyloxyethylester (4-MET), Trimellitsäureanhydrid-4-methacryloyloxyethylester (4-META), Pyromellitsäuredimethacrylat und Pyromellitsäureglyceroldimethacrylat.

**7.** Dentale polymerisierbare Zusammensetzung nach einem der vorangehenden Ansprüche, wobei Bestandteil (F) Additive ausgewählt ist aus der Gruppe bestehend aus rheologische Hilfsmittel, Farbmittel, vorzugsweise Farbpigmente, Aromen, Stabilisatoren, insbesondere Tageslichtstabilisatoren, Inhibitoren, Molekulargewichtsregler, Konservierungsmittel, vorzugsweise Parabene, grenzflächenaktive Substanzen, vorzugsweise Tenside, Mikrobizide, vorzugsweise Bakterizide, organische Polymere und Oligomere und Verbindungen mit hohen Molekulargewichten, Verdickungsmittel, dentale Arzneimittel und Weichmacher.

**8.** Dentale, polymerisierbare Zusammensetzung nach einem der vorangehenden Ansprüche, wobei Bestandteil (G) Lösungsmittel ausgewählt ist aus der Gruppe bestehend aus Wasser, Toluol, Xylol, Isooctan, Aceton, Butanon, Methylisobutylketon, Ethylacetat, Butylacetat, Tetrahydrofuran, N-Methylpyrrolidon, Dimethylacetamid und Dimethylformamid, Ethanol, Propanole, Butanole, Pentanole, Hexanole, Cyclohexanol, Heptanole, Octanole, Nonanole, Decanole, vorzugsweise ausgewählt aus der Gruppe bestehend aus mit Wasser mischbaren organischen Lösungsmitteln, vorzugsweise Aceton, Ethanol, n-Propanol und Isopropanol sowie deren Mischungen, wobei das Verhältnis des organischen Lösungsmittel(s)/gemisches zu Wasser vorzugsweise im Bereich von 1:1 bis 10:1, bevorzugt im Bereich von 2:1 bis 8:1 und weiter bevorzugt im Bereich von 3:1 bis 5:1 liegt.

**9.** Dentale, polymerisierbare Zusammensetzung nach einem der vorangehenden Ansprüche, wobei

der Brechungsindex $n_A$ der Gesamtmenge der polymerisierbaren Monomere (A) und (D) im Bereich von 1.45 bis 1.55, bevorzugt im Bereich von 1.48 bis 1.55, liegt und/oder
der Brechungsindex $n_B$ der Gesamtmenge der Füllstoffe (B) im Bereich von 1.50 bis 1.55 liegt und/oder
der Betrag der Differenz I $n_A$ - $n_B$| zwischen dem Brechungsindex $n_A$ der Gesamtmenge der polymerisierbaren Monomere (A) und (D) und dem Brechungsindex $n_B$ der Gesamtmenge der Füllstoffe (B) kleiner als 0,05, bevorzugt kleiner 0,03, besonders bevorzugt kleiner als 0,02 und ganz besonders bevorzugt kleiner als 0,01 ist und/oder
die Differenz $n_P$ - $n_A$ zwischen dem Brechungsindex $n_P$ der polymerisierten Harzmatrix aus (A) und (D) und des Brechungsindexes $n_A$ der Gesamtmenge der polymerisierbaren Monomere (A) und (D) vor der Polymerisation kleiner als 0,03 und bevorzugt kleiner als 0,02 ist.

**10.** Verfahren zur Herstellung einer polymerisierbaren dentalen Zusammensetzung nach den Ansprüchen 1 bis 9 mit den folgenden Schritten:

- Bereitstellen der Bestandteile (A), (B), (C), sowie optional der Bestandteile (D), (E), (F), (G) und
- Mischen der Bestandteile.

**11.** Kit, umfassend

- Ein, zwei oder mehr als zwei polymerisierbare, dentale Zusammensetzungen nach einem der Ansprüche 1 bis 9 in einer Spritze und/oder Compule,
- optional ein, zwei oder mehr als zwei Bondings,
- optional ein, zwei oder mehr als zwei Ätzgele,
- optional eine oder mehr als eine Farbskala,
- optional einen oder mehr als einen Pinsel.

## Claims

**1.** Dental polymerizable composition comprising

(A) polysiloxanes, wherein the polysiloxanes comprise a mixture of the condensates of the three silanes (a1), (a2) and (a3) and/or

a cocondensate of a mixture of the three silanes (a1), (a2) and (a3) and/or
a mixture of at least two of the cocondensates (a1)/(a2), (a1)/(a3) and (a2)/(a3) and/or
a mixture of the condensate of one of the three silanes (a1), (a2) or (a3) with the cocondensate of the other two silanes,
wherein the silane (a1) conforms to the formula $(R^1O)_aR^2_bSi[-A(-Y\{-B[-PG]_f\}_e)_d]_c$ wherein Y = -C(=O)NH-, -NHC(=O)-, -OC(=O)NH-, -NHC(=O)O-, -SC(=O)NH-, -NHC(=O)S-, -NHC(=O)NH-, -OC(=O)N(-C(=O)NH-)-, -SC(=O)N(-C(=O)NH-)-, -NHC(=O)N(-C(=O)NH-)-, -C(=O)NHC(=O)NH-, - NHC(=O)NHC(=O)-, wherein the bond on the left in the formula image is closer to the structure element A and the bond arranged on the right is closer to the structure element B,
PG = polymerizable group,
wherein the polymerizable group PG is selected from the group consisting of $-Z-C(=O)-CH=CH_2$ and $-Z-C(=O)-C(CH_3)=CH_2$ wherein Z is selected from the group consisting of O and NH,
A = an organic connecting group that connects Si to Y and has 1 to 20 carbon atoms,
B = an organic connecting group that connects Y to PG and has 1 to 20 carbon atoms, $R^1$ = H or C1- to C4-alkyl, $R^2$ = C1- to C4-alkyl,
a = 2 or 3,
b = 0 or 1,
c = 1 or 2,

d = 1 to 3,
e = 1 or 2,
f = 1 to 5,

$$a + b + c = 4,$$

and wherein the silane (a2) conforms to the formula $(R^1O)_aR^2_bSi[-A'(-PG)_f]_c$ wherein
PG = polymerizable group,
wherein the polymerizable group PG is selected from the group consisting of $-Z-C(=O)-CH=CH_2$ and $-Z-C(=O)-C(CH_3)=CH_2$ wherein Z is selected from the group consisting of O and NH,
A' = an organic connecting group that connects Si to PG and has 1 to 20 carbon atoms and does not contain any of the following groups: -C(=O)NH-, -NHC(=O)-, -OC(=O)NH-, -NHC(=O)O-, -SC(=O)NH-, -NHC(=O)S-, -NHC(=O)NH-, - OC(=O)N(-C(=O)NH-)-, -SC(=O)N(-C(=O)NH-)-, or - NHC(=O)N(-C(=O)NH-)-, -C(=O)NHC(=O)NH-, -NHC(=O)NHC(=O)-,
$R^1$ = H or C1- to C4-alkyl,
$R^2$ = C1- to C4-alkyl,
a = 2 or 3
b = 0 or 1
c = 1 or 2
f = 1 to 5,

$$a + b + c = 4,$$

and wherein the silane (a3) conforms to the formula $(R^1O)\ _aR^2_bSiAr_c$ wherein
$R^1$ = H or C1- to C4-alkyl,
$R^2$ = C1- to C4-alkyl,
Ar = aryl, wherein different Ar groups may be the same or different,
a = 2 or 3,
b = 0 or 1,
c = 1 or 2

$$a + b + c = 4,$$

(B) fillers and
(C) initiators and/or catalysts and/or activators for the polymerization;
wherein the proportions of silanes (a1), (a2) and (a3) in the cocondensate or in the mixture of condensates or cocondensates are

(a1) in an amount of 10% to 70% by weight,
(a2) in an amount of 10% to 70% by weight, and
(a3) in an amount of 5% to 60% by weight,

based in each case on the total amount of silanes (a1), (a2) and (a3).

**2.** Dental polymerizable composition according to Claim 1, additionally comprising

(D) organic polymerizable monomers that are not polysiloxanes according to the invention and/or
(E) organic monomers that contain acid groups and do not include a silicon atom, and/or
(F) additives and/or
(G) solvents.

**3.** Dental polymerizable composition according to either of the preceding claims, wherein the composition contains constituent

(A) in an amount of 5% to 99.99% by weight,
(B) in an amount of 0.3% to 92% by weight,
(C) in an amount of 0.01% to 5% by weight,

(D) in an amount of 0% to 94% by weight,
(E) in an amount of 0% to 20% by weight,
(F) in an amount of 0% to 20% by weight and
(G) in an amount of 0% to 70% by weight, and

wherein the weight figures are each based on the overall composition.

**4.** Dental polymerizable composition according to any of the preceding claims, wherein the Y group from silane (a1) is selected from the group consisting of $-(X)_x-C(=O)NH-$, $-NHC(=O)-(X)_x-$ and $-(X)_xC(=O)N(-C(=O)NH-)-$ wherein X is selected from the group consisting of O, S and NH, preferably O and S, and wherein the index x is either 0 or 1, preferably 1, and wherein the polymerizable group PG is selected from the group consisting of $Z-C(=O)-CH=CH_2$ and $-Z-C(=O)-C(CH_3)=CH_2$, and wherein Z is selected from the group consisting of O and NH, preferably O.

**5.** Dental polymerizable composition according to any of the preceding claims, wherein constituent (D) comprises organic polymerizable monomers that are not polysiloxanes according to the invention, preferably for reaction with the polysiloxanes according to the invention, and wherein (D) is selected from the group consisting of

monofunctional or polyfunctional (meth)acrylate monomers, preferably from esters of (meth)acrylic acid having alkyl groups of 1 to 12 carbon atoms and from esters of (meth)acrylic acid containing aromatic groups having 6 to 12 carbon atoms, wherein the alkyl groups and aromatic groups forming the esters may contain substituents such as hydroxyl groups and ether bonds, and polymerizable monomers that are hydroxyl compounds having at least one ethylenic double bond, and
polyfunctional (meth)acrylate monomers, preferably from di(meth)acrylates of alkylene glycol having 2 to 20 carbon atoms, di(meth)acrylates of oligomers of alkylene glycol, polyalkylene glycol di(meth)acrylate, di(meth) acrylates of bisphenol A or of diglycidyl ether of bisphenol A, and
polymerizable compounds based on a central polyalicyclic structural element, such as 3(4), 8(9)-bis((meth) acryloyloxymethyl) tricyclo [5.2.1.$0^{2,6}$] decane, alkoxylated 3(4), 8(9)-bis((meth)acryloyloxymethyl)tricyclo [5.2.1.$0^{2,6}$] decane, 2,3-bis((meth)acryloyloxymethyl)bicyclo[2.2.1]heptane, alkoxylated 2,3-bis((meth)acryloyloxymethyl)bicyclo[2.2.1]heptane, 1,3,5-tri-(meth)acryloyloxytricyclo[3.3.1.$1^{3,7}$]decane, alkoxylated tri(meth) acryloyloxytricyclo[3.3.1.$1^{3,7}$]decane and (meth) acrylic esters of tricyclo[S.2.1.$0^{2,6}$]decane-3(4),8(9)-dimethanol, alkoxylated tricyclo[5.2.1.$0^{2,6}$]decane-3(4),8(9)-dimethano1, bicyclo[2.2.1]heptane-2,3-dimethanol, alkoxylated bicyclo[2.2.1]heptane-2,3-dimethanol, adamantane-1,3,5-triol, alkoxylated adamantane-1,3,5-triol, with urethane, urea, amide, allophanate, acylurea or biuret groups between the polyalicyclic structural element and the (meth)acrylic esters, and
urethane (meth)acrylates, reaction products of 2 mol of a (meth)acrylate having a hydroxyl group and one mole of a diisocyanate, and
ethylene glycol di(meth)acrylate, diethylene glycol di(meth)acrylate, hexane-1,6-diol di(meth)acrylate (HEDMA), triethylene glycol di(meth)acrylate (TEGDMA), dodecane-1,12-diol di(meth)acrylate, bisphenol A di(meth)acrylate, alkoxylated bisphenol A di(meth)acrylate, bisphenol B di(meth)acrylate, alkoxylated bisphenol B di(meth) acrylate, bisphenol C di(meth)acrylate, alkoxylated bisphenol C di(meth)acrylate, bisphenol F di(meth)acrylate, alkoxylated bisphenol F di(meth)acrylate, polyethylene glycol di(meth)acrylate, 7,7,9-trimethyl-3,14-dioxa-4,13-dioxo-5,12-diazahexadecane-1,16-dioxydi(meth)acrylate (UDMA), butanediol di(meth)acrylate, tetraethylene glycol di(meth)acrylate, neopentyl glycol di(meth)acrylate, 2-hydroxypropyl 1,3-di(meth)acrylate, 3-hydroxypropyl 1,2-di(meth)acrylate, pentaerythritol di(meth)acrylate, di(meth)acrylates of dihydroxymethyltricyclo [5.2.1.$0^{2,6}$]decane, 2-hydroxyethyl (meth)acrylate, 2-hydroxypropyl (meth)acrylate, 3-hydroxypropyl (meth)acrylate, 1,2-dihydroxypropyl (meth)acrylate, 1,3-dihydroxypropyl (meth)acrylate, 2,3-dihydroxypropyl (meth)acrylate, 2,2-bis[4-[3-(meth)acryloyloxy-2-hydroxypropoxy]phenyl]propane (bis-GMA), trimethylolpropane tri(meth)acrylate, trimethylolethane tri(meth)acrylate, pentaerythritol tri(meth)acrylate, trimethylolmethane tri(meth)acrylate, pentaerythritol tetra(meth)acrylate, ditrimethylolpropane tetra(meth)acrylate, pentaerythritol hexa(meth)acrylate, butylene glycol di(meth)acrylate, propylene glycol di(meth)acrylate, nonanediol di(meth) acrylate, decanediol di(meth)acrylate, glycerol mono(meth)acrylate, glycerol di(meth)acrylate, trimethylolpropane mono(meth)acrylate, trimethylolpropane di(meth)acrylate, sorbitol mono-, di-, tri-, tetra- or penta(meth) acrylate, methyl (meth)acrylate, ethyl (meth)acrylate, propyl (meth)acrylate, butyl (meth)acrylate, hexyl (meth) acrylate, tetrahydrofuryl (meth)acrylate, lauryl (meth)acrylate, cyclohexyl (meth)acrylate, allyl (meth)acrylate, glycidyl (meth)acrylate, 2-ethoxyethyl (meth)acrylate, methoxy polyethylene glycol(meth)acrylate, isobornyl (meth)acrylate, 2-(N,N-dimethylamino)ethyl (meth)acrylate, N-methylol(meth)acrylamide, diacetone(meth)acrylamide, 2,2-bis[4-(meth)acryloyloxyphenyl]propane, 2,2-bis[4-(meth)acryloyloxyethoxyphenyl]propane, 2,2-bis[4-(meth)acryloyloxydiethoxyphenyl]propane, 2,2-bis[4-(meth)acryloyloxytriethoxyphenyl]propane 2,2-bis[4-(meth)acryloyloxytetraethoxyphenyl]propane, 2,2-bis[4-(meth)acryloyloxypentaethoxyphenyl]propane, 2,2-bis[4-(meth)acryloyloxydipropoxyphenyl]propane, 2,2-bis[4-(meth)acryloyloxyethoxyphenyl]-2-[4-(meth)acryloyloxydiethoxyphenyl]propane, 2-[4-(meth)acryloyloxydiethoxyphenyl]-2-[4-(meth)acryloyloxytriethoxyphenyl]propane, 2-[4-(meth)acryloyloxdipropoxyphenyl]-2-[4-(meth)acryloyloxytriethoxyphenyl]propane, 2,2-bis [4-(meth)acryloyloxyisopropoxy phenyl]propane, hydroxypivalic acid neopentyl glycol di(meth)acrylate, acetoacetoxyethyl (meth)acrylate, polypropylene glycol di(meth)acrylate, glycerol alkoxylate dimethacrylate, neopentyl glycol (meth)acrylate, N,N-(1,2-dihydroxyethylene)bisacrylamide, 2,2-bis[4-(meth)acryloyloxypentaethoxyphenyl]propane, 2,2-bis[4-(meth)acryloyloxypolyethoxyphenyl]propane, diethylene glycol di(meth)acrylate, dipentaerythritol tetra(meth)acrylate, dipentaerythritol hexa (meth) acrylate, N,N-(2,2,4-trimethylhexamethylene)bis[2-(aminocarboxy)propane-1,3-diol] tetra (meth) acrylate, the condensation product of 3, (4)-(meth) acryloxymethyl-8, (9)-hydroxymethyltricyclo[5.2.1.0$^{2\ 6}$]decane with dicarboxylic acids, 2-ethylhexyl (meth)acrylate, tridecyl (meth)acrylate, stearyl (meth)acrylate, benzyl (meth)acrylate, methoxy diethylene glycol(meth)acrylate, dicyclopentenyl (meth)acrylate, phenyl (meth)acrylate, pentaerythritol mono(meth)acrylate, dipentaerythritol mono(meth)acrylate, and caprolactone-modified tetrahydrofurfuryl (meth)acrylate.

**6.** Dental polymerizable composition according to any of the preceding claims, wherein constituent (E) is monomers that contain acid groups and do not include a silicon atom, selected from the group consisting of 10-(meth)acryloyloxydecyl dihydrogenphosphate (10-MDP), 2-(meth)acryloyloxyethyl dihydrogenphosphate, 6-(meth)acryloyloxyhexyl dihydrogenphosphate, 4-(meth)acryloyloxybutyl dihydrogenphosphate, 8-(meth)acryloyloxyoctyl dihydrogenphosphate, 2-(meth)acryloyloxynonyl dihydrogenphosphate, 11-(meth)acryloyloxyundecyl dihydrogenphosphate, 20-(meth)acryloyloxyeicosyl dihydrogenphosphate, 1,3-di(meth)acryloyloxypropyl 2-dihydrogenphosphate, 2-(meth)acryloyloxyethylphenyl dihydrogenphosphate, di(2-(meth)acryloyloxyethyl) pyrophosphate, di(2-(meth)acryloyloxypropyl) pyrophosphate, di(2-(meth)acryloyloxybutyl) pyrophosphate, di(2-(meth)acryloyloxypentyl) pyrophosphate, di(2-(meth)acryloyloxyhexyl) pyrophosphate, di(2-(meth)acryloyloxydecyl) pyrophosphate, mono-, di- and/or triesters of phosphoric acid with hydroxy-C2-C8-alkyl methacrylate, glyceryl dimethacrylate phosphate, pentaerythritol trimethacrylate phosphate, dipentaerythritol pentaacrylate phosphate, tetramethacryloyloxyethyl pyrophosphate, trimellitic acid 4-methacryloyloxyethyl ester (4-MET), trimellitic anhydride 4-methacryloyloxyethyl ester (4-META), pyromellitic acid dimethacrylate, pyromellitic acid glycerol dimethacrylate, methacryloyloxyethyl phthalate, methacryloyloxyethyl maleate, methacryloyloxyethyl succinate, 1,3-glycerol dimethacrylate maleate and dioxyethoxymethacrylic acid ethylenediaminetetraacetic ester, wherein (E) is preferably selected from the group consisting of 10-(meth)acryloyloxydecyl dihydrogenphosphate (10-MDP), glyceryl dimethacrylate phosphate, pentaerythritol trimethacrylate phosphate, dipentaerythritol pentaacrylate phosphate, tetramethacryloyloxyethyl pyrophosphate, trimellitic acid 4-methacryloyloxyethyl ester (4-MET), trimellitic anhydride 4-methacryloyloxyethyl ester (4-META), pyromellitic acid dimethacrylate and pyromellitic acid glycerol dimethacrylate.

**7.** Dental polymerizable composition according to any of the preceding claims, wherein constituent (F) additives is selected from the group consisting of rheological aids, colourants, preferably colour pigments, flavours, stabilizers, in particular daylight stabilizers, inhibitors, molecular weight regulators, preservatives, preferably parabens, interface-active substances, preferably surfactants, microbicides, preferably bactericides, organic polymers and oligomers and compounds having high molecular weights, thickeners, dental medicaments and plasticizers.

**8.** Dental polymerizable composition according to any of the preceding claims, wherein constituent (G) solvent is selected from the group consisting of water, toluene, xylene, isooctane, acetone, butanone, methyl isobutyl ketone, ethyl acetate, butyl acetate, tetrahydrofuran, N-methylpyrrolidone, dimethylacetamide and dimethylformamide, ethanol, propanols, butanols, pentanols, hexanols, cyclohexanol, heptanols, octanols, nonanols, decanols, preferably selected from the group consisting of water-miscible organic solvents, preferably acetone, ethanol, n-propanol and isopropanol and mixtures thereof, wherein the ratio of the organic solvent(s)/mixture to water is preferably in the range of 1:1 to 10:1, preferably in the range from 2:1 to 8:1 and further preferably in the range from 3:1 to 5:1.

**9.** Dental polymerizable composition according to any of the preceding claims, wherein

the refractive index $n_A$ of the entirety of the polymerizable monomers (A) and (D) is in the range of 1.45 to 1.55, preferably in the range of 1.48 to 1.55, and/or
the refractive index $n_B$ of the entirety of the fillers (B) is in the range of 1.50 to 1.55 and/or the magnitude of the difference $|n_A - n_B|$ between the refractive index $n_A$ of the entirety of the polymerizable monomers (A) and (D) and the refractive index $n_B$ of the entirety of the fillers (B) is less than 0.05, preferably less than 0.03, more preferably less than 0.02 and most preferably less than 0.01, and/or
the difference $n_P - n_A$ between the refractive index $n_P$ of the polymerized resin matrix of (A) and (D) and the

refractive index $n_A$ of the entirety of the polymerizable monomers (A) and (D) prior to polymerisation is less than 0.03 and preferably less than 0.02.

**10.** Method of producing a polymerizable dental composition according to Claims 1 to 9, comprising the following steps:

- providing constituents (A), (B), (C) and optionally constituents (D), (E), (F), (G), and
- mixing the constituents.

**11.** Kit comprising

- one, two or more than two polymerizable dental compositions according to any of Claims 1 to 9 in a syringe and/or compule,
- optionally one, two or more than two adhesives,
- optionally one, two or more than two etching gels,
- optionally one or more than one shade guide,
- optionally one or more than one brush.

## Revendications

**1.** Composition dentaire polymérisable, comprenant

(A) des polysiloxanes, les polysiloxanes comprenant un mélange des produits de condensation des trois silanes (a1), (a2) et (a3) et/ou

un produit de cocondensation d'un mélange des trois silanes (a1), (a2) et (a3) et/ou
un mélange d'au moins deux des produits de cocondensation (a1)/(a2), (a1)/(a3) et (a2)/(a3) et/ou
un mélange du produit de condensation d'un des trois silanes (a1), (a2) et (a3) et du produit de cocondensation des deux autres silanes,
le silane (a1) correspondant à la formule $(R^1O)_aR^2_bSi[-A(-Y\{-B[-PG]_f\}_e)_d]_c$, dans laquelle Y = -C(=O)NH-, -NHC(=O)-, -OC(=O)NH-, -NHC(=O)O-, -SC(=O)NH-, -NHC(=O)S-, - NHC(=O)NH-, -OC(=O)N(-C(=O)NH-)-, -SC(=O)N(-C(=O)NH-)-, -NHC(=O)N(-C(=O)NH-)-, -C(=O)NHC(=O)NH-, - NHC(=O)NHC(=O)-,
la liaison à chaque fois agencée à gauche dans la formule étant plus proche de l'élément structural A et la liaison agencée à droite étant plus proche de l'élément structural B,
PG = groupe polymérisable,
le groupe polymérisable PG étant choisi dans le groupe constitué par $-Z-C(=O)-CH=CH_2$ et $-Z-C(=O)-C(CH_3)=CH_2$, Z étant choisi dans le groupe constitué par O et NH,
A = un groupe de liaison organique, qui relie Si à Y et qui présente 1 à 20 atomes de carbone,
B = un groupe de liaison organique, qui relie Y à PG et qui présente 1 à 20 atomes de carbone, $R^1$ = H ou C1-C4-alkyle,
$R^2$ = C1-C4-alkyle,
a = 2 ou 3,
b = 0 ou 1,
c = 1 ou 2,
d = 1 à 3,
e = 1 ou 2,
f = 1 à 5,

$$a + b + c = 4,$$

et le silane (a2) correspondant à la formule $(R^1O)_aR^2_eSi[-A'(-PG)_f]_c$, dans laquelle
PG = groupe polymérisable,
le groupe polymérisable PG étant choisi dans le groupe constitué par $-Z-C(=O)-CH=CH_2$ et $-Z-C(=O)-C(CH_3)=CH_2$, Z étant choisi dans le groupe constitué par O et NH,
A' = un groupe de liaison organique, qui relie Si à PG et qui présente 1 à 20 atomes de carbone et qui ne contient aucun des groupes -C(=O)NH-, -NHC(=O)-, - OC(=O)NH-, -NHC(=O)O-, -SC(=O)NH-, -NHC(=O)S-, - NHC(=O)NH-, -OC(=O)N(-C(=O)NH-)-, -SC(=O)N(-C(=O)NH-)-, ou -NHC(=O)N(-C(=O)NH-)-, -C(=O)NHC(=O)NH-, - NHC(=O)NHC(=O)-,

$R^1$ = H ou C1-C4-alkyle,
$R^2$ = C1-C4-alkyle,
a = 2 ou 3,
b = 0 ou 1,
c = 1 ou 2,
f = 1 à 5,

$$a + b + c = 4,$$

et le silane (a3) correspondant à la formule $(R^1O)R^2_bSiAr_c$, dans laquelle
$R^1$ = H ou C1-C4-alkyle,
$R^2$ = C1-C4-alkyle,
Ar = aryle, différents groupes aryle pouvant être identiques ou différents,
a = 2 ou 3,
b = 0 ou 1,
c = 1 ou 2,

$$a + b + c = 4,$$

(B) des charges et
(C) des initiateurs et/ou des catalyseurs et/ou des activateurs pour la polymérisation,
la proportion des silanes (a1), (a2) et (a3) dans le produit de cocondensation ou dans le mélange des produits de condensation ou des produits de cocondensation représentant

(a1) une quantité de 10 à 70% en poids,
(a2) une quantité de 10 à 70% en poids et
(a3) une quantité de 5 à 60% en poids,

à chaque fois par rapport à la quantité totale des silanes (a1), (a2) et (a3).

**2.** Composition dentaire polymérisable selon la revendication 1, comprenant en plus

(D) des monomères organiques polymérisables, qui ne sont pas des polysiloxanes selon l'invention et/ou
(E) des monomères organiques contenant des groupes acides qui ne présentent pas d'atome de Si et/ou
(F) des additifs et/ou
(G) des solvants.

**3.** Composition dentaire polymérisable selon l'une des revendications précédentes, la composition contenant les composants

(A) en une quantité de 5 à 99,99% en poids,
(B) en une quantité de 0,3 à 92% en poids,
(C) en une quantité de 0,01 à 5% en poids,
(D) en une quantité de 0 à 94% en poids,
(E) en une quantité de 0 à 20% en poids,
(F) en une quantité de 0 à 20% en poids et
(G) en une quantité de 0 à 70% en poids et

les indications de quantités se rapportant à chaque fois à la composition totale.

**4.** Composition dentaire polymérisable selon l'une des revendications précédentes, le groupe Y du silane (a1) étant choisi dans le groupe constitué par $-(X)_x-C(=O)NH-$, $-NHC(=O)-(X)_x-$ et $-(X)_xC(=O)N(-C(=O)NH-)-$, X étant choisi dans le groupe constitué par O, S et NH, de préférence par O et S et l'indice x valant soit 0, soit 1, de préférence 1, et le groupe polymérisable PG étant choisi dans le groupe constitué par $Z-C(=O)-CH=CH_2$ et - $Z-C(=O)-C(CH_3)=CH_2$ et Z étant choisi dans le groupe constitué par O et NH, de préférence O.

**5.** Composition dentaire polymérisable selon l'une des revendications précédentes, le constituant (D) comprenant des

monomères organiques polymérisables qui ne sont pas des polysiloxanes selon l'invention, de préférence pour la transformation avec les polysiloxanes selon l'invention et (D) étant choisi dans le groupe constitué par

les monomères monofonctionnels ou polyfonctionnels de (méth)acrylate, de préférence les esters de l'acide (méth)acrylique présentant des groupes alkyle de 1 à 12 atomes de carbone ainsi que les esters de l'acide (méth) acrylique qui contiennent des groupes aromatiques comprenant 6 à 12 atomes de carbone, les groupes alkyle et les groupes aromatiques, qui forment les esters, pouvant contenant des substituants tels que des groupes hydroxyle et des liaisons éther et

les monomères polymérisables qui sont des composés hydroxyle présentant au moins une double liaison éthylénique et

les monomères polyfonctionnels de (méth)acrylate, de préférence les di(méth)acrylates d'alkylèneglycol comprenant 2 à 20 atomes de carbone, les di(méth)acrylates d'oligomères de l'alkylèneglycol, le di(méth) acrylate de polyalkylèneglycol, les di(méth)acrylates du bisphénol A ou, selon le cas, du diglycidyléther du bisphénol A et

les composés polymérisables qui sont basés sur un élément structural central polyalicyclique, tels que le 3(4),8(9)-bis((méth)acryloyloxyméthyl)-tricyclo[5.2.1.02,6]décane, le 3(4),8(9)-bis((méth)acryloyloxyméthyl)-tricyclo[5.2.1.0$^{2,6}$]décane alcoxylé, le 2,3-bis((méth)acryloyloxyméthyl)-bicyclo[2.2.1]heptane, le 2,3-bis((méth)acryloyloxyméthyl)bicyclo[2.2.1]heptane alcoxylé, le 1,3,5-tri-(méth)acryloyloxytricyclo[3.3.1.1$^{3,7}$]décane, le tri(méth)acryloyloxy-tricyclo-[3.3.1.1$^{3,7}$]décane alcoxylé ainsi que les esters de l'acide (méth)acrylique du [5.2.1.0$^{2}$,$^{6}$]-décane-3(4),8(9)-diméthanol, du tricyclo[5.2.1.0$^{2,6}$]décane-3(4),8(9)-diméthanol alcoxylé, du bicyclo[2.2.1]heptane-2,3-diméthanol, du bicyclo[2.2.1]-heptane-2,3-diméthanol alcoxylé, du 1,3,5-adamantanetriol, du 1,3,5-adamantanetriol alcoxylé, des groupes uréthane, urée, amide, allophanate, acylurée ou biuret étant agencés entre l'élément structural polyalicyclique et les esters de l'acide (méth)acrylique et

les esters d'uréthane- (méth) acrylate, les produits de réaction de 2 moles d'un (méth)acrylate présentant un groupe hydroxyle et d'une mole d'un diisocyanate et

le di(méth)acrylate d'éthylèneglycol, le di(méth)acrylate de diéthylèneglycol, le di(méth) acrylate de 1,6-hexanediol (HEDMA), le di(méth) acrylate de triéthylèneglycol (TEGDMA), le di(méth)acrylate de 1,12-dodécanediol, le di(méth)acrylate de bisphénol A, le di(méth)acrylate de bisphénol A alcoxylé, le di(méth) acrylate de bisphénol B, le di(méth)acrylate de bisphénol B alcoxylé, le di(méth)acrylate de bisphénol C, le di(méth)acrylate de bisphénol C alcoxylé, le di(méth) acrylate de bisphénol F, le di(méth)acrylate de bisphénol F alcoxylé, le di(méth)acrylate de polyéthylèneglycol, le 1,16-dioxydi(méth)acrylate de 7,7,9-triméthyl-3,14-dioxa-4,13-dioxo-5,12-diazahexadécane (UDMA), le di(méth) acrylate de butanediol, le di(méth) acrylate de tétraéthylèneglycol, le di(méth)acrylate de néopentylglycol, le 1,3-di(méth)acrylate de 2-hydroxypropyle, le 1,2-di(méth) acrylate de 3-hydroxypropyle, le di(méth)acrylate de pentaérythritol, les di(méth)acrylates du dihydroxyméthyltricyclo[5.2.1.0$^{2,6}$]décane, le (méth)acrylate de 2-hydroxyéthyle, le (méth)acrylate de 2-hydroxypropyle, le (méth)acrylate de 3-hydroxypropyle, le (méth)acrylate de 1,2-dihydroxypropyle, le (méth)acrylate de 1,3-dihydroxypropyle, le (méth)acrylate de 2,3-dihydroxypropyle, le 2,2-bis[4-[3-(méth)acryloyloxy-2-hydroxypropoxy]phényl]propane (bis-GMA), le tri(méth)acrylate de triméthylolpropane, le tri(méth)acrylate de triméthyloléthane, le tri(méth)acrylate de pentaérythritol, le tri(méth)acrylate de triméthylolméthane, le tétra(méth)acrylate de pentaérythritol, le tétra(méth)acrylate de ditriméthylolpropane, l'hexa(méth)acrylate de pentaérythritol, le di(méth)acrylate de butylèneglycol, le di(méth)acrylate de propylèneglycol, le di(méth) acrylate de nonanediol, le di(méth)acrylate de décanediol, le mono(méth)acrylate de glycérol, le di(méth)acrylate de glycérol, le mono(méth)acrylate de triméthylolpropane, le di(méth)acrylate de triméthylolpropane, le mono(méth)acrylate, le di(méth)acrylate, le tri(méth)acrylate, le tétra(méth)acrylate ou penta(méth) acrylate de sorbitol, le (méth) acrylate de méthyle, le (méth)acrylate d'éthyle, le (méth)acrylate de propyle, le (méth)acrylate de butyle, le (méth) acrylate d'hexyle, le (méth)acrylate de tétrahydrofurfuryle, le (méth)acrylate de lauryle, le (méth)acrylate de cyclohexyle, le (méth)acrylate d'allyle, le (méth)acrylate de glycidyle, le (méth)acrylate de 2-éthoxyéthyle, le (méth)acrylate de méthoxypolyéthylèneglycol, le (méth)acrylate d'isobornyle, le (méth)acrylate de 2-(N,N-diméthylamino)éthyle, le N-méthylol (méth)acrylamide, le diacétone(méth)acrylamide, le 2,2-bis[4-(méth)acryloyloxyphényl]propane, le 2,2-bis[4-(méth)acryloyloxyéthoxyphényl]propane, le 2,2-bis[4-(méth)acryloyloxydiéthoxyphényl]propane, le 2,2-bis[4-(méth)acryloyloxytriéthoxyphényl]propane, le 2,2-bis[4-(méth)acryloyloxytétraéthoxyphényl]propane, le 2,2-bis[4-(méth)-acryloyloxypentaéthoxyphényl]propane, le 2,2-bis[4-(méth)acryloyloxydipropoxyphényl]propane, le 2,2-bis[4-(méth)acryloyloxyéthoxyphényl]-2-[4-(méth)acryloyloxydiéthoxyphényl]propane, le 2-[4-(méth)acryloyloxydiéthoxyphényl]-2-[4-(méth)acryloyloxytriéthoxyphényl]propane, le 2-[4-(méth)acryloyloxdipropoxyphényl]-2-[4-(méth)acryloyloxytriéthoxyphényl]propane, le 2,2-bis[4-(méth) acryloyloxyisopropoxyphényl]propane, le di(méth)acrylate de l'acide hydroxypivalique et du néopentylglycol, le (méth)acrylate d'acétoacétoxyéthyle, le di(méth)acrylate de polypropylèneglycol, le diméthacrylate d'alcoxylate de glycérol, le (méth)acrylate de néopentylglycol, le N,N-(1,2-dihydroxyéthylène)bisacrylamide, le 2,2-bis[4-(méth)acryloyloxypentaéthoxyphényl]propane, le 2,2-bis[4-(méth)acryloyloxypolyéthoxyphényl]-propane, le di(méth)acrylate de diéthylèneglycol, le tétra(méth)acrylate de dipentaérythritol, l'hexa(méth)acrylate de dipentaérythritol, le tétra(méth)acrylate de N,N-(2,2,4-triméthylhexaméthylène)bis[2-(aminocarboxy)propane-1,3-diol], le produit de condensation du 3, (4)-(méth) acryloxyméthyl-8, (9) - hydroxyméthyltricyclo[5.2.1.0$^{2,6}$]décane avec des acides dicarboxyliques, le (méth)acrylate de 2-éthylhexyle, le (méth)acrylate de tridécyle, le (méth) acrylate de stéaryle, le (méth)acrylate de benzyle, le (méth)acrylate de méthoxydiéthylèneglycol, le (méth) acrylate de dicyclopenténylе, le (méth)acrylate de phényle, le mono(méth)acrylate de pentaérythritol, le mono(méth)acrylate de dipentaérythritol ainsi que le (méth)acrylate de tétrahydrofurfuryle modifié par caprolactone.

6. Composition dentaire polymérisable selon l'une des revendications précédentes, le constituant (E), monomères contenant des groupes acides qui ne présentent pas d'atome de Si, étant choisi dans le groupe constitué par le dihydrogénophosphate de 10-(méth)acryloyloxydécyle (10-MDP), le dihydrogénophosphate de 2-(méth)acryloyloxyéthyle, le dihydrogénophosphate de 6-(méth)acryloyloxyhexyle, le dihydrogénophosphate de 4-(méth)acryloyloxybutyle, le dihydrogénophosphate de 8-(méth)acryloyloxyoctyle, le dihydrogénophosphate de 2-(méth)acryloyloxynonyle, le dihydrogénophosphate de 11-(méth)acryloyloxyundécyle, le dihydrogénophosphate de 20-(méth) acryloyloxyeicosyle, le 2-dihydrogénophosphate de 1,3-di(méth)acyloyloxypropyle, le dihydrogénophosphate de 2-(méth)-acryloyloxyéthylphényle, le pyrophosphate de di(2-(méth)acyloyloxyéthyle), le pyrophosphate de di(2-(méth)acyloyloxypropyle), le pyrophosphate de di(2-(méth)acyloyloxybutyle), le pyrophosphate de di(2-(méth)acyloyloxypentyle), le pyrophosphate de di(2-(méth)acyloyloxyhexyle), le pyrophosphate de di(2-(méth)acyloyloxydécyle), le monoester, le diester et/ou le triester de l'acide phosphorique avec du méthacrylate d'hydroxy-C2-C8-alkyle, le phosphate de diméthacrylate de glycéryle, le phosphate de triméthacrylate de pentaérythritol, le phosphate de pentaacrylate de dipentaérythritol, le pyrophosphate de tétraméthacryloxyéthyle, l'ester 4-méthacryloyloxyéthylique de l'acide trimellitique (4-MET), l'ester 4-méthacryloyloxyéthylique de l'anhydride de l'acide trimellitique (4-META), le diméthacrylate de l'acide pyromellitique, le diméthacrylate de l'acide pyromellitique et de glycérol, le phtalate de méthacryloyloxyéthyle, le maléate de méthacryloyloxyéthyle, le succinate de méthacryloyloxyéthyle, le maléate de diméthacrylate de 1,3-glycerol et le diester de l'acide oxyéthoxyméthacrylique-acide éthylènediaminetétraacétique, (E) étant de préférence choisi dans le groupe constitué par le dihydrogénophosphate de 10-(méth)acryloyloxydécyle (10-MDP), le phosphate de diméthacrylate de glycéryle, le phosphate de triméthacrylate de pentaérythritol, le phosphate de pentaacrylate de dipentaérythritol, le pyrophosphate de tétraméthacryloxyéthyle, l'ester 4-méthacryloyloxyéthylique de l'acide trimellitique (4-MET), l'ester 4-méthacryloyloxyéthylique de l'anhydride de l'acide trimellitique (4-META), le diméthacrylate de l'acide pyromellitique et le diméthacrylate de l'acide pyromellitique et de glycérol.

7. Composition dentaire polymérisable selon l'une des revendications précédentes, le constituant (F), additifs, étant choisi dans le groupe constitué par les adjuvants rhéologiques, les colorants, de préférence les pigments colorés, les aromes, les stabilisants, en particulier les stabilisants contre la lumière du jour, les inhibiteurs, les régulateurs du poids moléculaire, les conservateurs, de préférence les parabènes, les substances tensioactives, de préférence les tensioactifs, les microbiocides, de préférence les bactéricides, les polymères et oligomères organiques et les composés présentant des poids moléculaires élevés, les épaississants, les médicaments dentaires et les plastifiants.

8. Composition dentaire polymérisable selon l'une des revendications précédentes, le constituant (G), solvants, étant choisi dans le groupe constitué par l'eau, le toluène, le xylène, l'isooctane, l'acétone, la butanone, la méthylisobutylcétone, l'acétate d'éthyle, l'acétate de butyle, le tétrahydrofuranne, la N-méthylpyrrolidone, le diméthylacétamide et le diméthylformamide, l'éthanol, les propanols, les butanols, les pentanols, les hexanols, le cyclohexanol, les heptanols, les octanols, les nonanols, les décanols, de préférence choisi dans le groupe constitué par les solvants organiques miscibles à l'eau, de préférence l'acétone, l'éthanol, le n-propanol et l'isopropanol ainsi que leurs mélanges, le rapport du solvant organique/mélange de solvants organiques à l'eau étant de préférence situé dans la plage de 1:1 à 10:1, de préférence dans la plage de 2:1 à 8:1 et plus préférablement dans la plage de 3:1 à 5:1.

9. Composition dentaire polymérisable selon l'une des revendications précédentes, dans laquelle l'indice de réfraction $n_A$ de la quantité totale des monomères polymérisables (A) et (D) se situe dans la plage de 1,45 à 1,55, de préférence dans la plage de 1,48 à 1,55, et/ou
l'indice de réfraction $n_B$ de la quantité totale des charges (B) se situe dans la plage de 1,50 à 1,55 et/ou la valeur de la différence $|n_A - n_B|$ entre l'indice de réfraction $n_A$ de la quantité totale des monomères polymérisables (A) et (D) et l'indice de réfraction $n_B$ de la quantité totale des charges (B) est inférieure à 0,05, de préférence inférieure à 0,03, de manière particulièrement préférée inférieure à 0,02 et de manière tout particulièrement préférée inférieure à 0,01 et/ou la différence $n_P - n_A$ entre l'indice de réfraction $n_P$ de la matrice de résine polymérisée de (A) et de (D) et l'indice

de réfraction $n_A$ de la quantité totale des monomères polymérisables (A) et (D) avant la polymérisation est inférieure à 0,03 et de préférence inférieure à 0,02.

**10.** Procédé de préparation d'une composition dentaire polymérisables selon les revendications 1 à 9, présentant les étapes suivantes :

- mise à disposition des constituants (A), (B), (C), ainsi qu'éventuellement des constituants (D), (E), (F), (G) et
- mélange des constituants.

**11.** Kit, comprenant

- un, deux ou plus de deux compositions dentaires polymérisables selon l'une des revendications 1 à 9 dans une seringue et/ou une compule,
- éventuellement un, deux ou plus de deux promoteurs d'adhérence,
- éventuellement un, deux ou plus de deux gels de mordançage,
- éventuellement une échelle de couleur ou plus,
- éventuellement un pinceau ou plus.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- DE 2758414 A1
- DE 4416857 C1
- DE 19860364 C2
- EP 1874847 B1
- EP 1685182 B1
- WO 2013041723 A1
- WO 2013053693 A1
- DE 102014210432 A1
- DE 102014116389 A1
- DE 102014116402 A1
- JP H08311115 A
- US 20150299469 A1
- EP 0285133 A2
- WO 2012091154 A1
- JP 2016034912 A
- WO 2005011621 A1
- DE 102006019092 A1
- DE 3941629 C2
- DE 60116142 T2
- DE 3801511 C2
- DE 102006050153 A1
- EP 0184095 B1
- DE 4231579 C2
- EP 0366977 B1
- US 7081485 B2
- DE 3236026 A1
- US 20070027229 A1
- EP 0262629 B1
- EP 0073413 A
- US 7148382 B2
- US 5761169 A
- DE 19708294 A1
- EP 0057474 A
- EP 0047902 A
- EP 0007508 A
- DE 60029481 T2
- EP 0980682 B1
- EP 0948955 B1
- EP 1236459 B1
- EP 0173567 A2
- EP 1905415 A1
- US 4772530 A
- US 4954414 A
- US 4874450 A
- US 5055372 A
- US 5057393 A
- EP 1720506 A
- DE 102006019092
- DE 3941629
- EP 1839640 A
- DE 1495520
- WO 02092021 A
- WO 02092023 A
- EP 1872767 A
- EP 2070506 A
- EP 1881010 A
- DE 102007050763
- US 6288138 B
- DE 112006001049
- US 7214726 B
- EP 2070935 A
- EP 0059451 A
- EP 2133064 A1
- EP 0897710 A2
- WO 2005051332 A1
- US 9770528 B2
- DE 19648283 A1
- DE 3941629 A1
- EP 11183333 A
- EP 11183328 A
- EP 11183345 A
- EP 11183338 A
- EP 11183342 A
- EP 11188086 A
- EP 0948955 A1
- EP 0783880 B1
- DE 10119831 A1
- EP 1563821 A1
- EP 2374444 B1
- EP 2374445 B1
- EP 3090722 A1
- EP 2916801 B1
- EP 2748206 B1
- EP 2965742 A1
- EP 3058014 B1
- EP 3166569 B1
- EP 3046962 B1
- EP 2931756 B1
- EP 2623087 B1
- EP 2623086 B1
- DE 10147125 A1
- EP 1194110 B1
- DE 3246654 A1
- DE 10126476 A1
- DE 19711514 B4
- DE 3902417 A1
- DE 19961341 C2
- DE 19754029 A1

- DE 602004009552 T2
- DE 102008283306
- DE 69921231 T2
- DE 69231737 T2
- DE 69017484 T2
- DE 69725380 T2
- DE 69801010 T2
- DE 202010014676 U1
- DE 19941738 B4
- DE 102009046251 A1
- DE 2420351 C3
- DE 19860361 A1
- DE 4133494 A1
- DE 19903177 A1
- DE 10102297 A1
- DE 102006016474 A1

**In der Beschreibung aufgeführte Nicht-Patentliteratur**


- **MEGAN A. COLE et al.** Thiol-ene functionalized siloxanes for use as elastomeric dental impression materials. *DENTAL MATERIALS*, 01 April 2014, vol. 30, ISSN 0109-5641, 449-455
- **N. ILIE** ; **R. HICKEL**. *Clin. Oral Invest.*, 2009, vol. 13, 427-438
- Werkstoffkunde in der Zahnmedizin, Moderne Materialien und Technologien. Georg Thieme Verlag, 2018, vol. 208
- *CHEMICAL ABSTRACTS*, 28446-58-4
- *CHEMICAL ABSTRACTS*, 160031-60-7
- **DEBORAH HUCK-JONES** ; **RENATE HESSEMANN**. Chemische Identität einzelner Partike. *Nachrichten aus der Chemie*, September 2014, vol. 62, 886, 887
- **S.D. SILAB** ; **S. DORAN** ; **Y. YAGCI**. Photoinduced Electron Transfer Reactions for macromolecular Synthesis. *Chem. Rev.*, 2016, vol. 116, 10212-10275
- **WATTS DC**. Cash AJ. Determination of polymerization kinetics in visible-light cured materials: Methods development. *Dent Mater*, 1991, vol. 7, 281-287